# EUROPEAN PATENT APPLICATION

(11) **EP 4 628 484 A1**
(43) Date of publication of application: **08.10.2025**
(21) Application number: 23896907.5
(22) Date of filing: 30.11.2023
(51) Int. Cl.: C07D 311/18, A61K 31/37, A61P 35/00

(54) **BENZOPYRONE COMPOUNDS AS WELL AS PREPARATION METHOD THEREFOR AND USE THEREOF**

(30) Priority: 01.12.2022 CN 202211527029; 06.02.2023 CN 202310065010
(71) Applicant: Chengdu Di'Ao Jiuhong Pharmaceutical Factory, Chengdu, Sichuan 610041 (CN)
(72) Inventor: CUI, Wei, Chengdu, Sichuan 610041 (CN); CHENG, Xu, Chengdu, Sichuan 610041 (CN); WANG, Yuanqiang, Chengdu, Sichuan 610041 (CN); LIU, Lixin, Chengdu, Sichuan 610041 (CN); JIANG, Fan, Chengdu, Sichuan 610041 (CN); YU, Zhou, Chengdu, Sichuan 610041 (CN)
(74) Representative: Johnson, Stephen William
(86) International application number: PCT/CN2023/135650
(87) International publication number: WO 2024/114765

(57) **Abstract**

Provided in the present invention are benzopyrone compounds represented by formula (I) or pharmaceutically acceptable salts thereof, a preparation method therefor and the use thereof. The compounds have good inhibitory effect on POLRMT, and thus can be used for treating and/or preventing POLRMT-mediated diseases such as cancers.

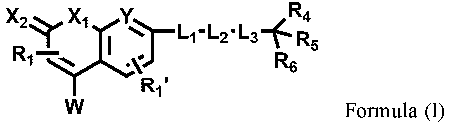

## Description

The present application claims priority to two applications, namely Chinese Patent Application No. 202211527029.6, filed on December 1, 2022, and Chinese Patent Application No. 202310065010.2, filed on February 6, 2023, the entire contents of which are incorporated herein by reference in their entirety.

### Technical Field

The present invention relates to the technical field of chemical medicine, and in particular to a benzopyrone-based compound, a preparation method therefor and the use thereof.

### Background Art

Cancers refer to diseases caused by the abnormal proliferation of cells. In addition to uncontrolled division, cancer cells may locally invade surrounding normal tissues and even metastasize to other parts of the body via the body's circulatory system or lymphatic system, causing damage to the body and even death. Cancers are characterized by high mortality, poor prognosis and costly treatment. Patients with cancers often suffer great physical pain, which is one of the most urgent problems in human health. For decades, with extensive research efforts, technological innovations and drug development, existing cancer therapies, including chemotherapy, radiotherapy and immunotherapy, still fail to meet the current cancer treatment needs. Moreover, issues such as severe side effects and drug resistance cannot be avoided. There is still an urgent need for additional and improved treatment regimens to combat cancers.

Interference with cancer metabolism is another principle for the treatment of cancers. Energy metabolism reprogramming promotes the rapid growth and proliferation of cells by regulating energy metabolism, which is considered a marker of malignant tumors. Targeting metabolic reprogramming has become an important direction in the development of anti-tumor drugs (Luengo et al., 2017, Cell Chem Biol 24, 1161-1180). Since the discovery of the Warburg effect, i.e., the growth rate of cancer cells is much greater than that of normal cells due to the difference in energy sources, with a preference for glycolysis instead of the mitochondrial oxidative phosphorylation process in normal cells, this has led to the hypothesis that oxidative phosphorylation (OXPHOS) is generally down-regulated in cancers. Research has also focused on the pharmacological inhibition of glycolysis. However, many glycolytic inhibitors (e.g., 2-deoxyglucose) do not appear to have a significant effect on tumor growth as expected. This is indeed the case for many cancers, but in some cancers, this hypothesis is being increasingly challenged. There is growing evidence that mitochondrial metabolism is not impaired, including in cases of leukemia, lymphoma, pancreatic ductal adenocarcinoma, the high OXPHOS subtype of melanoma and endometrial cancer (Luengo et al., 2017, Cell Chem Biol 24, 1161-1180; Moreno-Sanchez et al., 2007, FEBS J 274, 1393-1418). Several recent studies have provided strong evidence that tumor metabolism is heterogeneous, with some tumors being powered by oxidative phosphorylation (OXPHOS) (Hu et al., 2013, Nat Biotechnol 31, 522-529; Roesch et al., 2013, Cancer Cell 23, 811-825; Sriskanthadevan et al., 2015, Blood 125, 2120-2130). Elevated OXPHOS levels, increased mitochondrial contribution to total cellular energy, greater entry of fatty acid and glucose-derived carbons into the tricarboxylic acid (TCA) cycle and increased lipogenesis are critical for enhancing tumor growth (Birsoy et al., 2015, Cell 162, 540-551; Martinez-Reyes et al., 2020, Nature 585, 288-292; Sullivan et al., 2015, Cell 162, 552-563), suggesting that targeting OXPHOS will lead to effective cancer treatments.

Studies have shown that acquired treatment resistance can shift tumor cells from utilizing glycolysis to preferentially utilizing OXPHOS. This metabolic heterogeneity enables tumors to adapt to environmental changes and survive. For example, the inhibition of OXPHOS can overcome resistance to chemotherapy (Cannavino et al., 2014, J Physiol 592, 4575-4589; Farge et al., 2017, Cancer Discov 7, 716-735; Lee et al., 2017, Cell Metab 26, 633-647 e637) and tyrosine kinase inhibitors (Zhang et al., 2019, Sci Transl Med 11). OXPHOS is up-regulated in some gene mutation subtypes, including RB1 deficiency, SMARCA4 mutant tumor, PTEN mutation, etc. (Ashton et al., 2018, Clin Cancer Res 24, 2482-2490), as well as tumor subtypes with high OXPHOS typing such as the high OXPHOS type of diffuse large B-cell lymphoma (Caro et al., 2012, Cancer Cell 22, 547-560). Tumor types that have been reported to demonstrate elevated oxidative phosphorylation include acute lymphoblastic leukemia (ALL), colorectal cancer (CRC), glioma, diffuse large B-cell lymphoma, endometrial cancer, esophageal squamous cell carcinoma (ESCC), head and neck cancer, non-Hodgkin's lymphoma, ovarian cancer, papillary thyroid carcinoma, prostate cancer, salivary gland oncocytoma, etc. (Ashton et al., 2018, Clin Cancer Res 24, 2482-2490; Xu et al., 2020, J Med Chem 63, 14276-14307).

These evidences suggest a novel use of OXPHOS inhibitors, i.e., for treating cancers in which OXPHOS is upregulated or alleviating tumor hypoxia to improve the therapeutic effect. Alleviating tumor hypoxia can be achieved in cancers in which OXPHOS is not upregulated, so such inhibitors can be widely applied. Cancer-related studies such as inhibitors targeting respiratory chain complexes I-V and mitochondrial ribosome mechanisms, inhibitors for controlling the translation of respiratory chain subunits and inhibitors of related enzymes have been reported (Ashton et al., 2018; Clin Cancer Res 24, 2482-2490; Xu et al., 2020, J Med Chem 63, 14276-14307).

Recently, mitochondrial RNA polymerase (POLRMT, also known as mtRNAP) has come into the spotlight as a regulatory gene to control the OXPHOS process and has become a new target for targeting tumor metabolism. For example, the inhibition of POLRMT can inhibit AML tumor growth, which has been validated in a mouse CDX model, and the upregulation of the POLRMT gene is positively correlated with poor AML prognosis (Bralha et al., 2015, Oncotarget 6, 37216-37228). POLRMT is responsible for mitochondrial gene expression and provides RNA primers to initiate the replication of the mitochondrial genome and the transcription of 13 subunits of the OXPHOS complex (complexes I, III and IV), serving as an RNA primase for mitochondrial DNA replication. The inhibition of POLRMT affects the transcription of mtDNA, resulting in a decrease in mtDNA expression and a decline in OXPHOS levels.

Therefore, the development of drugs that specifically inhibit POLRMT is of great significance for cancer research.

### Summary of the Invention

The technical problem to be solved by the present invention is how to develop drugs that specifically inhibit POLRMT to effectively treat and intervene in cancers.

Specifically, to solve the above technical problem, the present invention provides the following technical solutions.

A benzopyrone-based compound represented by formula (I), or stereoisomer thereof, or salt thereof, or prodrug thereof, or deuteride thereof, or hydrate thereof, or solvate thereof, 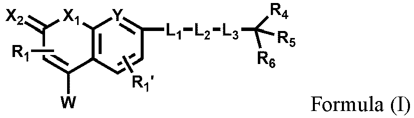
wherein X₁ and X₂ are each independently selected from the group consisting of O or NH group;
Y is selected from the group consisting of CR group or N, wherein R is H or C₁-C₃ alkyl; R₁ and R₁' may be identical or different and are each independently selected from the group consisting of hydrogen, alkyl, cycloalkyl, aryl, heterocyclyl or heteroaryl;
L₁ and L₃ are each independently selected from the group consisting of -O-, -S-, -NR₃-, -CR₂R₃-, -CR₂(R₃), -CO-, -SO- or -SO₂- groups, L₂ is a direct linkage, -CR₂R₃-, -CR₂(R₃)- or -C(R₂R₃)-, wherein R₂ is selected from the group consisting of H or C₁-C₄ linear alkyl; R₃ is selected from the group consisting of H or C₁-C₄ linear alkyl; or R₂ and R₃ in a group of -CR₂(R₃) together form an oxo group;
R₄, R₅ and R₆ are each independently selected from the group consisting of H, halogen, -C-OR₈(R₉), -CR₈(R₉), -CN, -NO₂, -OR₈, -NR₈R₉, -SR₈, -COR₈, -SOR₈, -SO₂R₈, -NR₈COR₉, -CONR₈R₉, -OCOR₈, -COOR₈, -OCONR₈R₉, -NR₈CONR₉R₁₀, -NR₈COOR₉, -NR₈SO₂R₉, -SO₂NR₈R₉, -OSO₂R₈, -SO₃R₈, linear alkyl, linear heteroalkyl, cycloalkyl, heterocycloalkyl, alkenyl, alkynyl, aryl or heteroaryl, or aromatic fused heterocyclyl, wherein R₈, R₉ and R₁₀ are each independently selected from the group consisting of H, linear alkyl, linear heteroalkyl, cycloalkyl, heterocycloalkyl, aryl or heteroaryl; wherein the linear alkyl, linear heteroalkyl, cycloalkyl, heterocycloalkyl, aryl or heteroaryl may be independently substituted with one or more R₁₁ selected from the group consisting of halogen, cyano, hydroxyl, mercapto, ether group, nitro, alkoxy, amino, amine group, carboxyl, sulfonic acid group, ester group, acyloxy, amide, sulfonic acid ester group, sulfonamido, linear alkyl, linear heteroalkyl, cycloalkyl, heterocycloalkyl, aryl or heteroaryl, or aromatic fused heterocyclyl, wherein each of the linear alkyl, linear heteroalkyl, cycloalkyl, heterocycloalkyl, aryl or heteroaryl, or aromatic fused heterocyclyl may be independently substituted with one or more halogen, cyano, hydroxyl, mercapto, ether group, nitro, alkoxy, amino, amine group, carboxyl, sulfonic acid group, ester group, amide, sulfonic acid ester group, sulfonamido, alkyl or haloalkyl; or R₄ and R₅ together form an oxo group;
or, any two or three of R₄, R₅ and R₆ together with the carbon atom to which L₃ is attached form a cyclic group A to which L₃ is attached: 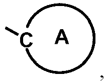 wherein the cyclic group A is any one selected from the group consisting of the following cyclic groups: aromatic ring group, saturated or unsaturated cycloalkyl, monoheterocyclyl, fused heterocyclyl, benzoheterocyclyl, spirocyclic group, bridged cyclic group and their cyclic groups substituted with R₁₁;
W is selected from the group consisting of hydrogen, H, alkyl, -CR₈ₐR₉ₐR₁₀ₐ, -NR₈ₐR₉ₐ, -OR₁₀ₐ, or a carbon atom-containing cyclic group B1 that may be substituted with one or two or more R₇ and is attached to a benzopyrone ring through the carbon atom: 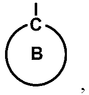 or a nitrogen atom-containing cyclic group B2 that is attached to a benzopyrone ring through the nitrogen atom: 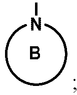 here, R₈ₐ, R₉ₐ and R₁₀ₐ are each independently selected from the group consisting of H, halogen, alkyl, cyano, hydroxyl, mercapto, ether group, nitro, alkoxy, amino, amine group, carboxyl, sulfonic acid group, ester group, acyloxy, amide, sulfonic acid ester group, sulfonamido, cycloalkyl, heterocycloalkyl, aryl or heteroaryl, or aromatic fused heterocyclyl; the cyclic group B1 and the cyclic group B2 are each independently selected from the group consisting of cycloalkyl, heterocycloalkyl, aryl or heteroaryl;
wherein R₇ is selected from the group consisting of H, halogen, -CN, -NO₂, -OR₈, -NR₈R₉, -SR₈, -COR₈, -SOR₈, -SO₂R₈, -NR₈COR₉, -CONR₈R₉, -OCOR₈, -COOR₈, -OCONR₈R₉, -NR₈CONR₉R₁₀, -NR₈COOR₉, -NR₈SO₂R₉, -SO₂NR₈R₉, -OSO₂R₈, -SO₃R₈, linear alkyl, linear heteroalkyl, cycloalkyl, heterocycloalkyl, aryl or heteroaryl, or aromatic fused heterocyclyl, preferably halogen, alkyl, cyano, hydroxyl, mercapto, ether group, nitro, alkoxy, amino, amine group, carboxyl, sulfonic acid group, ester group, amide, acyloxy, sulfonic acid ester group, sulfonamido, cycloalkyl, heterocycloalkyl, aryl or heteroaryl; wherein each of the linear alkyl, linear heteroalkyl, cycloalkyl, heterocycloalkyl, aryl or heteroaryl, or aromatic fused heterocyclyl is independently substituted with one or two or more R₁₁, and R₈, R₉ and R₁₀ have the same meaning as R₈, R₉ and R₁₀ present in R₄, R₅ and R₆; and
formula (I) does not comprise the following compound:

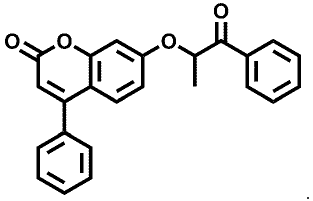

In an embodiment, the compound, or stereoisomer thereof, or salt thereof, or prodrug thereof, or deuteride thereof, or hydrate thereof, or solvate thereof of the present invention as described above,
wherein the X₁ is -O- group, X₂ is -O- group, Y is -CR- group, R₁ and R₁' are both hydrogen;
or, X₁ is -NH- group, X₂ is -O- group, Y is -CR- group, R₁ and R₁' are both hydrogen;
or, X₁ is -O- group, X₂ is -NH- group, Y is -CR- group, R₁ and R₁' are both hydrogen;
or, X₁ is -O- group, X₂ is -O- group, Y is -N- group, R₁ and R₁' are both hydrogen.

In an embodiment, the compound, or stereoisomer thereof, or salt thereof, or prodrug thereof, or deuteride thereof, or hydrate thereof, or solvate thereof of the present invention as described above, wherein the -L₁-L₂-L₃- as a whole is any one selected from the group consisting of -O-CR₂(R₃)-CO-, -O-CR₂R₃-CO-, -CR₂(R₃)-CR₂(R₃)-CO-, -NR₃-CR₂(R₃)-CO-, -S-CR₂(R₃)-CO-, -SO₂-CR₂(R₃)-CO-, -O-CR₂(R₃)-CR₂R₃-, -O-CR₂(R₃)-SO₂- and -CR₂(R₃)-NR₃-CO-;
wherein the R₂ is preferably one of H, methyl and ethyl, more preferably methyl; R₃ is preferably methyl or H, more preferably H.

In an embodiment, the compound, or stereoisomer thereof, or salt thereof, or prodrug thereof, or deuteride thereof, or hydrate thereof, or solvate thereof of the present invention as described in any one of the preceding paragraphs, wherein the -L₁-L₂-L₃- as a whole is any one selected from the group consisting of -O-CH(CH₃)-CO-, -0-CR₂(R₃)-CO-, -CR₂R₃-CH(CH₃)-CO-, -NR₃-CH(CH₃)-CO-, -S-CH(CH₃)-CO-, -SO₂-CH(CH₃)-CO-, -O-CR₂(R₃)-CR₂(R₃)-, and -O-CH(CH₃)-SO₂- or -CR₂(R₃)-NR₃-CO-.

In an embodiment, the compound, or stereoisomer thereof, or salt thereof, or prodrug thereof, or deuteride thereof, or hydrate thereof, or solvate thereof of the present invention as described in any one of the preceding paragraphs, wherein the cyclic group A is any one selected from the group consisting of the following cyclic groups: C₃-C₁₂ cycloalkyl, C₃-C₁₂ heterocycloalkyl, C₅-C₁₂ aryl and C₅-C₁₂ heteroaryl, preferably C₃-C₁₂ cycloalkyl, phenyl, naphthyl, anthranyl, phenanthryl, anthraquinonyl, furyl, pyrrolyl, thienyl, pyrazolyl, imidazolyl, oxazolyl, thiazolyl, isothiazolyl, pyridyl, pyranyl, thiopyranyl, pyridazinyl, pyrimidinyl, pyrazinyl, piperazinyl, indolyl, benzimidazolyl, carbazolyl, thiazolinyl, quinolinyl, isoquinolinyl, purinyl, acridinyl, phenazinyl and phenothiazinyl;
more preferably, the cyclic group A is any one selected from the group consisting of the following cyclic groups: 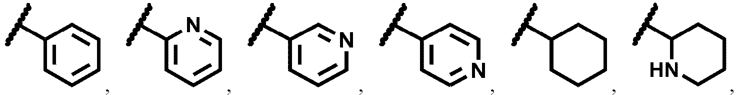 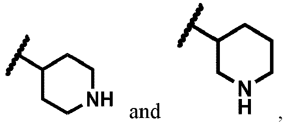 wherein these cyclic groups A may be substituted with one or two or more groups selected from the group consisting of halogen, cyano, hydroxyl, mercapto, ether group, nitro, alkoxy, amino, amine group, carboxyl, sulfonic acid group, ester group, amide, sulfonic acid ester group, sulfonamido, linear alkyl, linear heteroalkyl, cycloalkyl, heterocycloalkyl, aryl and heteroaryl.

In an embodiment, the compound, or stereoisomer thereof, or salt thereof, or prodrug thereof, or deuteride thereof, or hydrate thereof, or solvate thereof of the present invention as described in any one of the preceding paragraphs, wherein the R₄, R₅ and R₆ are each independently selected from the group consisting of -CR₈(R₉), R₈ is H, linear alkyl, cyano or cycloalkyl, and R₉ is linear alkyl, linear heteroalkyl, cycloalkyl, heterocycloalkyl, aryl or heteroaryl substituted with halogen, cyano, hydroxyl, mercapto, ether group, nitro, alkoxy, amino, amine group, carboxyl, sulfonic acid group, ester group, acyl, amide, sulfonic acid ester group and sulfonamido, where R₉ is attached to the carbon atom to which L₃ is attached through the oxygen atom, nitrogen atom or carbon atom on the substituent.

In an embodiment, the compound, or stereoisomer thereof, or salt thereof, or prodrug thereof, or deuteride thereof, or hydrate thereof, or solvate thereof of the present invention as described in any one of the preceding paragraphs, wherein the substituted cyclic group B1 and the substituted cyclic group B2 are each independently selected from the group consisting of halocycloalkyl, haloheterocycloalkyl, haloaryl and haloheteroaryl;
further preferably, the substituted cyclic group B1 and the substituted cyclic group B2 are each independently 2-chloro-4-fluorophenyl, chlorophenyl, methyl-substituted phenyl, amide-substituted phenyl, amino-substituted phenyl or phenylalkyl.

In an embodiment, the compound, or stereoisomer thereof, or salt thereof, or prodrug thereof, or deuteride thereof, or hydrate thereof, or solvate thereof of the present invention as described in any one of the preceding paragraphs, wherein the cyclic group B1 is C₅-C₁₀ aryl, C₅-C₁₀ heteroaryl, C₃-C₁₀ cycloalkyl or C₃-C₁₀ heterocycloalkyl.

In an embodiment, the compound, or stereoisomer thereof, or salt thereof, or prodrug thereof, or deuteride thereof, or hydrate thereof, or solvate thereof of the present invention as described in any one of the preceding paragraphs, wherein the compound of formula (I) is selected from the group consisting of compounds represented by any one of the following structural formulas: 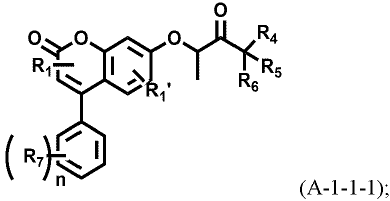 wherein the R₇ is preferably any one or two or more of F, Cl, Br, I, OH, OR₂, C₁₋₃ alkyl, sulfonic acid group, nitro, amino and amine group; R₁ and R₁' are independently selected from the group consisting of hydrogen, C₁₋₄ alkyl, C₁₋₄ alkoxy; wherein R₂ is the same as defined in claim 1; n is an integer from 0 to 5; 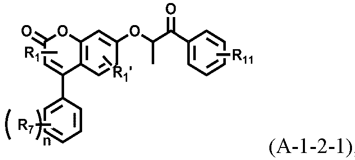 wherein the R₇ is preferably any one or two or more of F, Cl, Br, I, OH, OR₂ₐ, C₁₋₃ alkyl, sulfonic acid group, nitro, amino or amine group; R₁₁ is preferably any one or two or more of F, Cl, Br, I, C₁₋₄ alkoxy, acyl, or C₁₋₃ alkyl, sulfonic acid group, nitro, amino, cyano, amine group, -C₀₋₄ alkyl-COOH, ester group, acyloxy, ether group, amide, aminoacyl, cycloalkyl-substituted aminoacyl, or aralkyl-substituted aminoacyl, carboxyl-substituted C₁₋₃ alkoxy, carboxyl-substituted amine group, cycloalkyl-substituted C₁₋₃ alkoxy, acyloxy-containing C₃₋₅ cycloalkyl or 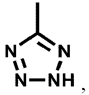 wherein R₁₁ may form a benzene fused azacyclic or oxacyclic structure with the benzene ring to which it is attached; R₁ and R₁' are independently selected from the group consisting of hydrogen, C₁₋₄ alkyl, C₁₋₄ alkyloxy; R₂ₐ is selected from the group consisting of C₁-C₄ alkyl, C₁-C₄ alkenyl, C₁-C₂ alkoxy-substituted C₁-C₄ alkyl, C₁-C₄ alkyl-substituted formyl; n is an integer from 0 to 5; 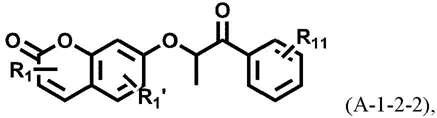 wherein R₁₁ is preferably -C₀₋₄ alkyl-COOH; R₁ and R₁' are independently selected from the group consisting of hydrogen, C₁₋₄ alkyl, C₁₋₄ alkoxy, C₁₋₄ alkylphenyl; 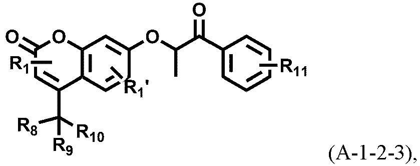 wherein 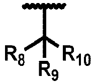 is preferably C₁₋₄ alkyl and C₁₋₄ alkylphenyl, more preferably methyl, isobutyl or benzyl; R₁₁ is preferably -C₀₋₄ alkyl-COOH; R₁ and R₁' are independently selected from the group consisting of hydrogen, C₁₋₄ alkyl, C₁₋₄ alkoxy, C₁₋₄ alkylphenyl; 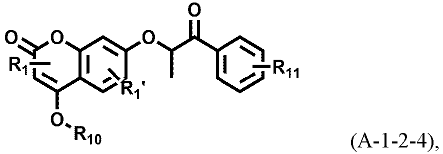 wherein R₁₀ is preferably C₁₋₄ alkyl, hydroxyl-substituted C₁₋₄ alkyl, C₁₋₄ alkoxy or hydroxyl-substituted C₁₋₄ alkoxy; R₁₁ is preferably -C₀-C₄ alkyl-COOH; R₁ and R₁' are independently selected from the group consisting of hydrogen, C₁₋₄ alkyl, C₁₋₄ alkoxy, C₁₋₄ alkylphenyl; 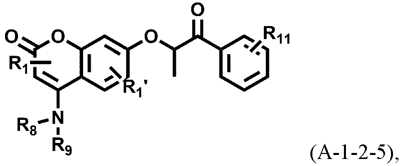 wherein R₈ and R₉ are independently selected from the group consisting of hydrogen or C₁₋₄ alkyl; R₁₁ is preferably -C₀-C₄ alkyl-COOH; R₁ and R₁' are independently selected from the group consisting of hydrogen, C₁₋₄ alkyl, C₁₋₄ alkoxy, C₁₋₄ alkylphenyl; 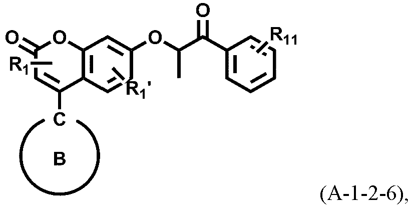 wherein the cyclic group, i.e., cyclic group B1, is preferably selected from the group consisting of C₃₋₁₂ cycloalkyl, 4- to 10-membered heterocyclyl or 5- to 10-membered aryl; R₁₁ is preferably -C₀-C₄ alkyl-COOH; the heterocyclyl is further preferably any one of furyl, pyrrolyl, thienyl, pyrazolyl, imidazolyl, oxazolyl, thiazolyl, isothiazolyl, pyridyl, pyranyl, thiopyranyl, pyridazinyl, pyrimidinyl, pyrazinyl, piperazinyl, indolyl, benzimidazolyl, carbazolyl, thiazolinyl, quinolinyl, isoquinolinyl, purinyl, acridinyl, phenazinyl and phenothiazinyl; R₁ and R₁' are both preferably hydrogen, C₁₋₄ alkyl, C₁₋₄ alkoxy, C₁₋₄ alkylphenyl; the cyclic group B1 is optionally substituted with one or two or more of hydrogen, halogen, hydroxyl, C₁₋₆ alkyl, C₁₋₆ alkyl-substituted hydroxyl, amino and amine group, or is unsubstituted; 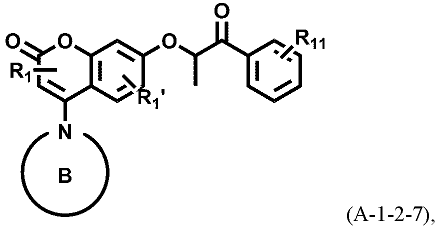 wherein the cyclic group, i.e., cyclic group B2, is preferably selected from the group consisting of C₃₋₁₂ cycloalkyl, 4- to 10-membered heterocyclyl or 5- to 10-membered aryl; R₁₁ is preferably -C₀-₄ alkylcarboxyl; the heterocyclyl is further preferably any one of furyl, pyrrolyl, thienyl, pyrazolyl, imidazolyl, oxazolyl, thiazolyl, isothiazolyl, pyridyl, pyranyl, thiopyranyl, pyridazinyl, pyrimidinyl, pyrazinyl, piperazinyl, indolyl, benzimidazolyl, carbazolyl, thiazolinyl, quinolinyl, isoquinolinyl, purinyl, acridinyl, phenazinyl and phenothiazinyl; R₁ and R₁' are both preferably hydrogen; the cyclic group B2 is optionally substituted with one or two or more of hydrogen, halogen, hydroxyl, C₁₋₆ alkyl, C₁₋₆ alkyl-substituted hydroxyl, amino and amine group, or is unsubstituted; 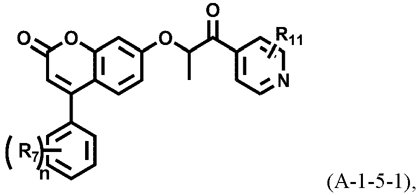 wherein the R₇ is preferably any one or two or more of F, Cl, Br, I, OH, OR₂ₐ, C₁₋₆ alkyl, sulfonic acid group, nitro, amino and amine group, and more preferably, the benzene ring in the structural formula is substituted with both chlorine and fluorine; R₁₁ is preferably any one or two or more of F, Cl, Br, I, oxo, C₁₋₃ alkoxy or alkyl, cycloalkyl, sulfonic acid group, nitro, amino, cyano, amine group, -C₀-₄ alkyl-carboxyl, ester group, acyloxy and ether group; R₁ and R₁' are independently selected from the group consisting of hydrogen, C₁₋₄ alkyl, C₁₋₄ alkoxy, C₁₋₄ alkylphenyl; n is an integer from 0 to 5; R₂ₐ is selected from the group consisting of C₁-C₄ alkyl, C₁-C₄ alkenyl, C₁-C₂ alkoxy-substituted C₁-C₄ alkyl, C₁-C₄ alkyl-substituted formyl; 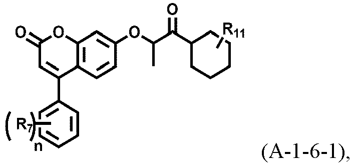 wherein the R₇ is preferably any one or two or more of F, Cl, Br, I, OH, OR₂ₐ, C₁₋₆ alkyl, sulfonic acid group, nitro, amino or amine group, and more preferably, the benzene ring in the structural formula is substituted with both chlorine and fluorine; R₁₁ is preferably any one or two or more of F, Cl, Br, I, oxo, C₁₋₆ alkoxy, C₁₋₆ alkyl, cycloalkyl, sulfonic acid group, nitro, amino, cyano, amine group or -C₀-₄ alkyl-carboxyl; R₁ and R₁' are independently selected from the group consisting of hydrogen, C₁₋₄ alkyl, C₁₋₄ alkoxy, C₁₋₄ alkylphenyl; n is an integer from 0 to 5; R₂ₐ is selected from the group consisting of C₁-C₄ alkyl, C₁-C₄ alkenyl, C₁-C₂ alkoxy-substituted C₁-C₄ alkyl, C₁-C₄ alkyl-substituted formyl; 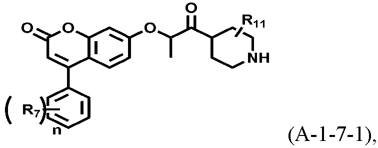 wherein the R₇ is preferably any one or two or more of F, Cl, Br, I, OH, OR₂ₐ, C₁₋₆ alkyl, sulfonic acid group, nitro, amino or amine group, and more preferably, the benzene ring in the structural formula is substituted with both chlorine and fluorine; R₁₁ is preferably any one or two or more of F, Cl, Br, I, oxo, C₁₋₆ alkoxy, C₁₋₆ alkyl, C₃₋₁₀ cycloalkyl, sulfonic acid group, hydroxyl, hydroxyl-substituted C₁₋₆ alkyl, nitro, amino, cyano, amine group or -C₀-₄ alkylcarboxyl; n is an integer from 0 to 5; R₂ₐ is selected from the group consisting of C₁-C₄ alkyl, C₁-C₄ alkenyl, C₁-C₂ alkoxy-substituted C₁-C₄ alkyl, C₁-C₄ alkyl-substituted formyl; 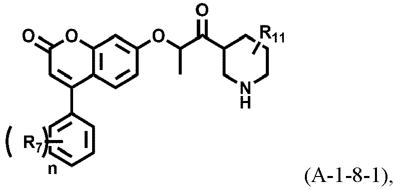 wherein the R₇ is preferably any one or two or more of F, Cl, Br, I, OH, OR₂ₐ, C₁₋₆ alkyl, C₃₋₁₀ cycloalkyl, sulfonic acid group, nitro, amino or amine group, and more preferably, the benzene ring in the structural formula is substituted with both chlorine and fluorine; R₁₁ is preferably any one or two or more of F, Cl, Br, I, oxo, C₁₋₃ alkoxy or alkyl, cycloalkyl, sulfonic acid group, hydroxyl, nitro, amino, cyano, amine group, -C₀-₄ alkylcarboxyl; n is an integer from 0 to 5; R₂ₐ is selected from the group consisting of C₁-C₄ alkyl, C₁-C₄ alkenyl, C₁-C₂ alkoxy-substituted C₁-C₄ alkyl, C₁-C₄ alkyl-substituted formyl; 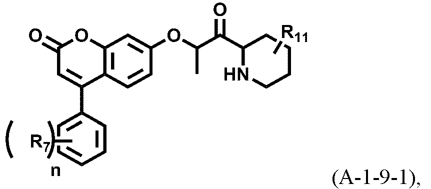 wherein the R₇ is preferably any one or two or more of F, Cl, Br, I, OH, OR₂ₐ, or C₁₋₃ alkyl, sulfonic acid group, nitro, amino and amine group, and more preferably, the benzene ring in the structural formula is substituted with both chlorine and fluorine; R₁₁ may be hydrogen, or any one or two or more of F, Cl, Br, I, oxo, C₁₋₆ alkoxy, C₁₋₆ alkyl, C₃₋₁₀ cycloalkyl, sulfonic acid group, hydroxyl, nitro, amino, cyano, amine group and -C₀-₄ alkylcarboxyl; n is an integer from 0 to 5; R₂ₐ is selected from the group consisting of C₁-C₄ alkyl, C₁-C₄ alkenyl, C₁-C₂ alkoxy-substituted C₁-C₄ alkyl, C₁-C₄ alkyl-substituted formyl; 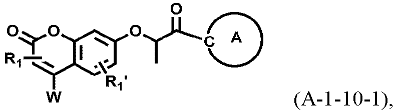 wherein the cyclic group A is aryl, cycloalkyl, heterocycloalkyl or heteroaryl which may be substituted or unsubstituted with one or two or more groups selected from the group consisting of halogen, cyano, hydroxyl, mercapto, ether group, nitro, alkoxy, amino, amine group, -C₀-₄ alkyl-carboxyl, sulfonic acid group, ester group, amide, sulfonic acid ester group, sulfonamido, linear alkyl, linear heteroalkyl, cycloalkyl and heterocycloalkyl; wherein the cycloalkyl is preferably C₃₋₆ cycloalkyl; the heterocycloalkyl is preferably any one of oxiranyl, oxetanyl, aziridinyl, azetidinyl and thietanyl; the heteroaryl comprises 5-membered heteroaryl such as furyl, thienyl, pyrrolyl, oxazolyl, thiazolyl, imidazolyl or pyrazolyl, or oxygen-containing imidazolyl or pyrazolyl, 6-membered heteroaryl such as pyridyl, pyrimidinyl, pyranyl, pyridazinyl, pyrazinyl, pyranone-forming group or pyranyl, benzoheterocyclyl such as benzofuranyl, benzothienyl, benzopyrrolyl, indolyl, quinolyl, isoquinolyl, benzopyranyl, a group formed by benzo-γ-pyranone, heterocycloheterocyclyl such as purinyl; W is preferably 2-chloro-4-fluoro-substituted phenyl or 2-methylphenyl; R₁ and R₁' are independently selected from the group consisting of hydrogen, C₁₋₄ alkyl, C₁₋₄ alkoxy, C₁₋₄ alkylphenyl; 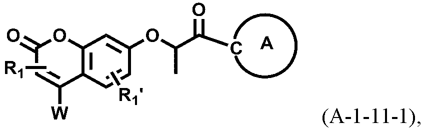 wherein the cyclic group A is preferably fused cyclic group or aromatic fused heterocyclic group, and more preferably, the benzoheterocyclyl is benzofuranyl, benzothienyl, benzopyrrolyl, benzoheterocyclyl such as indolyl, quinolinyl, isoquinolinyl, benzopyranyl, a group formed by benzo-γ-pyrone; W is preferably 2-chloro-4-fluoro-substituted phenyl or 2-methylphenyl; R₁ and R₁' are independently selected from the group consisting of hydrogen, C₁₋₄ alkyl, C₁₋₄ alkoxy, C₁₋₄ alkylphenyl; 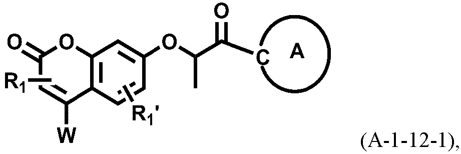 wherein the cyclic group A is preferably selected from the group consisting of spirocyclic group or bridged cyclic group; W is preferably 2-chloro-4-fluoro-substituted benzene or 2-methylphenyl; R₁ and R₁' are independently selected from the group consisting of hydrogen, C₁₋₄ alkyl, C₁₋₄ alkoxy, C₁₋₄ alkylphenyl; 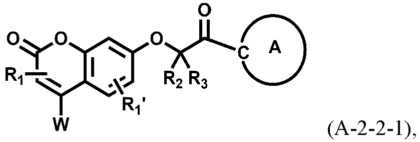 wherein the cyclic group A is preferably phenyl, halophenyl, -C₀-₄ alkylcarboxyl-substituted phenyl, further preferably phenyl or carboxyl-substituted phenyl; W is preferably 2-chloro-4-fluoro-substituted benzene or 2-methylphenyl; R₁ and R₁' are independently selected from the group consisting of hydrogen, C₁₋₄ alkyl, C₁₋₄ alkoxy, C₁₋₄ alkylphenyl; 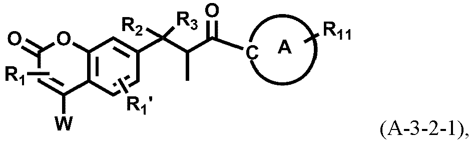 wherein the cyclic group A is preferably phenyl substituted or unsubstituted with any one or two or more of F, Cl, Br, I, oxo, C₁₋₆ alkoxy, C₁₋₆ alkyl, C₃₋₁₀ cycloalkyl, sulfonic acid group, nitro, amino, cyano, amine group or -C₀₋₄ alkyl-carboxyl as R₁₁; further preferably, the cyclic group A is phenyl substituted with R₁₁ as carboxyl group; W is preferably 2-chloro-4-fluoro-substituted benzene or 2-methylphenyl; R₁ and R₁' are independently selected from the group consisting of hydrogen, C₁₋₄ alkyl, C₁₋₄ alkoxy, C₁₋₄ alkylphenyl; 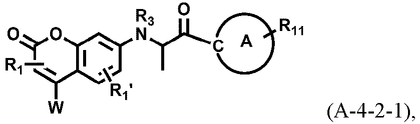 wherein the cyclic group A is preferably phenyl substituted or unsubstituted with any one or two or more of F, Cl, Br, I, oxo, C₁₋₆ alkoxy, C₁₋₆ alkyl, C₃₋₁₀ cycloalkyl, sulfonic acid group, nitro, amino, cyano, amine group or -C₀-₄ alkyl-carboxyl as R₁₁; further preferably, the cyclic group A is phenyl substituted with R₁₁ as carboxyl group; W is preferably 2-chloro-4-fluoro-substituted benzene or 2-methylphenyl; R₁ and R₁' are independently selected from the group consisting of hydrogen, C₁₋₄ alkyl, C₁₋₄ alkoxy, C₁₋₄ alkylphenyl; 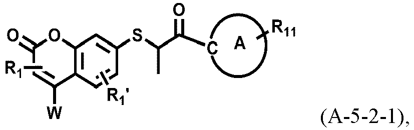 wherein the cyclic group A is preferably phenyl substituted or unsubstituted with any one or two or more of F, Cl, Br, I, oxo, C₁₋₆ alkoxy, C₁₋₆ alkyl, C₃₋₁₀ cycloalkyl, sulfonic acid group, nitro, amino, cyano, amine group or -C₀-₄ alkyl-carboxyl as R₁₁; further preferably, the cyclic group A is phenyl substituted with R₁₁ as carboxyl group; W is preferably 2-chloro-4-fluoro-substituted benzene or 2-methylphenyl; R₁ and R₁' are independently selected from the group consisting of hydrogen, C₁₋₄ alkyl, C₁₋₄ alkoxy, C₁₋₄ alkylphenyl; 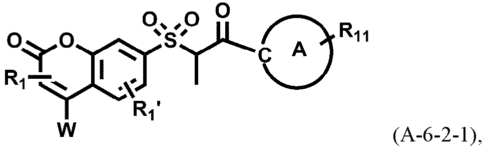 wherein the cyclic group A is preferably phenyl substituted or unsubstituted with any one or two or more of F, Cl, Br, I, oxo, C₁₋₆ alkoxy, C₁₋₆ alkyl, C₃₋₁₀ cycloalkyl, sulfonic acid group, nitro, amino, cyano, amine group or -C₀-₄ alkylcarboxyl as R₁₁; further preferably, the cyclic group A is phenyl substituted with R₁₁ as carboxyl group; W is preferably 2-chloro-4-fluoro-substituted benzene; R₁ and R₁' are independently selected from the group consisting of hydrogen, C₁₋₄ alkyl, C₁₋₄ alkoxy, C₁₋₄ alkylphenyl; 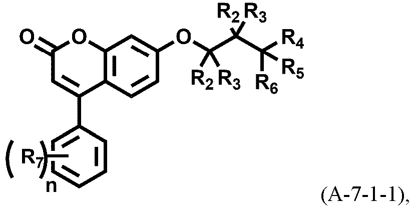 wherein R₇ is selected from the group consisting of hydrogen, C₁₋₄ alkyl, C₁₋₄ alkoxy; n is an integer from 0 to 5; R₂ and R₃ are independently selected from the group consisting of hydrogen, C₁₋₄ alkyl; R₄ and R₅ together form an oxo group; 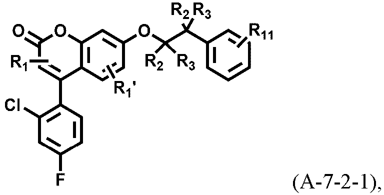 wherein R₂ and R₃ are each independently preferably hydrogen, C₁₋₆ alkyl; or one set of R₂ and R₃ together form an oxo group, and the other set of R₂ and R₃ is independently preferably hydrogen, C₁₋₆ alkyl; R₁ and R₁' are independently selected from the group consisting of hydrogen, C₁₋₄ alkyl, C₁₋₄ alkoxy, C₁₋₄ alkylphenyl; 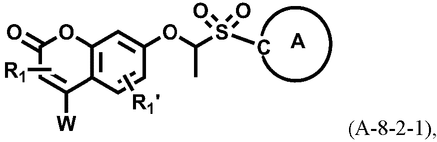 wherein the cyclic group A is preferably phenyl that may be substituted or unsubstituted with any one or two or more of F, Cl, Br, I, oxo, C₁₋₆ alkoxy, C₁₋₆ alkyl, C₃₋₁₀ cycloalkyl, sulfonic acid group, nitro, amino, cyano, amine group or carboxyl; further preferably, the cyclic group A is phenyl substituted with R₁₁ as carboxyl group; W is preferably 2-chloro-4-fluoro-substituted benzene or 2-methylphenyl; R₁ and R₁' are both preferably hydrogen; 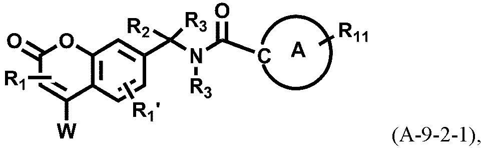 wherein the cyclic group A is preferably phenyl substituted with any one or two or more of F, Cl, Br, I, oxo, C₁₋₆ alkoxy, C₁₋₆ alkyl, C₃₋₁₀ cycloalkyl, sulfonic acid group, nitro, amino, cyano, amine group or C₀-₄ alkylcarboxyl as R₁₁; further preferably, the cyclic group A is phenyl substituted with R₁₁ as carboxyl group; W is preferably 2-chloro-4-fluoro-substituted benzene or 2-methylphenyl; R₁ and R₁' are independently selected from the group consisting of hydrogen, C₁₋₄ alkyl, C₁₋₄ alkoxy, C₁₋₄ alkylphenyl; 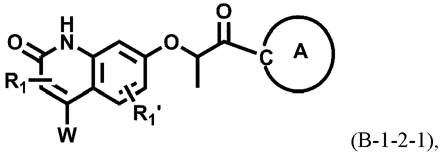 wherein the cyclic group A is preferably phenyl substituted or unsubstituted with any one or two or more of F, Cl, Br, I, oxo, C₁₋₆ alkoxy, C₁₋₆ alkyl, C₃₋₁₀ cycloalkyl, sulfonic acid group, nitro, amino, cyano, amine group or C₀-₄ alkylcarboxyl; further preferably, the cyclic group A is phenyl substituted with R₁₁ as carboxyl group; W is preferably 2-chloro-4-fluoro-substituted benzene or 2-methylphenyl; R₁ and R₁' are independently selected from the group consisting of hydrogen, C₁₋₄ alkyl, C₁₋₄ alkoxy, C₁₋₄ alkylphenyl; 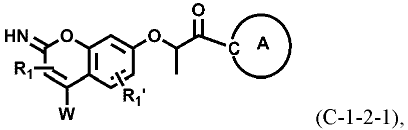 wherein the cyclic group A is preferably phenyl substituted with any one or two or more of F, Cl, Br, I, oxo, C₁₋₆ alkoxy, C₁₋₆ alkyl, C₃₋₁₀ cycloalkyl, sulfonic acid group, nitro, amino, cyano, amine group or C₀-₄ alkylcarboxyl; further preferably, the cyclic group A is phenyl substituted with R₁₁ as carboxyl group; W is preferably 2-chloro-4-fluoro-substituted benzene or 2-methylphenyl; R₁ and R₁' are independently selected from the group consisting of hydrogen, C₁₋₄ alkyl, C₁₋₄ alkoxy, C₁₋₄ alkylphenyl; and 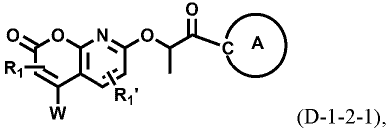 wherein the cyclic group A is preferably phenyl substituted with any one or two or more of F, Cl, Br, I, oxo, C₁₋₆ alkoxy, C₁₋₆ alkyl, C₃₋₁₀ cycloalkyl, sulfonic acid group, nitro, amino, cyano, amine group or C₀-₄ alkylcarboxyl; further preferably, the cyclic group A is phenyl substituted with R₁₁ as carboxyl group; W is preferably 2-chloro-4-fluoro-substituted benzene or 2-methylphenyl; R₁ and R₁' are independently selected from the group consisting of hydrogen, C₁₋₄ alkyl, C₁₋₄ alkoxy, C₁₋₄ alkylphenyl.

Also provided in the present invention is a pharmaceutical composition including the compound, or stereoisomer thereof, or salt thereof, or prodrug thereof, or deuteride thereof, or hydrate thereof, or solvate thereof as described in any one of the preceding paragraphs; or pharmaceutically acceptable salt thereof, and pharmaceutically acceptable carriers.

Also provided in the present invention is the use of the compound, or stereoisomer thereof, or salt thereof, or prodrug thereof, or deuteride thereof, or hydrate thereof, or solvate thereof as described in any one of the preceding paragraphs; or pharmaceutically acceptable salt thereof, or the pharmaceutical composition as described above, in the manufacture of drug as POLRMT inhibitor. Also provided in the present invention is the use of the compound, or stereoisomer thereof, or salt thereof, or prodrug thereof, or deuteride thereof, or hydrate thereof, or solvate thereof as described in any one of the preceding paragraphs; or pharmaceutically acceptable salt thereof, or the pharmaceutical composition as described above, in the manufacture of drug for diseases associated with abnormally high expression of POLRMT in oxidative phosphorylation.

In a more specific embodiment, the use described in the present invention is characterized in that the diseases are cancers; preferably, the cancers are melanoma, metastatic melanoma, pancreatic cancer, pancreatic ductal adenocarcinoma, prostate cancer, lung cancer, hepatocellular carcinoma, lymphoma, leukemia, multiple myeloma, breast cancer, glioma, glioblastoma, cervical cancer, renal cancer, colorectal cancer or ovarian cancer.

The present invention has the following beneficial technical effects:
1) The compounds of the present invention have better inhibitory activity against ovarian cancer cells than the compounds disclosed in the prior art.
2) The compounds of the present invention have good pharmacokinetic properties, e.g., good parameters such as Cmax and AUC.

### Brief Description of the Drawings

FIG. 1 shows BLI imaging results of the MOLM-13-luc orthotopic mouse tumor model in Experimental Example 4;
FIG. 2 shows the survival curves of the MOLM-13-luc orthotopic mouse tumor model in Experimental Example 4.

### Detailed Description of the Invention

To develop drugs for specifically inhibiting POLRMT to effectively treat or prevent cancers, or halt the progression of cancers, the present invention provides the following sets of technical solutions.

### First Set of Technical Solutions

It is an object of the present invention to provide a benzopyrone-based compound, as represented by general formula α-(I), general formula α-(II), general formula α-(III) and the like, and pharmaceutically acceptable salt thereof.

It is another object of the present invention to provide a pharmaceutical composition comprising any one of the compounds described above or pharmaceutically acceptable salts thereof, and pharmaceutically acceptable carriers.

It is another object of the present invention to provide the use of the compounds of the above general formulas or pharmaceutically acceptable salts thereof as POLRMT inhibitors or in the manufacture of drugs for diseases associated with abnormally high expression of POLRMT in oxidative phosphorylation.

It is another object of the present invention to provide the use of the compounds of the above general formulas or pharmaceutically acceptable salts thereof in the manufacture of drugs for treating and/or preventing cancers, such as melanoma, metastatic melanoma, pancreatic cancer, pancreatic ductal adenocarcinoma, prostate cancer, lung cancer, hepatocellular carcinoma, lymphoma, leukemia, myeloma, breast cancer, glioma, glioblastoma, cervical cancer, renal cancer, colorectal cancer or ovarian cancer.

It is another object of the present invention to provide a preparation method for the compounds of the above general formulas.

To achieve the above objectives, the technical solutions adopted by the present invention are as follows.

In some embodiments of the present invention, provided are compounds represented by general formula (I) or pharmaceutically acceptable salts thereof: 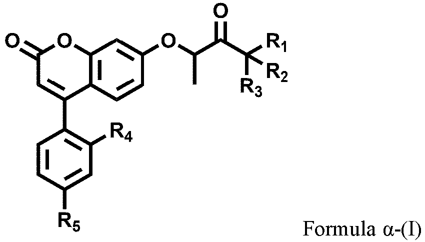
wherein R₁, R₂ and R₃ are independently selected from the group consisting of hydrogen, C₁-C₆ alkyl, and R₁, R₂ and R₃ are not all hydrogen;
or, R₁ and R₂ are attached to each other to form a ring as shown below, and R₃ is hydrogen or is absent: 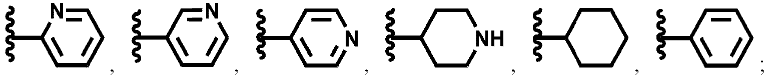 optionally,
the ring formed by the attachment of R₁ and R₂ to each other is substituted with m R' groups;
m is selected from the group consisting of 0, 1, 2, 3, 4 or 5;
R' is selected from the group consisting of hydrogen, amino, carboxyl, C₁-₆ alkylcarboxyl;
R₄ and R₅ are independently selected from the group consisting of hydrogen, halogen, C₁-₆ alkyl.

In some embodiments of the present invention, provided are compounds represented by general formula (II) or pharmaceutically acceptable salts thereof: 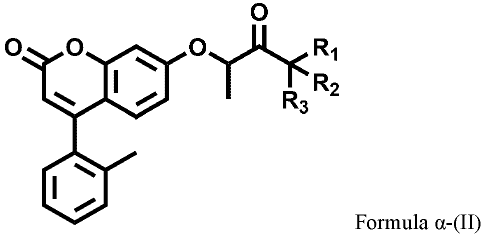
wherein R₁, R₂ and R₃ are independently selected from the group consisting of hydrogen, C₁-C₆ alkyl, and R₁, R₂ and R₃ are not all hydrogen; or, R₁ and R₂ are attached to each other to form a ring as shown below, and R₃ is hydrogen or is absent: 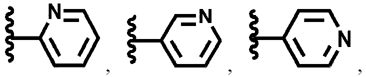 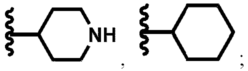
optionally, the ring formed by R₁ and R₂ is substituted with m R' groups, m is selected from the group consisting of 0 or 1, and the R' is selected from the group consisting of hydrogen, amino, carboxyl or C₁-C₆ alkylcarboxyl.

In some embodiments of the present invention, provided are compounds represented by general formula (III) or pharmaceutically acceptable salts thereof: 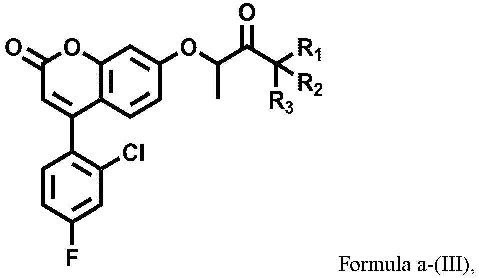
wherein R₁ and R₂ are attached to form a ring as shown below, and R₃ is hydrogen or is absent: 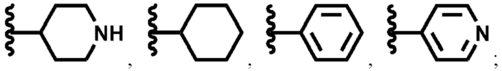
optionally, the ring formed by R₁ and R₂ is substituted with m R' groups, m is selected from the group consisting of 0 or 1, and the R' is selected from the group consisting of hydrogen, amino, carboxyl or C₁-C₆ alkylcarboxyl.

In some embodiments of the present invention, provided are compounds as shown below, but are not limited to the following specific compounds:

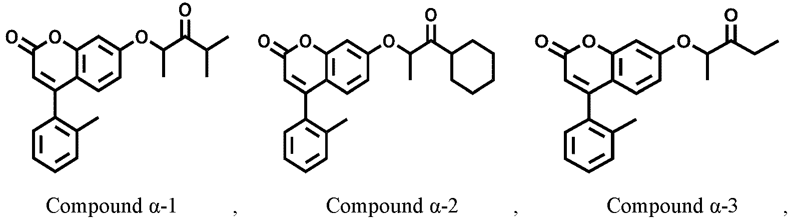

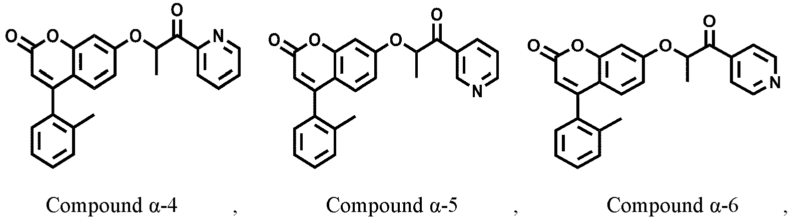

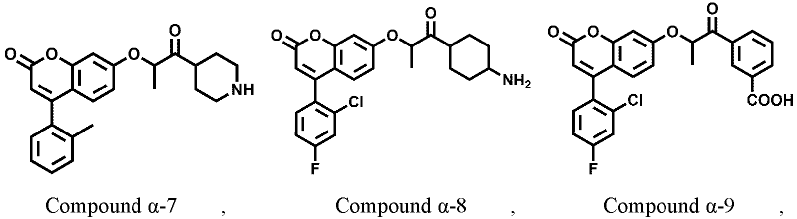

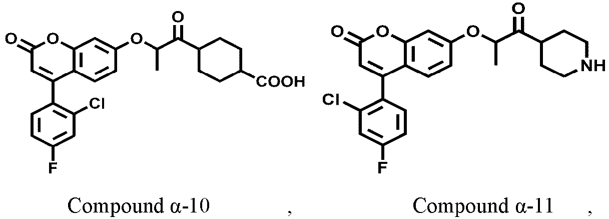

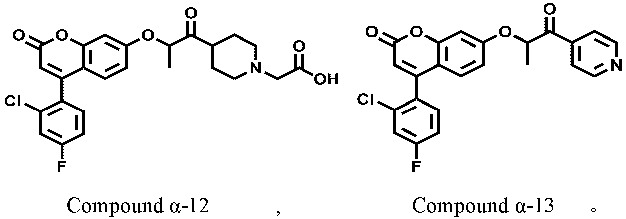

In some embodiments of the present invention, provided is a pharmaceutical composition comprising at least one of the compounds of the present invention described herein or pharmaceutically acceptable salts thereof, and pharmaceutically acceptable carriers, additives or excipients.

In some embodiments of the present invention, provided is the use of the compounds of the present invention described herein or pharmaceutically acceptable salts thereof, and the above pharmaceutical composition, in the manufacture of POLRMT inhibitors or drugs for diseases associated with abnormally high expression of POLRMT in oxidative phosphorylation.

In some embodiments of the present invention, provided is the use of the compounds of the present invention described herein or pharmaceutically acceptable salts thereof, stereoisomers thereof, solvates thereof, or hydrates thereof, and the above pharmaceutical composition, in the manufacture of drugs for treating and/or preventing cancers; preferably the use in the manufacture of drugs for treating and/or preventing melanoma, metastatic melanoma, pancreatic cancer, pancreatic ductal adenocarcinoma, prostate cancer, lung cancer, hepatocellular carcinoma, lymphoma, leukemia, myeloma, breast cancer, glioma, glioblastoma, cervical cancer, renal cancer, colorectal cancer or ovarian cancer.

### Second Set of Technical Solutions

Provided in the present invention is a benzopyrone-based compound, as general formula (I'), and pharmaceutically acceptable salt thereof.

It is another object of the present invention to provide a pharmaceutical composition comprising any one of the compounds described above or pharmaceutically acceptable salts thereof, and pharmaceutically acceptable carriers.

It is another object of the present invention to provide the use of the compounds of the above general formula or pharmaceutically acceptable salts thereof as POLRMT inhibitors or in the manufacture of drugs for diseases associated with abnormally high expression of POLRMT in oxidative phosphorylation.

It is another object of the present invention to provide the use of the compounds of the above general formula or pharmaceutically acceptable salts thereof in the manufacture of drugs for treating and/or preventing cancers, such as melanoma, metastatic melanoma, pancreatic cancer, pancreatic ductal adenocarcinoma, prostate cancer, lung cancer, hepatocellular carcinoma, lymphoma, leukemia, myeloma, breast cancer, glioma, glioblastoma, cervical cancer, renal cancer, colorectal cancer or ovarian cancer.

It is another object of the present invention to provide a preparation method for the compounds of the above general formula.

In some embodiments of the present invention, provided are compounds represented by general formula (I') or pharmaceutically acceptable salts thereof: 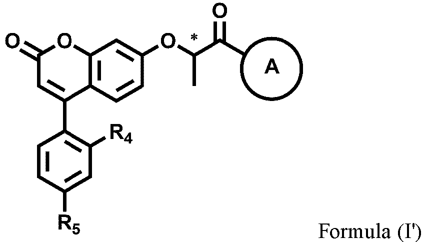
wherein ring A is optionally selected from the group consisting of 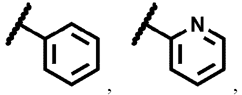 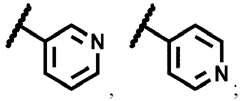
the configuration of the * carbon is R or S;
ring A is optionally substituted with any one or two or more of R'₁, R'₂, R'₃, R'₄ and R'₅;
the R'₁, R'₂, R'₃, R'₄ and R'₅ are independently any one selected from the group consisting of hydrogen, halogen, carboxyl and C₁-₆ alkylcarboxyl;
R₄ and R₅ are independently any one selected from the group consisting of hydrogen, halogen and C₁-₆ alkyl.

In some embodiments of the present invention, provided are compounds or pharmaceutically acceptable salts thereof, selected from any one of the following compounds: 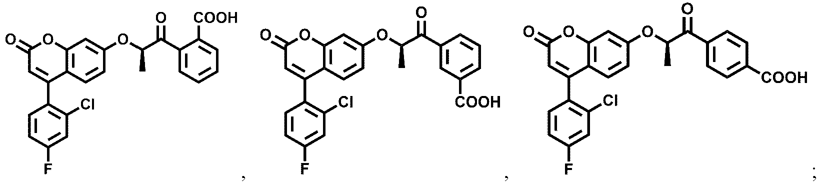 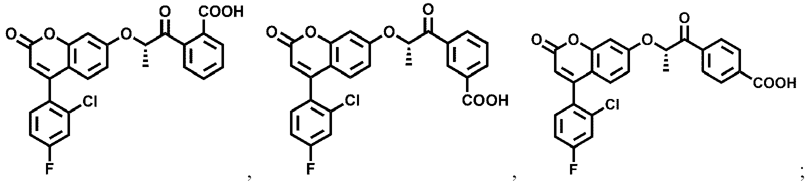 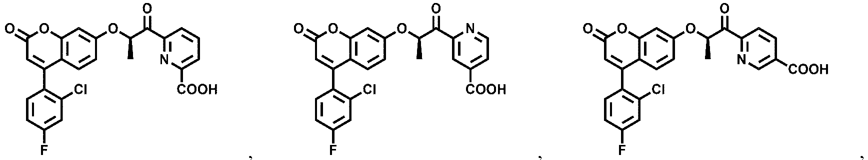 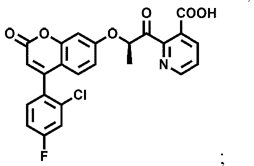 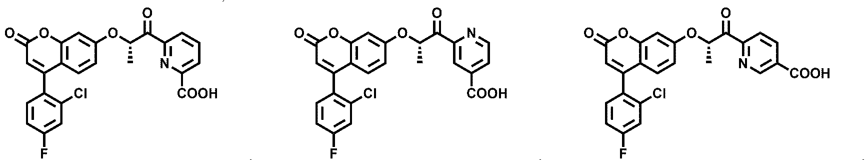 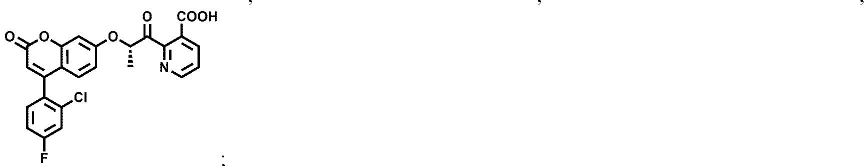 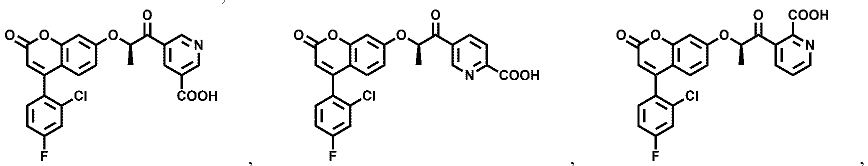 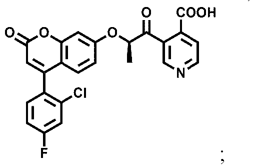 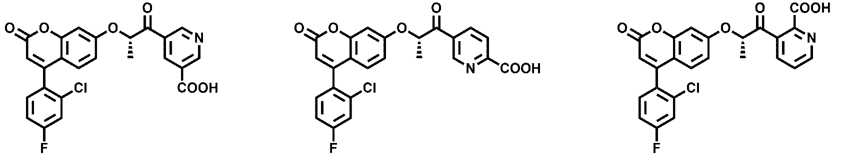 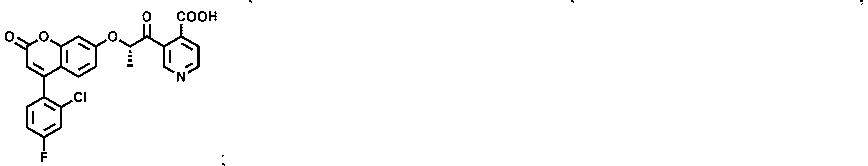 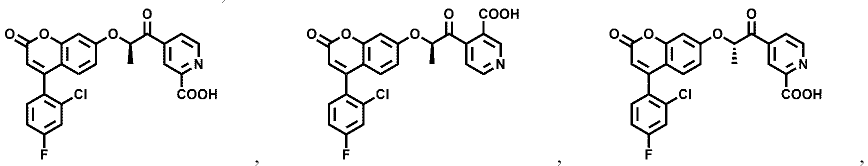 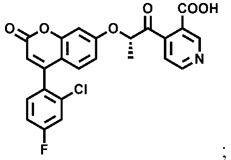 wherein the salts are optionally sodium, potassium or ammonium salts.

**Table β-1: A1-1 series compounds**

| 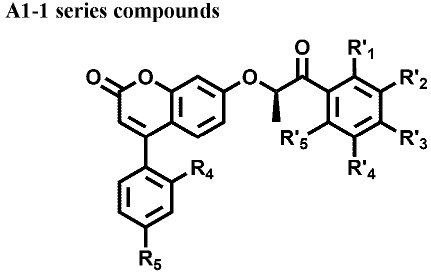 | | | | | | | | | |
|---|---|---|---|---|---|---|---|---|---|
| **No.** | **R'₁** | **R'₂** | **R'₃** | **R'₄** | **R'₅** | **R₄** | **R₅** | **Salt type** | **Mass spectrum** |
| **1** | -COOH | H | H | H | H | Cl | F | / | [M+H]⁺: 467.0 |
| **2** | | | | | | | | Na⁺ | - |
| **3** | | | | | | | | K⁺ | - |
| **4** | | | | | | | | NH₄⁺ | - |
| **5** | H | -COOH | H | H | H | Cl | F | / | [M-H]⁻: 465.0 |
| **6** | | | | | | | | Na⁺ | - |
| **7** | | | | | | | | K⁺ | - |
| **8** | | | | | | | | NH₄⁺ | - |
| **9** | H | H | -COOH | H | H | Cl | F | / | [M+H]⁺: 467.2 |
| **10** | | | | | | | | Na⁺ | - |
| **11** | | | | | | | | K⁺ | - |
| **12** | | | | | | | | NH₄⁺ | - |

**Table β-2: A2-1 series compounds**

| 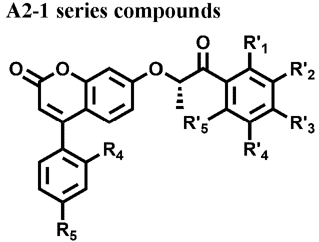 | | | | | | | | | |
|---|---|---|---|---|---|---|---|---|---|
| **No.** | **R'₁** | **R'₂** | **R'₃** | **R'₄** | **R'₅** | **R₄** | **R₅** | **Salt type** | **Mass spectrum** |
| **1** | -COOH | H | H | H | H | Cl | F | / | [M+H]⁺: 467.0 |
| **2** | | | | | | | | Na⁺ | - |
| **3** | | | | | | | | K⁺ | - |
| **4** | | | | | | | | NH₄⁺ | - |
| **5** | H | -COOH | H | H | H | Cl | F | / | [M-H]⁻: 465.0 |
| **6** | | | | | | | | Na⁺ | - |
| **7** | | | | | | | | K⁺ | - |
| **8** | | | | | | | | NH₄⁺ | - |
| **9** | H | H | -COOH | H | H | Cl | F | / | [M+H]⁺: 467.2 |
| **10** | | | | | | | | Na⁺ | - |
| **11** | | | | | | | | K⁺ | - |
| **12** | | | | | | | | NH₄⁺ | - |

**Table β-3: A1-2 series compounds**

| 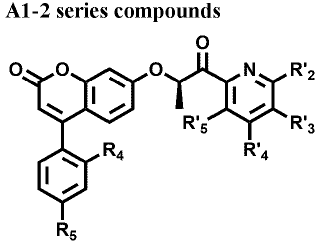 | | | | | | | | | |
|---|---|---|---|---|---|---|---|---|---|
| **No.** | **R'₁** | **R'₂** | **R'₃** | **R'₄** | **R'₅** | **R₄** | **R₅** | **Salt type** | **Mass spectrum** |
| **1** | / | -COOH | H | H | H | Cl | F | / | [M-H]⁻: 466.1 |
| **2** | | | | | | | | Na⁺ | - |
| **3** | | | | | | | | K⁺ | - |
| **4** | | | | | | | | NH₄⁺ | - |
| **5** | / | H | -COOH | H | H | Cl | F | / | [M-H]⁻: 466.1 |
| **6** | | | | | | | | Na⁺ | - |
| **7** | | | | | | | | K⁺ | - |
| **8** | | | | | | | | NH₄⁺ | - |
| **9** | / | H | H | -COOH | H | Cl | F | / | [M-H]⁻: 466.1 |
| **10** | | | | | | | | Na⁺ | - |
| **11** | | | | | | | | K⁺ | - |
| **12** | | | | | | | | NH₄⁺ | - |
| **13** | / | H | H | H | -COOH | Cl | F | / | [M-H]⁻: 466.1 |
| **14** | | | | | | | | Na⁺ | - |
| **15** | | | | | | | | K⁺ | - |
| **16** | | | | | | | | NH₄⁺ | - |

**Table β-4: A2-2 series compounds**

| 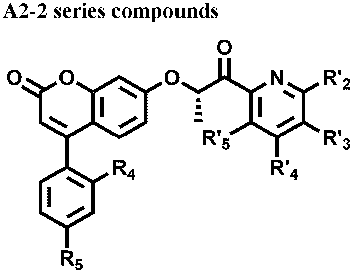 | | | | | | | | | |
|---|---|---|---|---|---|---|---|---|---|
| **No.** | **R'₁** | **R'₂** | **R'₃** | **R'₄** | **R'₅** | **R₄** | **R₅** | **Salt type** | **Mass spectrum** |
| **1** | / | -COOH | H | H | H | Cl | F | / | [M-H]⁻: 466.1 |
| **2** | | | | | | | | Na⁺ | - |
| **3** | | | | | | | | K⁺ | - |
| **4** | | | | | | | | NH₄⁺ | - |
| **5** | / | H | -COOH | H | H | Cl | F | / | [M-H]⁻: 466.1 |
| **6** | | | | | | | | Na⁺ | - |
| **7** | | | | | | | | K⁺ | - |
| **8** | | | | | | | | NH₄⁺ | - |
| **9** | / | H | H | -COOH | H | Cl | F | / | [M-H]⁻: 466.1 |
| **10** | | | | | | | | Na⁺ | - |
| **11** | | | | | | | | K⁺ | - |
| **12** | | | | | | | | NH₄⁺ | - |
| **13** | / | H | H | H | -COOH | Cl | F | / | [M-H]⁻: 466.1 |
| **14** | | | | | | | | Na⁺ | - |
| **15** | | | | | | | | K⁺ | - |
| **16** | | | | | | | | NH₄⁺ | - |

**Table β-5: A1-3 series compounds**

| 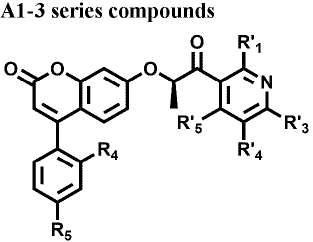 | | | | | | | | | |
|---|---|---|---|---|---|---|---|---|---|
| **No.** | **R'₁** | **R'₂** | **R'₃** | **R'₄** | **R'₅** | **R₄** | **R₅** | **Salt type** | **Mass spectrum** |
| **1** | -COOH | / | H | H | H | Cl | F | / | [M-H]⁻: 466.1 |
| **2** | | | | | | | | Na⁺ | - |
| **3** | | | | | | | | K⁺ | - |
| **4** | | | | | | | | NH₄⁺ | - |
| **5** | H | / | -COOH | H | H | Cl | F | / | [M-H]⁻: 466.1 |
| **6** | | | | | | | | Na⁺ | - |
| **7** | | | | | | | | K⁺ | - |
| **8** | | | | | | | | NH₄⁺ | - |
| **9** | H | / | H | -COOH | H | Cl | F | / | [M-H]⁻: 466.1 |
| **10** | | | | | | | | Na⁺ | - |
| **11** | | | | | | | | K⁺ | - |
| **12** | | | | | | | | NH₄⁺ | - |
| **13** | H | / | H | H | -COOH | Cl | F | / | [M-H]⁻: 466.1 |
| **14** | | | | | | | | Na⁺ | - |
| **15** | | | | | | | | K⁺ | - |
| **16** | | | | | | | | NH₄⁺ | - |

**Table β-6: A2-3 series compounds**

| 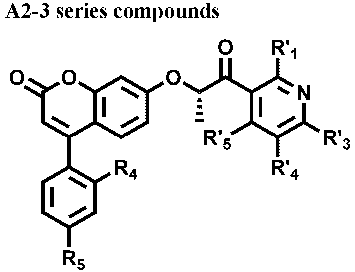 | | | | | | | | | |
|---|---|---|---|---|---|---|---|---|---|
| **No.** | **R'₁** | **R'₂** | **R'₃** | **R'₄** | **R'₅** | **R₄** | **R₅** | **Salt type** | **Mass spectrum** |
| **1** | -COOH | / | H | H | H | Cl | F | / | [M-H]⁻: 466.1 |
| **2** | | | | | | | | Na⁺ | - |
| **3** | | | | | | | | K⁺ | - |
| **4** | | | | | | | | NH₄⁺ | - |
| **5** | H | / | -COOH | H | H | Cl | F | / | [M-H]⁻: 466.1 |
| **6** | | | | | | | | Na⁺ | - |
| **7** | | | | | | | | K⁺ | - |
| **8** | | | | | | | | NH₄⁺ | - |
| **9** | H | / | -H | -COOH | H | Cl | F | / | [M-H]⁻: 466.1 |
| **10** | | | | | | | | Na⁺ | - |
| **11** | | | | | | | | K⁺ | - |
| **12** | | | | | | | | NH₄⁺ | - |
| **13** | H | / | H | H | -COOH | Cl | F | / | [M-H]⁻: 466.1 |
| **14** | | | | | | | | Na⁺ | - |
| **15** | | | | | | | | K⁺ | - |
| **16** | | | | | | | | NH₄⁺ | - |

**Table β-7: A1-4 series compounds**

| 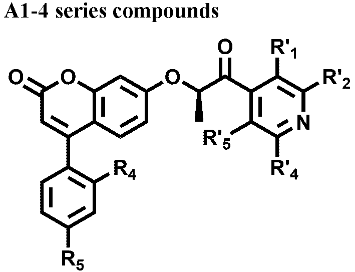 | | | | | | | | | |
|---|---|---|---|---|---|---|---|---|---|
| **No.** | **R'₁** | **R'₂** | **R'₃** | **R'₄** | **R'₅** | **R₄** | **R₅** | **Salt type** | **Mass spectrum** |
| **1** | -COOH | H | / | H | H | Cl | F | / | [M-H]⁻: 466.1 |
| **2** | | | | | | | | Na⁺ | - |
| **3** | | | | | | | | K⁺ | - |
| **4** | | | | | | | | NH₄⁺ | - |
| **5** | H | -COOH | / | H | H | Cl | F | / | [M-H]⁻: 466.0 |
| **6** | | | | | | | | Na⁺ | - |
| **7** | | | | | | | | K⁺ | - |
| **8** | | | | | | | | NH₄⁺ | - |

**Table β-8: A2-4 series compounds**

| 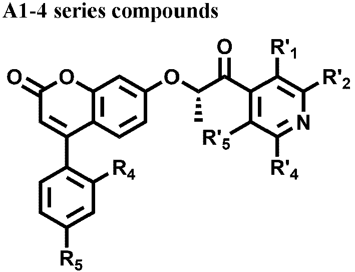 | | | | | | | | | |
|---|---|---|---|---|---|---|---|---|---|
| **No.** | **R'₁** | **R'₂** | **R'₃** | **R'₄** | **R'₅** | **R₄** | **R₅** | **Salt type** | **Mass spectrum** |
| **1** | -COOH | H | / | H | H | Cl | F | / | [M-H]⁻: 466.1 |
| **2** | | | | | | | | Na⁺ | - |
| **3** | | | | | | | | K⁺ | - |
| **4** | | | | | | | | NH₄⁺ | - |
| **5** | H | -COOH | / | H | H | Cl | F | / | [M-H]⁻: 466.0 |
| **6** | | | | | | | | Na⁺ | - |
| **7** | | | | | | | | K⁺ | - |
| **8** | | | | | | | | NH₄⁺ | - |

### Third Set of Technical Solutions

Provided in the present invention is a benzopyrone-based compound, or stereoisomer thereof, or salt thereof, or prodrug thereof, or deuteride thereof, or hydrate thereof, or solvate thereof, 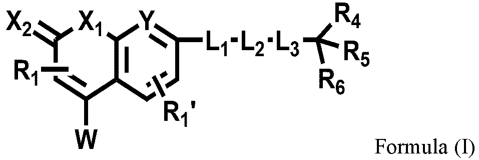
wherein X₁ and X₂ are each independently selected from the group consisting of O or NH group;
Y is selected from the group consisting of CR group or N, wherein R is H or C₁-C₃ alkyl; R₁ and R₁' may be identical or different and are each independently selected from the group consisting of hydrogen, alkyl, cycloalkyl, aryl, heterocyclyl or heteroaryl;
L₁ and L₃ are each independently selected from the group consisting of -O-, -S-, -NR₃-, -CR₂R₃-, -CR₂(R₃), -CO-, -SO- or -SO₂- groups, L₂ is a direct linkage, -CR₂R₃-, -CR₂(R₃)- or -C(R₂R₃)-, wherein R₂ is selected from the group consisting of H or C₁-C₄ linear alkyl; R₃ is selected from the group consisting of H or C₁-C₄ linear alkyl; or R₂ and R₃ in a group of -CR₂(R₃) together form an oxo group;
R₄, R₅ and R₆ are each independently selected from the group consisting of H, halogen, -C-OR₈(R₉), -CR₈(R₉), -CN, -NO₂, -OR₈, -NR₈R₉, -SR₈, -COR₈, -SOR₈, -SO₂R₈, -NR₈COR₉, -CONR₈R₉, -OCOR₈, -COOR₈, -OCONR₈R₉, -NR₈CONR₉R₁₀, -NR₈COOR₉, -NR₈SO₂R₉, -SO₂NR₈R₉, -OSO₂R₈, -SO₃R₈, linear alkyl, linear heteroalkyl, cycloalkyl, heterocycloalkyl, alkenyl, alkynyl, aryl or heteroaryl, or aromatic fused heterocyclyl, wherein R₈, R₉ and R₁₀ are each independently selected from the group consisting of H, linear alkyl, linear heteroalkyl, cycloalkyl, heterocycloalkyl, aryl or heteroaryl; wherein the linear alkyl, linear heteroalkyl, cycloalkyl, heterocycloalkyl, aryl or heteroaryl may be independently substituted with one or more R₁₁ selected from the group consisting of halogen, cyano, hydroxyl, mercapto, ether group, nitro, alkoxy, amino, amine group, carboxyl, sulfonic acid group, ester group, acyloxy, amide, sulfonic acid ester group, sulfonamido, linear alkyl, linear heteroalkyl, cycloalkyl, heterocycloalkyl, aryl or heteroaryl, or aromatic fused heterocyclyl, wherein each of the linear alkyl, linear heteroalkyl, cycloalkyl, heterocycloalkyl, aryl or heteroaryl, or aromatic fused heterocyclyl may be independently substituted with one or more halogen, cyano, hydroxyl, mercapto, ether group, nitro, alkoxy, amino, amine group, carboxyl, sulfonic acid group, ester group, amide, sulfonic acid ester group, sulfonamido, alkyl or haloalkyl; or R₄ and R₅ together form an oxo group;
or, any two or three of R₄, R₅ and R₆ together with the carbon atom to which L₃ is attached form a cyclic group A to which L₃ is attached: 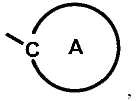 wherein the cyclic group A is any one selected from the group consisting of the following cyclic groups: aromatic ring group, saturated or unsaturated cycloalkyl, monoheterocyclyl, fused heterocyclyl, benzoheterocyclyl, spirocyclic group, bridged cyclic group and their cyclic groups substituted with R₁₁;
W is selected from the group consisting of hydrogen, H, alkyl, -CR₈ₐR₉ₐR₁₀ₐ, -NR₈ₐR₉ₐ, -OR₁₀ₐ, or a carbon atom-containing cyclic group B1 that may be substituted with one or two or more R₇ and is attached to a benzopyrone ring through the carbon atom: 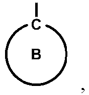 or a nitrogen atom-containing cyclic group B2 that is attached to a benzopyrone ring through the nitrogen atom: 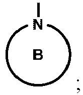 here, R₈ₐ, R₉ₐ and R₁₀ₐ are each independently selected from the group consisting of H, halogen, alkyl, cyano, hydroxyl, mercapto, ether group, nitro, alkoxy, amino, amine group, carboxyl, sulfonic acid group, ester group, acyloxy, amide, sulfonic acid ester group, sulfonamido, cycloalkyl, heterocycloalkyl, aryl or heteroaryl, or aromatic fused heterocyclyl; the cyclic group B1 and the cyclic group B2 are each independently selected from the group consisting of cycloalkyl, heterocycloalkyl, aryl or heteroaryl;
wherein R₇ is selected from the group consisting of H, halogen, -CN, -NO₂, -OR₈, -NR₈R₉, -SR₈, -COR₈, -SOR₈, -SO₂R₈, -NR₈COR₉, -CONR₈R₉, -OCOR₈, -COOR₈, -OCONR₈R₉, -NR₈CONR₉R₁₀, -NR₈COOR₉, -NR₈SO₂R₉, -SO₂NR₈R₉, -OSO₂R₈, -SO₃R₈, linear alkyl, linear heteroalkyl, cycloalkyl, heterocycloalkyl, aryl or heteroaryl, or aromatic fused heterocyclyl, preferably halogen, alkyl, cyano, hydroxyl, mercapto, ether group, nitro, alkoxy, amino, amine group, carboxyl, sulfonic acid group, ester group, amide, acyloxy, sulfonic acid ester group, sulfonamido, cycloalkyl, heterocycloalkyl, aryl or heteroaryl; wherein each of the linear alkyl, linear heteroalkyl, cycloalkyl, heterocycloalkyl, aryl or heteroaryl, or aromatic fused heterocyclyl is independently substituted with one or two or more R₁₁, and R₈, R₉ and R₁₀ have the same meaning as R₈, R₉ and R₁₀ present in R₄, R₅ and R₆; and
formula (I) does not comprise the following compound:

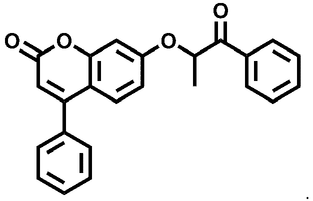

Among them, in an embodiment of the present invention, specifically provided are various benzopyrone-based compounds as shown in Table γ-1 below, as well as a preparation method and the use in the manufacture of drugs as POLRMT inhibitors:

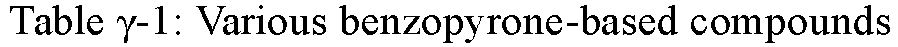

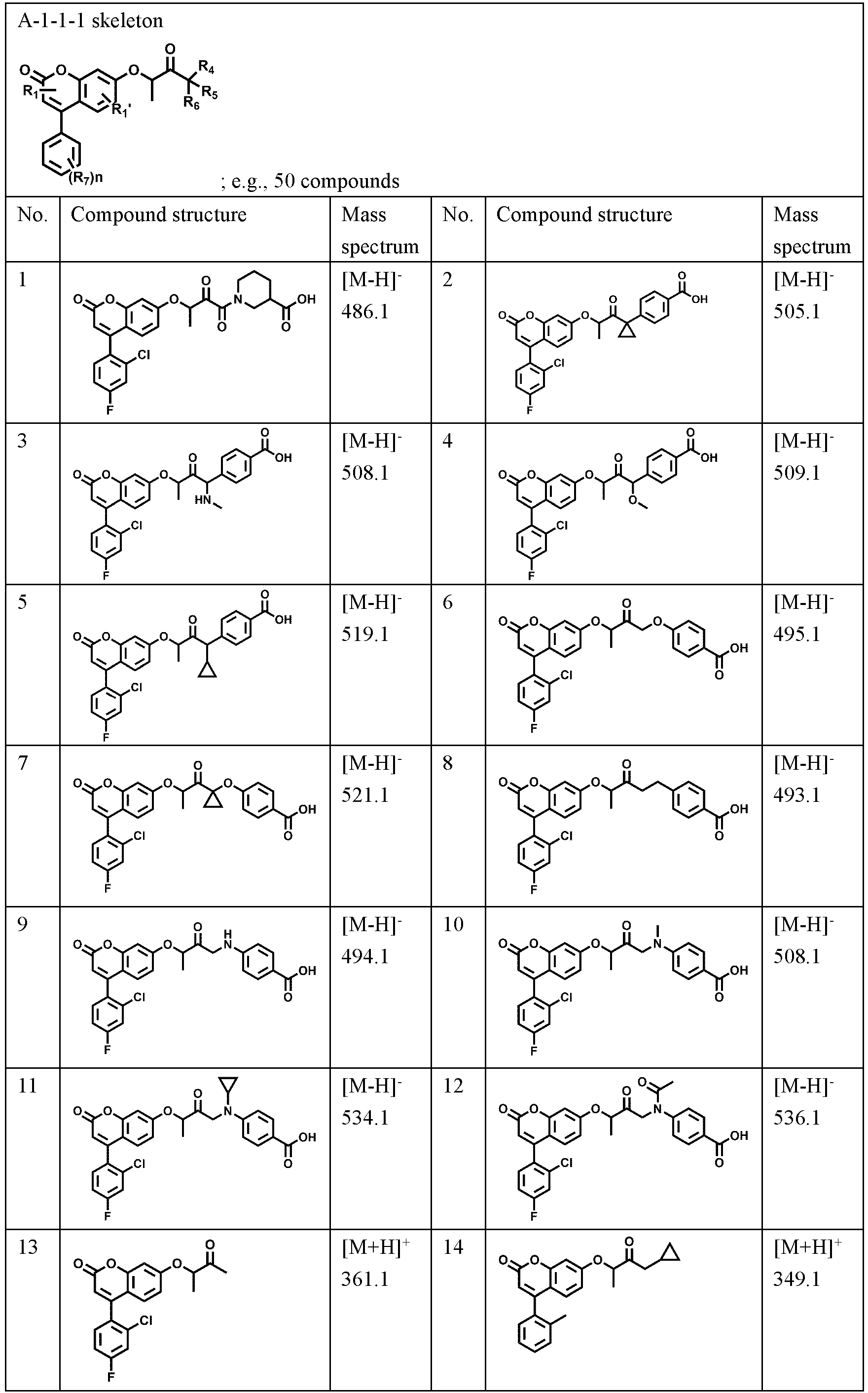

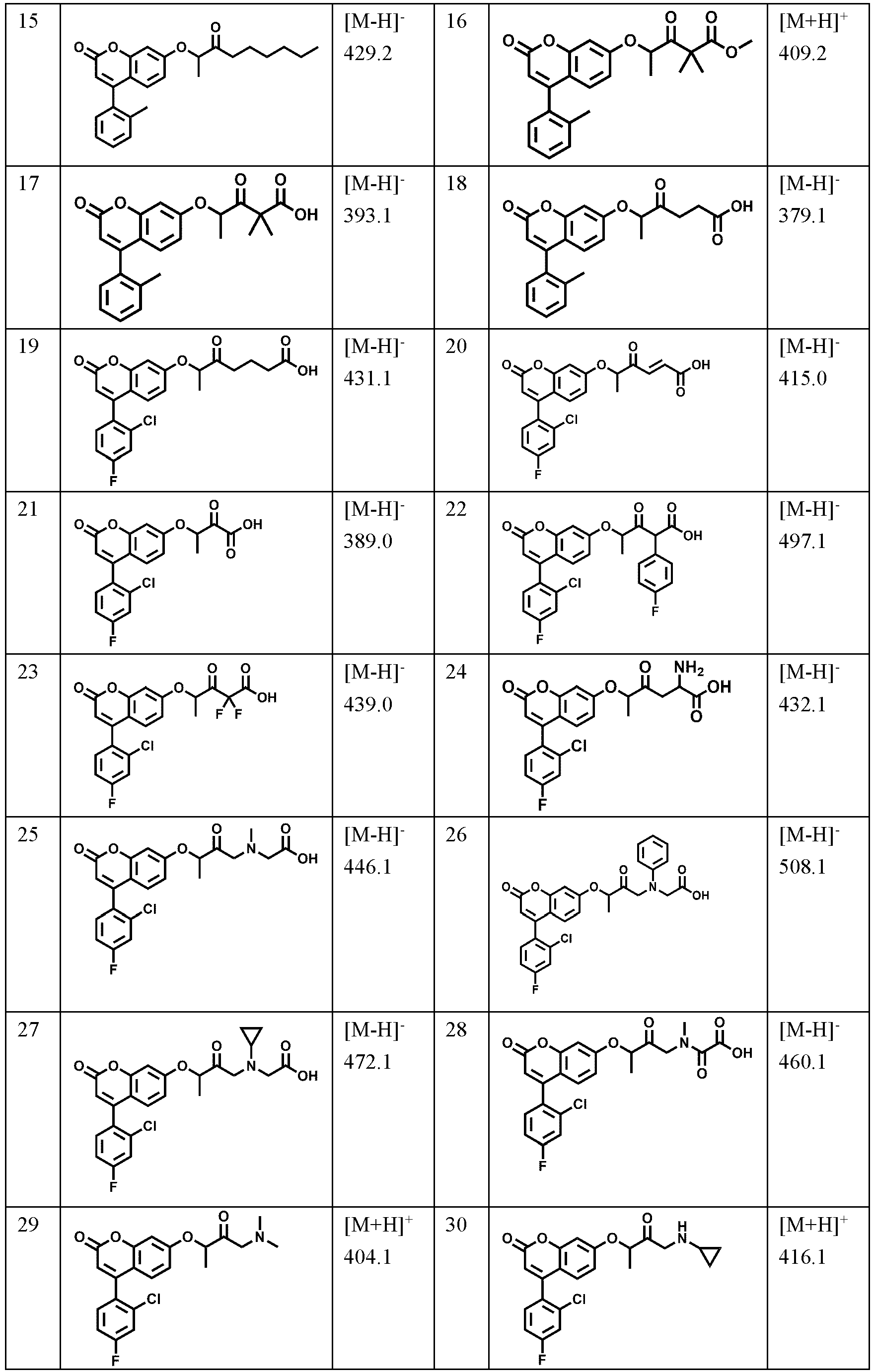

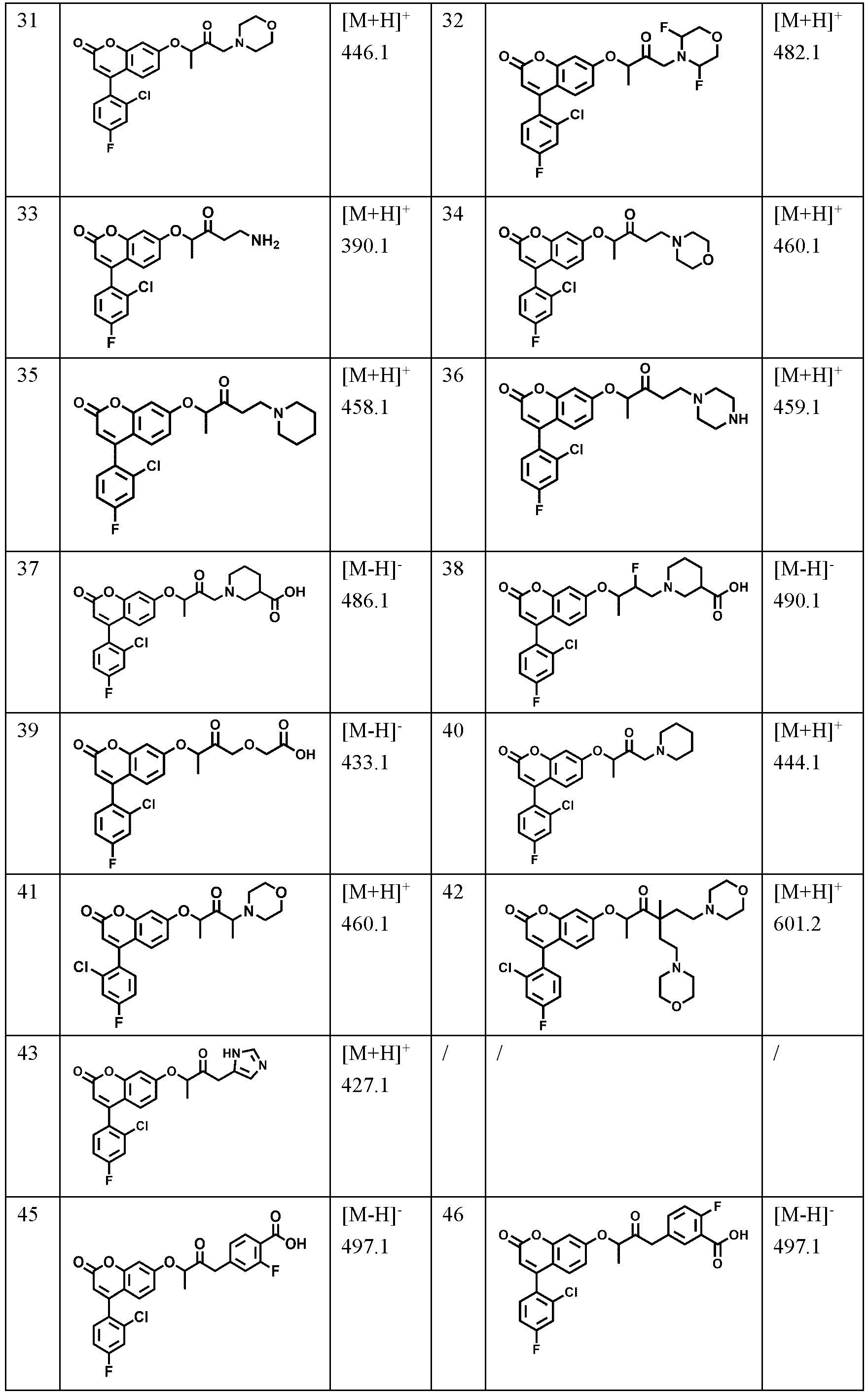

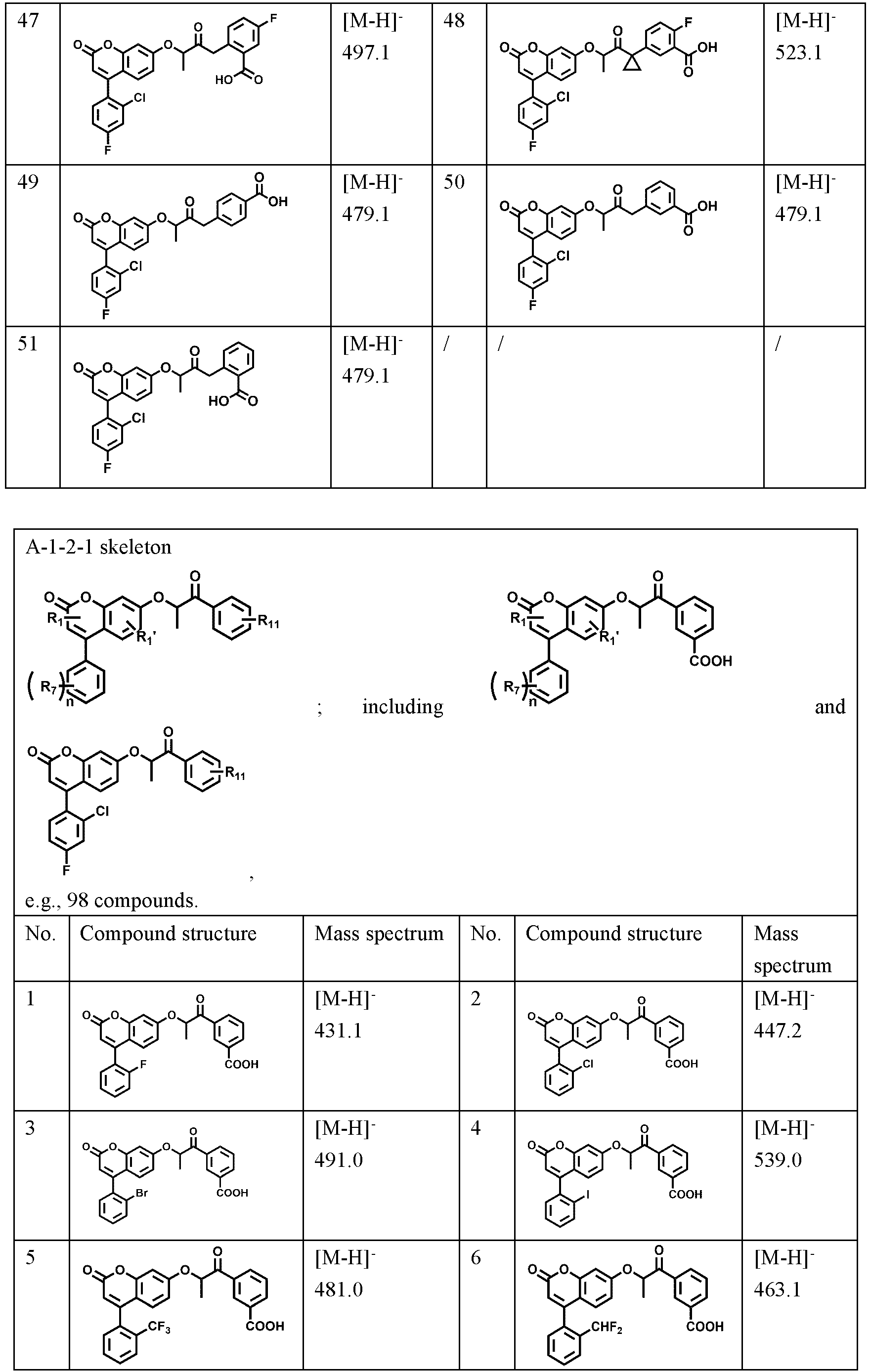

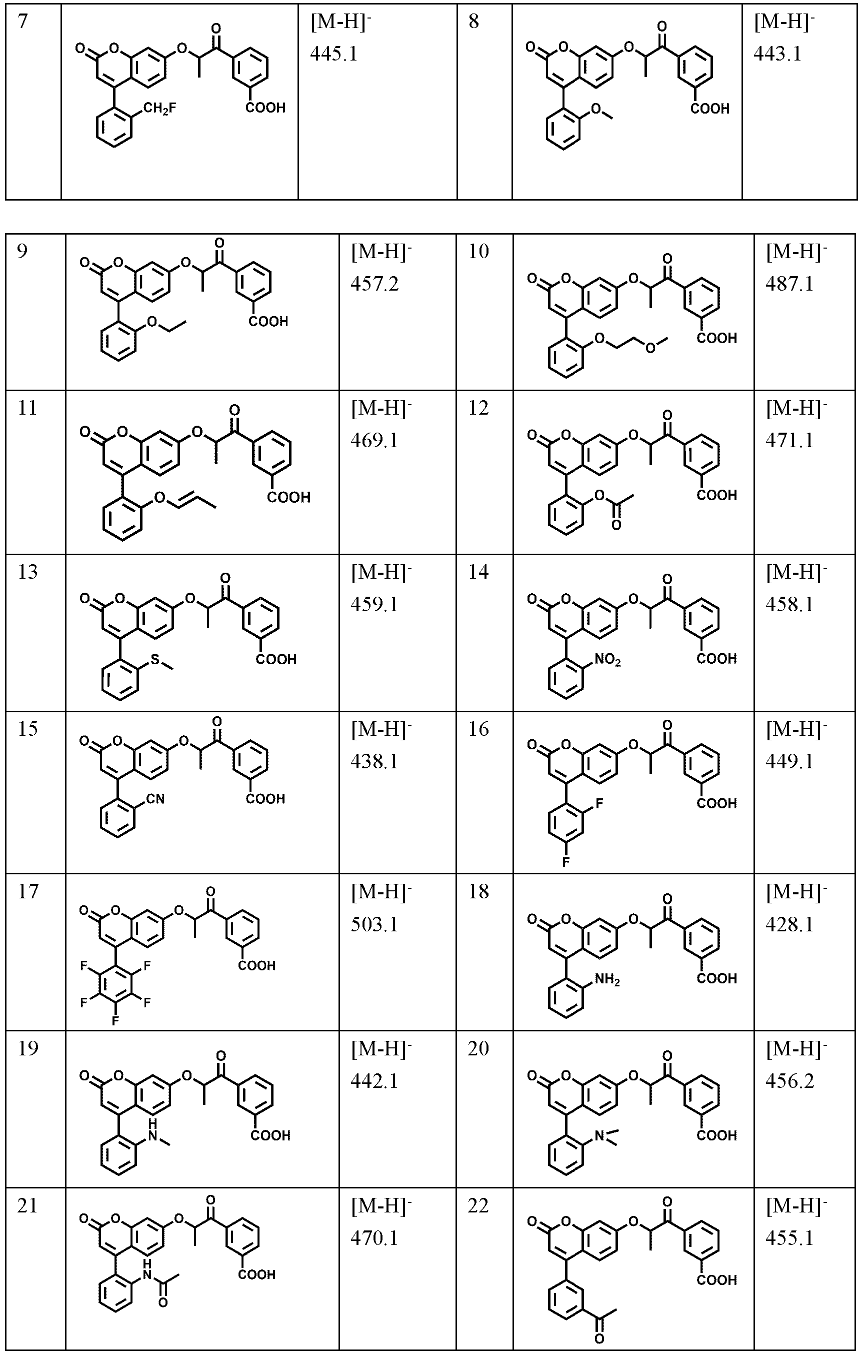

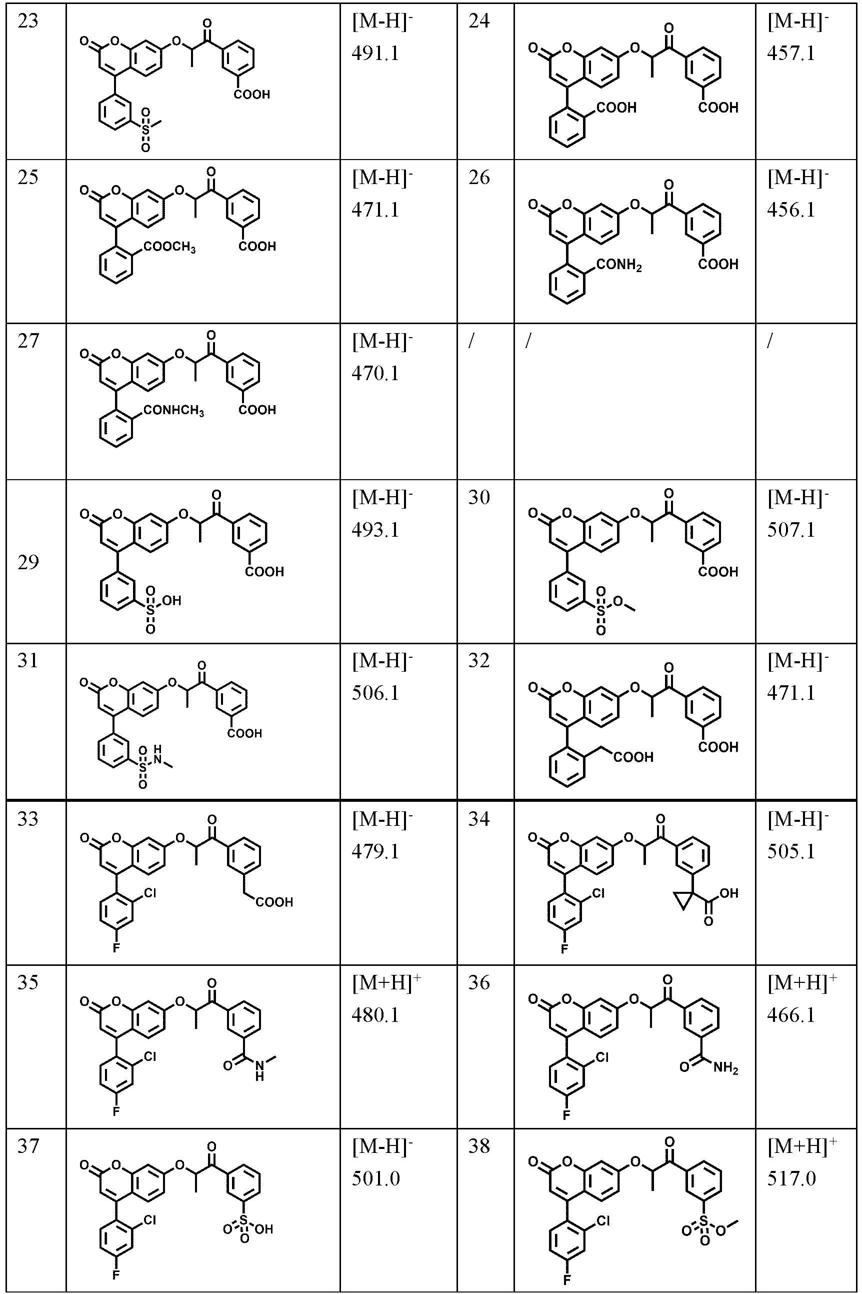

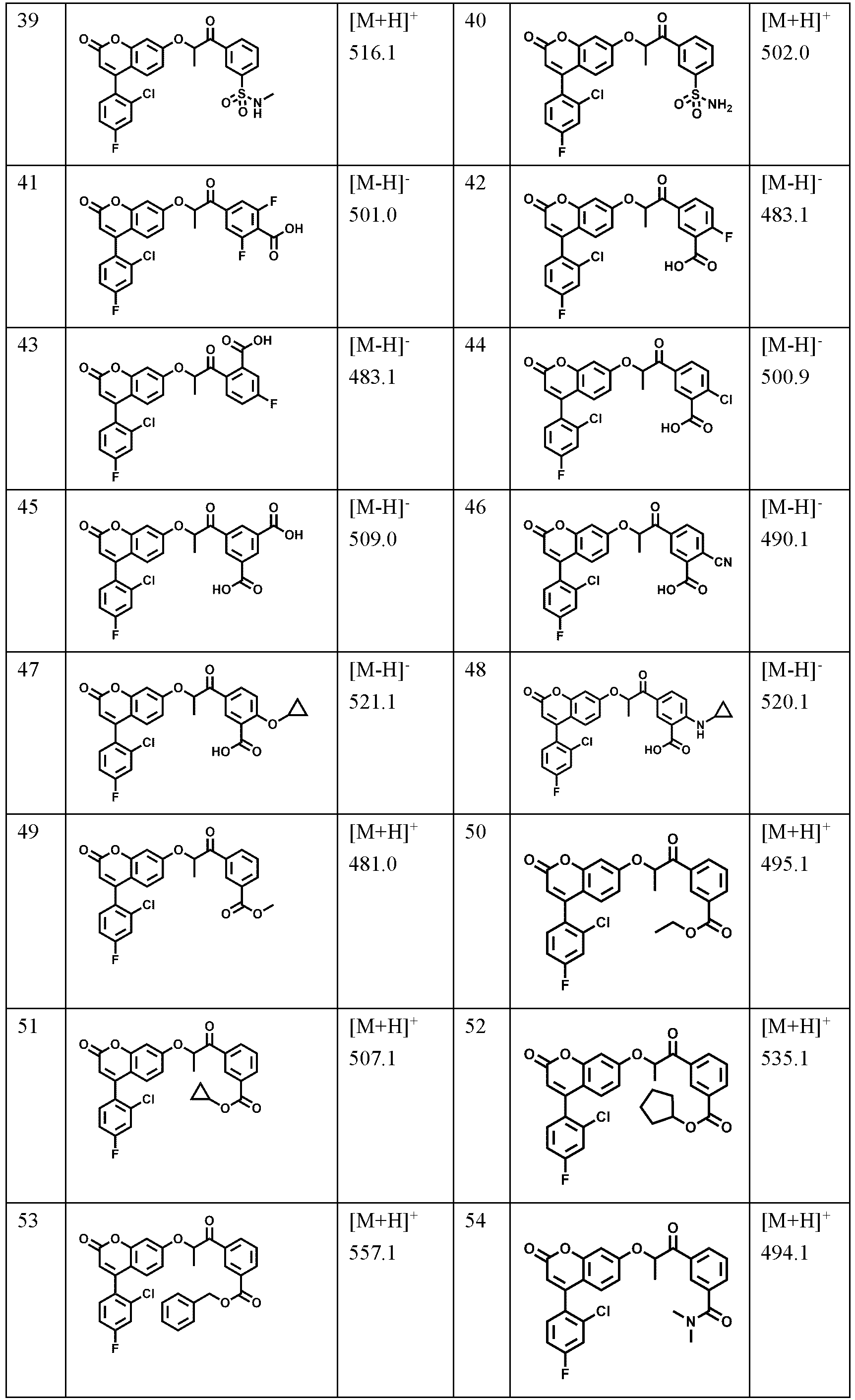

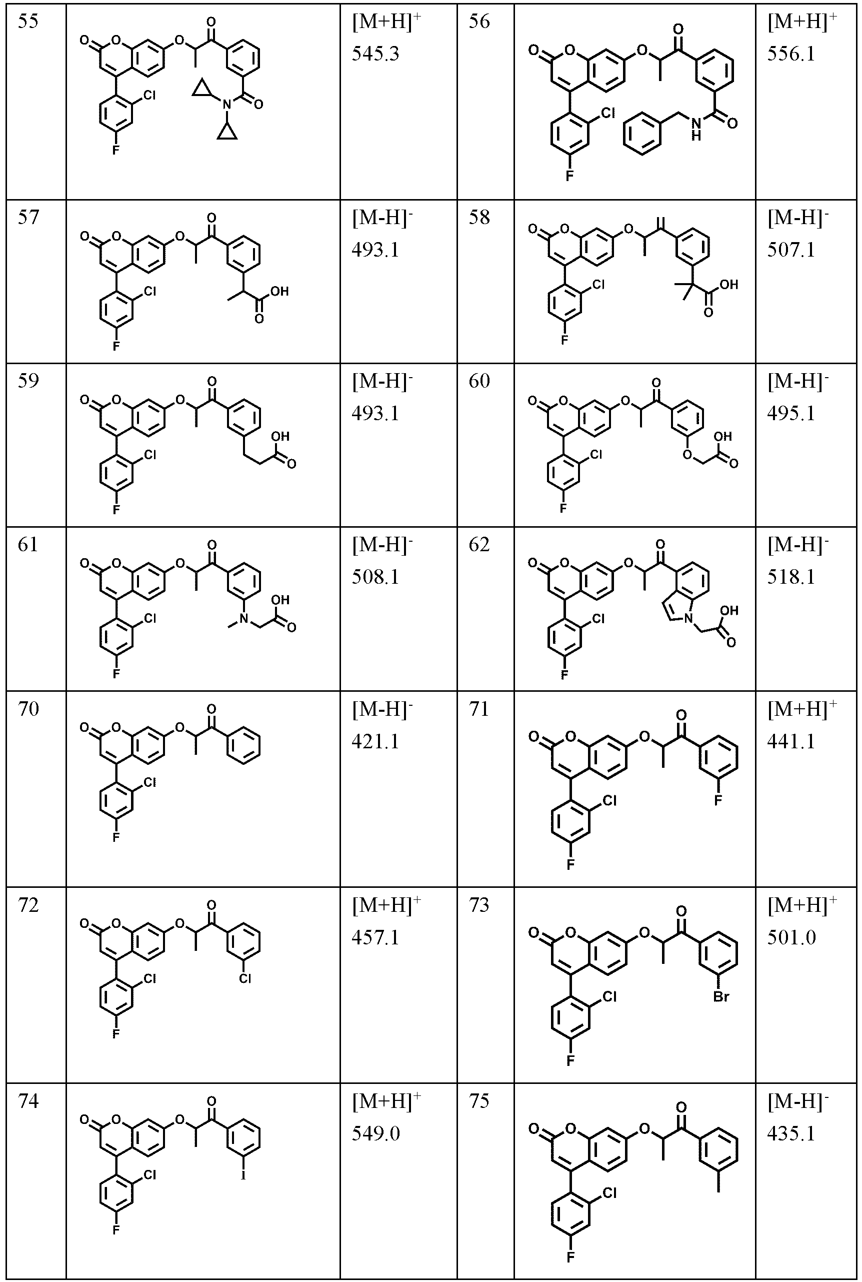

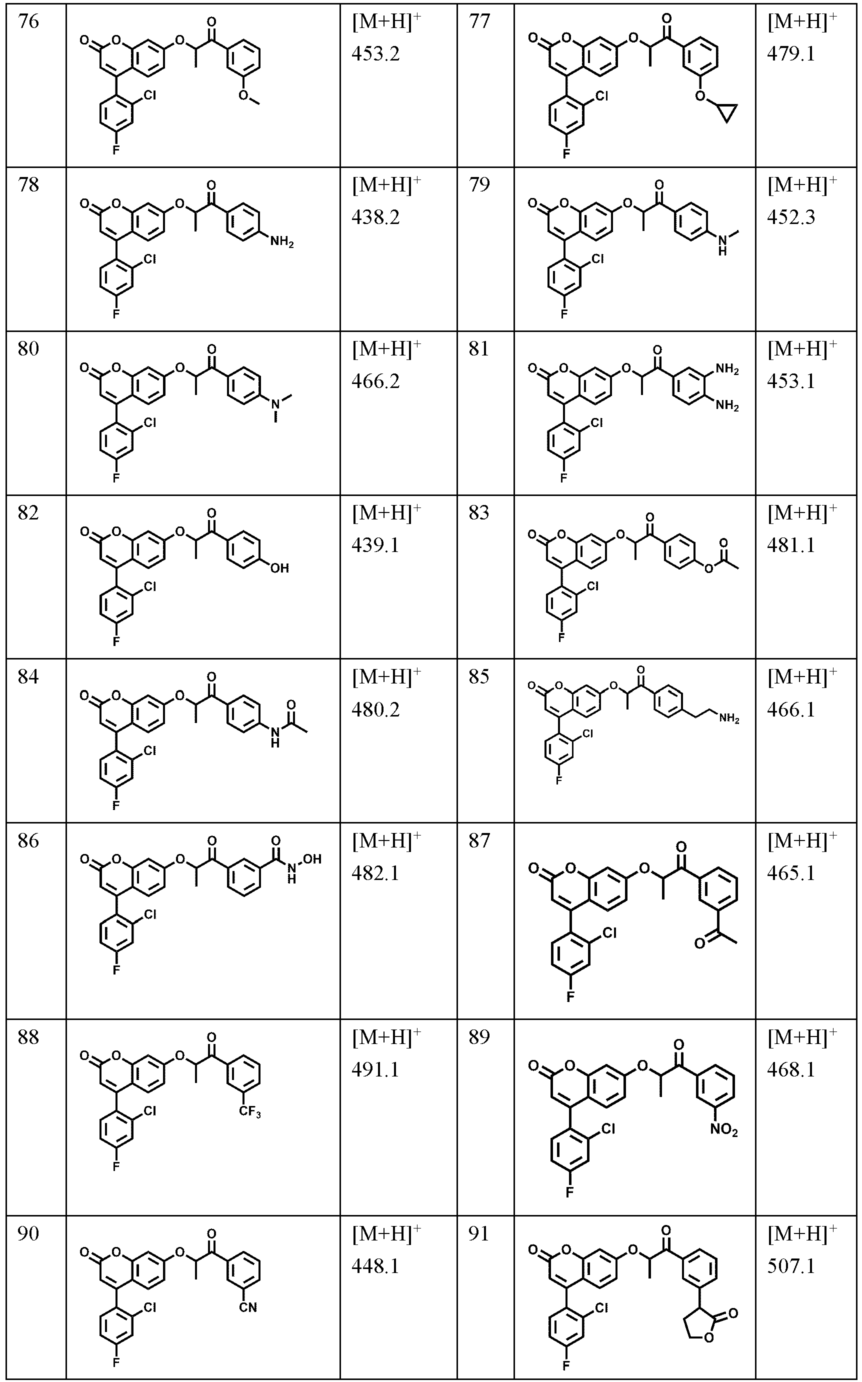

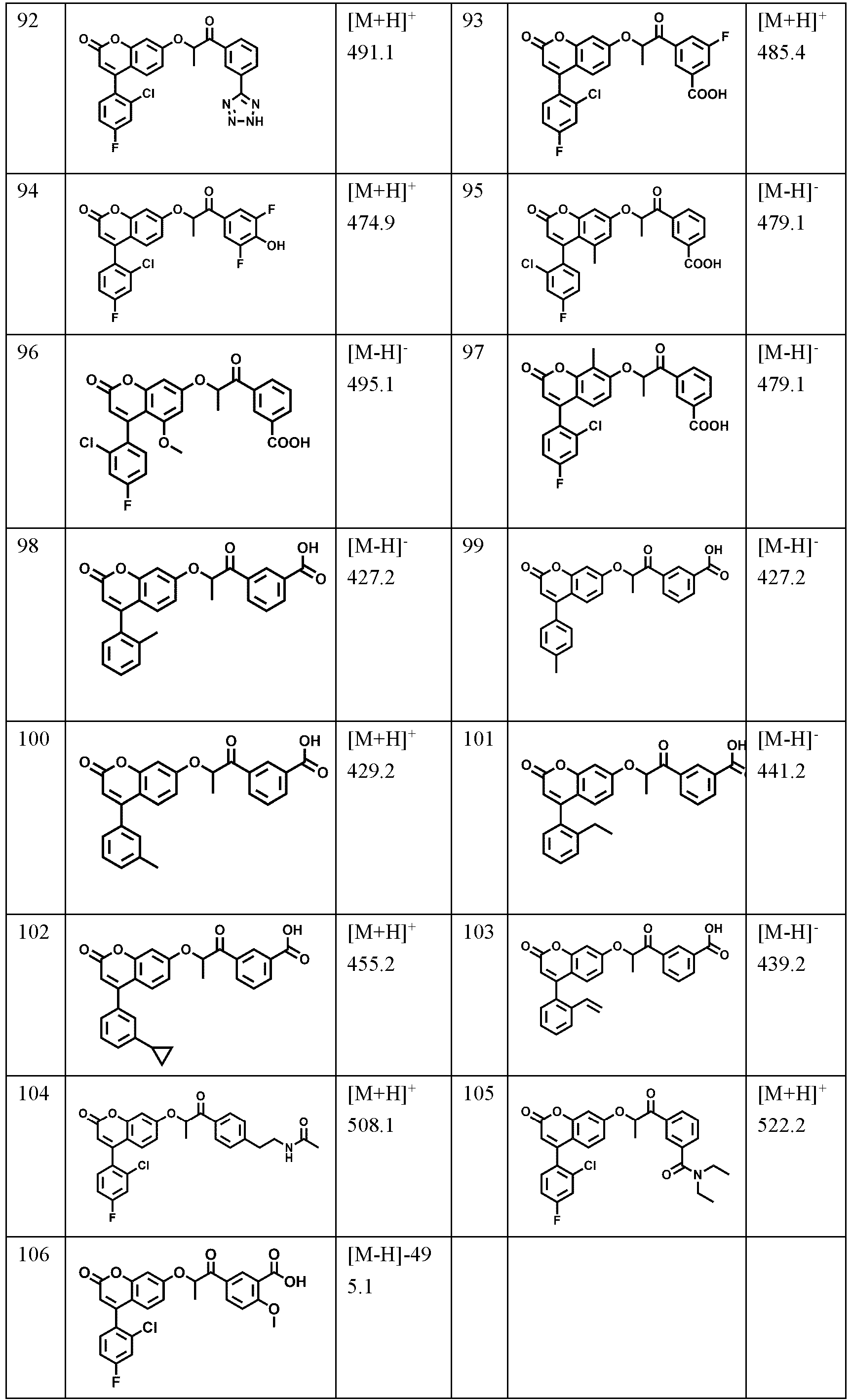

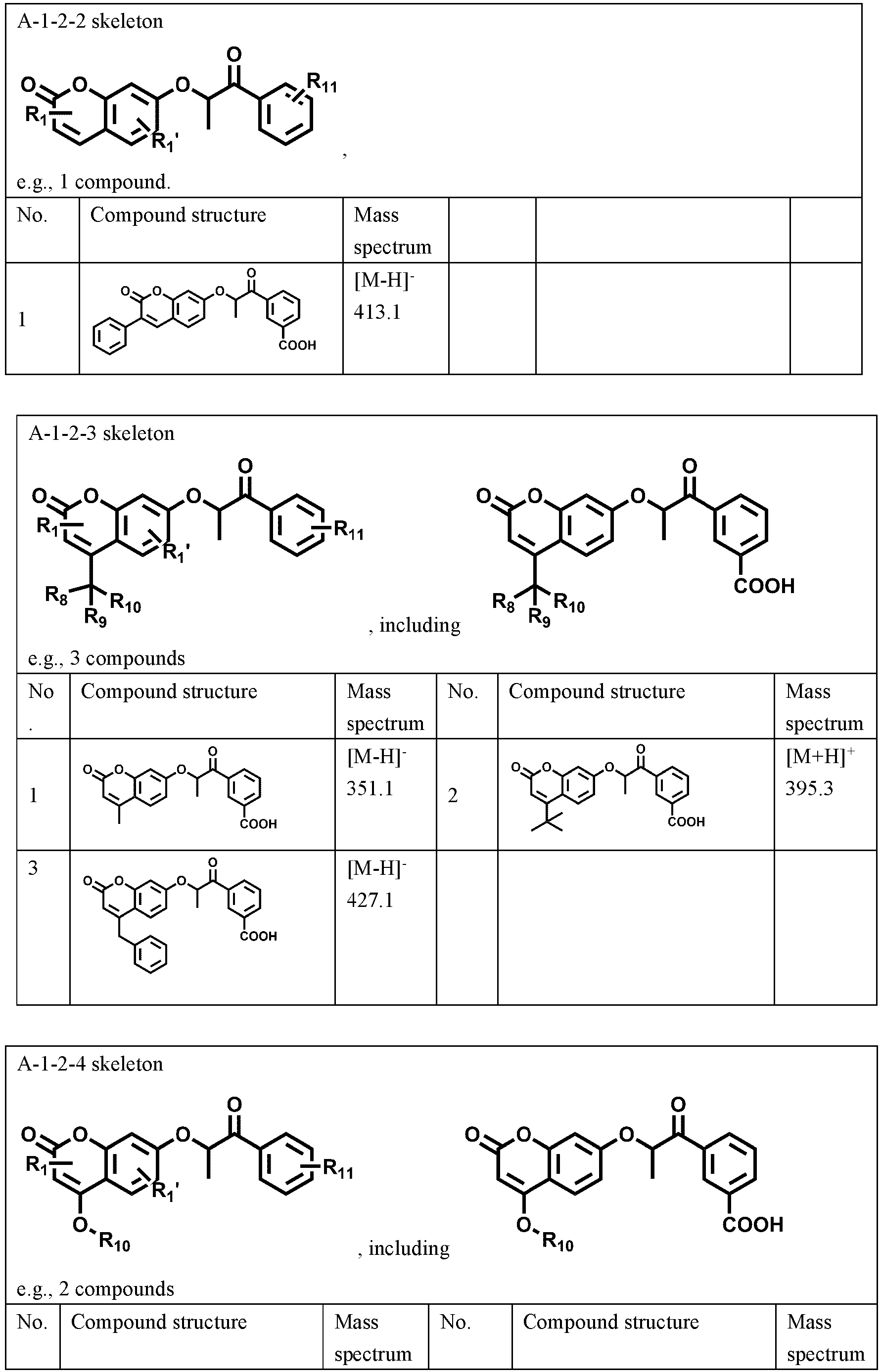

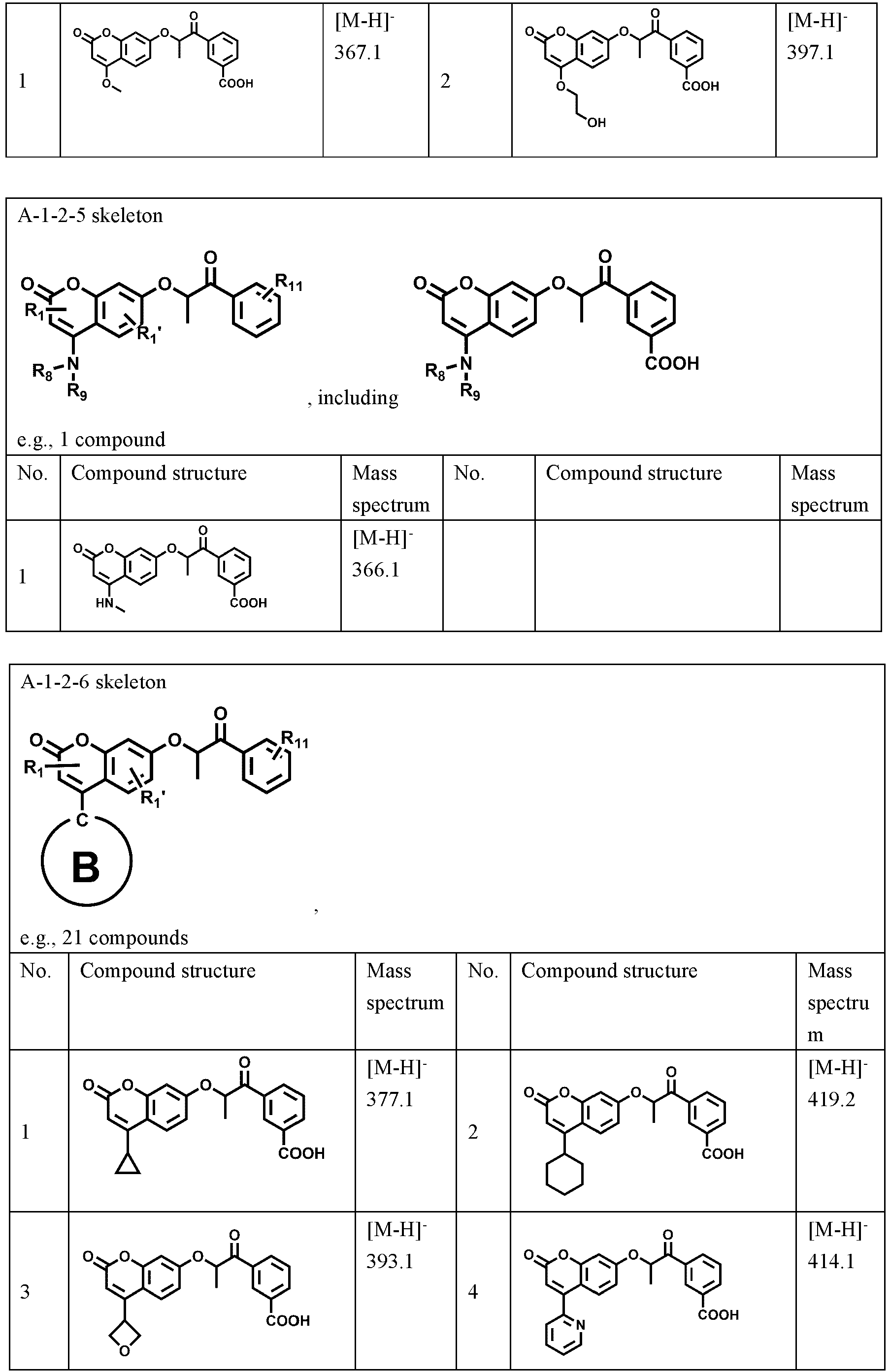

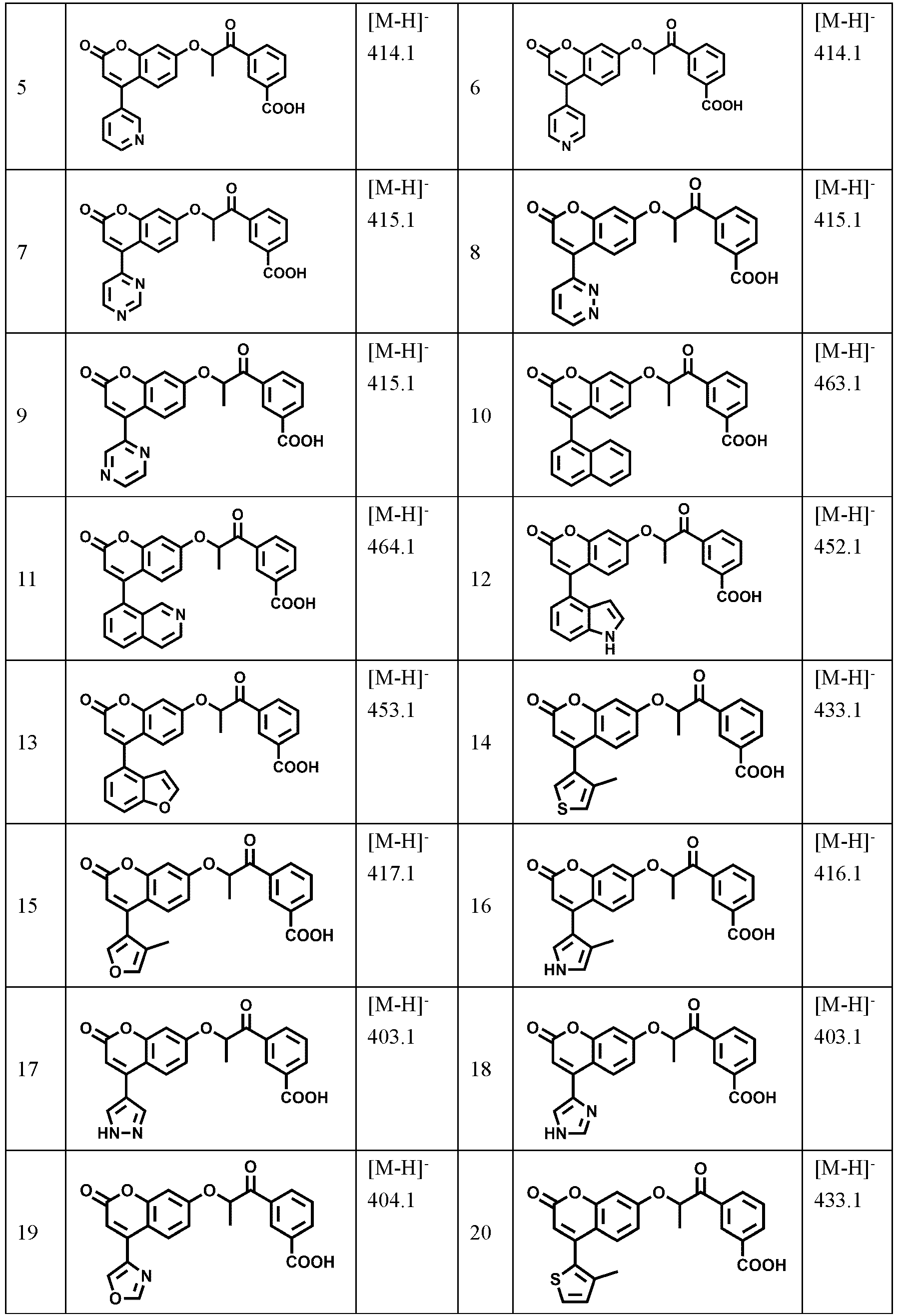

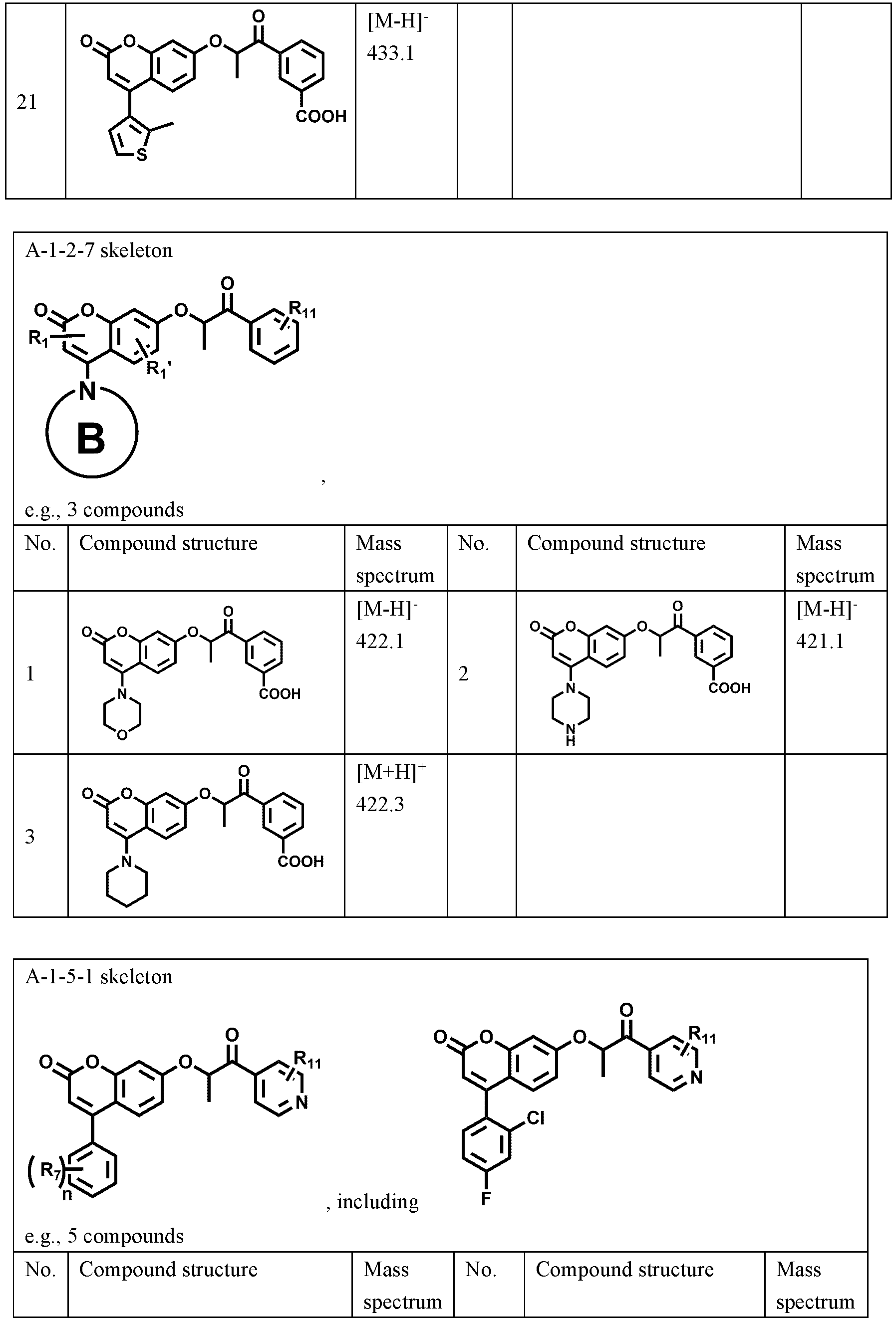

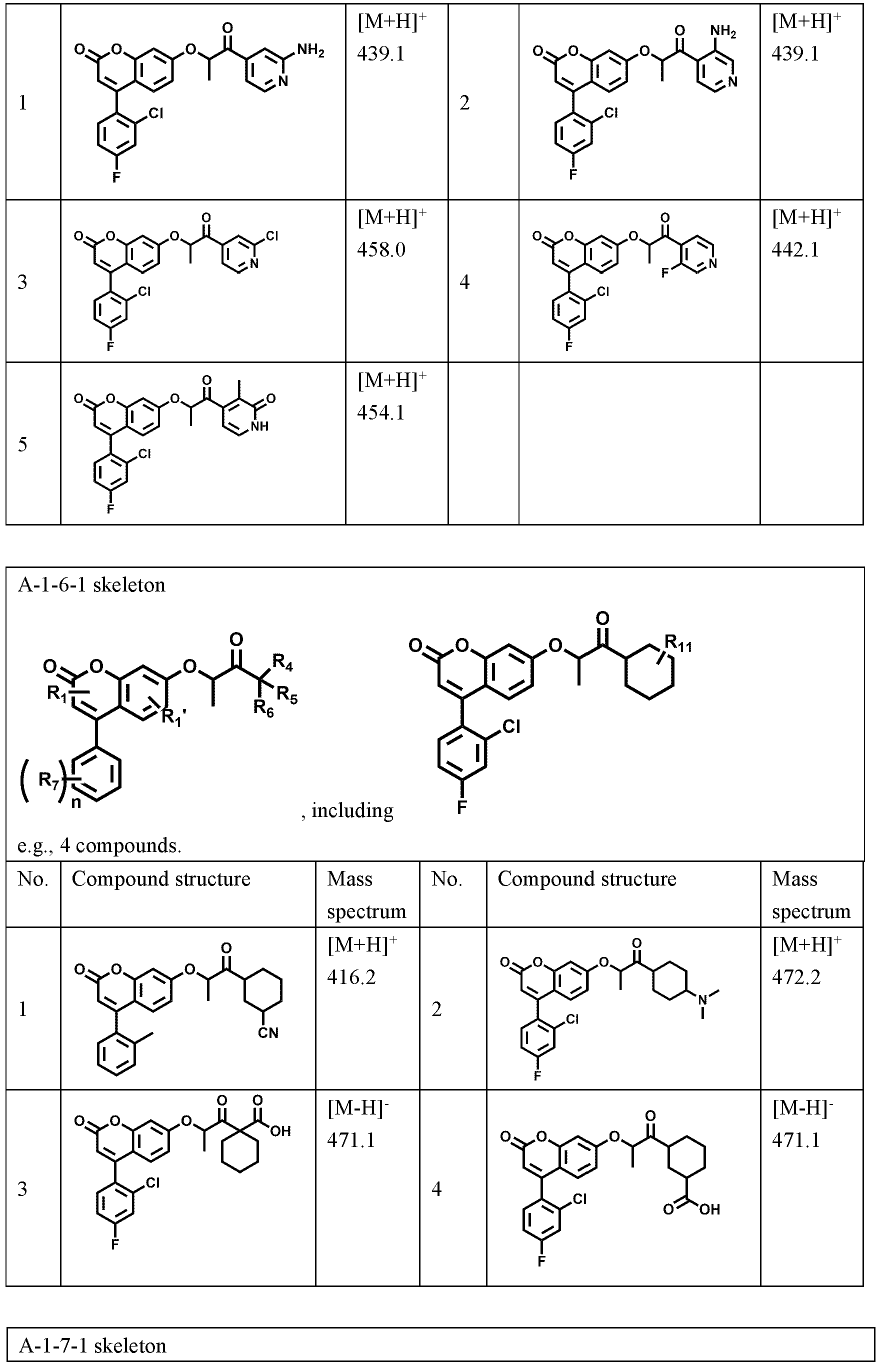

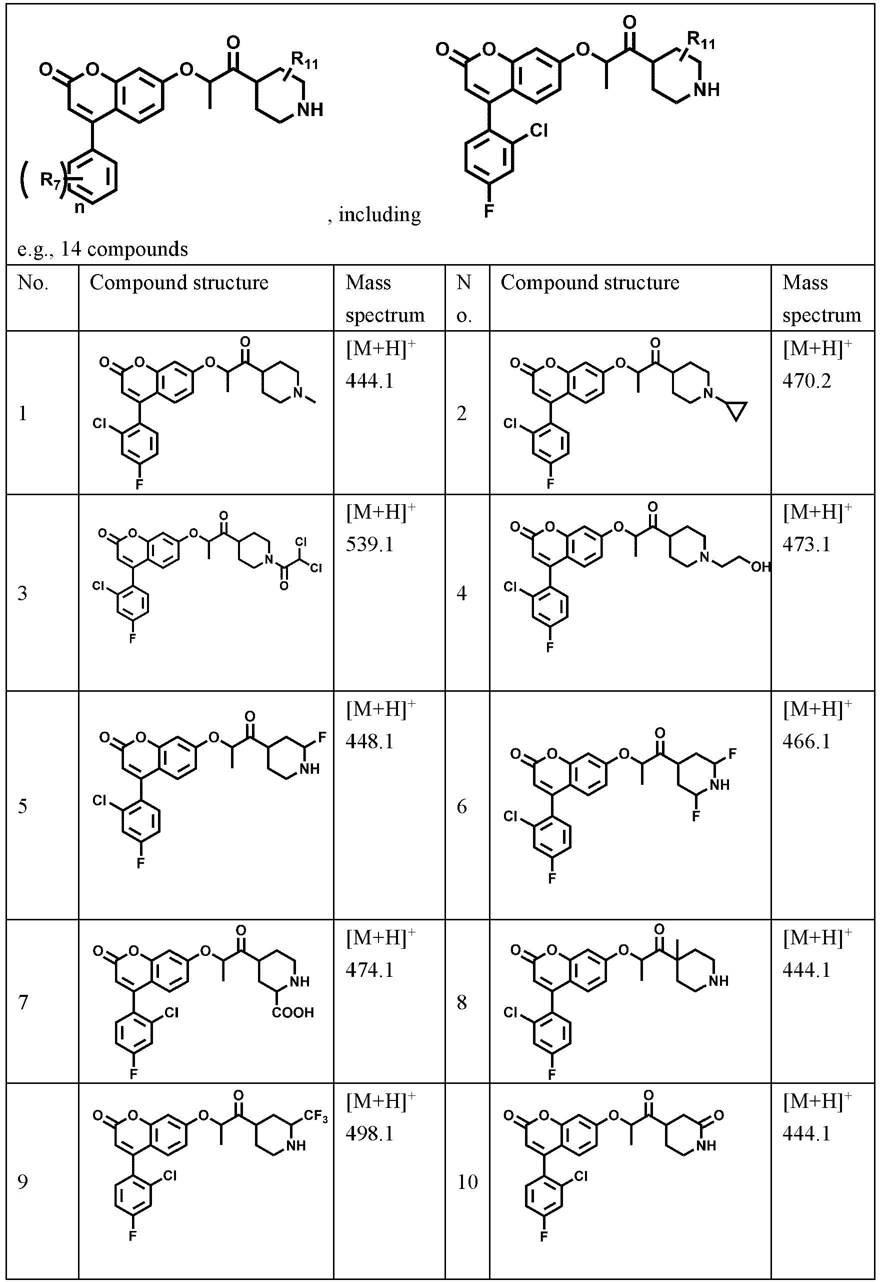

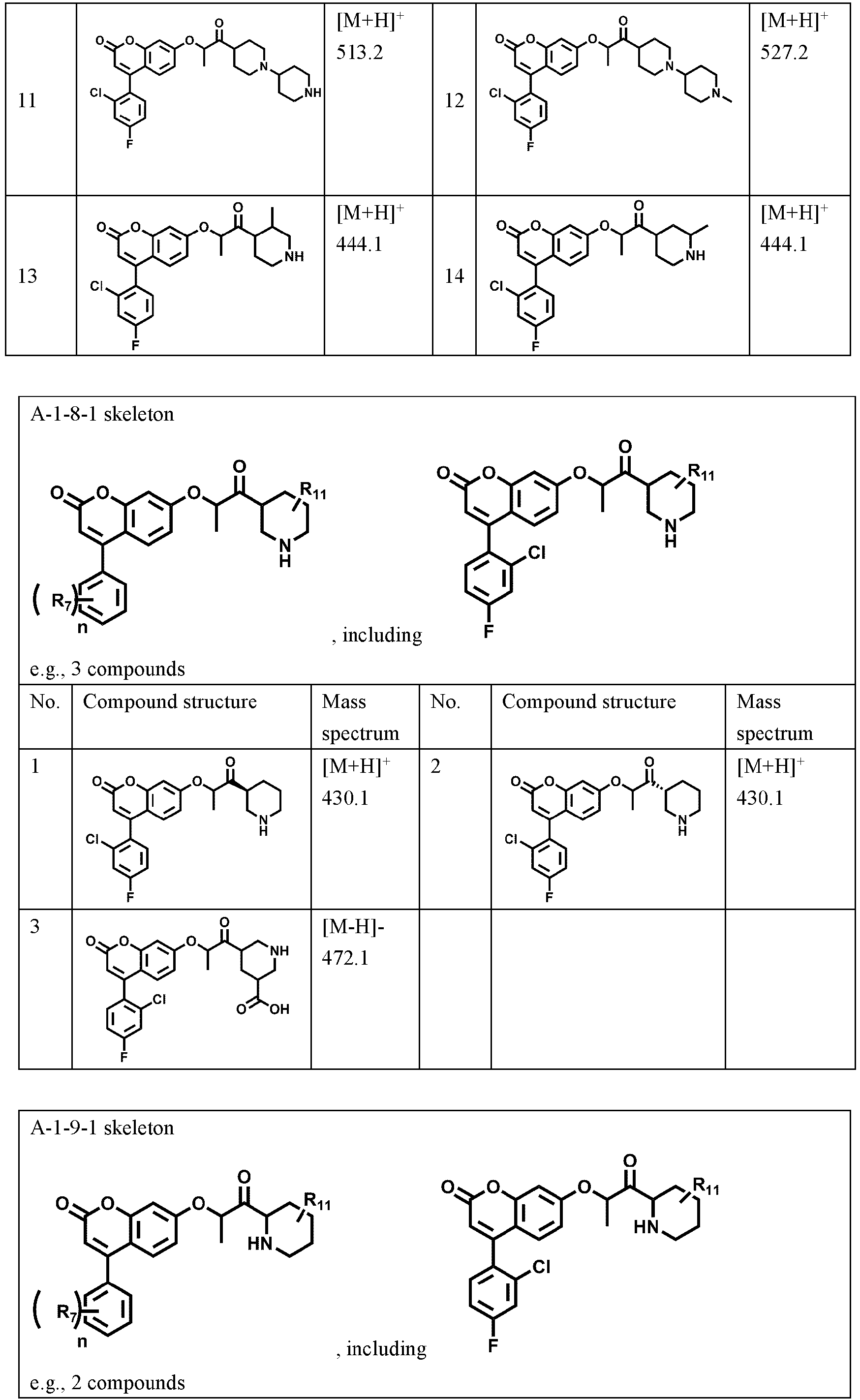

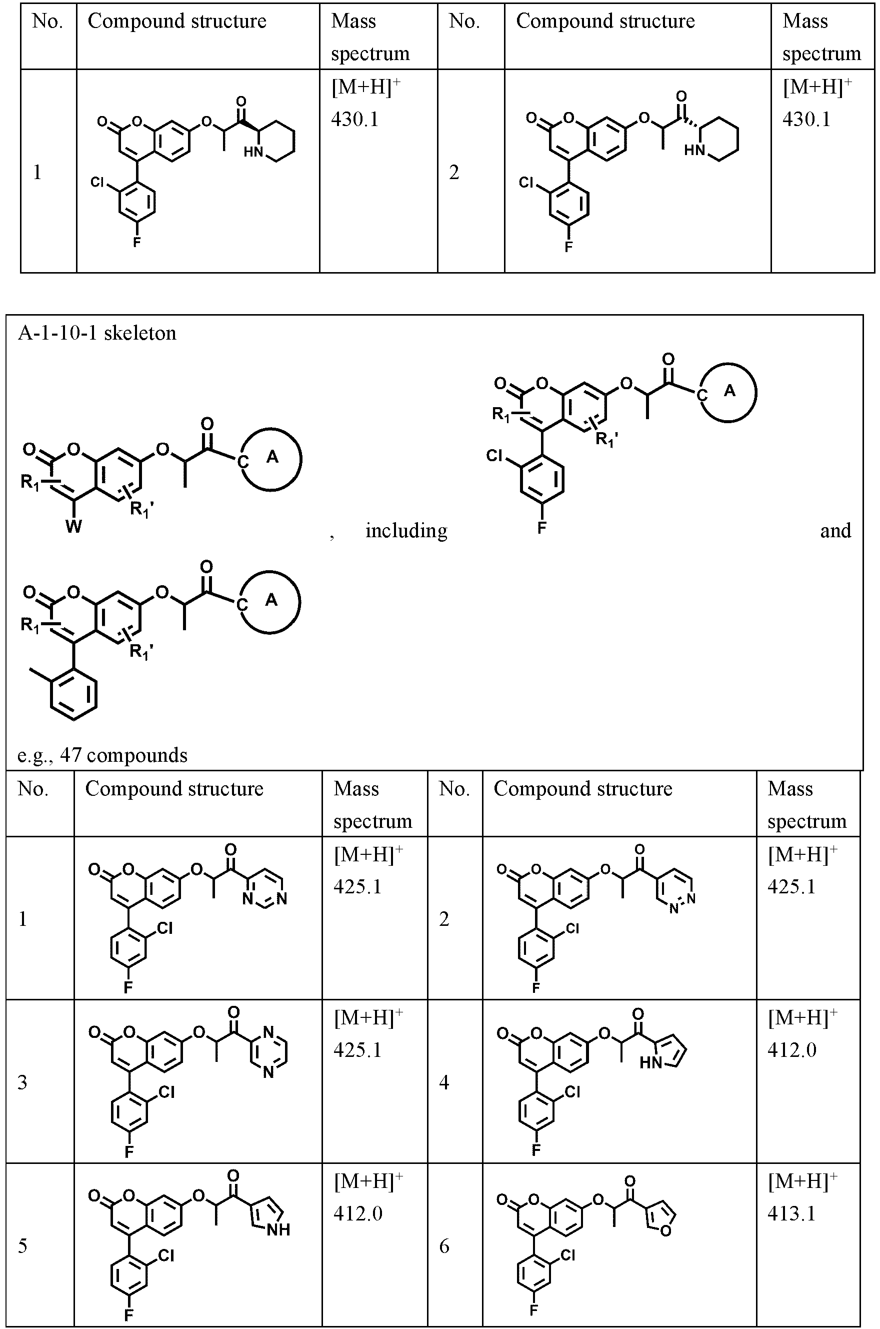

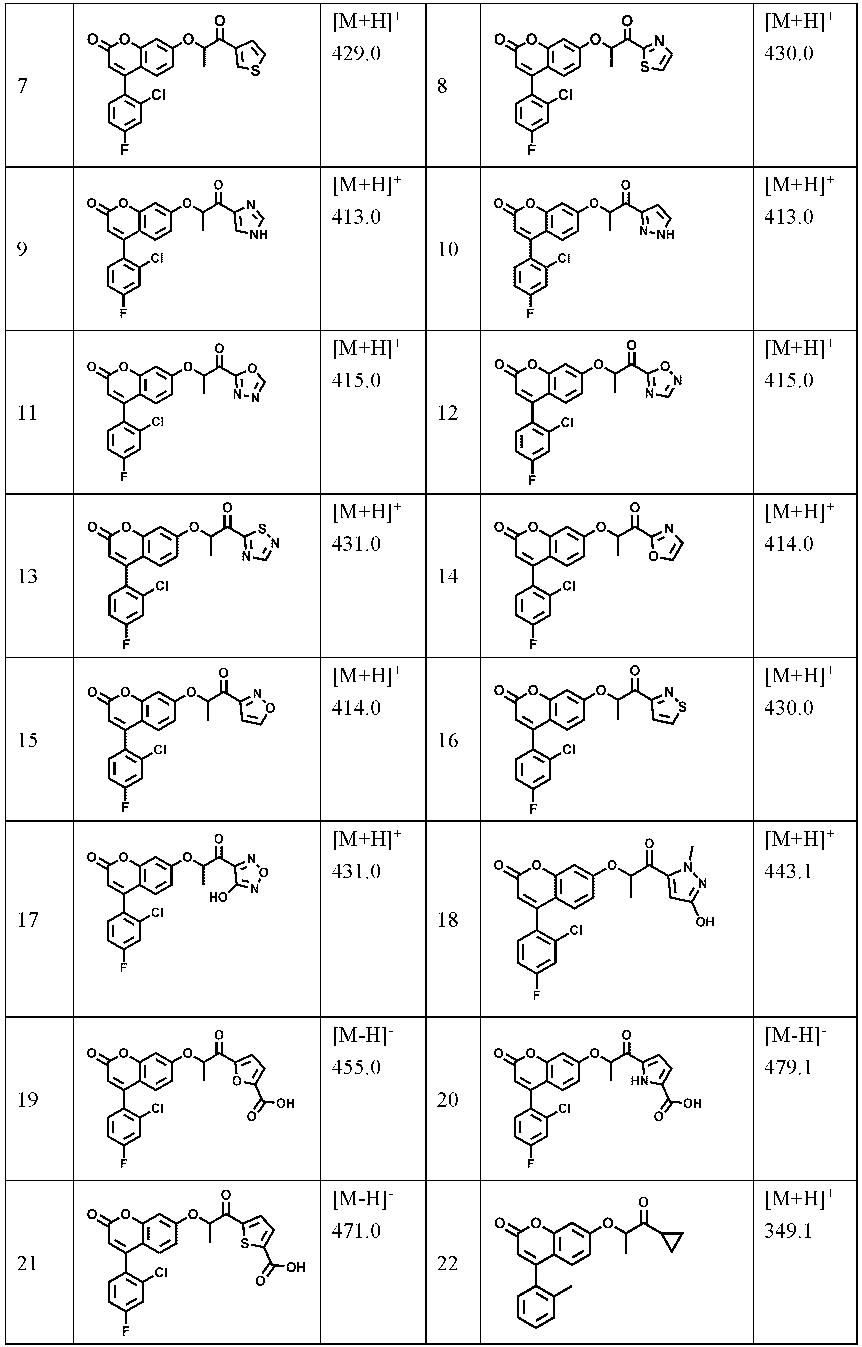

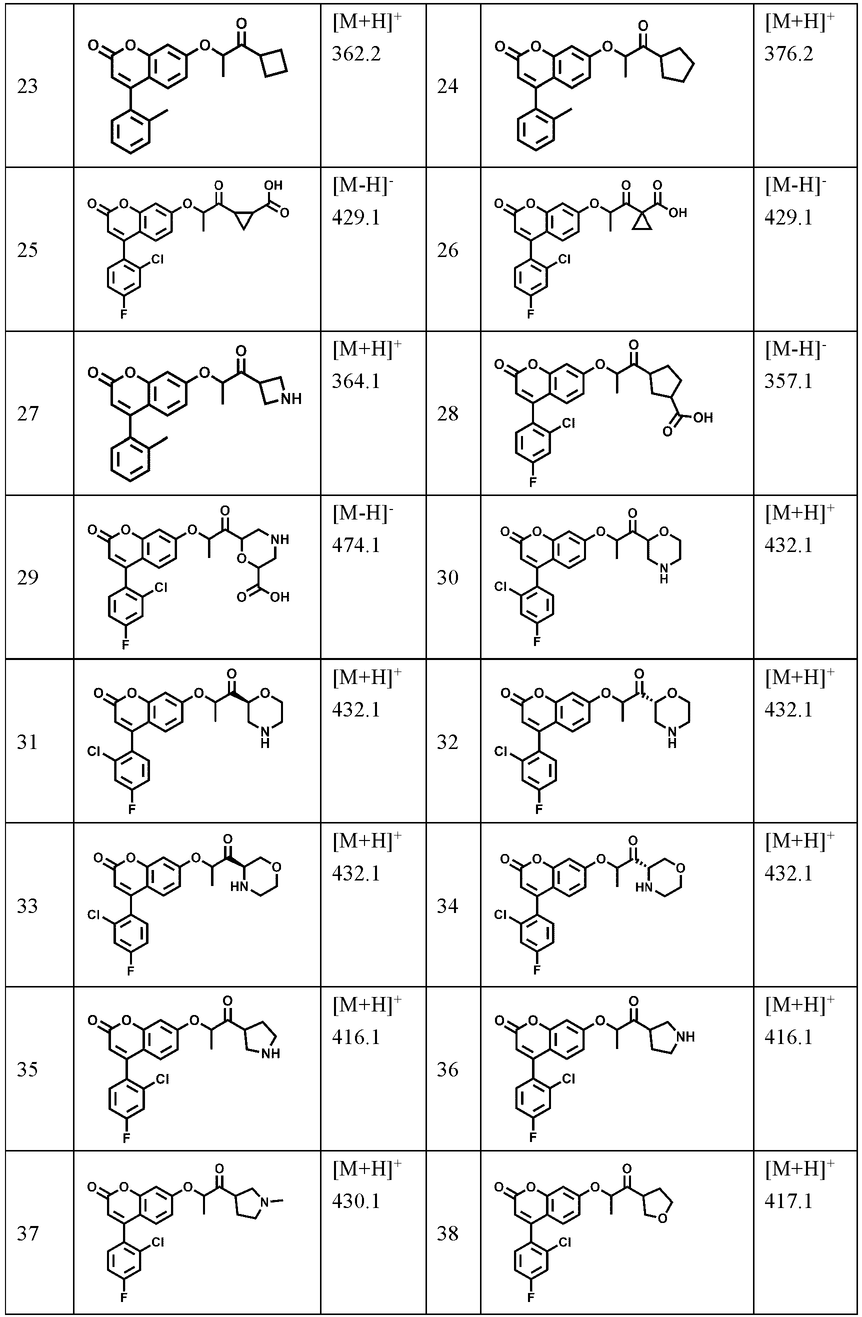

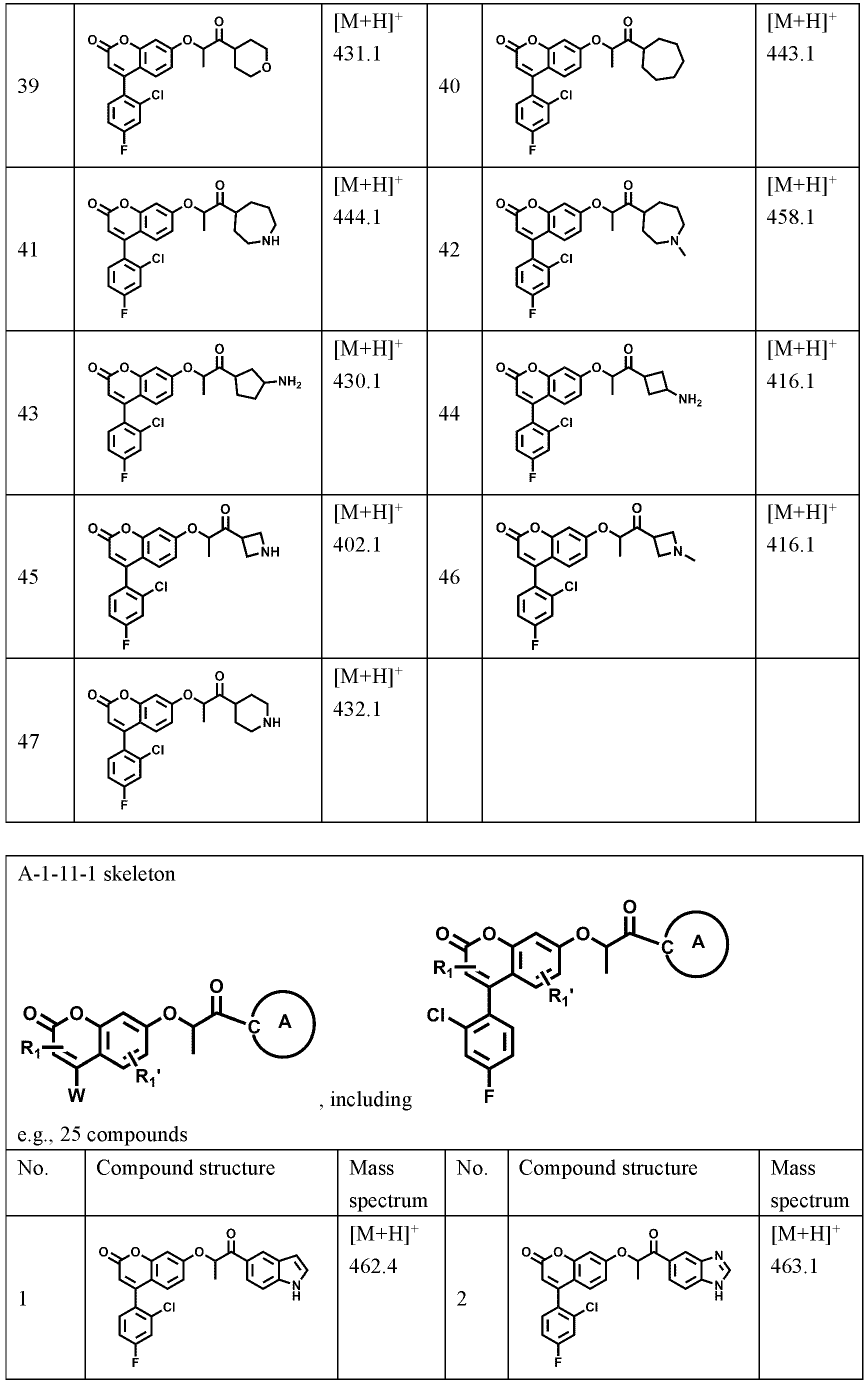

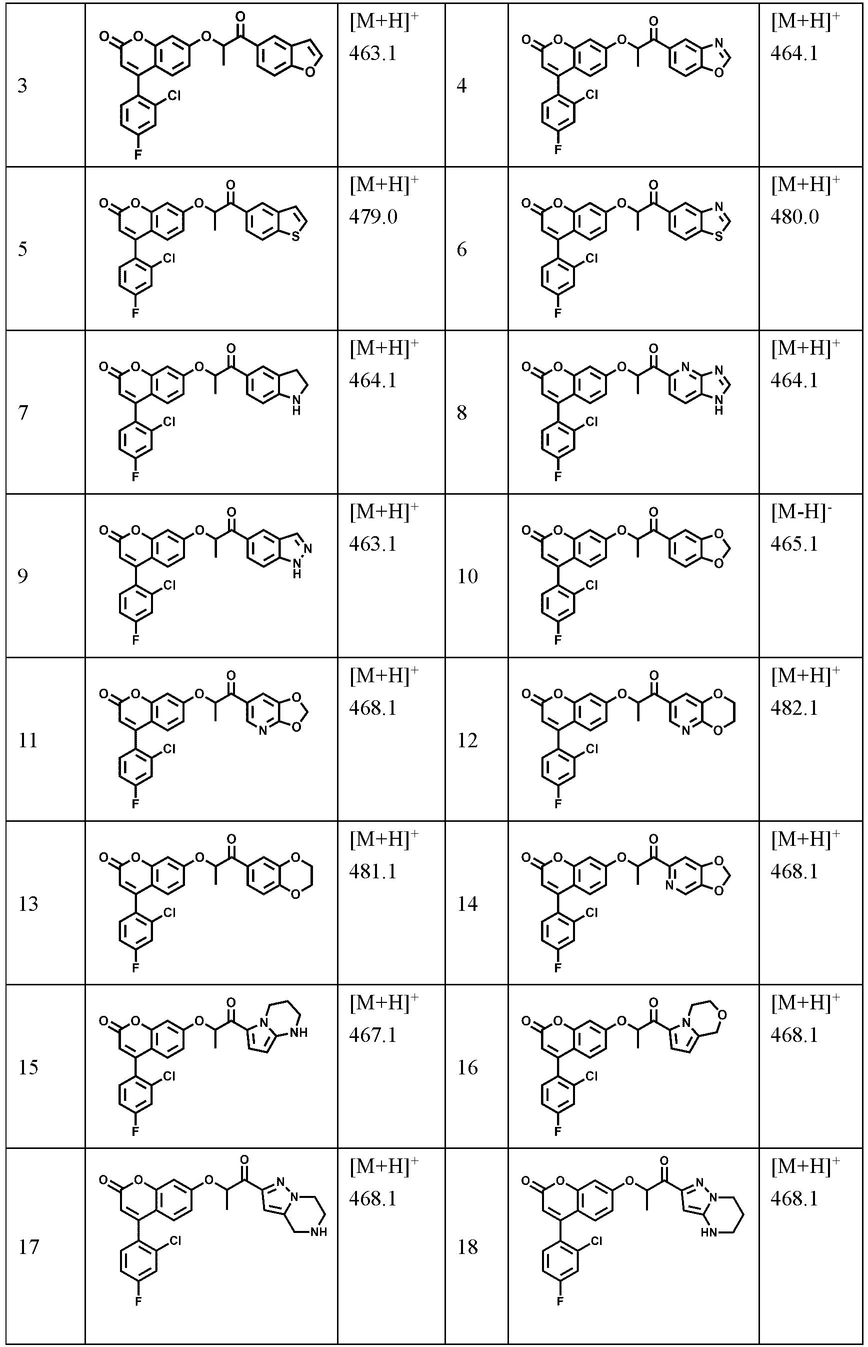

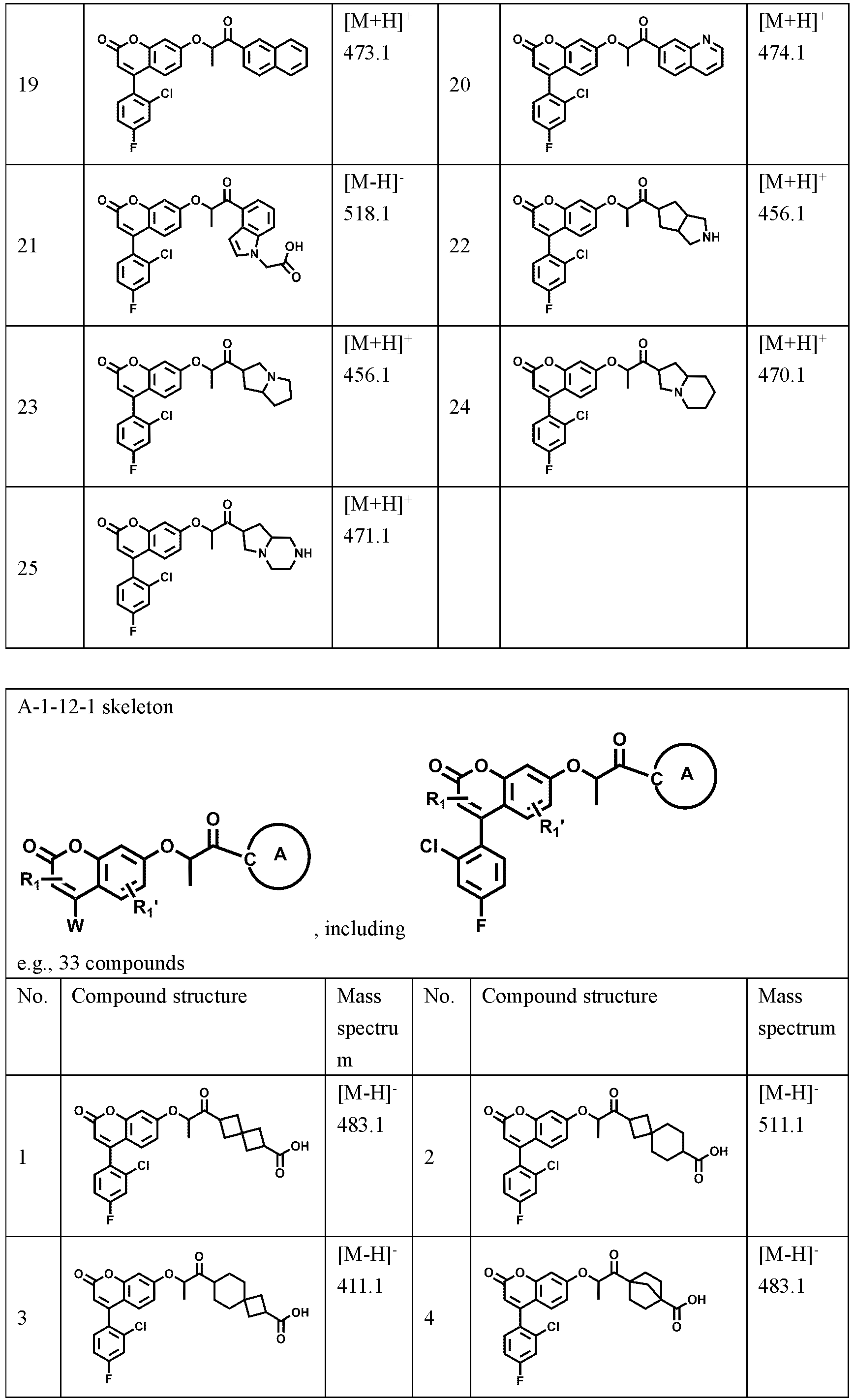

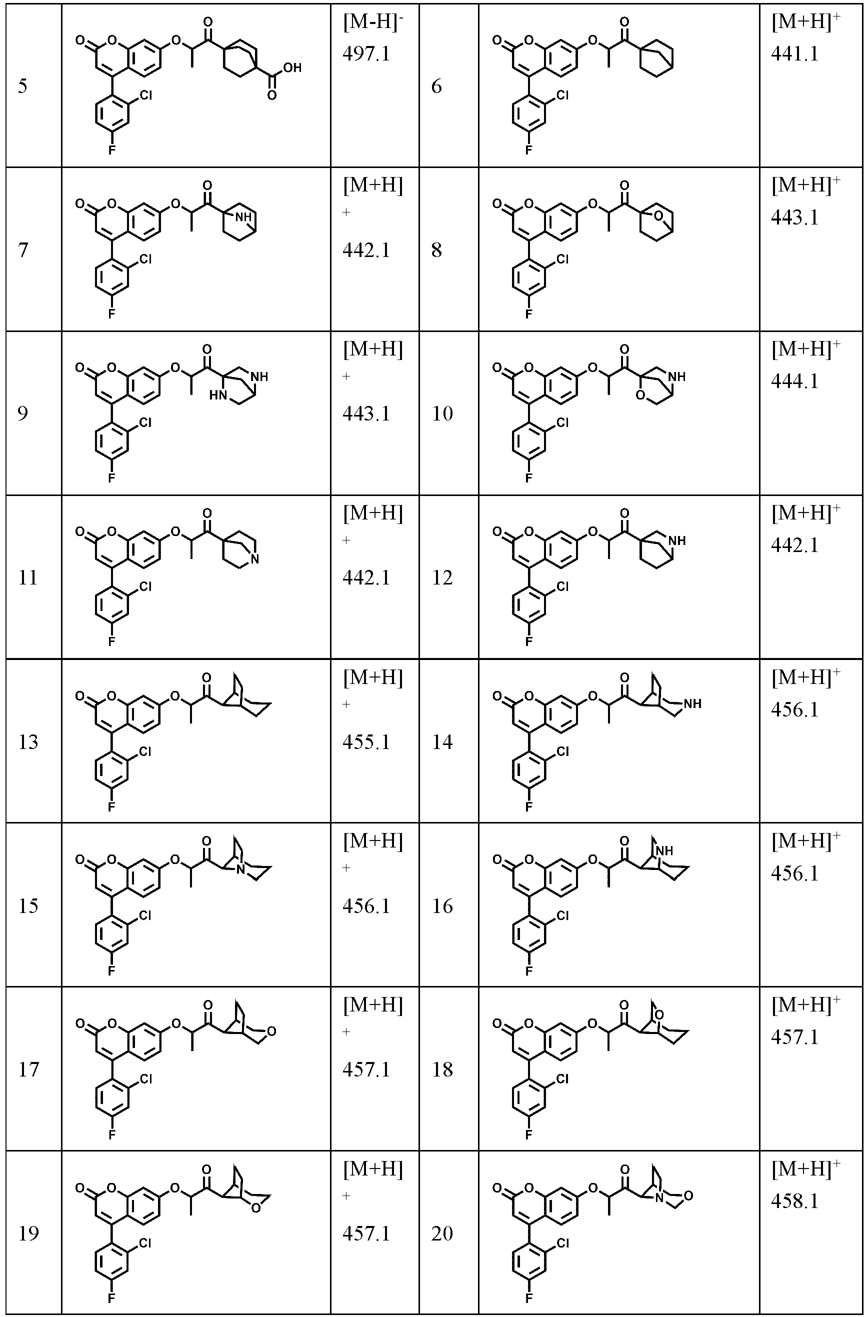

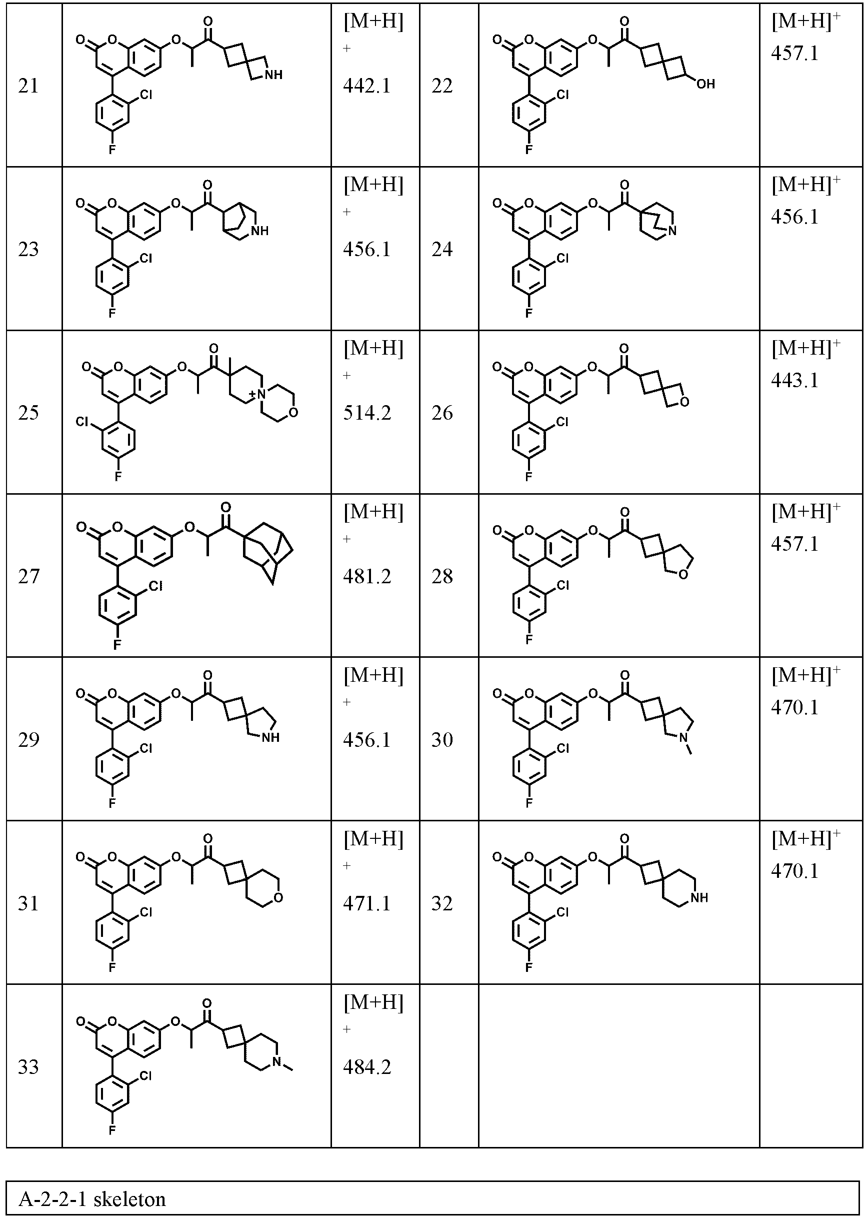

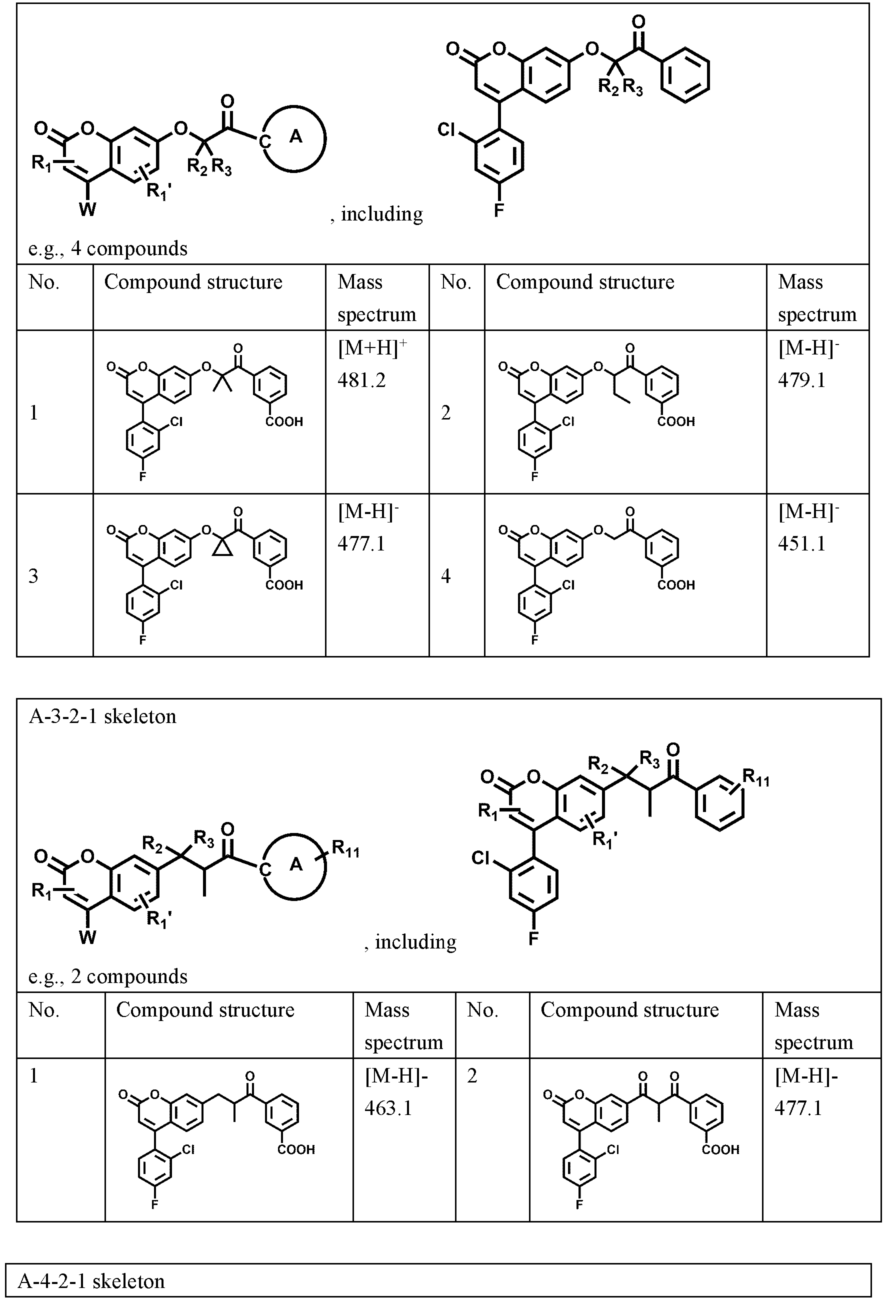

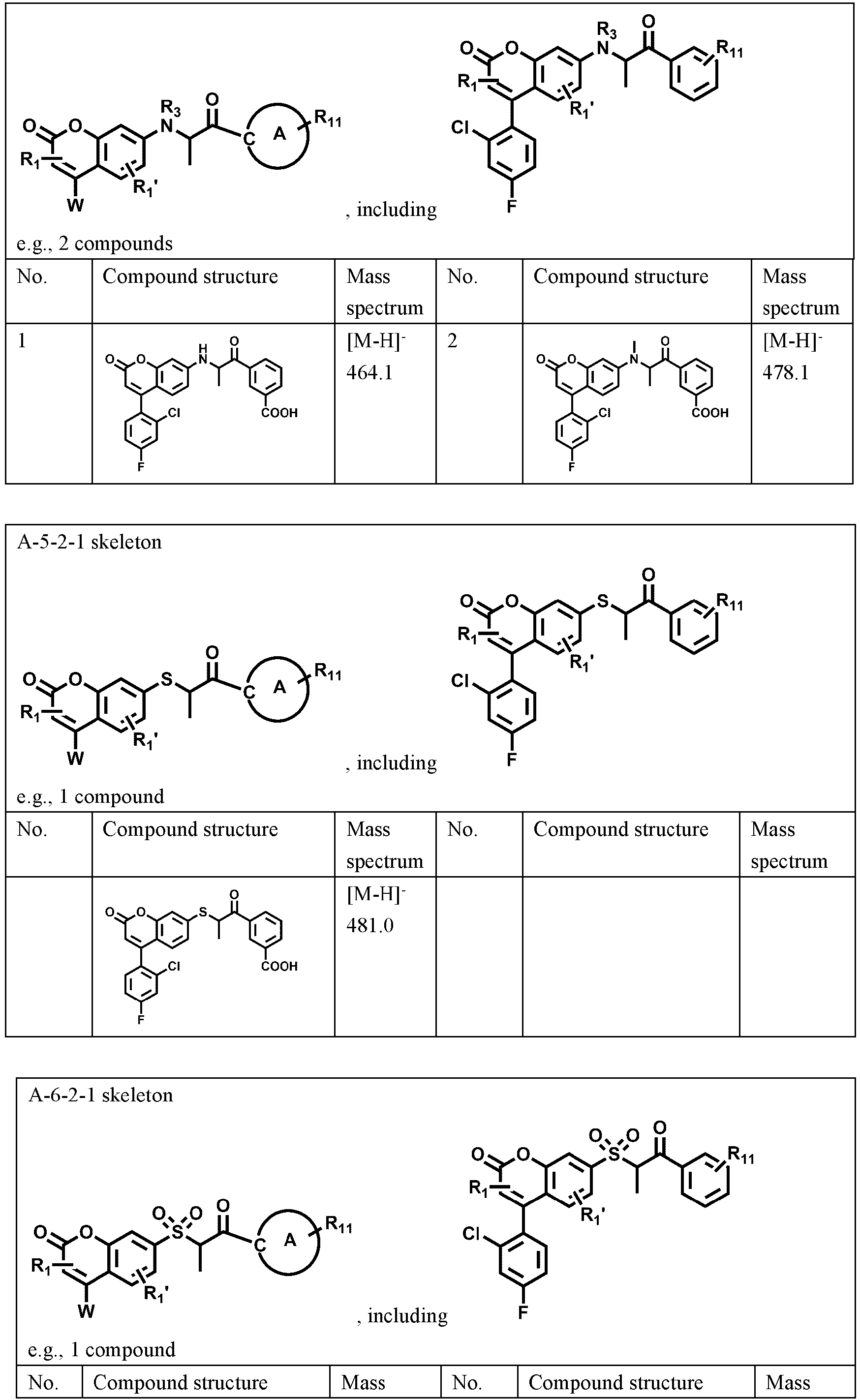

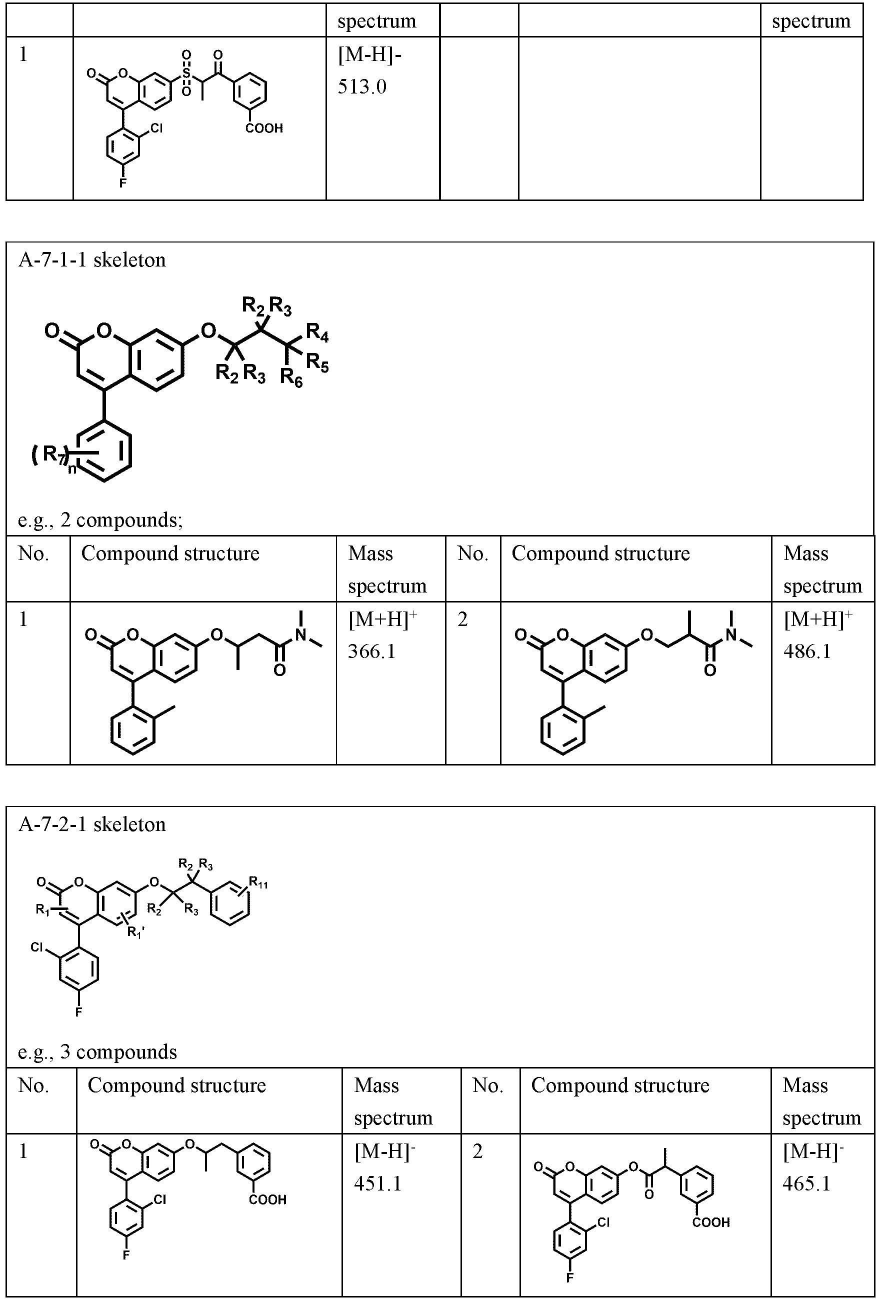

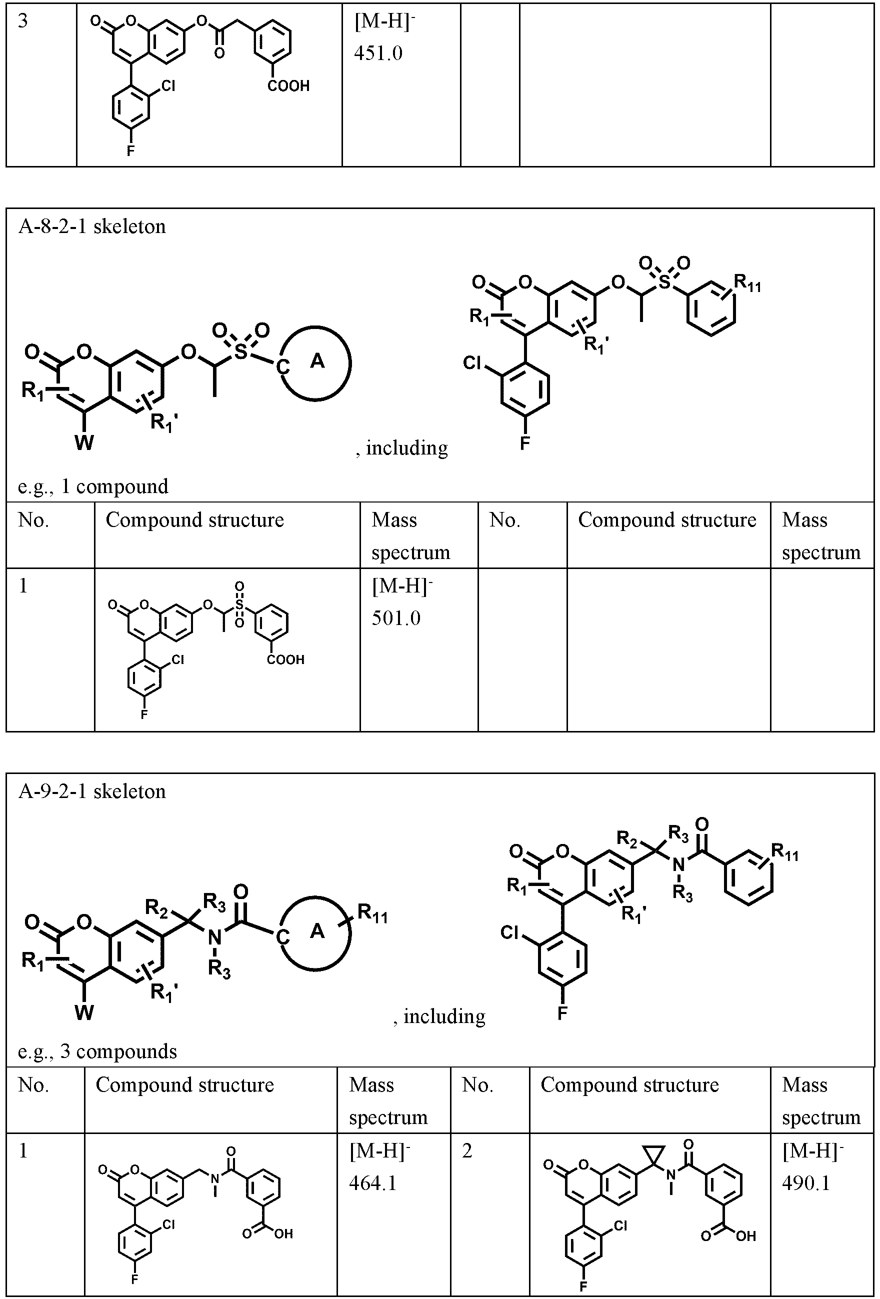

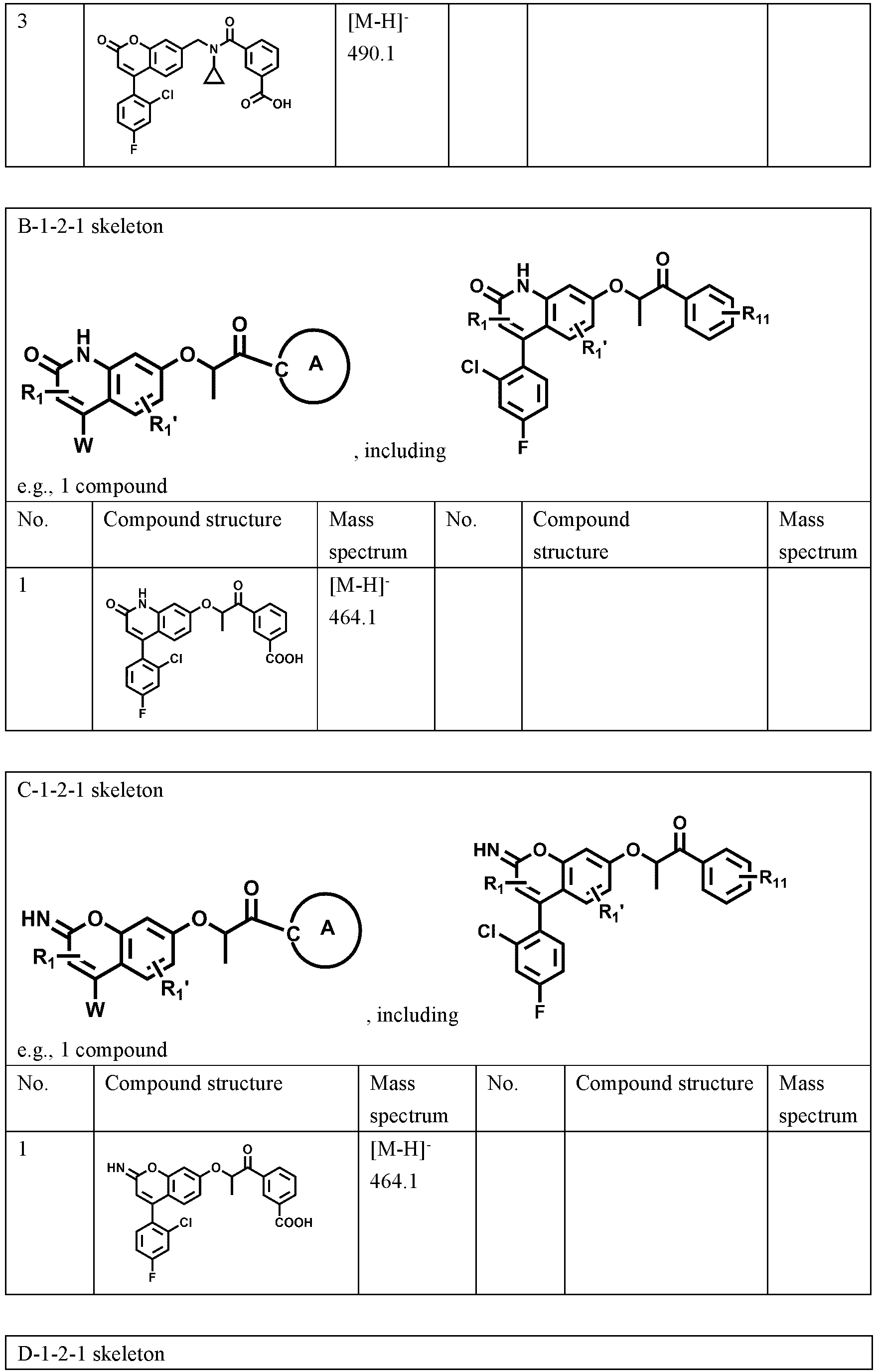

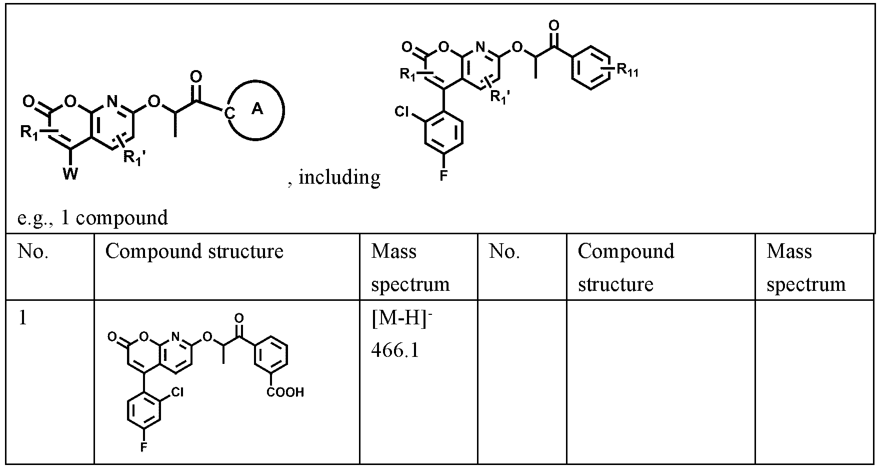

### Terms in the present invention

The following terms and the like are used to describe the present invention. It is to be understood that a term that is not specifically defined is given a meaning consistent with the use of the term in the context of the present invention, as would be understood by a person of ordinary skill in the art.

The term "alkyl" used in the present invention refers to a saturated aliphatic hydrocarbon group, which term includes both straight-chain and branched hydrocarbon groups. For example, "Cₘ₋ₙ alkyl" refers to an alkyl group having from m to n carbon atoms (m and n are integers), such as C₁-₆ alkyl. "C₁-₆ alkyl" refers to an alkyl group having from 1 to 6 carbon atoms, for example, an alkyl group having 1 carbon atom, 2 carbon atoms, 3 carbon atoms, 4 carbon atoms, 5 carbon atoms and 6 carbon atoms, including, but not limited to, methyl, ethyl, propyl (e.g., n-propyl, isopropyl), butyl (e.g., n-butyl, isobutyl, tert-butyl), pentyl (e.g., n-pentyl, isopentyl, neopentyl), hexyl (e.g., n-hexyl), etc.

The subscript numbers and/or letters "n" or "na" in Xₙ, Lₙ, Rₙ and Rₙₐ in the present invention are merely numbers given to distinguish different groups (or substituents or atoms) and do not indicate the number of these groups. For example, the subscript numbers and/or letters such as 1, 2 and 2a in the substituents such as X₁, X₂, R₂, R₂ₐ, L₁, L₂, L₃, R₃, R₃, R₄, R₅, R₆, R₇, R₈, R₉, R₁₀, R₈ₐ, R₉ₐ, R₁₀ₐ and R₁₁ are merely numbers given to distinguish different substituents or groups or atoms. Specifically, illustrative, but non-limiting, examples of the attachment of these R substituents are as follows.

For example, "-C(R₂R₃)-" may refer to a form in which R₂ is directly attached to the C atom therein and R₃ is attached to R₂ and not directly attached to the C atom. The structural formula is

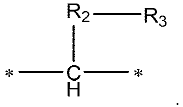

For example, "-CR₂R₃-" may refer to the -C-R₂₋R₃- structure.

For example, "-CR₈(R₉)" may refer to a structure in which R₉ is attached only to the C atom, including, for example:

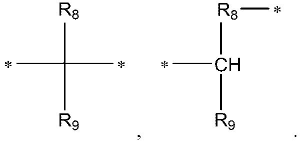

For example, "-C-OR₈(R₉)" may refer to a structure in which R₉ is directly attached to the carbon atom and not attached to R₈.

For example, "-NR₃-" may refer to

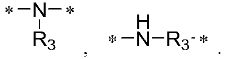

For example, "-NR₈R₉" may refer to

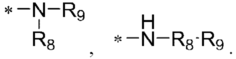

The term "oxo" used in the present invention refers to "O=".

The term "halogen" used in the present invention refers to fluorine, chlorine, bromine or iodine, preferably fluorine and chlorine.

The term "carboxyl" used in the present invention refers to -COOH.

The term "alkylcarboxyl" used in the present invention refers to an alkyl group having a carboxy substituent group.

The "heterocyclyl" used in the present invention refers to any group formed by saturated or unsaturated oxacyclic, azacyclic and thiacyclic compounds, including, for example, heterocycloalkyl and heteroaryl, including, but not limited to, the following groups: heterocycloalkyl such as oxiranyl, oxetanyl, aziridinyl, azetidinyl, thietanyl and hydrogenated aromatic heterocyclyl (e.g., tetrahydrofuranyl, tetrahydropyrrolyl, tetrahydrothienyl, tetrahydropyranyl, piperidinyl, tetrahydrothiopyranyl, dioxanyl, piperazinyl, hexahydropyrazinyl, morpholinyl, dithianyl, etc.); "heteroaryl" including, but not limited to, five-membered heteroaryl, six-membered heteroaryl, benzoheteroaryl, etc., such as any one of furyl, pyrrolyl, thienyl, pyrazolyl, imidazolyl, oxazolyl, thiazolyl, isothiazolyl, pyridyl, oxygen-containing imidazolyl or pyrazolyl, pyranyl, thiopyranyl, pyridazinyl, pyrimidinyl, pyrazinyl, piperazinyl, indolyl, benzimidazolyl, carbazolyl, thiazolinyl, quinolinyl, isoquinolinyl, purinyl, acridinyl, phenazinyl and phenothiazinyl, or a partially hydrogenated aromatic heterocyclyl thereof; benzoheterocyclyl such as benzofuranyl, benzothienyl, benzopyrrolyl, indolyl, quinolinyl, isoquinolinyl, benzopyranyl, a group formed by benzo-γ-pyrone, and fused benzoheterocyclyl, as well as heterocycloheterocyclyl such as purinyl; the "aryl" refers to any one of phenyl, naphthyl, anthryl, phenanthryl; "heteroaryl", also referred to herein as "heteroaromatic group" or "heteroaromatic ring group" or "aromatic heterocyclyl", refers to any of the above aryl groups substituted on a carbon atom with an oxygen atom or a nitrogen atom, such as anthraquinonyl.

The term "benzopyrone-based compound" refers to compounds having a benzopyrone ring, a structure where one carbon atom of the benzene ring in the benzopyrone ring is replaced with a nitrogen atom, a derivative structure formed by replacing either of two oxygen atoms of the pyranone ring in the benzopyrone ring with a nitrogen atom, and any of the various structures formed by substituting the above structures. The "coumarin ring" refers to the benzopyrone ring in the "benzopyrone-based compound" as described above, and includes the structure where one carbon atom of the benzene ring is replaced with a nitrogen atom or the structure where either of two oxygen atoms of the pyranone ring is replaced with a nitrogen atom as described above.

The term "pharmaceutically acceptable salts" used in the present invention refers to salts of compounds of the present invention, prepared from the compounds with the specified substituents found in the present invention and pharmaceutically acceptable acids or bases.

The term "pharmaceutically acceptable carriers" refers to any formulation carrier or medium capable of delivering an effective amount of an active substance of the present invention without interfering with the biological activity of the active substance and without toxic and side effects to hosts or patients. Representative carriers include water, oils, vegetables and minerals, cream bases, lotion bases, ointment bases, etc. These bases include a suspending agent, tackifier, transdermal enhancer, etc.

### Preparation Examples

To further describe in detail the preparation of the benzopyrone-based compound of the present invention and the technical effects thereof as drugs for specifically inhibiting POLRMT, the following description is given by way of illustration.

### First Set of Preparation Examples (Including Examples of the First Set of Technical Solutions)

### Preparation Example (I) 1: Compound α-1

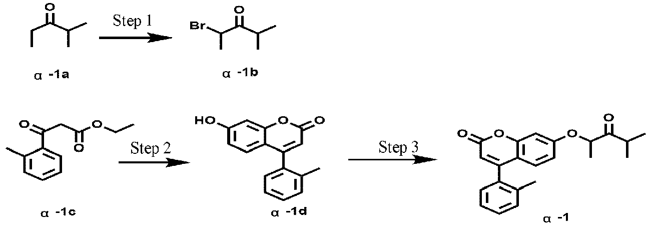

### Step 1

In a 100 mL three-necked flask under N₂ protection, α-1a (2.03 g, 20.3 mmol) and dry tetrahydrofuran (20 mL) were successively added. After sufficient dissolution, the system was cooled to 0°C, and then lithium hexamethyldisilazide (0.5 mL, 2.0 M/THF) was added dropwise. After completion of the dropwise addition, the reaction was carried out for 10 min. Then, the system was cooled to -78°C, and bromine (1 mL, 20 mmol) was slowly added dropwise. The reaction was carried out at the maintained temperature for 3 h and monitored by TLC until the starting materials had completely reacted. The reaction solution was added dropwise to saturated aqueous sodium bicarbonate solution and then extracted with diethyl ether (20 mL × 3). The organic phases were combined, washed successively with sodium thiosulfate solution and saturated brine, dried over anhydrous sodium sulfate, and concentrated under reduced pressure to give the crude product α-1b (3.58 g, 19.99 mmol, yield: 98%).

### Step 2

In a 200 mL single-necked flask, α-1c (20.62 g, 100 mmol) and methanesulfonic acid (65 mL) were successively added. After sufficient dissolution, resorcinol (10.392 g, 94 mmol) was added, and the temperature was raised to 45°C. The reaction was carried out for 4 h and monitored by TLC until the starting materials had completely reacted. The reaction solution was diluted with ethanol. Water was added for stirring, and a solid precipitated. The mixture was filtered to give the intermediate α-1d (18.0 g, 71.35 mmol, yield: 72%).

### Step 3

In a 100 mL single-necked flask, α-1d (0.73 g, 2.89 mmol) and N,N-dimethylformamide (10 mL) were successively added. After sufficient dissolution, cesium carbonate (2.36 g, 7.23 mmol) was added at room temperature, and then the reaction was carried out for 10 min. Then, α-1b (0.52 g, 2.89 mmol) was added, and the reaction was carried out for 4 h. The reaction was monitored by TLC until the starting materials had completely reacted. The reaction solution was diluted with water and extracted with ethyl acetate. The organic phase was dried over anhydrous sodium sulfate, concentrated under reduced pressure, and purified by column chromatography to give the product α-1 (0.32 g, 0.91 mmol, yield: 32%). MS-ESI calculated for [M+H]⁺ 351.1, found 351.4.

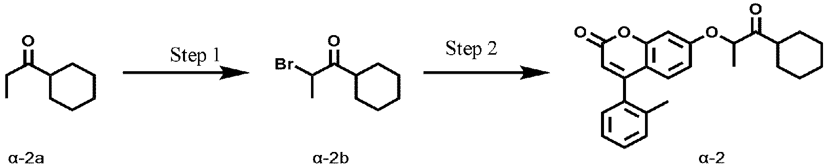

### Preparation Example (I) 2: Compound α-2

### Step 1

In a 100 mL single-necked flask, α-2a (1 g, 7.14 mmol) and carbon tetrachloride (10 mL) were successively added. After sufficient dissolution, the mixture was stirred for 5 min, and bromine (0.3 mL, 5.71 mmol) was added dropwise. The reaction was carried out for 4 h and monitored by TLC for completion. The reaction solution was added dropwise to saturated aqueous sodium bicarbonate solution and extracted with ethyl acetate. The organic phase was washed successively with sodium thiosulfate solution and saturated brine. The organic phase was dried over anhydrous sodium sulfate, concentrated using a rotary evaporator, and then separated and purified by a flash column to give α-2b (1.33 g, 6.07 mmol, yield: 85%).

### Step 2

In a 100 mL single-necked flask, α-1d (1.10 g, 4.36 mmol) and N,N-dimethylformamide (10 mL) were successively added. After sufficient dissolution, cesium carbonate (4.11 g, 12.6 mmol) was added at room temperature, and the reaction was carried out for 10 min. Then, α-2b (1.33 g, 6.07 mmol) was added, and the reaction was carried out for 4 h. The reaction was monitored by TLC until the starting materials had completely reacted. The reaction solution was concentrated using a rotary evaporator. Water was added, and a solid precipitated. The mixture was filtered and purified by column chromatography to give the product α-2 (1.00 g, 2.56 mmol, yield: 59%). MS-ESI calculated for [M+H]⁺ 391.1, found 391.4. ¹H NMR (400 MHz, DMSO-*d₆*) δ 7.48-7.37 (m, 2H), 7.34 (t, *J =* 7.3 Hz, 1H), 7.23 (d, *J =* 7.4 Hz, 1H), 6.98 (t, *J =* 2.5 Hz, 1H), 6.92-6.78 (m, 2H), 6.18 (s, 1H), 5.34 (qd, *J =* 6.8, 2.3 Hz, 1H), 2.77 (ddd, *J =* 11.2, 7.8, 3.3 Hz, 1H), 2.11 (d, *J =* 2.1 Hz, 3H), 1.78-1.58 (m, 4H), 1.47 (d, *J =* 6.8 Hz, 3H), 1.37-1.10 (m, 6H).

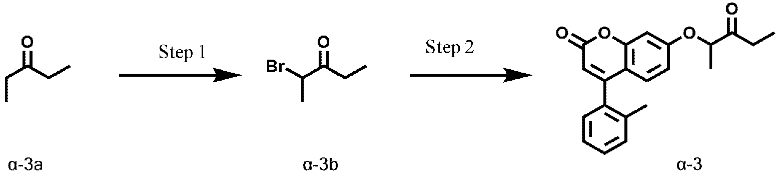

### Preparation Example (I) 3: Compound α-3

### Step 1

In a 250 mL single-necked flask, α-3a (3 g, 34.83 mmol) and diethyl ether (87 mL) were successively added. After sufficient dissolution, bromine (1.8 mL, 34.83 mmol) was added dropwise at room temperature. The reaction was carried out for 4 h and monitored by TLC until the starting materials had completely reacted. The reaction solution was added dropwise to saturated aqueous sodium bicarbonate solution and extracted with ethyl acetate. The organic phase was washed successively with sodium thiosulfate solution and saturated brine, dried over anhydrous sodium sulfate, and concentrated using a rotary evaporator to give the crude product α-3b (4.6 g, 27.87 mmol, yield: 80%), which could be directly used in the next step without purification.

### Step 2

In a 200 mL single-necked flask, α-1d (1.21 g, 4.8 mmol) and N,N-dimethylformamide (20 mL) were successively added. After sufficient dissolution, cesium carbonate (4.07 g, 12.5 mmol) was added at room temperature, and the reaction was carried out for 10 min. Then, α-3b (1.32 g, 8 mmol) was added, and the reaction was carried out for 8 h. The reaction was monitored by TLC until the starting materials had completely reacted. The reaction solution was filtered. The filtrate was diluted with water and extracted with ethyl acetate. The organic phase was dried over anhydrous sodium sulfate, concentrated using a rotary evaporator, and then separated and purified by a flash column to give the desired product (0.70 g, 2.08 mmol, yield: 43%). MS-ESI calculated for [M+H]⁺ 337.1, found 337.4. ¹H NMR (400 MHz, DMSO-*d₆*) δ 7.38 (dt, *J =* 29.0, 8.3 Hz, 3H), 7.24 (d, *J* = 7.7 Hz, 1H), 7.01 (s, 1H), 6.86 (s, 2H), 6.19 (s, 1H), 5.17 (q, *J* = 7.0 Hz, 1H), 2.84-2.52 (m, 2H), 2.11 (s, 3H), 1.47 (d, *J =* 6.0 Hz, 3H), 0.94 (t, *J =* 6.2 Hz, 3H).

### Preparation Example (I) 4: Compound α-4

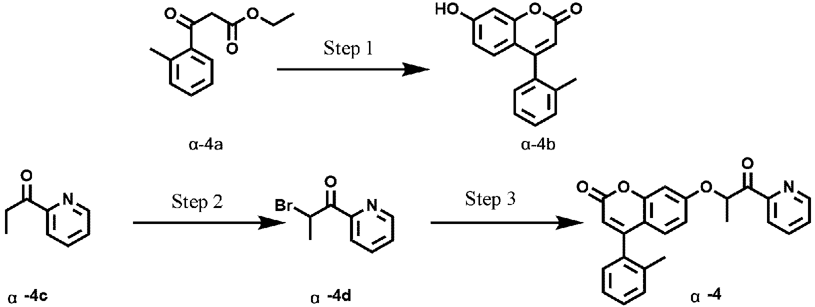

In a dry 100 mL three-necked flask, α-4a (7.00 g, 33.90 mmol) was dissolved in perchloric acid (20 mL). Then, resorcinol (4.50 g, 40.7 mmol) was added. The reaction was carried out at room temperature for 16 h until it reached completion. After completion of the reaction, water (40 mL) was added under an ice-water bath to quench the reaction. The mixture was extracted with ethyl acetate, dried, and concentrated. The residue obtained after concentration under reduced pressure was purified by silica gel column chromatography to give α-4b (6.94 g, 27.51 mmol, yield: 81.1%).

MS-ESI calculated for [M+H]+ 253.1, found 253.0.

¹H NMR (600 MHz, DMSO-d6) δ 10.64 (s, 1H), 7.44-7.37 (m, 2H), 7.34 (t, *J =* 7.4, 1H), 7.23 (d, *J =* 7.5, 1H), 6.84-6.79 (m, 2H), 6.73 (dd, *J* = 8.7, 2.3 Hz, 1H), 6.09 (s, 1H), 2.11 (s, 3H).

### Step 2

In a dry 50 mL three-necked flask, compound α-4c (1.00 g, 7.40 mmol) was dissolved in acetic acid:carbon tetrachloride = 1:1 (10 mL). Then, bromine (3.55 g, 22.21 mmol) was added. The reaction was carried out at room temperature for 16 h until it reached completion. After completion of the reaction, saturated sodium sulfite solution (10 mL) and saturated sodium bicarbonate solution (20 mL) were added under an ice-water bath to quench the reaction. The mixture was extracted with ethyl acetate, dried, and concentrated to give the crude product α-4d (1.10 g, 5.14 mmol, yield: 63.39%).

MS-ESI calculated for [M+H]+ 214.1, found 214.0.

### Step 3

In a dry 50 mL three-necked flask, compound α-4d (407.0 mg, 1.9 mmol), α-4b (400.0 mg, 1.59 mmol) and potassium carbonate (438.0 mg, 3.17 mmol) were dissolved in acetonitrile (10 mL). The reaction was carried out at room temperature for 16 h until it reached completion. After completion of the reaction, water (20 mL) was added to quench the reaction. The mixture was extracted with ethyl acetate, dried, and concentrated. The residue obtained after concentration under reduced pressure was purified by silica gel column chromatography to give the product α-4 as a white solid (69.0 mg, 0.18 mmol, yield: 11%).

MS-ESI calculated for [M+H]+ 386.4, found 386.0.

¹H NMR (600 MHz, DMSO-d6) δ 8.83 (d, *J =* 4.7, 1H), 8.09 (t, *J =* 7.7, 1H), 8.01 (d, *J =* 7.8, 1H), 7.81-7.75 (m, 1H), 7.43-7.37 (m, 2H), 7.33 (q, *J* = 7.3, 1H), 7.25-7.20 (m, 1H), 6.91-6.79 (m, 3H), 6.43 (q, *J =* 6.8 Hz, 1H), 6.17 (s, 1H), 2.10 (d, *J =* 5.1 Hz, 3H), 1.63 (d, *J =* 6.8 Hz, 3H).

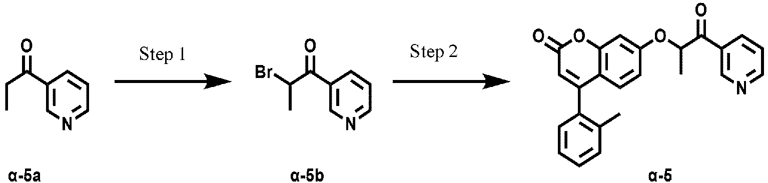

### Preparation Example (I) 5: Compound α-5

### Step 1

In a dry 50 mL three-necked flask, compound α-5a (500.0 mg, 3.70 mmol) was dissolved in diethyl ether (5 mL). Then, bromine (1.18 mg, 7.4 mmol) was added. The reaction was carried out at 50°C for 20 h until it reached completion. After completion of the reaction, the mixture was concentrated under reduced pressure to give the crude product α-5b (500 mg, 2.34 mmol, yield: 63.1%).

MS-ESI calculated for [M+H]⁺ 214.1, found 214.0.

### Step 2

In a dry 50 mL three-necked flask, compound α-5b (500 mg, 2.34 mmol), α-4b (250 mg, 0.99 mmol) and *N,N*-diisopropylethyl amine (645.0 mg, 5.0 mmol) were dissolved in acetone (10 mL). The reaction was carried out at room temperature for 16 h until it reached completion. After completion of the reaction, water (20 mL) was added to quench the reaction. The mixture was extracted with ethyl acetate, dried, and concentrated. The residue obtained after concentration under reduced pressure was purified by silica gel column chromatography followed by high-pressure preparative chromatography to give the product α-5 (70 mg, 0.18 mmol, yield: 18%, white solid).

MS-ESI calculated for [M+H]⁺ 386.4, found 386.0.

¹H NMR (600 MHz, DMSO-*d₆*) δ 9.24 (s, 1H), 8.87 (d, *J =* 4.2 Hz, 1H), 8.41 (dq, *J =* 8.0, 2.2 Hz, 1H), 7.64 (dd, *J =* 8.0, 4.8 Hz, 1H), 7.45-7.37 (m, 2H), 7.36-7.31 (m, 1H), 7.25-7.22 (m, 1H), 7.11 (s, 1H), 6.93-6.87 (m, 2H), 6.23 (p, *J =* 6.7 Hz, 1H), 6.20 (s, 1H), 2.11 (d, *J =* 3.4 Hz, 3H), 1.59 (dd, *J =* 6.8, 1.3 Hz, 3H).

### Preparation Example (I) 6: Compound α-6

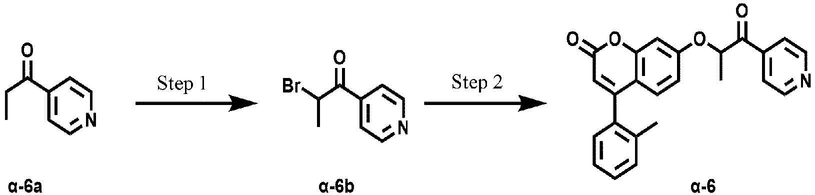

### Step 1:

In a dry 50 mL three-necked flask, compound α-6a (500 mg, 3.70 mmol) was dissolved in diethyl ether (5 mL). Then, bromine (1.18 mg, 7.40 mmol) was added. The reaction was carried out at 50°C for 20 h until it reached completion. After completion of the reaction, the mixture was concentrated under reduced pressure to give the crude product α-6b (500.0.0 mg, 2.34 mmol, yield: 63%).

MS-ESI calculated for [M+H]⁺ 214.06, found 214.0.

### Step 2:

In a dry 50 mL three-necked flask, compound α-6b (500 mg, 2.34 mmol), α-4b (250.0 mg, 0.99 mmol) and N,N-diisopropylethyl amine (645.0 mg, 5.0 mmol) were dissolved in acetone (10 mL). The reaction was carried out at room temperature for 16 h until it reached completion. After completion of the reaction, water (20 mL) was added to quench the reaction. The mixture was extracted with ethyl acetate, dried, and concentrated. The residue obtained after concentration under reduced pressure was purified by silica gel column chromatography followed by high-pressure preparative chromatography to give the product α-6 (60.0 mg, 0.16 mmol, yield: 16%, white solid).

MS-ESI calculated for [M+H]+ 386.4, found 386.0.

¹H NMR (600 MHz, DMSO-d₆) δ 8.86 (d, *J* = 17.4, 4.7, 2H), 7.95-7.84 (m, 2H), 7.45-7.37 (m, 2H), 7.37 -7.30 (m, 1H), 7.26-7.21 (m, 1H), 7.11 *(d, J =* 2. 0 Hz, 1H), 6.92 - 6.86 (m, 2H), 6.26 - 6.19 (m, 2H), 2.11 (*d, J =* 3.7 Hz, 3H), 1.57 (dd, *J =* 6.7, 1.2 Hz, 3H).

### Preparation Example (I) 7: Compound α-7

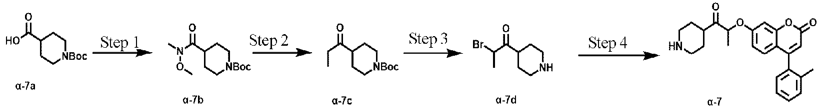

### Step 1:

In a dry 100 mL three-necked flask, compound α-7a (4.00 g, 17.45 mmol) was dissolved in N,N-dimethylformamide (40 mL). Then, dimethylhydroxylamine hydrochloride (2.00 g, 20.90 mmol), 2-(7-azabenzotriazole)-N,N,N',N'-tetramethyluronium hexafluorophosphate (7.96 g, 20.90 mmol) and N,N-diisopropylethyl amine (6.75 g, 52.35 mmol) were added. The reaction was carried out at room temperature for 3 h until it reached completion. After completion of the reaction, water (40 mL) was added to quench the reaction. The mixture was extracted with ethyl acetate, dried, and concentrated. The residue obtained after concentration under reduced pressure was purified by medium-pressure reverse phase chromatography to give the product α-7b (4.75 g, 17.44 mmol, yield: 99%).

MS-ESI calculated for [M+H]+ 273.2, found 273.0.

### Step 2:

In a dry 100 mL three-necked flask, compound α-7b (4.50 g, 16.52 mmol) was dissolved in tetrahydrofuran (40 mL). Then, ethylmagnesium bromide (1M in tetrahydrofuran) (24.80 mmol) was added. The reaction was carried out at room temperature for 18 h until it reached completion. After completion of the reaction, water (40 mL) was added to quench the reaction. The mixture was extracted with ethyl acetate, dried, and concentrated. The residue obtained after concentration under reduced pressure was purified by medium-pressure reverse phase chromatography to give the product α-7c (3.50 g, 14.50 mmol, yield: 87%).

MS-ESI calculated for [M+H]+ 242.3, found 242.0.

### Step 3:

In a dry 50 mL three-necked flask, compound α-7c (2.00 g, 8.29 mmol) was dissolved in diethyl ether (10 mL). Then, bromine (1.45 g, 9.1 mmol) was added. The reaction was carried out at 40°C for 4 h until it reached completion. After completion of the reaction, the mixture was concentrated under reduced pressure to give the crude product α-7d (1.50 g, 6.84 mmol, yield: 82%).

MS-ESI calculated for [M+H]+ 220.1, found 220.0.

### Step 4:

In a dry 50 mL three-necked flask, the crude compound α-7d (650 mg, 2.95 mmol) was dissolved in acetone (20 mL). Then, α-4b (500.0 mg, 1.98 mmol) and potassium carbonate (821 mg, 5.94 mmol) were added. The reaction was carried out at room temperature for 16 h until it reached completion. After completion of the reaction, water (20 mL) was added to quench the reaction. The mixture was extracted with ethyl acetate, dried, and concentrated. The residue obtained after concentration under reduced pressure was purified by silica gel column chromatography followed by high-pressure preparative chromatography to give the product α-7 (50.0 mg, 0.13 mmol, yield: 4%, white solid). The obtained product was dissolved in acetonitrile, and then adjust the pH of the solution to about 2 with dilute hydrochloric acid. Then, the mixture was lyophilized to give the hydrochloride salt.

MS-ESI calculated for [M+H]+ 392.4, found 392.1.

¹H NMR (600 MHz, DMSO-d₆) δ 9.11 (d, *J =* 11.2 Hz, 1H), 8.79 (q, *J =* 11.0 Hz, 1H), 7.46-7.39 (m, 2H), 7.35 (t, *J =* 7.5 Hz, 1H), 7.24 (d, *J =* 7.5 Hz, 1H), 7.06-7.01 (m, 1H), 6.91 - 6.83 (m, 2H), 6.21 (s, 1H), 5.49-5.33 (m, 1H), 3.27 (dd, *J =* 31.6, 12.7 Hz, 2H), 3.18 - 3.12 (m, 1H), 2.97 - 2.87 (m, 2H), 2.17 -2.08 (d, *J =* 3.2 Hz, 4H), 1.85 - 1.77 (m, 1H), 1.74 - 1.61 (m, 2H), 1.49 (d, *J =* 6.8 Hz, 3H).

### Preparation Example (I) 8: Compound α-8

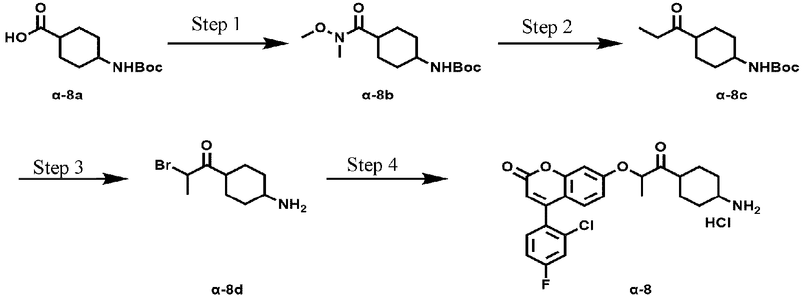

### Step 1

In a dry 100 mL three-necked flask, α-8a (5.00 g, 20.55 mmol), methoxymethanamine hydrochloride (2.203 g, 22.61 mmol), 1-propylphosphonic acid cyclic anhydride (13.07g, 41.1 mmol) and triethylamine (6.23 g, 61.65 mmol) were dissolved in N,N-dimethylformamide (35 mL). The reaction was carried out at room temperature for 1-2 h. The consumption of the starting materials was monitored by LCMS. After the starting materials were completely consumed, the mixture was diluted with water and extracted with ethyl acetate to give α-8b as a white flocculent compound (5.32 g, 18.6 mmol, yield: 75.96%, purity: 95%).
MS-ESI: [M+H]⁺ = 287.2

### Step 2

In a dry 100 mL three-necked flask, α-8b (3.60 g, 12.59 mmol) was dissolved in anhydrous tetrahydrofuran (25 mL). Ethylmagnesium bromide (2M, 18.89 mL, 37.76 mmol) was added dropwise under an ice-salt bath. After that, the reaction was carried out at room temperature for 14 h. 10% Hydrochloric acid was added to quench the reaction. The mixture was extracted with ethyl acetate three times. The organic phases were combined, washed successively with saturated sodium bicarbonate solution and saturated brine, dried over anhydrous sodium sulfate, and rotary evaporated to give a crude product. The crude product was purified by silica gel column chromatography (PE:EA = 4:1, R_{f}= 0.67, iodine fumigation) to give α-8c as a colorless oil (2.74 g, 10.72 mmol, yield: 51.82%, purity: 93%).
MS-ESI: [M+H]⁺ = 256.2

### Step 3

In a dry 100 mL three-necked flask, α-8c (2.00 g, 7.8 mmol) was dissolved in chloroform (30 mL). Bromine (1.88 g, 11.76 mmol) was added dropwise under an ice-salt bath. After that, the reaction was carried out at room temperature for 14 h. LCMS and TLC monitoring showed no starting material remaining (the starting material has no fluorescence). The reaction solution was poured into saturated sodium bicarbonate solution to quench the reaction, and extracted with ethyl acetate three times. The organic phases were combined, washed with saturated brine, dried over anhydrous sodium sulfate, and rotary evaporated to give the crude compound 4, α-8d as a pale yellow oil (1.85 g, 5.52 mmol, yield: 44.87%, purity: 80.34%).
MS-ESI: [M+H]⁺ = 234.2

### Step 4

Compound A1-1-1c (300.0 mg, 1.03 mmol) was dissolved in acetonitrile (10 mL). Then, α-8d (483.0 mg, 2.06 mmol) and potassium carbonate (427.3 mg, 3.09 mmol) were added. The reaction was carried out at room temperature for 2 h and monitored by LCMS and TLC. After 16 h, the mixture was diluted with water and extracted with ethyl acetate three times. The organic phases were combined, washed with saturated brine, dried over anhydrous sodium sulfate, and rotary evaporated to give a crude product. The crude product was purified by medium-pressure chromatography and preparative HPLC to give compound α-8 (42.0 mg, 87.40 µmol, yield: 8.5%).
MS-ESI: [M+H]⁺= 444.3

### Preparation Example (I) 9: Compound α-9

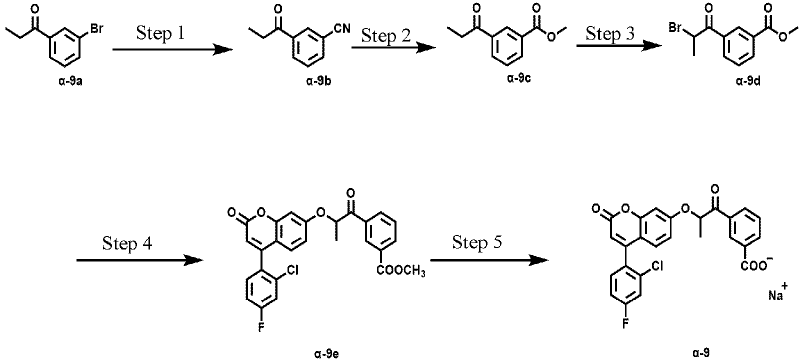

### Step 1

In a dry 500 mL three-necked flask, α-9a (10.00 g, 46.93 mmol) was dissolved in 35 mL of N,N-dimethylformamide. Cuprous cyanide (5.50 g, 61.40 mmol) was added and stirred under reflux for 6 h. After cooling back to room temperature, ferric chloride (4.70 g, 28.97 mmol) dissolved in water and 37% aqueous hydrochloric acid solution (10 mL) were added. The reaction mixture was warmed to 80°C and stirred for 0.5 h, then cooled back to room temperature and stirred overnight. LCMS and TLC monitoring showed that the starting materials had basically disappeared, indicating that the reaction was complete. The reaction solution was diluted with water and extracted with methyl tert-butyl ether. The organic phase was washed with saturated brine, dried over anhydrous sodium sulfate, concentrated, and purified by normal-phase column chromatography (PE:EA = 10:1) to give α-9b as a yellow solid (6.00 g, 37.69 mmol, yield: 80%).

¹H NMR (400 MHz, DMSO-d₆) δ 8.39 (td, *J =* 1.7, 0.6 Hz, 1H), 8.24 (ddd, *J =* 7.9, 1.8, 1.2 Hz, 1H), 8.10 (dt, *J =* 7.8, 1.3 Hz, 1H), 7.74 (td, *J =* 7.8, 0.6 Hz, 1H), 3.11 (q, *J* = 7.1 Hz, 2H), 1.09 (t, *J =* 7.1 Hz, 3H).

### Step 2

In a dry 250 mL three-necked flask, 30 mL of methanol was added, and the system was cooled to -78°C. Acetyl chloride (30 mL) was added dropwise, and α-9b (6.00 g, 37.69 mmol) was added to the reaction flask. The temperature was slowly raised, paying attention to safety. Care was taken that the reaction solution rapidly evolved HCl gas at around -40°C. The temperature was continuously raised to room temperature, and the mixture was stirred for 2 h. The reaction was monitored by LCMS for completion. The reaction solution was concentrated by rotary evaporation to give the imine intermediate. Then, 0.5% aqueous TFA solution was added to the flask. The mixture was stirred at room temperature overnight, concentrated by rotary evaporation, and purified by normal-phase column chromatography (PE:EA = 10:1) to give α-9c as a white solid (1.00 g, 5.2 mmol, yield: 13.8%). The spot corresponding to the product was above that of the starting materials.
MS-ESI: [M+H]⁺ = 193.0
¹H NMR (400 MHz, DMSO-d₆) δ 8.39 (s, 1H), 8.12-7.99 (m, 2H), 7.61 (t, *J =* 7.8 Hz, 1H), 3.82 (s, 3H), 3.10 (q, *J =* 7.1 Hz, 2H), 1.10 (t, *J =* 7.2 Hz, 3H).

### Step 3

In a dry 50 mL three-necked flask, α-9c (800 mg, 4.16 mmol) was dissolved in 10 mL of ultra-dry dichloromethane and protected with nitrogen. Bromine (3.33 g, 20.83 mmol, 1.06 mL) and hydrogen bromide (244 µL, 4.16 mmol) were added at 0°C. The mixture was allowed to return to room temperature and stirred for 2 h. LCMS monitoring showed that the starting materials had completely disappeared, indicating that the reaction was complete. Saturated sodium bicarbonate solution was added. The mixture was extracted, separated, and dried over anhydrous sodium sulfate. The organic phase was directly concentrated to give α-9d as a yellow oil (800 mg, 2.95 mmol, yield: 70%). The water was removed using an oil pump until dry, and the resulting product was directly used in the next step.
MS-ESI: [M+H]⁺ = 271.2
¹H NMR (400 MHz, DMSO-d₆) δ 8.54 (td, *J =* 1.8, 0.5 Hz, 1H), 8.35-8.28 (m, 1H), 8.26-8.17 (m, 1H), 7.73 (td, *J* = 7.8, 0.6 Hz, 1H), 5.87 (p, *J =* 6.2 Hz, 1H), 3.91 (s, 3H), 1.80 (d, *J* = 6.5 Hz, 3H).

### Step 4

In a dry 50 mL three-necked flask, compound α-9d (300.0 mg, 1.11 mmol) was dissolved in 6 mL of N,N-dimethylformamide and added to the three-necked flask. A1-1-1c (450 mg, 1.55 mmol) and cesium carbonate (840 mg, 2.58 mmol) were added and stirred under nitrogen protection at room temperature for 2 h. The reaction was monitored by LCMS for completion. Water and ethyl acetate were added, and the mixture was washed with saturated brine. The organic phase was concentrated to give a crude product. The crude product was dissolved in N,N-dimethylformamide and purified by MPLC to give α-9e as a yellow solid (310 mg, 0.64 mmol, yield: 41.5%).
MS-ESI: [M+H]⁺= 481.2
¹H NMR (400 MHz, DMSO-d₆) δ 8.55 (t, *J =* 1.8 Hz, 1H), 8.36 (dq, *J* = 7.8, 1.6 Hz, 1H), 8.26 (dt, *J =* 7.8, 1.4 Hz, 1H), 7.76 (t, *J* = 7.8 Hz, 1H), 7.69 (dt, *J =* 8.9, 2.3 Hz, 1H), 7.55 (ddd, *J =* 8.6, 6.1, 1.7 Hz, 1H), 7.42 (tdd, *J =* 8.5, 4.4, 2.5 Hz, 1H), 7.10 (dd, *J =* 6.4, 2.4 Hz, 1H), 6.98 -6.84 (m, 2H), 6.35-6.23 (m, 2H), 3.90 (s, 3H), 1.59 (dd, *J =* 6.8, 1.1 Hz, 3H).

### Step 5

In a dry 100 mL three-necked flask, α-9e (200.0 mg, 442 µmol) was dissolved in 1 mL of tetrahydrofuran, and 2 mL of aqueous hydrochloric acid solution (6M) was added. The mixture was heated to 100°C and stirred for 16 h. The reaction was monitored by LCMS for completion. The reaction solution was directly concentrated and rotary evaporated to give a crude product. The crude product was dissolved in N,N-dimethylformamide and purified by MPLC to give α-9 as a white solid (55.0 mg, 112.5 µmol, yield: 26%).
MS-ESI: [M+H]⁺ = 467.1
¹H NMR (400 MHz, DMSO-d6*)* δ 8.50 (t, *J =* 1.7 Hz, 1H), 8.15 (*dt, J =* 7.6, 1.4 Hz, 1H), 8.01 (dq, *J =* 7.8, 1.7 Hz, 1H), 7.68 (dt, *J =* 8.9, 2.7 Hz, 1H), 7.55 (ddd, *J =* 8.8, 6.1, 2.9 Hz, 1H), 7.47 (t, *J* = 7.6 Hz, 1H), 7.45-7.38 (m, 1H), 7.01 (dd, *J =* 4.6, 2.4 Hz, 1H), 6.94 (dd, *J =* 8.9, 1.5 Hz, 1H), 6.87 (ddd, J= 8.9, 6.4, 2.4 Hz, 1H), 6.29 *(d, J =* 1.4 Hz, 1H), 6.27-6.16 (m, 1H), 1.58 (dd, J = 6.7, 1.2 Hz, 3H).

### Preparation Example (I) 10: Compound α-10

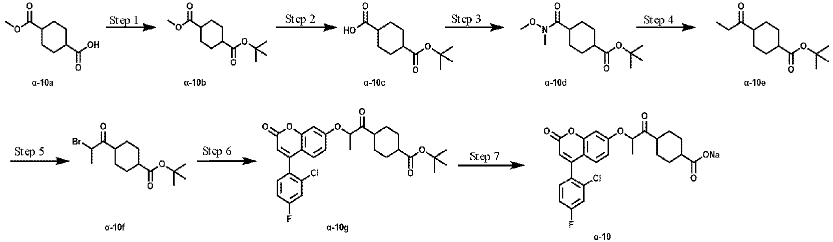

### Step 1

In a dry 100 mL three-necked flask, compound α-10a (5.00 g, 26.85 mmol) was dissolved in tert-butanol (70 mL). 4-Dimethylaminopyridine (1.31 g, 10.74 mmol) and di-tert-butyl dicarbonate (15.24 g, 69.82 mmol) were added. The reaction was carried out at room temperature for 2 h. The reaction was monitored by TLC for completion (PE:EA = 4:1, Rf = 0.77, iodine fumigation). The reaction solution was rotary evaporated, and then dissolved in ethyl acetate, washed successively with 1M hydrochloric acid, saturated sodium bicarbonate solution and saturated brine, dried over anhydrous sodium sulfate, and rotary evaporated to give the crude product α-10b as a white solid (6.4 g, 26.41 mmol, yield: 98%, purity: 80%).

¹H NMR (400 MHz, DMSO-*d*₆) δ 3.58 (s, 3H), 2.33-2.20 (m, 1H), 2.20-2.08 (m, 1H), 1.96-1.81 (m, 4H), 1.38 (s, 9H), 1.36-1.26 (m, 4H).

### Step 2

In a dry 250 mL three-necked flask, compound α-10b (6.40 g, 26.41 mmol) was dissolved in a mixed solvent of methanol (128 mL) and water (77 mL). Sodium hydroxide (5.28 g, 132.06 mmol) was added, and the reaction was carried out at room temperature for 1 h. Most of the methanol was removed by rotary evaporation under reduced pressure, and the residue was washed with ethyl acetate twice. The aqueous phase was adjusted to pH = 2-3 with concentrated hydrochloric acid and then extracted with ethyl acetate three times. The organic phases were combined, washed with saturated brine, dried over anhydrous sodium sulfate, and rotary evaporated to give α-10c as a white solid (5.46 g, 23.92 mmol, yield: 90%, purity: 100%).

¹H NMR (400 MHz, DMSO-d₆) δ 12.05 (s, 1H), 2.23- .04 (m, 2H), 1.97-1.79 (m, 4H), 1.38 (s, 8H), 1.34 - 1.26 (m, 4H).

### Step 3

In a dry 100 mL three-necked flask, compound α-10c (5.43 g, 23.79 mmol) and methoxymethanamine hydrochloride (2.55 g, 26.16 mmol) were dissolved in N,N-dimethylformamide (55 mL). Benzotriazole-N,N,N',N'-tetramethyluronium hexafluorophosphate (9.92 g, 26.16 mmol), 1-hydroxybenzotriazole (3.53 g, 26.16 mmol) and N,N-diisopropylethyl amine (15.37 g, 118.93 mmol) were added under an ice bath. The reaction was carried out at room temperature for 2 h. Water was added to quench the reaction. The mixture was extracted with ethyl acetate three times. The organic phases were combined, washed successively with 1M hydrochloric acid, 1M sodium hydroxide and saturated brine, dried over anhydrous sodium sulfate, and rotary evaporated to give a crude product. The crude product was purified by silica gel column chromatography (PE:EA = 4:1, Rf = 0.46, iodine fumigation) to give α-10d as a colorless oil (5.96 g, 21.96 mmol, yield: 92%, purity: 95%).

### Step 4

In a dry 100 mL three-necked flask, compound α-10d (5.46 g, 20.12 mmol) was dissolved in anhydrous tetrahydrofuran (23 mL) and added dropwise to ethylmagnesium bromide (2M, 25.15 mL, 50.30 mmol) under an ice-salt bath. After that, the reaction was carried out at room temperature for 1 h. 10% Hydrochloric acid was added to quench the reaction. The mixture was extracted with ethyl acetate three times. The organic phases were combined, washed successively with saturated sodium bicarbonate solution and saturated brine, dried over anhydrous sodium sulfate, and rotary evaporated to give a crude product. The crude product was purified by silica gel column chromatography (PE:EA = 4:1, Rf = 0.67, iodine fumigation) to give α-10e as a colorless oil (4.21 g, 17.52 mmol, yield: 87%, purity: 95%).

### Step 5

In a dry 50 mL three-necked flask, compound α-10e (500 mg, 2.08 mmol) was dissolved in methanol (7 mL). Then, 48% hydrobromic acid (15 µL) was added, and bromine (339 mg, 2.12 mmol, 108.7 µL) was added dropwise. After that, the reaction was carried out at room temperature for 16 h. TLC showed no starting material remaining (no color development of the product). The reaction solution was poured into saturated sodium bicarbonate solution to quench the reaction, and extracted with ethyl acetate three times. The organic phases were combined, washed with saturated brine, dried over anhydrous sodium sulfate, and rotary evaporated to give the crude compound α-10f as a pale yellow oil (596.0 mg, 1.87 mmol, yield: 89%, purity: 50%).

¹H NMR (400 MHz, Chloroform-d) δ 4.52 *(qd, J =* 6.8, 1.4 Hz, 1H), 2.89-2.76 (m, 1H), 2.37-2.13 (m, 1H), 2.12-1.90 (m, 4H), 1.73 (d, *J* = 6.8, Hz, 3H), 1.66-1.24 (m, 13H).

### Step 6

In a dry 50 mL three-necked flask, compound α-10f (494.0 mg, 1.55 mmol) and A1-1-1c (300 mg, 1.03 mmol) were dissolved in N,N-dimethylformamide (7.5 mL). Cesium carbonate (873 mg, 2.68 mmol) was added. The reaction was carried out at room temperature for 2 h and monitored by LCMS for completion. The mixture was diluted with water and extracted with ethyl acetate three times. The organic phases were combined, washed with saturated brine, dried over anhydrous sodium sulfate, and rotary evaporated to give a crude product. The crude product was purified by medium-pressure reverse phase chromatography to give α-10g as a white solid (211.0 mg, 379 µmol, yield: 38%, purity: 95%).
MS-ESI: [M+Na]⁺= 551.1

### Step 7

In a dry 50 mL three-necked flask, compound α-10g (180 mg, 340 µmol) was dissolved in dichloromethane (2.7 mL). Trifluoroacetic acid (0.9 mL) was added. The reaction was carried out at room temperature for 1 h and monitored by LCMS for completion. The reaction solution was rotary evaporated. The residue was dissolved in acetonitrile (1 mL) and adjusted to pH = 8-9 with saturated sodium bicarbonate. The mixture was purified by medium-pressure reverse phase chromatography to give α-10 as a white solid (45 mg, 91 µmol, yield: 26%, purity: 98.31%).
MS-ESI: [M-Na]⁻ = 471.2
¹H NMR (400 MHz, DMSO-d₆) δ 7.70 (dd, *J =* 8.6, 2.2 Hz, 1H), 7.57 (ddd, *J* = 8.6, 6.1, 1.4 Hz, 1H), 7.43 (tdd, *J =* 8.5, 2.6, 1.0 Hz, 1H), 6.99 (dd, *J =* 4.1, 2.5 Hz, 1H), 6.95 (dd, *J =* 8.9, 2.3 Hz, 1H), 6.88-6.81 (m, 1H), 6.31 (s, 1H), 5.36 (qd, *J =* 6.9, 4.1 Hz, 1H), 2.71-2.64 (m, 1H), 1.94 (dd, *J=* 26.4, 11.0 Hz, 2H), 1.85-1.83 (m, 1H), 1.77-1.71 (m, 1H), 1.65-1.63 (m, 1H), 1.47 (d, *J* = 6.8 Hz, 3H), 1.35-1.09 (m, 4H).

### Preparation Example (I) 11: Compound α-11

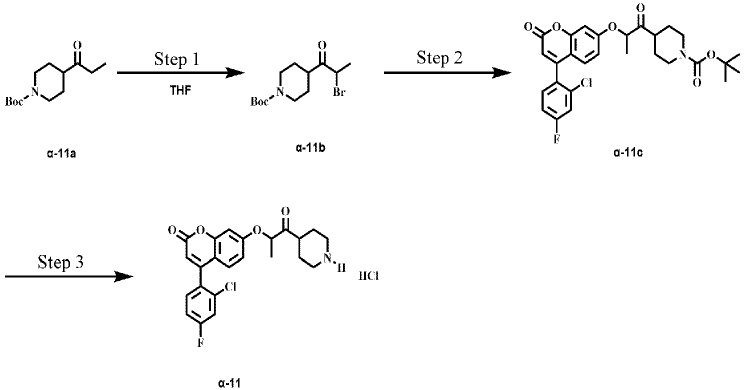

### Step 1

In a dry 100 mL three-necked flask, α-11a (2.0 g, 8.29 mmol) and tetrahydrofuran (30 mL) were successively added. After sufficient dissolution, pyridinium tribromide (2.92 g, 2.07 mmol) was added. After that, the reaction was carried out at room temperature overnight and monitored by TLC until the starting materials had completely reacted. After completion of the reaction, the reaction system was filtered. The filtrate was collected, concentrated using a rotary evaporator, and then separated and purified by a flash column to give α-11b (2.08 g, 6.50 mmol, yield: 78%).

### Step 2

In a dry 100 mL three-necked flask, α-11b (1.00 g, 3.12 mmol), A1-1-1c (760.0 mg, 2.61 mmol) and dry acetone (30 mL) were successively added. After sufficient dissolution, potassium carbonate (0.72 g, 5.21 mmol) and tetrabutylammonium iodide (0.96 g, 2.60 mmol) were successively added, and then the temperature was slowly raised to 25°C. The reaction was carried out at the maintained temperature for 6 h and monitored by TLC until the starting materials had completely reacted. After completion of the reaction, the reaction solution was washed with water (30 mL) and then extracted with ethyl acetate (50 mL × 3). The organic phases were combined, washed with saturated brine (100 mL), then dried over anhydrous sodium sulfate, concentrated using a rotary evaporator, and then separated and purified by a flash column to give α-11c (1.28 g, 2.42 mmol, yield: 92%).

### Step 3

In a dry and clean eggplant-shaped flask, α-11c (1.36 g, 2.56 mmol) was added. Then, a solution of hydrogen chloride in ethyl acetate (3.0 M, 20 mL) was slowly added dropwise and stirred at room temperature. The progress of the reaction was monitored by TLC. After completion of the reaction, a white suspension was obtained. A solid was filtered and dried to give the desired product α-11 (1.10 g, 2.36 mmol, yield: 92%, purity: 97%).
MS-ESI: [M+H]⁺ = 430.0
¹H NMR: (400 MHz, Methanol-d4) δ 7.50 - 7.42 (m, 2H), 7.29 (td, *J =* 8.3, 2.5 Hz, 1H), 7.04 (d, *J* = 8.6 Hz, 1H), 6.99 (s, 1H), 6.92 (d, *J =* 8.6 Hz, 1H), 6.23 (s, 1H), 5.24 (d, *J =* 6.7 Hz, 1H), 3.51 - 3.37 (m, 2H), 3.31 - 3.23 (m, 2H), 3.11 (m, 2H), 2.24 (d, *J =* 14.4 Hz, 1H), 1.95 (d, *J =* 14.3 Hz, 1H), 1.85 (t, *J =* 12.1 Hz, 2H), 1.56 *(d, J =* 6.4 Hz, 3H).

### Preparation Example (I) 12: Compound α-12

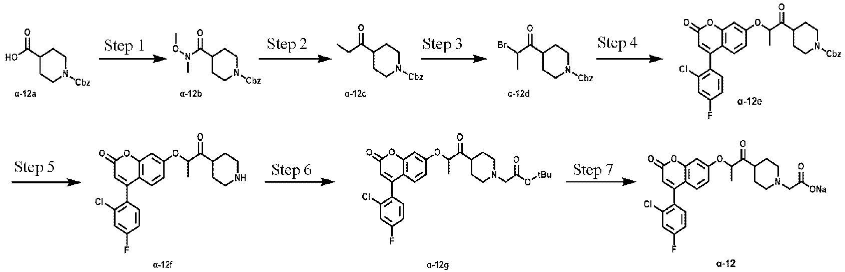

### Step 1

In a dry 100 mL three-necked flask, compound α-12a (5.00 g, 18.99 mmol) and methoxymethanamine hydrochloride (2.04 g, 20.89 mmol) were dissolved in N,N-dimethylformamide (50 mL). Benzotriazole-N,N,N:N' -tetramethyluronium hexafluorophosphate (7.92 g, 20.89 mmol), 1-hydroxybenzotriazole (2.82 g, 20.89 mmol) and N,N-diisopropylethyl amine (12.27 g, 94.95 mmol) were added under an ice bath. The reaction was carried out at room temperature for 16 h and monitored by LCMS for completion. Water was added to quench the reaction. The mixture was extracted with ethyl acetate three times. The organic phases were combined, washed successively with 1M hydrochloric acid, 1M sodium hydroxide and saturated brine, dried over anhydrous sodium sulfate, and rotary evaporated to give a crude product. The crude product was purified by medium-pressure reverse phase chromatography to give α-12b as a colorless oil (5.24 g, 17.10 mmol, yield: 90%, purity: 90%).
MS-ESI: [M+H]⁺ = 307.1

### Step 2

In a dry 100 mL three-necked flask, compound α-12b (5.30 g, 17.30 mmol) was dissolved in anhydrous tetrahydrofuran (21 mL) and added dropwise to ethylmagnesium bromide (2M in tetrahydrofuran, 21.63 mL, 43.25 mmol) under an ice-salt bath. After that, the reaction was carried out at room temperature for 1 h and monitored by TLC for completion (PE:EA = 4:1, Rf = 0.41). 10% Hydrochloric acid was added to quench the reaction. The mixture was extracted with ethyl acetate three times. The organic phases were combined, washed successively with saturated sodium bicarbonate and saturated brine, dried over anhydrous sodium sulfate, and rotary evaporated to give a crude product. The crude product was purified by silica gel column chromatography to give α-12c as a colorless oil (3.79 g, 13.76 mmol, yield: 79%, purity: 90%).
MS-ESI: [M+H]⁺ = 276.0

### Step 3

In a dry 50 mL three-necked flask, compound α-12c (500.0 mg, 1.82 mmol) was dissolved in methanol (7.5 mL). Then, 48% hydrobromic acid (15 µL) was added, and bromine (296.0.0 mg, 1.85 mmol, 95 µL) was added dropwise. After that, the reaction was carried out at room temperature for 16 h and monitored by LCMS for completion. The reaction solution was poured into saturated sodium bicarbonate solution to quench the reaction, and extracted with ethyl acetate three times. The organic phases were combined, washed with saturated brine, dried over anhydrous sodium sulfate, and rotary evaporated to give the crude product α-12d as a pale yellow oil (542.0 mg, 1.53 mmol, yield: 84%, purity: 70%).
MS-ESI: [M+H]⁺ = 354.0

### Step 4

In a dry 50 mL three-necked flask, compound α-12d (418 mg, 1.18 mmol, purity: 70%) and compound A1-1-1c (160 mg, 550 µmol) were dissolved in N,N-dimethylformamide (4 mL). Cesium carbonate (466 mg, 1.43 mmol) was added. The reaction was carried out at room temperature for 3 h and monitored by LCMS for completion. The mixture was diluted with water and extracted with ethyl acetate three times. The organic phases were combined, washed with saturated brine, dried over anhydrous sodium sulfate, and rotary evaporated to give a crude product. The crude product was purified by medium-pressure reverse phase chromatography to give α-12e as a white solid (251.0 mg, 455 µmol, yield: 82%, purity: 95%).
MS-ESI: [M+H]⁺ =564.1

### Step 5

In a dry 50 mL three-necked flask, compound α-12e (256 mg, 453.90 µmol) was dissolved in methanol (6 mL). Palladium hydroxide on carbon (78 mg, 20%) was added. After purging with hydrogen gas three times, the reaction was carried out at normal pressure and room temperature for 1 h and monitored by LCMS for completion. The mixture was filtered, and the filter cake was washed with methanol. The filtrates were combined and rotary evaporated to give a crude product. The crude product was purified by medium-pressure reverse phase chromatography to give α-12f as a white solid (132 mg, 307 µmol, yield: 67%, purity: 95%).
MS-ESI: [M+H⁺= 430.0

### Step 6

In a dry 50 mL three-necked flask, compound α-12f (110 mg, 255.89 µmol) was dissolved in N,N-dimethylformamide (2.2 mL). Tert-butyl bromoacetate (54.9.0 mg, 281.48 µmol) and triethylamine (28 mg, 281 µmol) were added. The reaction was carried out at room temperature for 2 h and monitored by LCMS for completion. The reaction solution was purified by medium-pressure reverse phase chromatography to give α-12g as a white solid (135 mg, 248 µmol, yield: 96%, purity: 99%).
MS-ESI: [M+H]⁺= 544.3, [M ^{t}Bu]⁺= 488.3

### Step 7

In a dry 50 mL three-necked flask, compound α-12g (110 mg, 202.2 µmol) was dissolved in dichloromethane (1.8 mL). Trifluoroacetic acid (0.6 mL) was added. The reaction was carried out at room temperature for 4 h and monitored by LCMS for completion. The reaction solution was rotary evaporated. The residue was dissolved in acetonitrile (1 mL) and adjusted to pH = 8-9 with saturated sodium bicarbonate. The mixture was purified by medium-pressure reverse phase chromatography to give α-12 as a white solid (42 mg, 82 µmol, yield: 40%, purity: 95.37%).

MS-ESI: [M-Na]⁻ = 486.2.

¹H NMR (400 MHz, DMSO-d₆) δ 7.70 (ddd, *J =* 8.9, 2.6, 1.0 Hz, 1H), 7.57 (ddd, *J =* 8.6, 6.1, 1.6 Hz, 1H), 7.43 (tdd, *J =* 8.5, 2.6, 1.2 Hz, 1H), 7.04 - 7.00 (m, 1H), 7.00 - 6.89 (m, 1H), 6.85 (ddd, *J =* 9.1, 6.9, 2.5 Hz, 1H), 6.32 (s, 1H), 5.38 (qd, *J =* 6.8, 3.5 Hz, 1H), 2.93 (dd, *J =* 24.8, 11.2 Hz, 2H), 2.73 (s, 2H), 2.71-2.64 (m, 1H), 2.15 - 1.99 (m, 2H), 1.88 (d, *J* = 12.5 Hz, 1H), 1.61 - 1.43 (m, 6H).

¹⁹F NMR (376 MHz, *DMSO-d₆DMSO-D6)* δ 109.97(s, 1F).

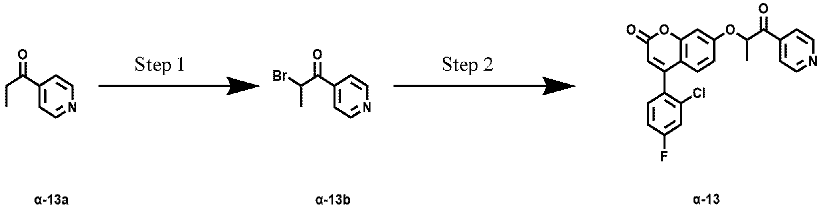

### Preparation Example (I) 13: Compound α-13

### Step 1

In a dry 50 mL three-necked flask, compound α-13a (500.0 mg, 3.70 mmol) was added. Then, hydrobromic acid (4 mL) was added, and bromine (621.0 mg, 3.88 mmol, 200 µL) was added dropwise to the flask at 15-20°C. After that, the mixture was stirred at 40°C for 1 h, heated to 80°C, and stirred for another 1 h. After the reaction was monitored by LCMS to be complete, the mixture was cooled to 25-30°C. Methyl tert-butyl ether (12 mL) was added to the reaction solution. The mixture was stirred at 25-30°C for 5 minutes and then filtered under vacuum. The filter cake was washed with methyl tert-butyl ether (10 mL) and dried to give a product. The crude product was used in the next step without purification. α-13b (900.0 mg, 3.05 mmol, yield: 82%, purity: 91.5%, HBr)
MS-ESI: [M+H]⁺ = 214.0

### Step 2

In a dry 50 mL three-necked flask, acetone (2.40 mL), α-13b (183.0 mg, 620 µmol, HBr) and A1-1-1c (120.0 mg, 413 µmol) were added. Diisopropylethyl amine (53.3 mg, 413 µmol, 71.9 µL, 1.00 eq) was added dropwise at 20°C and then stirred at 20°C for 16 h. After the reaction was monitored by LCMS to be complete, water (10 mL) was added to the reaction solution. The mixture was extracted with ethyl acetate (10 mL × 3) and washed with saturated brine (15 mL). The organic layer was dried over anhydrous sodium sulfate and concentrated under vacuum to give a crude product. The crude product was purified by Pre-HPLC (Column: Waters *bridge 150*25 mm 10 µm; Mobile phase: [water (ammonium bicarbonate)-acetonitrile; B%: 43%-73%, 14 minutes). The mixture was freeze-dried to give the product α-13 (60.0 mg, 142 µmol, yield: 34%, purity: 95.3%).
MS-ESI: [M+H]⁺= 424.1

### Second Set of Preparation Examples (Including Examples of the Second Set of Technical Solutions)

### Preparation Example (II) 1: Compound A1-1-1

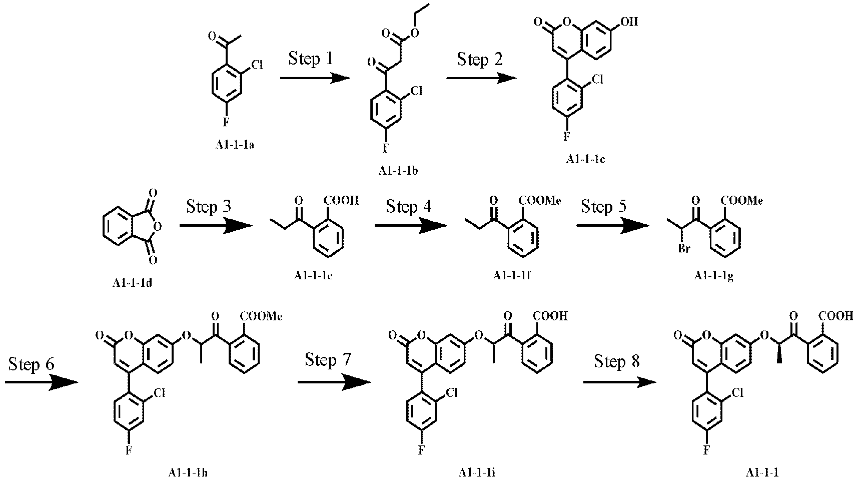

### Step 1

Diethyl carbonate (136.90 g, 1.16 mol) was dissolved in tetrahydrofuran (500 mL). The system was purged with nitrogen. Sodium hydride (23.18 g, 579.44 mmol) was added at 0°C, and the reaction was allowed to proceed for 1 h. Then, compound A1-1-1a (50 g, 289.72 mmol) was added. The reaction solution was transferred to 55°C and reacted for 8 h. The reaction solution was poured into 2 M aqueous hydrochloric acid solution (400 mL) and extracted with ethyl acetate. The extracted organic phase was washed with saturated brine (400 mL) twice and preliminary purified by column chromatography to give the crude product A1-1-1b, which was directly used in the next step.

### Step 2

The crude product A1-1-1b was dissolved in methanesulfonic acid (400 mL). Resorcinol (45.01 g, 408.75 mmol) was added. The reaction was carried out at 45°C for 3 h to give a black viscous reaction solution. TLC showed that the starting materials had been completely consumed. The reaction solution was slowly poured into ethanol (200 mL), and then the resulting system was poured into water (500 mL). The mixture was extracted with ethyl acetate (300 mL × 3). The organic phases were combined, washed with saturated brine (500 mL) once, dried over anhydrous sodium sulfate, and purified by column chromatography to give A1-1-1c (40.00 g, 137.61 mmol, total yield for two steps: 47.5%).

¹HNMR(400 MHz, Chloroform-d) 7.35-7.29(m, 1 H), 7.26 - 7.21 (m, 1 H), 7.20 - 7.10 (m, 1 H), 7.02 - 7.08 (m, 1 H), 6.76 - 6.82 (m, 1 H), 6.40 - 6.52 (m, 1 H), 6.21 (s, 1 H), 5.51 (s, 1 H).

### Step 3

In a 250 mL three-necked flask under nitrogen protection, A1-1-1d (13.32 g, 89.93 mmol), cuprous bromide (0.91 g, 6.36 mmol) and anhydrous tetrahydrofuran (120 mL) were successively added. After sufficient dissolution, the system was cooled to -20°C. Then, a solution of ethylmagnesium bromide in tetrahydrofuran (50 mL, 2.0 M) was slowly added dropwise. After completion of the dropwise addition, the reaction was carried out at this temperature for 1 h. Then, the temperature was slowly raised to 30°C, and the reaction was allowed to proceed for 2 h. The reaction was monitored by TLC until the starting materials had completely reacted. Then, saturated aqueous ammonium chloride solution (100 mL) was added to quench the reaction. The mixture was extracted with ethyl acetate (50 mL × 3). The organic phases were combined, washed with saturated brine, dried over anhydrous sodium sulfate, concentrated using a rotary evaporator, and then separated and purified by a flash column to give A1-1-1e (13.0 g, 72.96 mmol, yield: 81%).
MS-ESI: [M-H]⁻= 177.1

### Step 4

In a 100 mL three-necked flask, A1-1-1e (13.0 g, 72.96 mmol), anhydrous potassium carbonate (20.17 g, 145.92 mmol) and N,N-dimethylformamide (50 mL) were successively added. The reaction solution was heated to 60°C. Iodomethane (20.71 g, 145.92 mmol) was slowly added dropwise. After completion of the dropwise addition, the reaction was carried out at this temperature for 6 h and monitored by TLC until the starting materials had completely reacted. Then, the reaction solution was diluted with water (50 mL) and extracted with ethyl acetate (50 mL × 3). The organic phases were combined, washed with saturated brine, dried over anhydrous sodium sulfate, concentrated using a rotary evaporator, and then separated and purified by a flash column to give A1-1-1f (5.9 g, 30.70 mmol, yield: 42%).

¹H NMR (400 MHz, Chloroform-d) δ 7.93 (dd, *J =* 7.7, 1.4 Hz, 1H), 7.59 (td, *J =* 7.5, 1.3 Hz, 1H), 7.51 (td, *J =* 7.6, 1.4 Hz, 1H), 7.36 (dd, *J =* 7.5, 1.4 Hz, 1H), 3.91 (s, 3H), 2.83 (q, *J* = 7.3 Hz, 2H), 1.25 (t, *J* = 7.3 Hz, 3H).

### Step 5

In a 100 mL three-necked flask, A1-1-1f (5.9 g, 30.70 mmol) and dichloromethane (15 mL) were added at room temperature. Hydrogen bromide (1.86 mL, 34.24 mmol) and bromine (4.38 mL, 85.60 mmol) were successively and slowly added dropwise. The reaction was carried out at room temperature for 4 h and monitored by TLC until the starting materials had completely reacted. Then, the reaction solution was quenched with saturated aqueous sodium thiosulfate solution (100 mL) and extracted with dichloromethane (30 mL × 3). The organic phases were combined, washed with saturated brine, dried over anhydrous sodium sulfate, concentrated using a rotary evaporator, and then separated and purified by a flash column to give A1-1-1g (1.7 g, 6.27 mmol, yield: 20%). ¹H NMR (400 MHz, Chloroform-d) δ 8.02 (dd, *J =* 8.1, 1.3 Hz, 1H), 7.65 (td, *J =* 7.5, 1.4 Hz, 1H), 7.55 (ddt, *J =* 8.7, 3.8, 1.5 Hz, 2H), 4.85 (q, *J* = 6.6 Hz, 1H), 3.93 (s, 3H), 2.00 (d, *J* = 6.6 Hz, 3H).

### Step 6

In a 100 mL three-necked flask, A1-1-1g (1.7 g, 6.27 mmol) and acetone (5 mL) were added at room temperature. Then, A1-1-1c (1.92 g, 6.61 mmol), tetrabutylammonium iodide (2.44 g, 6.61 mmol) and anhydrous potassium carbonate (1.83 g, 13.22 mmol) were successively added and stirred at room temperature overnight. The reaction was monitored by TLC until the starting materials had completely reacted. Then, the reaction solution was diluted with water (25 mL) and extracted with ethyl acetate (30 mL × 3). The organic phases were combined, washed with saturated brine, dried over anhydrous sodium sulfate, concentrated using a rotary evaporator, and then separated and purified by a flash column to give A1-1-1h (1.17 g, 2.43 mmol, yield: 39%). MS-ESI: [M+H]⁺ = 481.2

### Step 7

In a 100 mL three-necked flask, A1-1-1 h (1.17 g, 2.43 mmol) and methanol (20 mL) were added. Then, aqueous sodium hydroxide solution (20 mL, 0.5 M) was added, and the reaction was carried out at this temperature for 3 h. The reaction was monitored by TLC until the starting materials had completely reacted. Then, the reaction solution was adjusted to pH 2-3 with dilute aqueous hydrochloric acid solution (1.0 M) and extracted with ethyl acetate (30 mL × 3). The organic phases were combined, washed with saturated brine, dried over anhydrous sodium sulfate, concentrated using a rotary evaporator, and then separated and purified by a flash column to give A1-1-1i (290.0 mg, 0.62 mmol, yield: 25%).

### Step 8

Compound A1-1-1i was resolved by SFC to give A1-1-1. Instrument: SFC-80 (Waters); Model of resolution column: CHIRALPAK AD (30*250 mm 5 µm) (Daicel); Resolution conditions: ethanol (0.2% TFA), 75% CO₂; Flow rate: 45 mL:min. ESI-LCMS: m:z 467.0[M+H]⁺. ¹H NMR (400 MHz, DMSO-d₆) δ 8.40 (d, *J =* 31.2 Hz, 1H), 7.88 - 7.66 (m, 4H), 7.63 - 7.51 (m, 1H), 7.43 (d, *J =* 8.6 Hz, 1H), 7.10 (s, 1H), 6.94 - 6.63 (m, 2H), 6.29 (d, *J =* 6.4 Hz, 1H), 5.12 (dd, *J =* 86.7, 6.7 Hz, 1H), 1.39 (dd, *J =* 40.3, 6.2 Hz, 3H).

### Preparation Example (II) 2: Compound A2-1-1

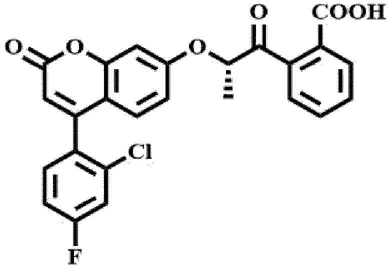

Compound A1-1-1i was resolved by SFC to give A2-1-1. Instrument: SFC-80 (Waters); Model of resolution column: CHIRALPAK AD (30×250 mm 5 µm) (Daicel); Resolution conditions: ethanol (0.2% TFA), 75% CO₂; Flow rate: 45 mL:min.

### Preparation Example (II) 3: Compound A1-1-5

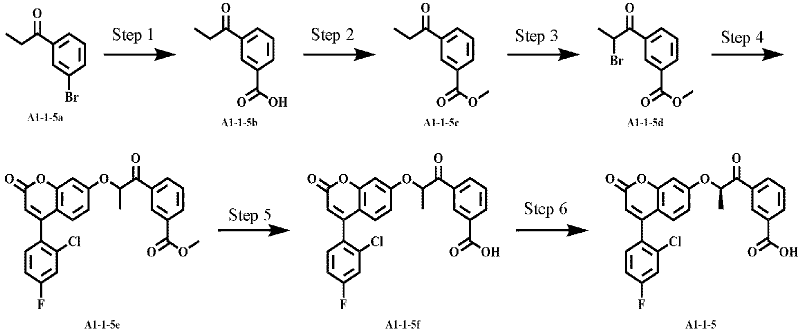

### Step 1

Palladium acetate (0.63 g, 2.82 mmol) and XantPhos (1.63 g, 2.82 mmol) were added to a dry three-necked flask. After nitrogen protection, A1-1-5a (20 g, 93.87 mmol), *N*,*N*-dimethylformamide (250 mL) and DCC (3.87 g, 18.77 mmol) were added. The system was put under nitrogen protection again. Formic acid (30.25 g, 657.09 mmol) and triethylamine (19.00 g, 26.02 mmol) were added, and the reaction was carried out at 110°C for 12 h. TLC showed that the starting materials had basically reacted completely. Water (500 mL) and methyl tert-butyl ether (500 mL) were added. The mixture was stirred uniformly, filtered through diatomite, and separated. The aqueous phase was extracted three times with methyl tert-butyl ether (300 mL × 3). The organic phases were combined and washed with saturated aqueous sodium chloride solution (500 mL). The organic phase was dried over anhydrous sodium sulfate, filtered, and concentrated to give the crude product A1-1-5b, which was directly used in the next step.

### Step 2

The crude product A1-1-5b was dissolved in methanol (200 mL). DMAP (5.14 g, 42.09 mmol), N,N-diisopropylethyl amine (21.76 g, 168.36 mmol) and 1-ethyl-(3-dimethylaminopropyl)carbodiimide hydrochloride (24.21 g, 126.27 mmol) were added. The reaction was carried out at room temperature for 12 h. TLC showed that the starting materials had basically reacted completely. After concentration, ethyl acetate (200 mL) and saturated aqueous sodium chloride solution (200 mL) were added, and the layers were separated. The aqueous phase was extracted with ethyl acetate (100 mL × 2) twice. The organic phases were combined and washed successively with 1 M aqueous hydrogen chloride solution (100 mL), saturated aqueous sodium chloride solution (200 mL), saturated aqueous sodium bicarbonate solution (200 mL) and saturated aqueous sodium chloride solution (200 mL). The resulting organic phase was dried over anhydrous sodium sulfate, concentrated, and purified by column chromatography to give A1-1-5c (8.00 g, 41.62 mmol, total yield for two steps: 44%).

### Step 3

A1-1-5c (8.00 g, 41.62 mmol) was dissolved in tetrahydrofuran (100 mL). Pyridinium tribromide (26.62 g, 83.24 mmol) was added. The reaction was carried out at 45°C for 3 h. TLC showed that the starting materials had basically reacted completely. After filtration and concentration, ethyl acetate (100 mL) and saturated aqueous sodium chloride solution (100 mL) were added, and the layers were separated. The aqueous phase was extracted with ethyl acetate (50 mL × 2) twice. The organic phases were combined, dried over anhydrous sodium sulfate, concentrated, and purified by column chromatography to give A1-1-5d (6.50 g, 23.98 mmol, yield: 57%).

### Step 4

A1-1-5d (6.5 g, 23.98 mmol), A1-1-1c (6.97 g, 23.98 mmol) and potassium carbonate (8.29 g, 59.95 mmol) were added to acetone (100 mL), and the reaction was carried out at room temperature overnight. TLC showed that the starting materials had basically reacted completely. After concentration, ethyl acetate (100 mL) and water (100 mL) were added, and the layers were separated. The aqueous phase was extracted with ethyl acetate (100 mL × 2) twice. The organic phases were combined, washed with 1 M aqueous hydrogen chloride solution (100 mL), dried over anhydrous sodium sulfate, filtered, concentrated, and purified by column chromatography to give A1-1-5e (7.01 g, 14.58 mmol, yield: 60%).

¹H NMR (400 MHz, Chloroform-d) δ 8.61 (q, *J* = 1.6 Hz, 1H), 8.18 (ddt, *J* = 21.2, 7.9, 1.6 Hz, 2H), 7.53 (t, *J =* 7.8 Hz, 1H), 7.21 - 7.16 (m, 2H), 7.11 - 7.00 (m, 1H), 6.87 (dd, *J =* 8.8, 3.8 Hz, 1H), 6.70 (ddd, *J =* 18.7, 9.6, 2.5 Hz, 2H), 6.08 (d, *J =* 1.1 Hz, 1H), 5.58 (q, *J =* 6.9 Hz, 1H), 3.88 (s, 3H), 1.70 (d, *J =* 6.9 Hz, 3H).

### Step 5

A1-1-5e (3 g, 6.24 mmol) was dissolved in a mixed solution of methanol (50 mL) and tetrahydrofuran (100 mL). Then, 2 M aqueous sodium hydroxide solution (50 mL) was added under an ice bath. The reaction was allowed to proceed for 25 min. TLC showed that the reaction was complete. After the addition of 1 M hydrochloric acid (120 mL), the mixture was extracted with ethyl acetate (200 mL). Then, the aqueous phase was washed with ethyl acetate (150 mL × 2) twice. The organic phases were combined, washed with saturated sodium chloride (150 mL), dried over anhydrous sodium sulfate, filtered, concentrated, and purified by column chromatography to give A1-1-5f (1.34 g, 2.87 mmol, yield: 46%, purity: 96.45%).

### Step 6

Compound A1-1-5f was resolved by SFC to give A1-1-5. Instrument: SFC-80 (Waters); Model of resolution column: CHIRALPAK AD (30*250 mm 5 µm) (Daicel); Resolution conditions: ethanol (0.2% TFA), 75% CO₂; Flow rate: 45 mL:min.

¹ HNMR: (400 MHz, Chloroform-d), 8.81 (s, 1H), 8.35 (dd, *J =* 26.9, 7.8 Hz, 2H), 7.68 (t, *J =* 7.8 Hz, 1H), 7.33 - 7.28 (m, 2H), 7.17 - 7.10 (m, 1H), 6.97 (dd, *J =* 8.8, 4.0 Hz, 1H), 6.81 (dd, *J* = 11.2, 5.2, 2H), 6.20 (s, 1H), 5.65 (q, *J =* 6.8 Hz, 1H), 1.82 (d, *J =* 6.8 Hz, 3H).

MS (ES): for C₂₅H₁₆ClFO₆, theoretical value: 466.1; measured value: 465.0 (M-H)⁻.

### Preparation Example (II) 4: Compound A2-1-5

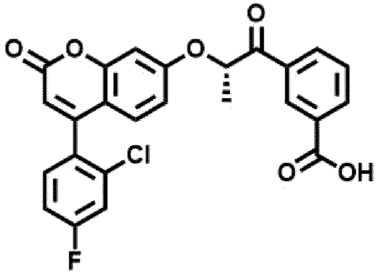

Compound A1-1-5f was resolved by SFC to give A2-1-5. Instrument: SFC-80 (Waters); Model of resolution column: CHIRALPAK AD (30×250 mm 5 µm) (Daicel); Resolution conditions: ETOH ethanol (0.2% FATFA), 75% CO₂; Flow rate: 45 mL:min.

### Preparation Example (II) 5: Compound A1-1-6

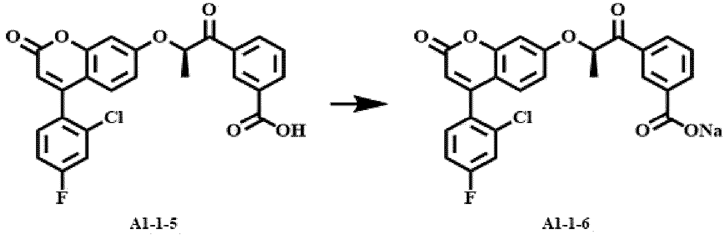

A1-1-5 (100.0 mg, 214 µM) was dissolved in N,N-dimethylformamide (0.5 mL). Then, aqueous sodium carbonate solution was added to adjust the solution to alkaline. The mixture was purified by medium-pressure reverse phase chromatography (MeCN:H₂0) to give A1-1-6 as a white solid (63.0 mg, 129 µM, yield: 60%). ¹H NMR (400 MHz, DMSO-d₆) δ 8.50 (t, *J =* 1.7 Hz, 1H), 8.15 (dt, *J =* 7.6, 1.4 Hz, 1H), 8.01 (dq, *J =* 7.8, 1.7 Hz, 1H), 7.68 (dt, *J =* 8.9, 2.7 Hz, 1H), 7.55 (ddd, *J* = 8.8, 6.1, 2.9 Hz, 1H), 7.47 (t, *J =* 7.6 Hz, 1H), 7.45 - 7.38 (m, 1H), 7.01 (dd, *J =* 4.6, 2.4 Hz, 1H), 6.94 (dd, *J =* 8.9, 1.5 Hz, 1H), 6.87 (ddd, *J =* 8.9, 6.4, 2.4 Hz, 1H), 6.29 (d, *J =* 1.4 Hz, 1H), 6.27 - 6.16 (m, 1H), 1.58 (dd, *J* = 6.7, 1.2 Hz, 3H). MS (ES): for the free acid C₂₅H₁₆ClFO₆ of C₂₅H₁₅ClFO₆Na, theoretical value: 466.1; measured value: 467.0 (M+H)⁺.

### Preparation Example (II) 6: Compound A1-1-9

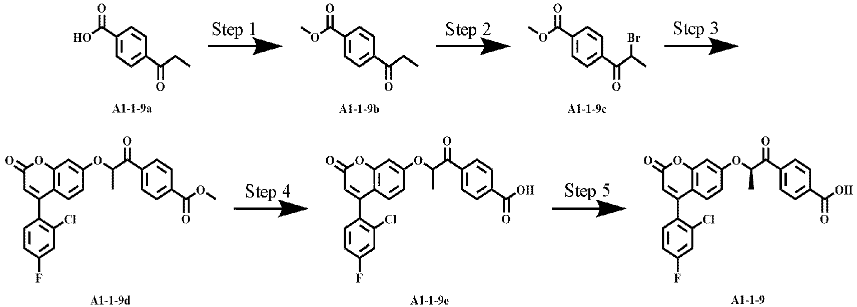

### Step 1

In a dry 100 mL three-necked flask, A1-1-9a (2.0 g, 11.22 mmol) and dry N,N-dimethylformamide (30 mL) were successively added. After sufficient dissolution, iodomethane (4.78 g, 33.66 mmol) and sodium bicarbonate (2.83 g, 33.66 mmol) were successively added, and then the temperature was slowly raised to 30°C. The reaction was carried out at the maintained temperature for 6 h and monitored by TLC until the starting materials had completely reacted. After completion of the reaction, water (50 mL) was added to the reaction solution. Then, the mixture was extracted with ethyl acetate (100 mL × 2). The organic phases were combined, washed successively with water (50 mL × 2) and saturated brine, dried over anhydrous sodium sulfate, concentrated under reduced pressure, concentrated using a rotary evaporator, and then separated and purified by a flash column to give A1-1-9b (2.0 g, 10.41 mmol, yield: 92%).

### Step 2

In a dry 100 mL three-necked flask, A1-1-9b (2.0 g, 10.41 mmol) and dry tetrahydrofuran (30 mL) were successively added. After sufficient dissolution, 2-pyrrolidone (1.06 g, 12.49 mmol) and pyrrolidone hydrotribromide (5.09 g, 15.62 mmol) were successively added, and then the temperature was slowly raised to 50°C. The reaction was carried out at the maintained temperature for 3 h and monitored by TLC until the starting materials had completely reacted. After completion of the reaction, the reaction solution was added dropwise to saturated aqueous sodium metabisulfite solution (20 mL) and stirred for 5 min. Then, water (50 mL) was added, and the mixture was extracted with ethyl acetate (50 mL × 3). The organic phases were combined, washed with saturated brine (50 mL × 2), then dried over anhydrous sodium sulfate, concentrated using a rotary evaporator, and then separated and purified by a flash column to give A1-1-9c (2.10 g, 7.75 mmol, yield: 74%).

### Step 3

In a dry 100 mL three-necked flask, A1-1-9c (2.00 g, 7.38 mmol), A1-1-1c (2.13 g, 7.38 mmol) and dry acetone (30 mL) were successively added. After sufficient dissolution, potassium carbonate (2.04 g, 14.76 mmol) and tetrabutylammonium iodide (2.73 g, 7.38 mmol) were successively added, and then the temperature was slowly raised to 25°C. The reaction was carried out at the maintained temperature for 6 h and monitored by TLC until the starting materials had completely reacted. After completion of the reaction, water (50 mL) was added to the reaction solution. Then, the mixture was extracted with ethyl acetate (50mL × 3). The organic phases were combined, washed with saturated brine (100 mL), dried over anhydrous sodium sulfate, concentrated using a rotary evaporator, and then separated and purified by a flash column to give A1-1-9d (2.14 g, 4.45 mmol, yield: 60%).

### Step 4

In a dry 100 mL three-necked flask, A1-1-9d (1.0 g, 2.08 mmol) and tetrahydrofuran (30 mL) were successively added. After sufficient dissolution, aqueous NaOH solution (2.0 M, 15 mL) was slowly added dropwise. Then, methanol (10 mL) was added to the reaction solution to aid dissolution. After that, the reaction was carried out at room temperature for 10 minutes and monitored by TLC until the starting materials had completely reacted. After completion of the reaction, the reaction solution was adjusted to acidic (pH: 3-5) with dilute aqueous hydrochloric acid solution (1.0 M) and then extracted with ethyl acetate (50 mL × 3). The organic phases were combined, washed with saturated brine (100 mL), dried over anhydrous sodium sulfate, concentrated using a rotary evaporator, and then separated and purified by a flash column to give compound A1-1-9e (380.0 mg, 0.81 mmol, yield: 39%).

### Step 5

Compound A1-1-9e was resolved by SFC to give A1-1-9. Instrument: SFC-80 (Waters); Model of resolution column: CHIRALPAK AD (30*250 mm 5 µm) (Daicel); Resolution conditions: ethanol (0.2% TFA), 75% CO₂; Flow rate: 45 mL:min.
MS-ESI: [M+H]⁺ = 467.2
¹H NMR (400 MHz, DMSO-d₆) δ 12.99 (s, 1H), 7.94 - 7.86 (m, 2H), 7.66 - 7.61 (m, 1H), 7.57 - 7.33 (m, 4H), 7.13 (dd, *J =* 8.2, 2.4 Hz, 1H), 6.69 (s, 1H), 6.33 - 6.28 (m, 1H), 6.21 (s, 1H), 4.61 (m, 1H), 1.34 - 1.22 (m, 3H).

### Preparation Example (II) 7: Compound A2-1-9

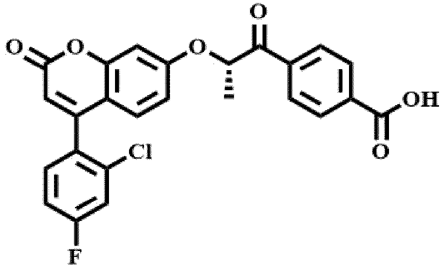

Compound A1-1-9e was resolved by SFC to give A2-1-9. Instrument: SFC-80 (Waters); Model of resolution column: CHIRALPAK AD (30×250 mm 5 µm) (Daicel); Resolution conditions: ETOH ethanol (0.2% FATFA), 75% CO₂; Flow rate: 45 mL:min.

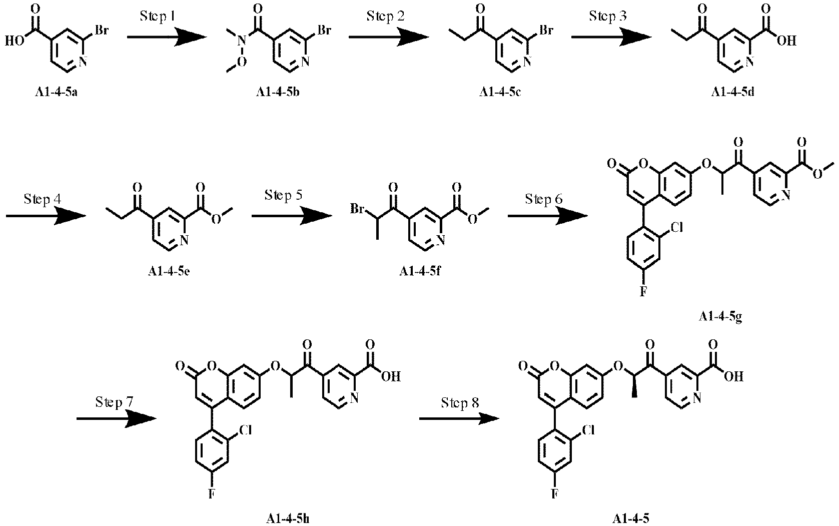

### Preparation Example (II) 8: Compound A1-4-5

### Step 1

In a dry 500 mL three-necked flask, A1-4-5a (13.0 g, 64.35 mmol) and dry dichloromethane (130 mL) were successively added. After sufficient dissolution, methoxymethylamine hydrochloride (6.28 g, 64.35 mmol), 2-(7-azabenzotriazole)-N,N,N',N'-tetramethyluronium hexafluorophosphate (29.36 g, 77.22 mmol) and triethylamine (16.28 g, 160.88 mmol) were successively added. After that, the reaction was carried out at room temperature for 16 h and monitored by TLC until the starting materials had completely reacted. After completion of the reaction, water (150 mL) was added to the reaction solution. Then, the mixture was extracted with dichloromethane (200 mL × 3). The organic phases were combined, washed successively with purified water (300 mL) and saturated brine (300 mL), then dried over anhydrous sodium sulfate, concentrated using a rotary evaporator, and then separated and purified by a flash column to give A1-4-5b (14.3 g, 58.36 mmol, yield: 90%).

### Step 2

In a dry 500 mL three-necked flask, A1-4-5b (14.00 g, 57.13 mmol) and dry tetrahydrofuran (150 mL) were successively added. After sufficient dissolution, the system was purged with N₂ three times and cooled to -15°C. Ethylmagnesium bromide (11.2 mL, 85.70 mmol, 2.0 M in tetrahydrofuran) was slowly added dropwise, and then the temperature was slowly raised to room temperature. The reaction was carried out for 3 h and monitored by TLC until the starting materials had completely reacted. After completion of the reaction, the reaction solution was added dropwise to saturated aqueous ammonium chloride solution (50 mL) and stirred for 5 min. Then, water (50 mL) was added, and the mixture was extracted with ethyl acetate (150 mL × 3). The organic phases were combined, washed with saturated brine (100 mL), then dried over anhydrous sodium sulfate, concentrated using a rotary evaporator, and then separated and purified by a flash column to give A1-4-5c (8.66 g, 40.46 mmol, yield: 70%).

### Step 3

In a dry 250 mL three-necked flask, A1-4-5c (8.50 g, 39.71 mmol), Xantphos (4.60 g, 7.94 mmol), DCC (16.39 g, 79.42 mmol) and Pb(OAc)₂ (1.78 g, 7.94 mmol) were successively added. Then, dry N,N-dimethylformamide (150 mL) was added. The system was purged with nitrogen three times. Triethylamine (16.5 mL, 119.13 mmol) and formic acid (3 mL, 79.42 mmol) were successively and slowly added dropwise to the reaction solution, and then the temperature was slowly raised to 100°C. The reaction was carried out at the maintained temperature for 6 h and monitored by TLC until the starting materials had completely reacted. After completion of the reaction, water (100 mL) was added to the reaction solution. Then, the mixture was extracted with ethyl acetate (100 mL × 3). The organic phases were combined, washed with saturated brine (100 mL), then dried over anhydrous sodium sulfate, concentrated using a rotary evaporator, and then separated and purified by a flash column to give A1-4-5d (5.13 g, 29.64 mmol, yield: 72%), which was directly used in the next step.

### Step 4

In a dry 250 mL three-necked flask, A1-4-5d (5.00 g, 27.91 mmol) and dry N,N-dimethylformamide (80 mL) were successively added. After sufficient dissolution, iodomethane (5.94 g, 41.87 mmol) and potassium carbonate (7.71 g, 55.82 mmol) were successively added, and then the temperature was slowly raised to 25°C. The reaction was carried out at the maintained temperature for 6 h and monitored by TLC until the starting materials had completely reacted. After completion of the reaction, water (80 mL) was added to the reaction solution. Then, the mixture was extracted with ethyl acetate (100 mL × 3). The organic phases were combined, washed successively with purified water (100 mL × 2) and saturated brine (200 mL), then dried over anhydrous sodium sulfate, concentrated using a rotary evaporator, and then separated and purified by a flash column to give A1-4-5e (1.47 g, 7.61 mmol, yield: 27%).

### Step 5

In a dry 100 mL three-necked flask, A1-4-5e (1.40 g, 7.25 mmol) and dry tetrahydrofuran (30 mL) were successively added. After sufficient dissolution, 2-pyrrolidone (0.74 g, 8.70 mmol) and pyrrolidone hydrotribromide (3.54 g, 10.88 mmol) were successively added, and then the temperature was slowly raised to 50°C. The reaction was carried out at the maintained temperature for 3 h and monitored by TLC until the starting materials had completely reacted. After completion of the reaction, the reaction solution was added dropwise to saturated aqueous sodium metabisulfite solution (20 mL) and stirred for 5 min. Then, water (50 mL) was added, and the mixture was extracted with ethyl acetate (50 mL × 3). The organic phases were combined, washed with saturated brine (100 mL), then dried over anhydrous sodium sulfate, concentrated using a rotary evaporator, and then separated and purified by a flash column to give A1-4-5f (1.49 g, 5.47 mmol, yield: 76%).

### Step 6

In a dry 100 mL three-necked flask, A1-4-5f (1.40 g, 5.15 mmol), A1-1-1c (1.49 g, 5.15 mmol) and dry acetone (20 mL) were successively added. After sufficient dissolution, potassium carbonate (1.42 g, 10.30 mmol) and tetrabutylammonium iodide (1.90 g, 5.15 mmol) were successively added, and then the temperature was slowly raised to 25°C. The reaction was carried out at the maintained temperature for 6 h and monitored by TLC until the starting materials had completely reacted. After completion of the reaction, the reaction solution was washed with water (30 mL) and then extracted with ethyl acetate (50 mL × 3). The organic phases were combined, washed with saturated brine (100 mL), then dried over anhydrous sodium sulfate, concentrated using a rotary evaporator, and then separated and purified by a flash column to give A1-4-5g (1.35 g, 2.80 mmol, yield: 54%).

### Step 7

In a dry 100 mL three-necked flask, A1-4-5g (1.35 g, 2.80 mmol) and tetrahydrofuran (30 mL) were successively added. After sufficient dissolution, aqueous lithium hydroxide solution (1.0 M, 15 mL) was slowly added dropwise. Then, methanol (10 mL) was added to the reaction solution to aid dissolution. After that, the reaction was carried out at room temperature for 2 h and monitored by TLC until the starting materials had completely reacted. After completion of the reaction, the reaction solution was adjusted to acidic (pH: 4-5) with formic acid and then extracted with ethyl acetate (50 mL × 3). The organic phases were combined, washed with saturated brine (100 mL), then dried over anhydrous sodium sulfate, concentrated using a rotary evaporator, and then separated and purified by a flash column to give compound A1-4-5h (730 mg, 1.56 mmol, yield: 55%).
MS-ESI: [M+H]⁺= 468.0

### Step 8

Compound A1-4-5h was resolved by SFC to give A1-4-5. Instrument: SFC-80 (Waters); Model of resolution column: CHIRALPAK AD (30*250 mm 5 µm) (Daicel); Resolution conditions: ethanol (0.2% TFA), 75% CO₂; Flow rate: 45 mL:min.

### Preparation Example (II) 9: Compound A2-4-5

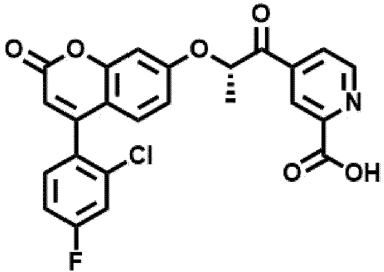

Compound A1-4-5h was resolved by SFC to give A2-4-5. Instrument: SFC-80 (Waters); Model of resolution column: CHIRALPAK AD (30×250 mm 5 µm) (Daicel); Resolution conditions: ETOH ethanol (0.2% FATFA), 75% CO₂; Flow rate: 45 mL:min.

### Third Set of Preparation Examples (Including Examples of the Third Set of Technical Solutions)

### Preparation Example (III) 1: Compound A-1-8-1-1

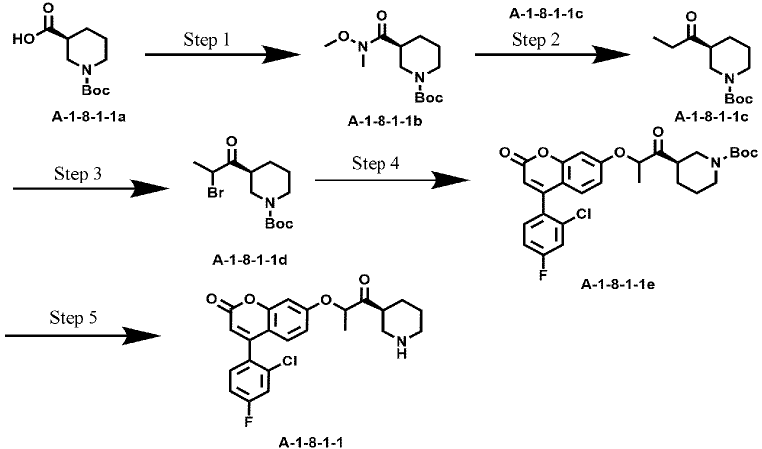

### Step 1

In a dry 100 mL three-necked flask, compound A-1-8-1-1a (5.98 g, 26.08 mmol) was dissolved in dichloromethane (20 mL). Then, N,O-dimethylhydroxylamine hydrochloride (3.03 g, 31.30 mmol), triethylamine (7.5 mL, 57.34 mmol) and 1-[bis(dimethylamino)methylene]-1H-1,2,3-triazolo[4,5-b]pyridinium 3-oxide hexafluorophosphate (11.89 g, 31.30 mmol) were added successively. The reactants were allowed to react at room temperature for 1 h. The reaction was monitored by TLC until the starting materials had completely reacted. Water (40 mL) was added to quench the reaction. Then, the mixture was extracted with ethyl acetate (50 mL × 3). The organic phases were combined, washed with saturated brine (100 mL), then dried over anhydrous sodium sulfate, concentrated using a rotary evaporator, and separated and purified by a flash column to give a concentrated organic phase. The concentrated organic phase was subjected to normal phase purification (PE:EA = 5:1, Rf = 0.4) to give the desired product A-1-8-1-1b as a colorless oil (6.50 g, 23.89 mmol, yield: 91.67%).

### Step 2

In a dry 100 mL three-necked flask, compound A-1-8-1-1b (6.50 g, 23.89 mmol) was dissolved in tetrahydrofuran (30 mL). Under nitrogen protection, ethylmagnesium bromide (28.66 mL, 28.66 mmol) was added dropwise. Then, the reaction was carried out at room temperature for 16 h.The reaction was monitored by TLC until the starting materials had completely reacted. 20 mL of saturated ammonium chloride was added to quench the reaction. Then, the mixture was extracted with ethyl acetate (50 mL × 3). After that, the organic phase was washed with saturated brine, dried over anhydrous sodium sulfate, rotary evaporated, and subjected to normal phase purification (PE:EA = 5:1, Rf = 0.6) to give the desired product A-1-8-1-1c as a colorless oil (5.00 g, 20.72 mmol, yield: 86.84%).

### Step 3

In a dry 100 mL three-necked flask, compound A-1-8-1-1c (1 g, 4.15 mmol) was dissolved in tetrahydrofuran (30 mL), and pyridinium tribromide (1.99 g, 6.22 mmol) was added. Then, the reaction was carried out at room temperature for 16 h. The spot plate test of the reaction solution indicated the formation of a new spot (PE:EA = 10:1, Rf = 0.7), and small amount of starting materials remained. H₂O (20 mL) was added to quench the reaction. Then, the mixture was extracted with ethyl acetate (20 mL × 3). After that, the organic phase was washed with saturated brine, dried over anhydrous sodium sulfate, and rotary evaporated to give the desired product A-1-8-1-1d as a yellow oil, which was directly used in the next step.

### Step 4

In a dry 100 mL three-necked flask, the crude compound A-1-8-1-1d (1.33 g, 4.15 mmol) was dissolved in acetone (30 mL). A1-1-1c (0.60 g, 2.07 mmol) and potassium carbonate (1.14 g, 8.30 mmol) were added. Then, the reaction was carried out at room temperature for 16 h. The spot plate test of the reaction solution indicated the formation of a new spot (PE:EA = 3:1, Rf = 0.5), and small amount of starting materials remained. 20 mL of water was added to quench the reaction. Then, the mixture was extracted with ethyl acetate (20 mL × 3). After that, the organic phase was washed with saturated brine, dried over anhydrous sodium sulfate, subjected to normal phase purification, and rotary evaporated to give the desired product A-1-8-1-1e as a yellow oil (0.43 g, 0.81 mmol, yield: 19%).

### Step 5

In a dry 100 mL three-necked flask, compound A-1-8-1-1e (0.43 g, 0.81 mmol) was dissolved in hydrochloric acid-ethyl acetate solution (3 M) (10 mL). Then, the reaction was carried out at room temperature for 1 h. The spot plate test of the reaction solution indicated the formation of a new spot, and the starting materials had completely reacted. 40 mL of saturated sodium bicarbonate was added to quench the reaction. Then, the mixture was extracted with ethyl acetate (20 mL × 3). After that, the organic phase was washed with saturated brine, dried over anhydrous sodium sulfate, subjected to normal phase purification (DCM:MeOH = 10:1, Rf = 0.5), and rotary evaporated to give the desired product A-1-8-1-1 as a white solid (0.29 g, 0.67 mmol, yield: 83.26%, purity: 95.33%).
MS-ESI: [M+H]⁺ = 430.1
¹H NMR (400 MHz, Chloroform-d) δ 7.32 (ddd, *J =* 8.4, 5.5, 3.0 Hz, 2H), 7.17 (t, *J* = 8.2 Hz, 1H), 7.02 (d, *J =* 6.9 Hz, 1H), 6.95 - 6.89 (m, 1H), 6.87 - 6.77 (m, 1H), 6.23 (d, *J =* 2.4 Hz, 1H), 5.02 - 4.89 (m, 1H), 3.73 (dt*, J =* 17.0, 8.5 Hz, 1H), 3.57 (d, *J =* 13.2 Hz, 1H), 3.41 (t, *J* = 13.5 Hz, 1H), 3.00 (dd, *J =* 20.6, 11.0 Hz, 1H), 2.89 (d, *J* = 12.9 Hz, 1H), 2.31 (s, 1H), 2.02 - 1.86 (m, 2H), 1.59 (dd, *J =* 13.5, 6.8 Hz, 3H), 1.27 (dd, *J =* 14.8, 10.8 Hz, 2H).

### Preparation Example (III) 2: Compound A-1-10-1-30

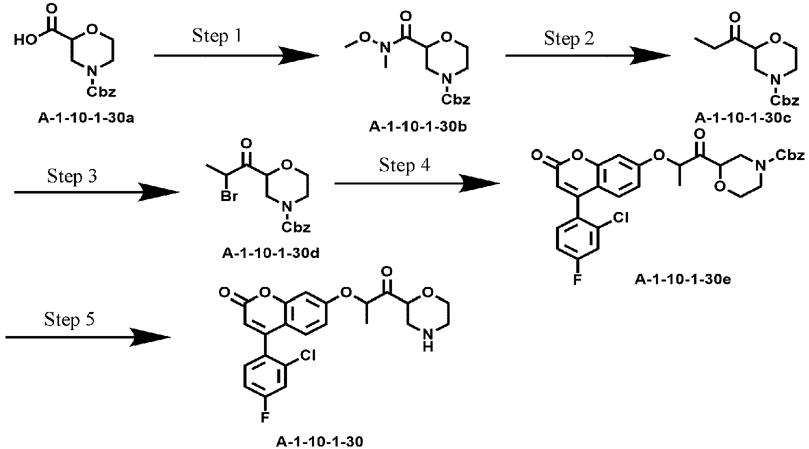

### Step 1

In a dry 500 mL three-necked flask, compound A-1-10-1-30a (4 g, 15.08 mmol) and 2-(7-azabenzotriazole)-N,N,N',N'-tetramethyluronium hexafluorophosphate (11.47 g, 30.16 mmol) were dissolved in N,N-dimethylformamide (100 mL). After stirring at room temperature for 5 minutes, triethylamine (4.58 g, 45.24 mmol) and methoxymethylamine hydrochloride (1.38 g, 22.62 mmol) were successively added. The mixture was stirred for 16 h, and TLC showed that the reaction was complete. The mixture was washed and extracted three times with ethyl acetate (100 mL), 50 mL of 5% aqueous hydrochloric acid solution and 100 mL of saturated brine. The organic phase was collected and rotary evaporated to give the desired product A-1-10-1-30b (4.50 g, 14.59 mmol, yield: 96.79%), which was directly used in the next step.

### Step 2

In a dry 500 mL three-necked flask, A-1-10-1-30b (4.5 g, 14.59 mmol) was dissolved in anhydrous tetrahydrofuran (100 mL). The system was purged with nitrogen at 0°C, and a solution of magnesium bromide in tetrahydrofuran (3.89 g, 29.18 mmol) was added. After that, the mixture was transferred to room temperature and reacted for 2-3 h. Acetic acid (5 mL) was added, followed by water (10 mL). After stirring for 5 min, the mixture was concentrated to dryness, mixed with silica gel, and subjected to column purification to give the desired product (PE:EA = 3:1) as pale yellow oil. HNMR (LJH-97-P1) confirmed it to be the desired product A-1-10-1-30c (2.2 g, 7.93 mmol, yield: 54.36%) as a pale yellow oil.

### Step 3

In a dry 100 mL three-necked flask, A-1-10-1-30c (2.2 g, 7.93 mmol) was dissolved in tetrahydrofuran (40 mL). Pyridinium tribromide (5.07 g, 15.86 mmol) was added. The reaction was carried out at 45°C for 16 h. TLC (PE:EA = 3:1, Rf = 0.49) showed a partial reaction. Ethyl acetate (40 mL) was added, and the mixture was washed with water (40 mL) twice. The organic phase was dried over anhydrous sodium sulfate and then concentrated to dryness to give the crude product A-1-10-1-30d (1.00 g, 2.81 mmol, yield: 35.39%), which was directly used in the next step.

### Step 4

In a dry 100 mL three-necked flask, compounds A-1-10-1-30d (1.00 g, 2.81 mmol) and A1-1-1c (0.82 g, 2.81 mmol) were dissolved in acetone (50 mL). Then, potassium carbonate (0.78 g, 5.62 mmol) was added. The reaction was carried out at room temperature for 24 h to give a brown-yellow suspension. The spot plate test of the reaction solution indicated that the reaction was complete (PE:EA = 1:1, Rf = 0.38). The reaction solution was dried, and the residue was added to 50 mL of ethyl acetate and 50 mL of water. The organic phase was extracted, collected, and dried to give a crude product. The crude product was subjected to column purification twice to give the desired product A-1-10-1-30e (1.20 g, 2.12 mmol, yield: 75%) as a pale yellow solid.

### Step 5

In a dry 100 mL three-necked flask, compound A-1-10-1-30e (0.6 g, 1.06 mmol) was dissolved in methanol (20 mL), and 350.0 mg of wet palladium on carbon (5%) was added. Then, the system was purged with hydrogen gas. Under a hydrogen atmosphere, the reaction was carried out at room temperature for 2 h. The spot plate test of the reaction solution indicated the formation of a new spot, with some starting materials remaining and an increase in impurity spots. The reaction was stopped. The mixture was filtered, subjected to normal phase purification (DCM:MeOH = 10:1, Rf = 0.5), and rotary evaporated to give the desired product A-1-10-1-30 as a white solid (0.15 g, 0.35 mmol, yield: 32.83%).
MS-ESI: [M+H]⁺ = 432.1
¹H NMR(400 MHz, Chloroform-d)δ=7.36-7.29 (m, 2H), 7.16 (s, 1H), 6.98 (d, *J* = 8.8 Hz, 1H), 6.85-6.72 (m, 2H), 6.21 (s, 1H), 5.39 (ddd, *J* = 17.5, 11.2, 7.3 Hz, 1H), 4.23 (t, *J* = 7.2 Hz, 1H), 4.03 (t, *J =* 11.1 Hz, 1H), 3.79-3.64 (m, 1H), 3.26 (dd, *J =* 28.4, 12.3 Hz, 1H), 3.06-2.86 (m, 2H), 2.79 (t, *J* = 11.3 Hz, 1H), 1.77 (s, 1H), 1.67-1.58 (m, 3H).

### Preparation Example (III) 3: Compound A-1-2-1-1

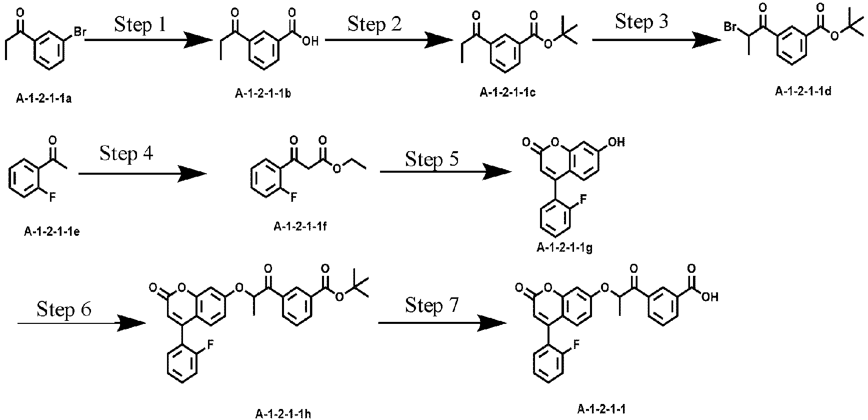

### Step 1

In a dry 500 mL three-necked flask, palladium acetate (0.63 g, 2.82 mmol) and the ligand 4,5-bis(diphenylphosphino)-9,9-dimethylxanthene (1.63 g, 2.82 mmol) were added, and the system was purged with nitrogen. Then, tert-butanol (20.87 g, 281.6 mmol), A-1-2-1a (30.00 g, 140.80 mmol) and triethylamine (28.50 g, 281.6 mmol) were added and dissolved in toluene (300 mL). The system was purged with nitrogen. After that, a mixture of formic acid (12.96 g, 281.6 mmol) and acetic anhydride (28.75 g, 281.6 mmol) was stirred at 30°C for 1.5 h and then added to the reaction flask. After that, the temperature was raised to 80°C, and the reaction was carried out for 4 h. After completion of the reaction, the insolubles in the reaction solution were filtered, and the reaction solution was washed with 300 mL of distilled water and 300 mL of saturated brine. The organic phase was dried over anhydrous sodium sulfate and concentrated to give a yellow-brown crude product. The crude product was slurried with PE and oven-dried to give the orange product A-1-2-1b (20.00 g, 112.24 mmol, yield: 79.72%).
MS-ESI: [M-H]⁻= 177.1

### Step 2

In a dry 500 mL three-necked flask, A-1-2-1b (20.00 g, 112.24 mmol), tert-butoxycarbonyl anhydride (49.00 g, 224.48 mmol) and 4-dimethylaminopyridine (6.86 g, 56.12 mmol) were mixed, dissolved in tert-butanol (200 mL), and stirred at room temperature for 12 h. Potassium carbonate (62.06 g, 448.96 mmol) was dissolved in water (50 mL) and poured into the reaction solution. Stirring was continued for 1 h, and the progress of the reaction was monitored by TLC. The mixture was extracted with ethyl acetate (100 mL × 3), then washed with saturated brine (100 mL × 2), dried over anhydrous sodium sulfate, loaded onto a column, and subjected to column chromatography ( PE:EA = 1-20%) to give A-1-2-1c as a colorless oily liquid (21.66 g, 92.45 mmol, yield: 82%).

¹H NMR (400 MHz, Chloroform-d) δ 8.52 (d, *J* = 1.9 Hz, 1H), 8.12 (ddt, *J* = 16.4, 7.8, 1.5 Hz, 2H), 7.50 (t, *J =* 7.7 Hz, 1H), 3.02 (q, *J =* 7.2 Hz, 2H), 1.60 (s, 9H), 1.22 (t, *J =* 7.2 Hz, 3H).

### Step 3

In a dry 500 mL single-necked flask, A-1-2-1c (23.50g, 100.30 mmol) was added and dissolved in 200 mL of tetrahydrofuran. Pyridinium tribromide (38.49 g, 120.00 mmol) was added in batches at 0°C. After that, the mixture was transferred to room temperature and stirred at room temperature for 5 h. The progress of the reaction was monitored by TLC. After completion of the reaction, the reaction solution was filtered, and the filtrate was rotary evaporated. Then, 200 mL of ethyl acetate was added. The mixture was washed successively with water (100 mL × 2), 100 mL of saturated sodium bicarbonate and 100 mL of saturated brine, dried over anhydrous sodium sulfate, rotary evaporated, and subjected to column chromatography (PE:EA = 10:1) to give A-1-2-1d as a yellow oily liquid (30.10 g, 96.11 mmol, yield: 95%).

### Step 4

In a dry 100 mL three-necked flask, diethyl carbonate (17.11 g, 144.80 mmol) was added and dissolved in tetrahydrofuran (50 mL), and the system was purged with nitrogen. The reactor was cooled to 0°C in a low-temperature reaction bath. Sodium hydride (2.90 g, 72.40 mmol) was added in batches, and the reaction was carried out for 1 h. Then, A-1-2-1e (5.00 g, 36.20 mmol) was added to the flask. After that, the temperature was slowly raised to room temperature, and the reaction was carried out for 2 h (preferably 2-4 h). After TLC showed that the reaction was complete, the reaction was quenched with 1 M hydrochloric acid solution at 0°C to pH = 1, while controlling the temperature not to exceed 10°C. The reaction solution was extracted with ethyl acetate (100 mL × 3), and the layers were separated. The organic phases were combined, washed with saturated sodium chloride solution (200 mL), dried over anhydrous sodium sulfate, and rotary evaporated to give the crude intermediate A-1-2-1f (4.20 g, 19.98 mmol, yield: 55.2%).

### Step 5

In a dry 100 mL three-necked flask, A-1-2-1f (4.20 g, 19.98 mmol) and resorcinol (2.2 g, 19.98 mmol) were added and dissolved in methanesulfonic acid (30 mL). The temperature was raised to 50°C, and the reaction was carried out for 4 h (preferably 4-8 h). After LCMS showed that the reaction was complete, the reaction solution was slowly added to ethanol (100 mL) while controlling the quenching temperature. After that, the reaction solution was added to water (100 mL) and extracted with ethyl acetate (200 mL × 3). The layers were separated. The organic phases were combined, washed with saturated sodium chloride solution (500 mL), dried over anhydrous sodium sulfate, and rotary evaporated to give the crude intermediate A-1-2-1g (3.0 g, 11.71 mmol, yield: 59%).
MS-ESI: [M-H]⁻ = 255.1

### Step 6

In a dry 100 mL three-necked flask, A-1-2-1g (1.64 g, 6.39 mmol), A-1-2-1d (2.0 g, 6.39 mmol), potassium carbonate (1.77 g, 12.78 mmol) and tetrabutylammonium iodide (2.36 g, 6.39 mmol) were successively added, dissolved in acetone (30 mL), and stirred at room temperature for 8 h (preferably 8-12 h). After LCMS showed that the reaction was complete, the insolubles were filtered, and the solvent was rotary evaporated. Water (30 mL) was added. The mixture was extracted with ethyl acetate (15 mL × 3), and the layers were separated. The organic phases were combined, washed with saturated sodium chloride solution (20 mL), dried over anhydrous sodium sulfate, and purified by silica gel column chromatography (PE:EA = 5:1) to give the intermediate A-1-2-1h (0.62 g, 1.27 mmol, yield: 19.9%). MS-ESI: [M-H]⁻ = 487.2

### Step 7

In a dry 100 mL three-necked flask, A-1-2-1h (0.62 g, 1.27 mmol) was added and dissolved in dichloromethane (10 mL). Trifluoroacetic acid (10 mL) was added and stirred for 4 h (preferably, the reaction time was 4-8 h). After LCMS showed that the reaction was complete, the solvent was rotary evaporated. The residue was purified by silica gel column chromatography (PE:EA = 2:1) to give the product A-1-2-1 (395.9 mg, 0.92 mmol, yield: 72%).
MS-ESI: [M-H]⁻= 431.1.
¹H NMR (400 MHz, Chloroform-d) δ 8.80 (t, *J =* 1.7 Hz, 1H), 8.36 (dt, *J =* 7.7, 1.5 Hz, 1H), 8.30 (dt, *J = 7.9,* 1.5 Hz, 1H), 7.65 (t, *J =* 7.8 Hz, 1H), 7.49 (dddd, *J =* 8.6, 7.0, 5.1, 2.2 Hz, 1H), 7.30 (ddd, *J = 8.3,* 6.8, 1.5 Hz, 2H), 7.21 (dd, *J =* 9.8, 8.3 Hz, 1H), 7.15 (dd, *J* = 9.1, 2.4 Hz, 1H), 6.81 (d, *J* = 7.9 Hz, 2H), 6.25 (s, 1H), 5.63 (q, *J =* 6.8 Hz, 1H), 1.80 (d, *J =* 6.9 Hz, 3H).

### Preparation Example (III) 4: Compound A-1-2-1-2

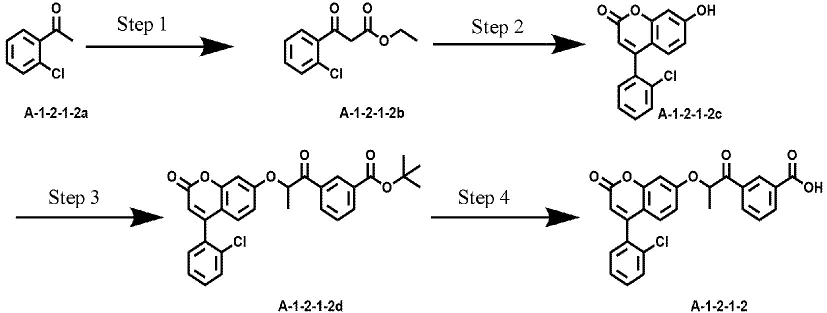

### Step 1

In a dry 100 mL three-necked flask, diethyl carbonate (15.28 g, 129.36 mmol) was added and dissolved in tetrahydrofuran (50 mL), and the system was purged with nitrogen. The reactor was cooled to 0°C in a low-temperature reaction bath. Sodium hydride (2.59 g, 64.68 mmol) was added in batches, and the reaction was carried out for 1 h. Then, A-1-2-1-2a (5.00 g, 32.34 mmol) was added to the flask. After that, the temperature was slowly raised to room temperature, and the reaction was carried out for 2 h (preferably 2-4 h). After TLC showed that the reaction was complete, the reaction was quenched with 1 M hydrochloric acid solution at 0°C to pH = 1, while controlling the temperature not to exceed 10°C. The reaction solution was extracted with ethyl acetate (100 mL × 3), and the layers were separated. The organic phases were combined, washed with saturated sodium chloride solution (200 mL), dried over anhydrous sodium sulfate, and rotary evaporated to give the crude intermediate A-1-2-1-2b (5.60 g, 24.71 mmol, yield: 77.4%).
¹H NMR (400 MHz, DMSO-d₆) δ 7.78 (d, *J* = 7.7 Hz, 1H), 7.55 *(d, J =* 4.3 Hz, 2H), 7.47 (dt, *J =* 8.2, 4.0 Hz, 1H), 4.14 (s, 2H), 4.09 (dd, *J =* 7.1, 2.7 Hz, 2H), 1.14 (t, *J =* 7.1 Hz, 3H)

### Step 2

In a dry 100 mL three-necked flask, A-1-2-1-2b (5.60 g, 24.71 mmol) and resorcinol (2.72 g, 24.71 mmol) were added and dissolved in methanesulfonic acid (30 mL). The temperature was raised to 50°C, and the reaction was carried out for 4 h (preferably 4-8 h). After LCMS showed that the reaction was complete, the reaction solution was slowly added to ethanol (100 mL) while controlling the quenching temperature. After that, the reaction solution was added to water (100 mL) and extracted with ethyl acetate (200 mL × 3). The layers were separated. The organic phases were combined, washed with saturated sodium chloride solution (500 mL), dried over anhydrous sodium sulfate, and rotary evaporated to give the crude intermediate A-1-2-1-2c (6.20 g, 22.74 mmol, yield: 78.2%). MS-ESI: [M-H]⁻ = 271.0

### Step 3

In a dry 100 mL three-necked flask, A-1-2-1-2c (1.74 g, 6.39 mmol), tert-butyl 3-(2-bromopropanoyl)benzoate (2.00 g, 6.39 mmol), potassium carbonate (1.77 g, 12.78 mmol) and tetrabutylammonium iodide (2.36 g, 6.39 mmol) were successively added, dissolved in acetone (30 mL), and stirred at room temperature for 8 h (preferably 8-12 h). After LCMS showed that the reaction was complete, the insolubles were filtered, and the solvent was rotary evaporated. Water (30 mL) was added. The mixture was extracted with ethyl acetate (15 mL × 3), and the layers were separated. The organic phases were combined, washed with saturated sodium chloride solution (20 mL), dried over anhydrous sodium sulfate, and purified by silica gel column chromatography (PE:EA = 5:1) to give the intermediate A-1-2-1-2d (0.90 g, 1.78 mmol, yield: 27.9%).
MS-ESI: [M-H]⁻ = 503.1

### Step 4

In a dry 100 mL three-necked flask, A-1-2-1-2d (0.90 g, 1.78 mmol) was added and dissolved in dichloromethane (10 mL). Trifluoroacetic acid (10 mL) was added and stirred for 4 h (preferably, the reaction time was 4-8 h). After LCMS showed that the reaction was complete, the solvent was rotary evaporated. The residue was purified by silica gel column chromatography (PE:EA = 2:1) to give the product A-1-2-1-2 (113 mg, 0.25 mmol, yield: 14%).
MS-ESI: [M-H]⁻ = 447.2
¹H NMR (400 MHz, Chloroform-d) δ 8.79 (q, *J* = 1.8 Hz, 1H), 8.36 (dq, *J =* 7.7, 1.3 Hz, 1H), 8.29 (dt, *J =* 8.0*,* 1.5 Hz, 1H), 7.65 (ddd, *J =* 8.4, 7.6, 1.1 Hz, 1H), 7.52 (dt, *J =* 7.9, 1.5 Hz, 1H), 7.46 - 7.32 (m, 2H), 7.29 - 7.20 (m, 1H), 6.97 (dd, *J =* 8.8, 4.0 Hz, 1H), 6.86 - 6.68 (m, 2H), 6.20 (d, *J*= 1.1 Hz, 1H), 5.63 (dd, *J =* 7.0, 1.1 Hz, 1H), 1.79 (d, *J* = 6.9 Hz, 3H).

### Preparation Example (III) 5: Compound A-1-2-6-1

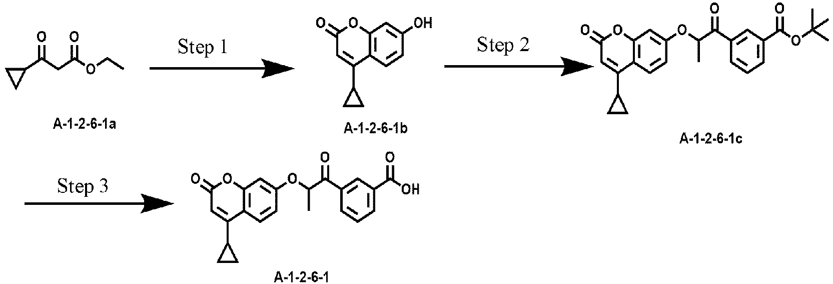

### Step 1

In a dry 100 mL three-necked flask, compound A-1-2-6-1a (2.50 g, 16.01 mmol) and resorcinol (1.94 g, 17.61 mmol) were weighed into the reaction flask and then dissolved in methanesulfonic acid (20 mL). The reaction was carried out at 50°C for 3 h to give a black reaction solution. The TLC spot plate showed that the starting materials had completely reacted. The reaction was then subjected to a work-up. The reaction solution was returned to room temperature, and 30 mL of ethanol was added. Solid sodium bicarbonate was added to adjust the pH to 6-7. Then, 200 mL of water was added. The mixture was extracted with ethyl acetate (100 mL). Then, the organic phase was collected, dried, concentrated, and then subjected to column purification (PE:EA = 3:1) to give the desired product A-1-2-6-1b as a yellow oil (3.00 g, 14.84 mmol, yield: 92.68%), which was used in the next step.

### Step 2

In a dry 100 mL three-necked flask, compound A-1-2-6-1b (3.00 g, 14.84 mmol), A-1-2-1-1d (5.58 g, 17.81 mmol) and potassium carbonate (5.13 g, 37.1 mmol) were dissolved in acetone (30 mL). Then, the reaction was carried out at room temperature to give a yellow turbid solution. TLC showed that a new spot had appeared and the starting materials had completely reacted. The reaction was then subjected to a work-up. The reaction solution was poured into ethyl acetate (50 mL) and then washed with saturated brine three times. The organic phase was collected, dried, concentrated, and then subjected to column purification (PE:EA = 1:1) to give the desired product A-1-2-6-1c as a yellow solid (1.5 g, 3.45 mmol, yield: 23.27%). The resulting product was directly used in the next step.

### Step 3

In a dry 100 mL three-necked flask, compound A-1-2-6-1c (1.50 g, 3.45 mmol) was dissolved in dichloromethane (20 mL). Then, trifluoroacetic acid (0.79 g, 6.90 mmol) was added. The reaction was carried out at room temperature for 2 h to give a clear yellow solution. The TLC spot plate showed that the starting materials had completely reacted. To the reaction solution, 50 mL of ethyl acetate and 50 mL of water were added. Then, the pH was adjusted to 4-5 with solid sodium bicarbonate. The reaction solution was extracted with ethyl acetate (40 mL × 2). The organic phases were collected, combined, dried over anhydrous sodium sulfate, and then rotary evaporated to give a crude product as a dark yellow oil. The compound was purified as follows. 50 mL of Ethyl acetate was added to the crude product. After dissolution, 25 mL of petroleum ether was slowly added while continuously stirring for 1 h to give a yellow turbid solution. Filtration was carried out to give a pale yellow solid. The solid was collected, and 50 mL of ethyl acetate was added. The mixture was refluxed for 3 h to be completely dissolved, concentrated, mixed with an adsorbent, and subjected to column purification (PE:EA = 2:1) to give the desired product. The desired product was mixed with 2.5 mL of acetonitrile and 25 mL of water and then freeze-dried to give the desired product A-1-2-6-1 as a white solid (265 mg, 0.70 mmol, purity: 99.26%, yield: 20.2%). MS-ESI: [M-H]⁻ = 377.1.

¹H NMR (400 MHz, DMSO-d₆) δ 8.55 (s, 1H), 8.31 (d, *J =* 8.0 Hz, 1H), 8.23 (d, *J =* 7.8 Hz, 1H), 7.97 (d, *J =* 8.8 Hz, 1H), 7.71 (t, *J =* 7.8 Hz, 1H), 6.98 (dt, *J =* 6.0, 2.4 Hz, 2H), 6.27 (q, *J =* 6.7 Hz, 1H), 5.89 (s, 1H), 2.29-2.18 (m, 1H), 1.59 (d, *J =* 6.7 Hz, 3H), 1.11-1.04 (m, 2H), 0.90-0.82 (m, 2H).

### Preparation Example (III) 6: Compound A-1-2-1-8

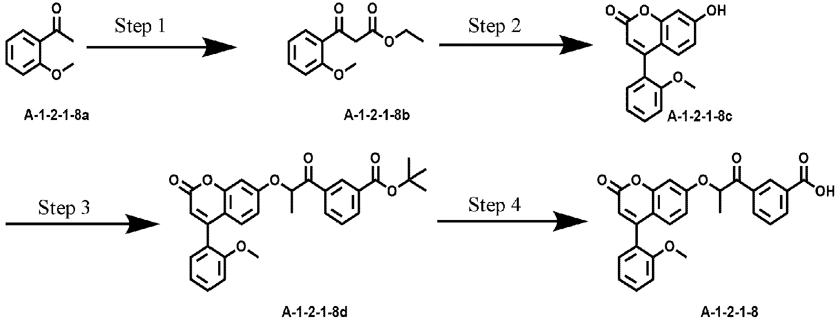

### Step 1

In a dry 100 mL three-necked flask, diethyl carbonate (15.73 g, 133.20 mmol) was added and dissolved in tetrahydrofuran (50 mL), and the system was purged with nitrogen. The reactor was cooled to 0°C in a low-temperature reaction bath. Sodium hydride (2.59 g, 66.60 mmol) was added in batches, and the reaction was carried out for 1 h. Then, A-1-2-1-8a (5.00 g, 33.30 mmol) was added to the flask. After that, the temperature was slowly raised to room temperature, and the reaction was carried out for 2 h (preferably 2-4 h). After TLC showed that the reaction was complete, the reaction was quenched with 1 M hydrochloric acid solution at 0°C to pH = 1, while controlling the temperature not to exceed 10°C. The reaction solution was extracted with ethyl acetate (100 mL × 3), and the layers were separated. The organic phases were combined, washed with saturated sodium chloride solution (200 mL), dried over anhydrous sodium sulfate, and rotary evaporated to give the crude intermediate A-1-2-1-8b (7.0 g, 22.05 mmol, yield: 94.60%).
MS-ESI: [M+H]⁺= 223.2

### Step 2

In a dry 100 mL three-necked flask, A-1-2-1-8b (7.00 g, 22.05 mmol) and resorcinol (2.43 g, 22.05 mmol) were added and dissolved in methanesulfonic acid (30 mL). The temperature was raised to 50°C, and the reaction was carried out for 4 h (preferably 4-8 h). After LCMS showed that the reaction was complete, the reaction solution was slowly added to ethanol (100 mL) while controlling the quenching temperature. After that, the reaction solution was added to water (100 mL) and extracted with ethyl acetate (200 mL × 3). The layers were separated. The organic phases were combined, washed with saturated sodium chloride solution (500 mL), dried over anhydrous sodium sulfate, and rotary evaporated to give the crude intermediate A-1-2-1-8c (5.50 g, 20.50 mmol, yield: 79.00%).
MS-ESI: [M-H]⁻ = 267.0

### Step 3

In a dry 100 mL three-necked flask, A-1-2-1-8c (1.54 g, 5.75 mmol), A-1-2-1-1d (2.00 g, 6.39 mmol), potassium carbonate (1.77 g, 12.78 mmol) and tetrabutylammonium iodide (2.36 g, 6.39 mmol) were successively added, dissolved in acetone (30 mL), and stirred at room temperature for 8 h (preferably 8-12 h). After LCMS showed that the reaction was complete, the insolubles were filtered, and the solvent was rotary evaporated. Water (30 mL) was added. The mixture was extracted with ethyl acetate (15 mL × 3), and the layers were separated. The organic phases were combined, washed with saturated sodium chloride solution (20 mL), dried over anhydrous sodium sulfate, and purified by silica gel column chromatography (PE:EA = 5:1) to give the intermediate A-1-2-1-8d (1.00 g, 2.00 mmol, yield: 31.29%).
MS-ESI: [M-^{t}Bu]⁻ = 445.2

### Step 4

In a dry 100 mL three-necked flask, A-1-2-1-8d (1.00 g, 2.00 mmol) was added and dissolved in dichloromethane (10 mL). Trifluoroacetic acid (10 mL) was added and stirred for 4 h (preferably, the reaction time was 4-8 h). After LCMS showed that the reaction was complete, the solvent was rotary evaporated. The residue was purified by silica gel column chromatography (PE:EA = 2:1) to give the product A-1-2-1-8 (329.5 mg, 0.74 mmol, yield: 36.80%, purity: 99.16%).

MS-ESI: [M-H]⁻ = 443.1.

¹H NMR (400 MHz, Chloroform-d) δ 8.80 (t, *J =* 1.8 Hz, 1H), 8.36 (dt, *J =* 7.8, 1.4 Hz, 1H), 8.29 (dt, *J* = 7.9, 1.5 Hz, 1H), 7.64 (t, *J =* 7.8 Hz, 1H), 7.45 (ddd, *J =* 8.4, 7.4, 1.8 Hz, 1H), 7.18 (dd, *J* = 7.5, 1.8 Hz, 1H), 7.10 - 6.96 (m, 3H), 6.76 (s, 2H), 6.20 (s, 1H), 5.62 (*d, J =* 8.0 Hz, 1H), 3.73 (s, 3H), 1.79 (d, *J =* 6.8 Hz, 3H).

### Preparation Example (III) 7: Compound A-1-2-1-9

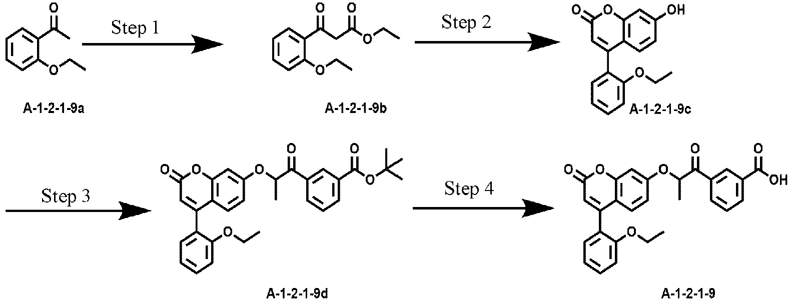

### Step 1

In a dry 100 mL three-necked flask, diethyl carbonate (14.39 g, 121.80 mmol) was added and dissolved in tetrahydrofuran (50 mL), and the system was purged with nitrogen. The reactor was cooled to 0°C in a low-temperature reaction bath. Sodium hydride (2.44 g, 60.90 mmol) was added in batches, and the reaction was carried out for 1 h. Then, A-1-2-1-9a (5.00 g, 30.45 mmol) was added to the flask. After that, the temperature was slowly raised to room temperature, and the reaction was carried out for 2 h (preferably 2-4 h). After TLC showed that the reaction was complete, the reaction was quenched with 1 M hydrochloric acid solution at 0°C to pH = 1, while controlling the temperature not to exceed 10°C. The reaction solution was extracted with ethyl acetate (100 mL × 3), and the layers were separated. The organic phases were combined, washed with saturated sodium chloride solution (200 mL), dried over anhydrous sodium sulfate, and rotary evaporated to give the crude intermediate A-1-2-1-9b (7.5 g, 22.22 mmol, yield: 83.40%).
MS-ESI: [M+H]⁺ = 237.1

### Step 2

In a dry 100 mL three-necked flask, A-1-2-1-9b (7.5 g, 22.22 mmol) and resorcinol (2.45 g, 22.22 mmol) were added and dissolved in methanesulfonic acid (30 mL). The temperature was raised to 50°C, and the reaction was carried out for 4 h (preferably 4-8 h). After LCMS showed that the reaction was complete, the reaction solution was slowly added to ethanol (100 mL) while controlling the quenching temperature. After that, the reaction solution was added to water (100 mL) and extracted with ethyl acetate (200 mL × 3). The layers were separated. The organic phases were combined, washed with saturated sodium chloride solution (500 mL), dried over anhydrous sodium sulfate, and rotary evaporated to give the crude intermediate A-1-2-1-9c (2.5 g, 8.86 mmol, yield: 33.88%).
MS-ESI: [M-H]⁻ = 281.1

### Step 3

In a dry 100 mL three-necked flask, A-1-2-1-9c (1.62 g, 5.57 mmol), A-1-2-1-1d (2.0 g, 6.39 mmol), potassium carbonate (1.77 g, 12.78 mmol) and tetrabutylammonium iodide (2.36 g, 6.39 mmol) were successively added, dissolved in acetone (30 mL), and stirred at room temperature for 8 h (preferably 8-12 h). After LCMS showed that the reaction was complete, the insolubles were filtered, and the solvent was rotary evaporated. Water (30 mL) was added. The mixture was extracted with ethyl acetate (15 mL × 3), and the layers were separated. The organic phases were combined, washed with saturated sodium chloride solution (20 mL), dried over anhydrous sodium sulfate, and purified by silica gel column chromatography (PE:EA = 5:1) to give the intermediate A-1-2-1-9d (2.00 g, 1.27 mmol, yield: 60.86%).
MS-ESI: [M-H]⁻ = 513.2

### Step 4

In a dry 100 mL three-necked flask, A-1-2-1-9d (2.00 g, 1.27 mmol) was added and dissolved in dichloromethane (10 mL). Trifluoroacetic acid (10 mL) was added and stirred for 4 h (preferably, the reaction time was 4-8 h). After LCMS showed that the reaction was complete, the solvent was rotary evaporated. The residue was purified by silica gel column chromatography (PE:EA = 2:1) to give the product A-1-2-1-9 (905.2 mg, 0.92 mmol, yield: 48.13 %, purity: 95.03%).

MS-ESI: [M-H]⁻ = 457.2.

¹H NMR (400 MHz, Chloroform-d) δ 8.80 (t, *J =* 1.8 Hz, 1H), 8.35 (dt, *J =* 7.8, 1.4 Hz, 1H), 8.29 (*dt, J* = 7.9, 1.5 Hz, 1H), 7.63 (t, *J =* 7.8 Hz, 1H), 7.42 (ddd, *J =* 8.3, 7.5, 1.8 Hz, 1H), 7.18 (dd, *J* = 7.5, 1.8 Hz, 1H), 7.10 (d, *J =* 8.8 Hz, 1H), 7.05 - 6.96 (m, 2H), 6.76 (s, 2H), 6.21 (s, 1H), 5.62 (d, *J* = 6.9 Hz, 1H), 3.99 (dq, *J =* 10.7, 7.1 Hz, 2H), 1.79 (d, *J =* 6.8 Hz, 3H), 1.14 (t, *J =* 7.0 Hz, 3H).

### Preparation Example (III) 8: Compound A-2-2-1-4

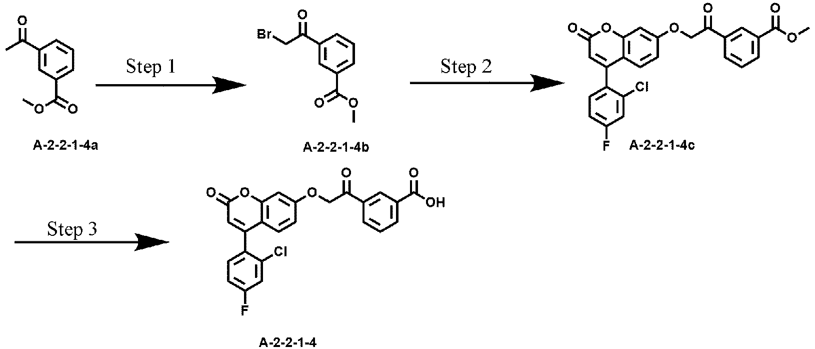

### Step 1

In a dry 100 mL three-necked flask, A-2-2-1-4a (2.00 g, 11.22 mmol) was added, and the system was purged with nitrogen. After that, anhydrous tetrahydrofuran (20 mL) was added for dissolution, and the system was cooled to 0°C. Pyridinium tribromide (4.31 g, 13.46 mmol) was added in batches, and then the temperature was raised to 25°C. The reaction was carried out for 6 h (preferably 6-8 h). After LCMS showed that the reaction was complete, the reaction solution was filtered. The filtrate was collected, diluted with water (20 mL), and extracted with ethyl acetate (20 mL × 3). The layers were separated. The organic phases were combined, washed successively with saturated sodium bicarbonate solution (50 mL) and saturated sodium chloride solution (50 mL), dried over anhydrous sodium sulfate, and purified by silica gel column chromatography (PE:EA = 2:1) to give the intermediate A-2-2-1-4b (1.10 g, 4.28 mmol, yield: 38.12%).
MS-ESI: [M-H]⁻= 255.0

### Step 2

In a dry 100 mL three-necked flask, A-2-2-1-4b (1.1 g, 4.28 mmol), A1-1-1c (1.12 g, 3.85 mmol), potassium carbonate (1.18 g, 8.56 mmol) and tetrabutylammonium iodide (1.58 g, 4.28 mmol) were successively added and dissolved in acetone (10 mL). The mixture was stirred at room temperature overnight. After LCMS showed that the reaction was complete, the mixture was diluted with water (20 mL) and extracted with ethyl acetate (20 mL × 3). The layers were separated. The organic phases were combined, washed with saturated sodium chloride solution (50 mL), dried over anhydrous sodium sulfate, and purified by silica gel column chromatography (PE:EA = 2:1) to give the intermediate A-2-2-1-4c (1.00 g, 2.14 mmol, yield: 50.06%).
MS-ESI: [M-H]⁻ = 465.1

### Step 3

In a dry 100 mL three-necked flask, A-2-2-1-4c (1.00 g, 2.14 mmol) was added and dissolved in 4 mL of tetrahydrofuran, and the system was cooled to 0°C. Then, 2 M sodium hydroxide solution (2 mL) was added dropwise and stirred for 1 h (preferably 1-2 h). After LCMS showed that the reaction was complete, the mixture was diluted with water (10 mL), and 2 M hydrochloric acid solution was added at 0°C to quench the reaction and adjust the pH to 1. The mixture was extracted with ethyl acetate (5 mL × 3), and the layers were separated. The organic phases were combined, washed with saturated sodium chloride solution (10 mL), dried over anhydrous sodium sulfate, and purified by silica gel column chromatography (DCM:MeOH = 15:1) to give the final product A-2-2-1-4 (117.4.0 mg, 0.08 mmol, yield: 10.29%, purity: 85.01%).
MS-ESI: [M-H]⁻ = 451.1
¹H NMR (400 MHz, DMSO-d₆) δ 8.52 (*t, J* = 1.8 Hz, 1H), 8.25 (ddt, *J =* 10.7, 7.8, 1.4 Hz, 2H), 7.76 - 7.64 (m, 2H), 7.57 (dd, *J =* 8.6, 6.1 Hz, 1H), 7.44 (td, *J =* 8.5, 2.6 Hz, 1H), 7.25 (d, *J =* 2.3 Hz, 1H), 6.99 - 6.93 (m, 2H), 6.32 (s, 1H), 5.83 (s, 2H).

### Preparation Example (III) 9: Compound A-1-2-1-33

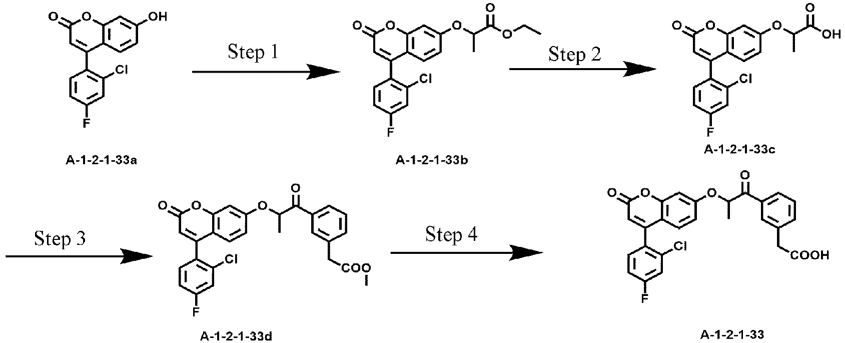

### Step 1

In a dry 500 mL three-necked flask, A-1-2-1-33a (20.00 g, 68.81 mmol) and ethyl (2S)-2-hydroxypropanoate (9.75 g, 82.57 mmol) were added and dissolved in tetrahydrofuran (300 mL). Under an ice bath, triphenylphosphine (21.66 g, 82.57 mmol) and diisopropyl azodicarboxylate (16.70 g, 82.57 mmol) were added. After stirring for 2 h under an ice bath, the reaction was monitored by TLC for completion. Water (200 mL) was added to quench the reaction. The mixture was extracted with ethyl acetate (200 mL × 3). The organic phases were combined, washed with saturated brine, dried over anhydrous sodium sulfate, concentrated, and purified by silica gel column chromatography (PE:EA = 10:1) to give the intermediate A-1-2-1-33b (30.00 g, crude).
MS-ESI: [M+H]+ = 391.1

### Step 2

In a 500 mL single-necked flask, A-1-2-1-33b (30.00 g, crude) was added and dissolved in tetrahydrofuran (150 mL) and methanol (50 mL). Aqueous sodium hydroxide solution (2 N, 100 mL) was added and stirred under an ice bath for 20 min. A sample was taken for the spot plate. TLC showed that the starting materials had completely reacted. The reaction was stopped. Water (100 mL) was added to the reaction solution. The reaction solution was extracted with ethyl acetate (100 mL × 3), and the aqueous phase was collected. Dilute hydrochloric acid (1 N, 200 mL) was added dropwise to the aqueous phase to quench the reaction and adjust the solution to weakly acidic. The mixture was extracted with ethyl acetate (100 mL × 3). The organic phases were combined, sampled for LCMS, and purified by silica gel column chromatography (DCM:MeOH = 15:1) to give the intermediate A-1-2-1-33c (14.40 g, crude).
MS-ESI: [M-H]⁻ = 361.1

### Step 3

In a dry 50 mL three-necked flask, A-1-2-1-33c (450 mg, 1.24 mmol) and (3-(2-methoxy-2-oxoethyl)phenyl)boronic acid (530.0.0 mg, 2.48 mmol) were added. After purging with nitrogen, tetrakis(triphenylphosphine)palladium (72 mg, 0.062 mmol) was added and dissolved in 5 mL of dioxane solution. Finally, dimethyl dicarbonate (330 mg, 2.48 mmol) was added. The reaction was carried out at 115°C for 2 h (preferably 2-4 h). After LCMS analysis showed that the reaction was complete, the mixture was cooled to room temperature and filtered. The filtrate was collected, and 10 mL of water was added. The mixture was extracted with ethyl acetate (10 mL × 3), washed with 20 mL of saturated brine, dried over anhydrous sodium sulfate, and purified by silica gel column chromatography (PE:EA = 3:1) to give the intermediate A-1-2-1-33d (400 mg, 0.81 mmol, yield: 65.15%).
MS-ESI: [M+H]⁺= 495.1

### Step 4

In a dry 50 mL three-necked flask, A-1-2-1-33d (400 mg, 0.81 mmol) was added and dissolved in 2 mL of tetrahydrofuran, and the system was cooled at 0°C. Then, 2 mL of 2M aqueous sodium hydroxide solution was added. The reaction was carried out for 30 min (preferably 0.5-2 h). After TLC showed that the reaction was complete, the mixture was adjusted to pH = 1 with 1M aqueous hydrochloric acid solution, extracted with ethyl acetate (5 mL × 3), washed with saturated brine (5 mL × 2), dried over anhydrous sodium sulfate, rotary evaporated, and purified by silica gel column chromatography (DCM:MeOH = 10:1) to give the desired product A-1-2-1-33 (86 mg, 0.178 mmol, yield: 22.0%).
MS-ESI: [M-H]⁻ = 479.1
¹H NMR (400 MHz, Chloroform-d) δ 8.01 - 7.94 (m, 2H), 7.60 - 7.55 (m, 1H), 7.49 (td, *J* = 7.7, 1.7 Hz, 1H), 7.30 - 7.27 (m, 1H), 7.26 - 7.23 (m, 1H), 7.12 (tdd, *J* = 8.3, 4.1, 2.5 Hz, 1H), 6.94 (dd, *J =* 8.8, 4.9 Hz, 1H), 6.83 - 6.78 (m, 1H), 6.77 - 6.71 (m, 1H), 6.17 (d, *J =* 0.9 Hz, 1H), 5.61 (*qd, J =* 6.9, 3.4 Hz, 1H), 3.74 (d, *J* = 1.9 Hz, 2H), 1.75 (d, *J* = 6.8 Hz, 3H).

### Preparation Example (III) 10: Compound A-1-10-1-21

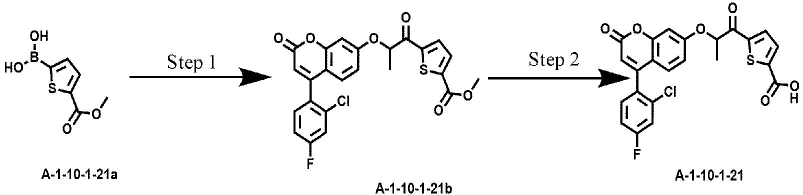

### Step 1

In a dry 50 mL three-necked flask, A-1-2-1-33c (450 mg, 1.24 mmol) and A-1-10-1-21a (460 mg, 2.48 mmol) were added. After purging with nitrogen, tetrakis(triphenylphosphine)palladium (72 mg, 0.062 mmol) was added and dissolved in 2 mL of dioxane solution. Finally, dimethyl dicarbonate (0.33 g, 2.48 mmol) was added. The reaction was carried out at 115°C for 2 h (preferably 2-4 h). After LCMS analysis showed that the reaction was complete, the mixture was cooled to room temperature and filtered. The filtrate was collected, and 10 mL of water was added. Then, the mixture was extracted with ethyl acetate (10 mL × 3), washed with 20 mL of saturated brine, dried over anhydrous sodium sulfate, and purified by silica gel column chromatography (PE:EA = 3:1) to give the intermediate A-1-10-1-21b (210 mg, 0.43 mmol, yield: 34.77%).
MS-ESI: [M-H]⁻ = 485.4

### Step 2

In a dry 50 mL three-necked flask, A-1-10-1-21b (210 mg, 0.43 mmol) was added and dissolved in 2 mL of tetrahydrofuran, and the system was cooled at 0°C. Then, 2 mL of 2M aqueous sodium hydroxide solution was added. The reaction was carried out for 30 min (preferably 0.5-2 h). After TLC showed that the reaction was complete, the mixture was adjusted to pH = 1 with 1M aqueous hydrochloric acid solution, extracted with ethyl acetate (5 mL × 3), washed with saturated brine (5 mL × 2), dried over anhydrous sodium sulfate, rotary evaporated, and purified by silica gel column chromatography (DCM:MeOH = 10:1) to give the desired product A-1-10-1-21 (52 mg, 0.109 mmol, yield: 25.3%).
MS-ESI: [M-H]⁻ = 471.0
¹H NMR (400 MHz, Chloroform-d) δ 7.93 (d, *J =* 4.1 Hz, 1H), 7.83 (d, *J =* 4.0 Hz, 1H), 7.29 (dd, *J =* 8.4, 2.5 Hz, 1H), 7.25 (d, *J = 5.9* Hz, 1H), 7.13 (tt, *J =* 8.3, 2.4 Hz, 1H), 6.98 (dd, *J =* 8.4, 2.5 Hz, 1H), 6.92 - 6.73 (m, 2H), 6.21 (d, *J =* 0.8 Hz, 1H), 5.25 (q, *J=* 6.8 Hz, 1H), 1.79 (d, *J =* 6.9 Hz, 3H).

### Preparation Example (III) 11: Compound A-1-10-1-19

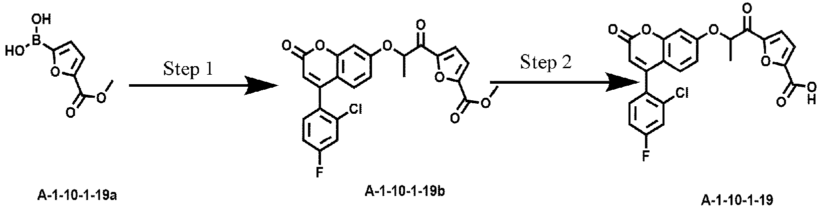

### Step 1

In a dry 50 mL three-necked flask, A-1-2-1-33c (500 mg, 1.38 mmol) and A-1-10-1-19a (469 mg, 2.76 mmol) were added. After purging with nitrogen, tetrakis(triphenylphosphine)palladium (80 mg, 0.069 mmol) was added and dissolved in 5 mL of dioxane solution. Finally, dimethyl dicarbonate (463 mg, 3.45 mmol) was added. The reaction was carried out at 115°C for 2 h (preferably 2-4 h). After LCMS analysis showed that the reaction was complete, the mixture was cooled to room temperature and filtered. The filtrate was collected, and 10 mL of water was added. Then, the mixture was extracted with ethyl acetate (10 mL × 3), washed with 20 mL of saturated brine, dried over anhydrous sodium sulfate, and purified by silica gel column chromatography (PE:EA = 3:1) to give the intermediate A-1-10-1-19b (317 mg, 0.67 mmol, yield: 65.15 %).
MS-ESI: [M+H]⁺ = 471.1

### Step 2

In a dry 50 mL three-necked flask, A-1-10-1-19b (317 mg, 0.67 mmol) was added and dissolved in 2 mL of tetrahydrofuran, and the system was cooled at 0°C. Then, 2 mL of 2M aqueous sodium hydroxide solution was added. The reaction was carried out for 30 min (preferably 0.5-2 h). After TLC showed that the reaction was complete, the mixture was adjusted to pH = 1 with 1M aqueous hydrochloric acid solution, extracted with ethyl acetate (5 mL × 3), washed with saturated brine (5 mL × 2), dried over anhydrous sodium sulfate, rotary evaporated, and purified by silica gel column chromatography (DCM:MeOH = 10:1) to give the desired product A-1-10-1-19 (104.8 mg, 0.23 mmol, yield: 31.67%).
MS-ESI: [M-H]⁻ = 455.0
¹H NMR (400 MHz, DMSO-d₆) δ 7.76-7.65 (m, 2H), 7.54 (dd, *J =* 8.6, 6.1 Hz, 1H), 7.41 (tt, *J* = 8.5, 3.2 Hz, 1H), 6.99 (dd, *J =* 6.6, 2.4 Hz, 1H), 6.98-6.91 (m, 2H), 6.87 (ddd, *J = 8.7,* 5.7, 2.5 Hz, 1H), 6.31 (s, 1H), 5.91-5.77 (m, 1H), 1.59 (d, *J =* 6.4 Hz, 3H).

### Preparation Example (III) 12: Compound A-1-2-1-44

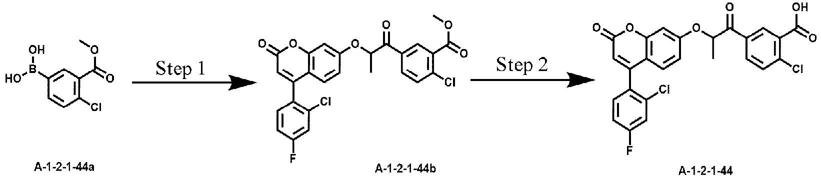

### Step 1

In a dry 100 mL three-necked flask, A-1-2-1-33c (1.00 g, 2.76 mmol) and A-1-2-1-44a (1.18 g, 5.52 mmol) were added. After purging with nitrogen, tetrakis(triphenylphosphine)palladium (0.16 g, 0.14 mmol) was added and dissolved in 15 mL of dioxane solution. Finally, dimethyl dicarbonate (0.74 g, 5.52 mmol) was added. The reaction was carried out at 115°C for 2 h. (5-(methoxycarbonyl)furan -2-yl)boronic acid (469 mg, 2.76 mmol). After purging with nitrogen, tetrakis(triphenylphosphine)palladium (80 mg, 0.069 mmol) was added and dissolved in 5 mL of dioxane solution. Finally, dimethyl dicarbonate (463 mg, 3.45 mmol) was added. The reaction was carried out at 115°C for 2 h (preferably 2-4 h). After LCMS analysis showed that the reaction was complete, the mixture was cooled to room temperature and filtered. The filtrate was collected, and 10 mL of water was added. Then, the mixture was extracted with ethyl acetate (10 mL × 3), washed with 20 mL of saturated brine, dried over anhydrous sodium sulfate, and purified by silica gel column chromatography (PE:EA = 3:1) to give the intermediate A-1-2-1-44b (0.33 g, 0.64 mmol, yield: 23.13%).
MS-ESI: [M-H]⁻ = 513.1

### Step 2

In a dry 50 mL three-necked flask, A-1-2-1-44b (300 mg, 0.58 mmol) was added and dissolved in 2 mL of tetrahydrofuran, and the system was cooled at 0°C. Then, 2 mL of 2M aqueous sodium hydroxide solution was added. The reaction was carried out for 30 min (preferably 0.5-2 h). After TLC showed that the reaction was complete, the mixture was adjusted to pH = 1 with 1M aqueous hydrochloric acid solution, extracted with ethyl acetate (5 mL × 3), washed with saturated brine (5 mL × 2), dried over anhydrous sodium sulfate, rotary evaporated, and purified by silica gel column chromatography (DCM:MeOH = 10:1) to give the desired product A-1-2-1-44 (15 mg, 0.03 mmol, yield: 4.66%).
LCMS = [M-H]⁻ = 498.9
¹H NMR (400 MHz, Chloroform-d) δ 8.69 (s, 1H), 8.13 (d, *J =* 8.4 Hz, 1H), 7.61 (d, *J =* 8.4 Hz, 1H), 7.30-7.27 (m, 1H), 7.24 (s, 1H), 7.16-7.05 (m, 1H), 6.95 (dd, *J =* 8.8, 3.0 Hz, 1H), 6.87-6.64 (m, 2H), 6.18 (s, 1H), 5.51 (d, *J =* 6.9 Hz, 1H), 1.78 (d, *J =* 6.8 Hz, 3H).

### Preparation Example (III) 13: Compound A-1-2-1-60

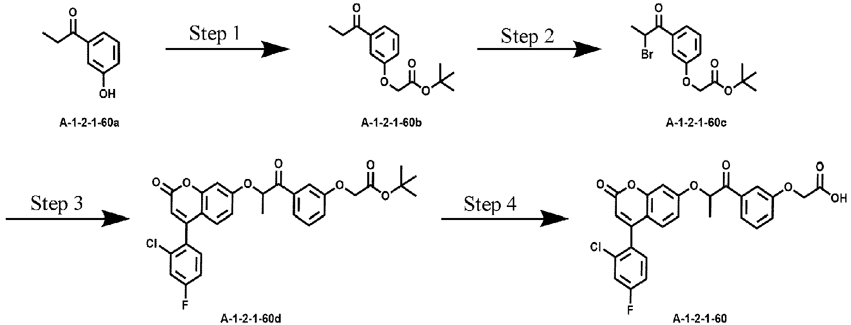

### Step 1

In a dry 500 mL three-necked flask, A-1-2-1-60a (20.00 g, 76.65mmol), tert-butyl 2-bromoacetate (1.36 g, 6.99 mmol) and potassium carbonate (1.61 g, 11.65mmol) were added, dissolved in 12 mL of acetone, and stirred at 25°C for 6 h (preferably 6-10 h). After LCMS showed that the reaction was complete, 10 mL of water was added. The mixture was extracted with ethyl acetate (10 mL × 3), and the layers were separated. The organic phases were combined, washed with saturated sodium chloride solution (20 mL), dried over anhydrous sodium sulfate, and purified by silica gel column chromatography (PE:EA = 5:1) to give the intermediate A-1-2-1-60b (1.20 g, 4.54 mmol, yield: 97.40%).
MS-ESI: [M-^{t}Bu]⁺ = 209.2

### Step 2

In a dry 100 mL three-necked flask, A-1-2-1-60b (1.20 g, 4.54 mmol) and pyridinium tribromide (1.45 g, 4.54 mmol) were dissolved in 12 mL of tetrahydrofuran and stirred at 25°C for 2 h (preferably 2-4 h). After LCMS showed that the reaction was complete, the insoluble salts were removed by filtration, and then water (10 mL) was added. The mixture was extracted with ethyl acetate (10 mL × 3), and the layers were separated. The organic phases were combined, washed with saturated sodium chloride solution (20 mL), dried over anhydrous sodium sulfate, and purified by silica gel column chromatography (PE:EA = 5:1) to give the intermediate A-1-2-1-60c (1.20 g, 3.83 mmol, yield: 77.01%).

¹H NMR (400 MHz, Chloroform-d) δ 7.62 (dt, *J =* 7.7, 1.4 Hz, 1H), 7.52 (dq, *J =* 2.7, 1.5 Hz, 1H), 7.39 (tt, *J =* 7.9, 1.7 Hz, 1H), 7.15 (ddt, *J =* 8.4, 2.8, 1.3 Hz, 1H), 5.27 (q, *J =* 6.6 Hz, 1H), 4.57 (q, *J =* 1.7 Hz, 2H), 1.88 (dt, *J =* 6.7, 1.6 Hz, 3H), 1.49 (t, *J =* 1.5 Hz, 9H).

### Step 3

In a dry 100 mL three-necked flask, A-1-2-1-60c (1.20 g, 3.83 mmol) was dissolved in 10 mL of acetone. Tetrabutylammonium iodide (1.41 g, 3.83 mmol) and potassium carbonate (1.06 g, 7.66 mmol) were added and stirred to disperse them. Then, dichloromethane was added. Finally, A-1-1-1c (0.85 g, 5.75 mmol) was added and stirred at room temperature overnight. After LCMS showed that the reaction was complete, the reaction solution was filtered. The filtrate was collected, diluted with 10 mL of water, and extracted with ethyl acetate (10 mL × 3). The layers were separated. The organic phases were combined, washed with saturated sodium chloride solution (20 mL), dried over anhydrous sodium sulfate, and purified by silica gel column chromatography (DCM:MeOH = 15:1, 1:1000 AcOH) to give the intermediate A-1-2-1-60d (0.86 g, 1.56 mmol, yield: 44.48%).
MS-ESI: [M-H]⁻ = 551.2

### Step 4

In a dry 50 mL three-necked flask, A-1-2-1-60d (860 mg, 1.56 mmol) was added and dissolved in trifluoroacetic acid (10 mL) and 10 mL of dichloromethane solution. The reaction was carried out at 25°C for 2 h (preferably 2-4 h). After TLC showed that the reaction was complete, water (20 mL) was added to quench the reaction. The reaction solution was extracted with ethyl acetate (20 mL × 3), washed with saturated brine (40 mL), dried over anhydrous sodium sulfate, and purified by silica gel column chromatography (PE:EA = 2:1) to give the desired product A-1-2-1-60a (386. mg, 0.78 mmol, yield: 49.53%).
MS-ESI: [M-H]⁻ = 495.1
¹H NMR (400 MHz, Chloroform-d) δ 7.68 (d, *J =* 7.6 Hz, 1H), 7.56 (dd, *J =* 5.3, 2.6 Hz, 1H), 7.44 (t, *J =* 8.0 Hz, 1H), 7.30-7.26 (m, 1H), 7.25-7.19 (m, 2H), 7.12 (ddd, *J =* 11.5, 7.8, 3.2 Hz, 1H), 6.94 (dd, *J* = 9.2, 3.6 Hz, 1H), 6.83-6.67 (m, 2H), 6.17 (s, 1H), 5.58 (q, *J=* 6.8 Hz, 1H), 4.74 (*d, J* = 1.4 Hz, 2H), 1.74 (dd, *J =* 6.7, 1.4 Hz, 3H).

### Preparation Example (III) 14: Compound A-1-2-1-93

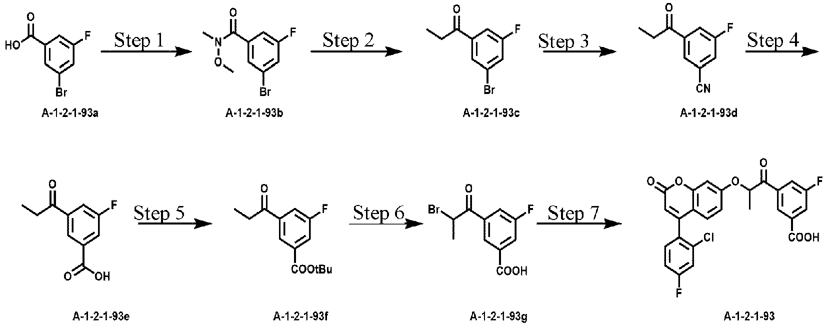

### Step 1

In a dry 100 mL three-necked flask, compound A-1-2-1-93a (4.10 g, 18.72 mmol) and me thoxymethanamine hydrochloride (1.39 g, 20.59 mmol) were dissolved in N,N-dimethylform amide (40 mL). Benzotriazole-N,N,N',N'-tetramethyluronium hexafluorophosphate (7.81 g, 20. 59 mmol), 1-hydroxybenzotriazole (2.78 g, 20.59 mmol) and N,N-diisopropylethyl amine (1 2.1 g, 93.6 mmol) were added under an ice bath. The reaction was carried out at room te mperature for 2 h. Water was added to quench the reaction. The mixture was extracted wi th ethyl acetate three times. The organic phases were combined, washed successively with 1M hydrochloric acid, 1M sodium hydroxide and saturated brine, dried over anhydrous so dium sulfate, and rotary evaporated to give a crude product. The crude product was purifi ed by silica gel column chromatography (0-10% EA:PE) to give A-1-2-1-93b as a colorles s oil (4.85 g, 17.58 mmol, yield: 93.91%, purity: 95%).
MS-ESI: [M+H]⁺ = 261.9

### Step 2

In a dry 100 mL three-necked flask, compound A-1-2-1-93b (4.35 g, 16.6 mmol) was dissolved in anhydrous tetrahydrofuran (87 mL). Ethylmagnesium bromide (2M, 12.45 mL, 24.9 mmol) was added dropwise under an ice bath. After that, the reaction was carried out at room temperature for 1 h. Saturated ammonium chloride was added to quench the reaction. The mixture was extracted with ethyl acetate three times. The organic phases were combined, washed successively with saturated sodium bicarbonate and saturated brine, dried over anhydrous sodium sulfate, and rotary evaporated to give a crude product. The crude product was purified by silica gel column chromatography (PE:EA = 20:1) to give A-1-2-1-93c as a white solid (3.87 g, 15.91 mmol, yield: 95.86%, purity: 95%).

¹H NMR (600 MHz, Chloroform-d) δ 7.88 (t, *J =* 1.6 Hz, 1H), 7.58 (ddd, *J =* 8.9, 2.4, 1.4 Hz, 1H), 7.44 (ddd, *J =* 7.7, 2.5, 1.7 Hz, 1H), 2.96 (q, *J =* 7.2 Hz, 2H), 1.23 (t, *J =* 7.2 Hz, 3H).

### Step 3

In a dry 100 mL three-necked flask, compound A-1-2-1-93c (3.90 g, 16.88 mmol) was dissolved in anhydrous N,N-dimethylformamide (87 mL). Cuprous cyanide (4.54 g, 50.64 mmol) was added. After purging with nitrogen four times, the system was refluxed at 185°C under nitrogen protection for three hours (a lot of bubbles were generated at the beginning of the reaction, and the bubbles disappeared after completion of the reaction). After removal of N,N-dimethylformamide by concentration under reduced pressure, 50 mL of water and 120 mL of ethyl acetate were added and stirred for 10 minutes. The mixture was filtered, and the filter cake was rinsed with ethyl acetate. The filtrate was allowed to stand for layer separation, and the aqueous phase was extracted with ethyl acetate once. The organic phases were combined, washed successively with water and saturated brine, dried over anhydrous sodium sulfate, and rotary evaporated to give a crude product. The crude product was purified by silica gel column chromatography (PE:EA = 20:1) to give A-1-2-1-93d as a white solid (2.08 g, 11.62 mmol, yield: 68.86%, purity: 99%).

¹H NMR (400 MHz, Chloroform-d) δ 8.04 (t, *J =* 1.4 Hz, 1H), 7.89 (ddd, *J =* 8.8, 2.5, 1.4 Hz, 1H), 7.55 (ddd, *J =* 7.5, 2.6, 1.4 Hz, 1H), 3.00 (q, *J =* 7.2 Hz, 2H), 1.26 (t, *J* = 7.2 Hz, 3H).

### Step 4

In a dry 100 mL three-necked flask, compound A-1-2-1-93d (2.08 g, 11.74 mmol) was dissolved in dioxane (17.60 mL). Aqueous sodium hydroxide solution (4M, 17.6 mL, 70.44 mmol) was added. The reaction was carried out at 95°C for 3 h. The mixture was cooled to room temperature, diluted with water, and washed with ethyl acetate once. The aqueous phase was adjusted to pH = 2-3 with concentrated hydrochloric acid and extracted with ethyl acetate twice. The organic phases were combined, washed with saturated brine, dried over anhydrous sodium sulfate, and rotary evaporated to give A-1-2-1-93e as a colored solid (2.03 g, 9.83 mmol, yield: 83.74%, purity: 95%).
MS-ESI: [M-H]⁻= 195.3

### Step 5

In a dry 100 mL three-necked flask, compound A-1-2-1-93e (1.93 g, 9.84 mmol) was dissolved in tert-butanol (19 mL). 4-Dimethylaminopyridine (481 mg, 3.94 mmol) and di-tert-butyl dicarbonate (2.58 g, 25.58 mmol) were added. The reaction was carried out at room temperature for 3 h. The reaction was monitored by TLC for completion. The reaction solution was rotary evaporated, dissolved with ethyl acetate, washed successively with 1M hydrochloric acid, saturated sodium bicarbonate solution and saturated brine, dried over anhydrous sodium sulfate, and rotary evaporated to give a crude product. The crude product was purified by silica gel column chromatography (PE:EA = 10:1) to give A-1-2-1-93f as a white solid (1.25 g, 4.71 mmol, yield: 47.84%, purity: 95%).

¹H NMR (400 MHz, Chloroform-d) δ 8.34 (t, *J =* 1.4 Hz, 1H), 7.83 (dddd, *J =* 11.9, 8.9, 2.6, 1.4 Hz, 2H), 3.03 (q, *J =* 7.2 Hz, 2H), 1.62 (s, 9H), 1.24 (t, *J =* 7.2 Hz, 3H).

### Step 6

In a dry 50 mL three-necked flask, compound A-1-2-1-93f (100 mg, 396.38 µmol) was dissolved in dichloromethane (1 mL). Then, 48% hydrobromic acid (4.5 µL) was added, and bromine (63 mg, 396.38 µmol, 20.4 µL) was added dropwise. After that, the reaction was carried out at room temperature for 16 h. An additional 1 equivalent of bromine (63 mg, 396.38 µmol, 20.4 µL) was added, and the reaction was allowed to proceed at room temperature for 3 h. The reaction was monitored by LCMS for completion. The reaction solution was dried over anhydrous sodium sulfate and rotary evaporated to give the crude compound A-1-2-1-93g as a pale yellow solid (107 mg, 350.09 µmol, yield: 88.32%, purity: 90%). MS-ESI: [M-H]⁻= 273.2.

### Step 7

In a dry 50 mL three-necked flask, compound A1-1-1-1c (101 mg, 368.11 µmol) was dissolved in anhydrous acetonitrile (1 mL). Triethylamine (112 mg, 1.10 mmol) and compound A-1-2-1-93g (107 mg, 368.11 µmol) were added. The reaction was carried out at room temperature for 5 h and monitored by LCMS for completion. The mixture was diluted with water and extracted with ethyl acetate three times. The organic phases were combined, washed with saturated brine, dried over anhydrous sodium sulfate, and rotary evaporated to give a crude product. The crude product was purified by medium-pressure reverse phase chromatography to give A-1-2-1-93 as a white solid (43 mg, 85.15 µmol, yield: 23.13%, purity: 95.34%).

MS-ESI: [M+H]⁺ = 485.4.

¹H NMR (400 MHz, DMSO-d₆) δ 13.65 (s, 1H), 8.37 (q, *J* = 1.3 Hz, 1H), 8.19 (*dq, J =* 9.1, 2.2 Hz, 1H), 7.99 (ddd, *J =* 8.7, 2.6, 1.4 Hz, 1H), 7.69 (dt, *J =* 8.9, 2.3 Hz, 1H), 7.56 (ddd, *J =* 8.6, 6.1, 2.6 Hz, 1H), 7.43 (tdd, *J =* 8.5, 4.1, 2.5 Hz, 1H), 7.13 (dd, *J =* 3.5, 2.3 Hz, 1H), 6.98 - 6.86 (m, 2H), 6.35 - 6.25 (m, 2H), 1.58 (d, *J =* 6.7 Hz, 3H).

### Preparation Example (III) 15: Compound A-1-2-1-40

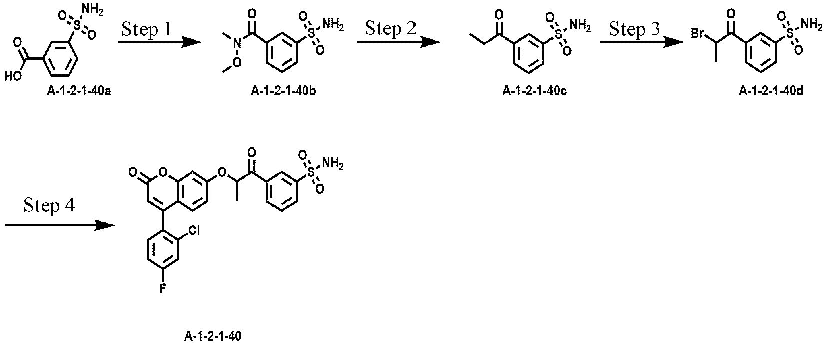

### Step 1

In a dry 100 mL three-necked flask, compound A-1-2-1-40a (2.00 g, 9.94 mmol), methoxymethylamine (1.05 g, 10.44 mmol), triethylamine (1.06 g, 10.44 mmol) and 1-(3-dimethylaminopropyl)-3-ethylcarbodiimide hydrochloride (2.00 g, 10.44 mmol) were dissolved in N,N-dimethylformamide (30 mL). The reaction was carried out at room temperature for 3 h. The N,N-dimethylformamide was removed by concentration, and water was added. Then, the mixture was extracted with ethyl acetate three times. The organic phases were combined and rotary evaporated. The crude product was purified by silica gel column chromatography to give A-1-2-1-40b (1.77 g, 6.17 mmol, yield: 62.10%, purity: 85%) as a colorless liquid.

MS-ESI: [M+H]⁺ = 244.9.

### Step 2

In a dry 10 mL three-necked flask, compound A-1-2-1-40b (1.77 g, 7.26 mmol) was dissolved in tetrahydrofuran (35 mL). Ethylmagnesium bromide (14.5 mL, 29.05 mmol) was added dropwise at 0°C. The reaction was carried out at room temperature for 2 h. A saturated ammonium chloride solution was added at 0°C to quench the reaction. The mixture was extracted with ethyl acetate three times. The organic phases were combined and rotary evaporated. The crude product was purified by silica gel column chromatography to give A-1-2-1-40c as a colorless liquid (718 mg, 3.37 mmol, yield: 46.36%, purity: 95%).

MS-ESI: [M+H]⁺ = 214.2.

### Step 3

In a dry 50 mL three-necked flask, compound A-1-2-1-40d (718 mg, 3.37 mmol) and hydrobromic acid (272 mg, 3.37 mmol) were dissolved in dichloromethane (15 mL). Bromine (538 mg, 3.37 mmol) was added at 0°C. The reaction was carried out at room temperature for 3 h. The solvent was removed by concentration. The crude product was purified by silica gel column chromatography to give A-1-2-1-40e as a colorless liquid (700 mg, 2.40 mmol, yield: 71.16%, purity: 100%).

MS-ESI: [M+H]⁺ = 292.2.

### Step 4

In a dry 50 mL three-necked flask, A1-1-1c (200 mg, 688 µmol), compound A-1-2-1-40e (201 mg, 688 µmol) and cesium carbonate (560 mg, 1.72 mmol) were dissolved in acetonitrile (10 mL). The reaction was carried out at room temperature for 2 h. The reaction solution was filtered and then purified by high-pressure reverse phase preparative chromatography and silica gel normal phase chromatography to give A-1-2-1-40 as a colored solid (45 mg, 88 µmol, yield: 12.86%, purity: 98.7%).

¹H NMR (400 MHz, DMSO-d₆) δ 8.42 (t, *J* = 1.8 Hz, 1H), 8.35 (ddd, *J =* 7.0, 2.9, 1.3 Hz, 1H), 8.13 (dt, *J =* 8.1, 1.2 Hz, 1H), 7.81 (t, *J =* 7.8 Hz, 1H), 7.69 (dt, *J =* 8.9, 2.4 Hz, 1H), 7.59 - 7.50 (m, 3H), 7.42 (tdd, *J =* 8.5, 4.1, 2.6 Hz, 1H), 7.09 (dd, *J =* 5.2, 2.4 Hz, 1H), 6.98 - 6.86 (m, 2H), 6.35 - 6.30 (m, 1H), 6.29 - 6.22 (m, 1H), 1.68 - 1.53 (m, 3H). ESI-LCMS: m:z 502.0[M+H]⁺.

### Preparation Example (III) 16: Compound A-1-2-1-94

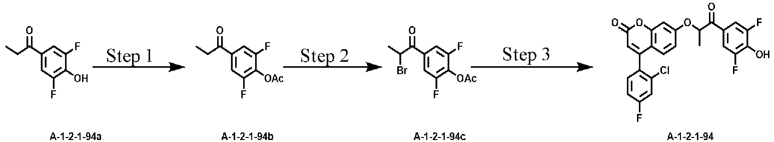

### Step 1

In a dry 50 mL three-necked flask, compound A-1-2-1-94a (500 mg, 2.69 mmol) and 4-dimethylaminopyridine (83 mg, 671 µmol) were dissolved in dichloromethane (20 mL). Acetyl chloride (232 mg, 2.95 mmol) was added dropwise at room temperature. The reaction was carried out at 25°C for 1.5 h. Dichloromethane was removed by concentration. The crude product was purified by silica gel column chromatography to give A-1-2-1-94b as a white solid (248 mg, 1.09 mmol, yield: 40.46%, purity: 98%).

MS-ESI: [M+H]⁺= 229.0.

### Step 2

In a dry 50 mL three-necked flask, compound A-1-2-1-94b (248 mg, 1.09 mmol) and hydrobromic acid (88 mg, 1.09 mmol) were dissolved in dichloromethane (8 mL). Bromine (521 mg, 3.26 mmol) was added at 0°C. The reaction was carried out at 20°C for 16 h. Dichloromethane was removed by concentration. The crude product was purified by silica gel column chromatography to give A-1-2-1-94c as a yellow solid (281 mg, 915 µmol, yield: 84.20%, purity: 95%).

MS-ESI: [M+H]⁺ = 307.0.

### Step 3

In a dry 50 mL three-necked flask, compounds A-1-2-1-94c (295 mg, 960 µmol), A1-1-1c (279 mg, 960 µmol) and cesium carbonate (782 mg, 2.40 mmol) were dissolved in acetonitrile (8 mL). The reaction was carried out at 40°C for 3 h. The reaction solution was filtered and then purified by high-pressure reverse phase preparative chromatography to give A-1-2-1-94 as a white solid (52 mg, 104 µmol, yield: 10.84%, purity: 95.1%).

MS-ESI: [M+H]⁺ = 474.9.

¹H NMR (600 MHz, DMSO-d₆) δ 11.59 (s, 1H), 7.82 (dt, *J =* 7.4, 1.4 Hz, 2H), 7.69 (dt, *J =* 8.8, 2.8 Hz, 1H), 7.55 (ddd, *J =* 8.5, 6.1, 2.4 Hz, 1H), 7.46 - 7.40 (m, 1H), 7.02 (dd, *J =* 8.0, 2.5 Hz, 1H), 6.94 (d, *J =* 8.9 Hz, 1H), 6.86 (ddd, *J =* 13.1, 8.9, 2.5 Hz, 1H), 6.31 (d, *J =* 1.3 Hz, 1H), 6.21 - 6.14 (m, 1H), 1.55 (dd, *J =* 6.7, 1.6 Hz, 3H).

### Preparation Example (III) 17: Compound A-1-11-1-1

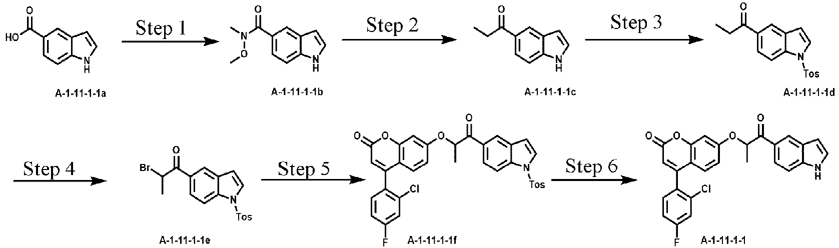

### Step 1

In a dry 100 mL three-necked flask, compound A-1-11-1-1a (4.00 g, 24.82 mmol) and N-methoxymethanamine hydrochloride (2.88 g, 29.78 mmol) were dissolved in N,N-dimethylformamide (30 mL). 2-(7-Azabenzotriazole)-N,N,N:N'-tetramethyluronium hexafluorophosphate (12.26 g, 32.27 mmol) and N,N-diisopropylethyl amine (9.62 g, 74.46 mmol) were successively added thereto and stirred at room temperature for 2 h. Water was added, and the mixture was extracted with ethyl acetate. The organic phases were combined, dried, filtered, and rotary evaporated to give a crude product. The crude product was purified by reverse phase chromatography to give A-1-11-1-1b as a white solid (3.30 g, 16.16 mmol, yield: 65.10%).
MS-ESI: [M+H]⁺ = 205.0

### Step 2

In a dry 100 mL three-necked flask, A-1-11-1-1b (2.00 g, 9.79 mmol) was dissolved in tetrahydrofuran (20 mL). Ethylmagnesium bromide (2 M, 24.48 mL) was slowly added to the reaction solution under an ice bath, and the temperature was raised to 50°C. The reaction was carried out for 10 h and then quenched with saturated aqueous ammonium chloride solution. The reaction solution was extracted with ethyl acetate. The organic phases were combined, dried, filtered, and purified by normal phase chromatography to give A-1-11-1-1c as a yellow oil (840 mg, 4.12 mmol, yield: 42.09%).
MS-ESI: [M+H]⁺ = 174.2

### Step 3

In a dry 50 mL three-necked flask, A-1-11-1-1c (860 mg, 4.97 mmol) was dissolved in tetrahydrofuran (10 mL). Sodium hydride (179 mg, 7.45 mmol) was added thereto under an ice bath and stirred under an ice bath for 0.5 h. Then, p-toluenesulfonyl chloride (644 mg, 7.45 mmol) was added. The reaction was carried out at room temperature for 2 h and then quenched with water. The mixture was extracted with ethyl acetate. The organic phases were combined, dried, filtered, and rotary evaporated to give a crude product. The crude product was purified by normal phase chromatography to give A-1-11-1-1d as a yellow solid (1.00 g, 3.05 mmol, yield: 61.52%).
MS-ESI: [M+H]⁺ = 328.0

### Step 4

In a dry 50 mL three-necked flask, compound A-1-11-1-1d (290 mg, 885.78 µmol) was dissolved in tetrahydrofuran (3 mL). Phenyltrimethyl ammonium tribromide (399 mg, 1.06 mmol) was added thereto, and the reaction was carried out at room temperature for 2 h. The mixture was filtered and rotary evaporated to give A-1-11-1-1e (359 mg, crude), which was directly used in the next step.
MS-ESI: [M+H]⁺ = 405.9

### Step 5

In a dry 50 mL three-necked flask, compounds A-1-11-1-1e (634 mg, crude) and A1-1-1c (381 mg, 1.30 mmol) were dissolved in acetonitrile (10 mL). Potassium carbonate (180 mg, 1.30 mmol) was added thereto. The reaction was carried out at 45°C for 2 h. The mixture was filtered, and the solvent was rotary evaporated. The resulting crude product was purified by normal phase chromatography to give A-1-11-1-1f (530 mg, 857.51 µmol, yield: 65.87%).
MS-ESI: [M+H]⁺ = 616.4

### Step 6

In a dry 50 mL three-necked flask, compound A-1-11-1-1f (250 mg, 404.48 µmol) was dissolved in methanol (10 mL) and tetrahydrofuran (5 mL). Cesium carbonate (263 mg, 808.97 µmol) was added. The reaction was carried out at 55°C for 2 h. The mixture was filtered, and the solvent was rotary evaporated. The residue was purified by reverse phase chromatography to give A-1-11-1-1 as a white solid (70 mg, 150.90 µmol, yield: 37.31%).
MS-ESI: [M+H]⁺ = 462.4
¹H NMR (600 MHz, DMSO-*d₆*) δ 11.59 (br.s, 1H), 8.51 (t, *J =* 2.1 Hz, 1H), 7.80 (dd, *J =* 8.5, 1.7 Hz, 1H), 7.68 (ddd, *J =* 8.9, 4.4, 2.6 Hz, 1H), 7.57 - 7.49 (m, 3H), 7.41 (qd, *J =* 8.3, 2.6 Hz, 1H), 7.01 - 6.85 (m, 3H), 6.65 (s, 1H), 6.34 - 6.22 (m, 2H), 1.61 (dd, *J =* 6.7, 1.7 Hz, 3H).

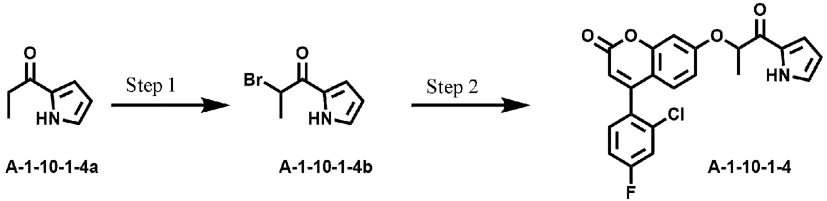

### Preparation Example (III) 18: Compound A-1-10-1-4

### Step 1

In a dry 50 mL three-necked flask, compound A-1-10-1-4a (1.20 g, 9.9 mmol) was dissolved in chloroform (15 mL) and ethyl acetate (15 mL). Copper dibromide (4.40 g, 19.9 mmol) was added at room temperature, and the temperature was raised to 65°C. The reaction was carried out for 5 h. The mixture was filtered, and the solvent was rotary evaporated. The resulting crude product was purified by reverse phase chromatography to give A-1-10-1-4b as a gray solid (500 mg, 2.47 mmol, yield: 24.78%).
MS-ESI: [M+H]⁺ = 202.0

### Step 2

In a dry 50 mL three-necked flask, compounds A-1-10-1-4b (100 mg, 494.92 µmol) and A1-1-1c (110 mg, 380.7 µmol) were dissolved in acetonitrile (5 mL). Potassium carbonate (131 mg, 0.95 mmol) was added. The reaction was carried out at 45°C for 10 h. The mixture was filtered and rotary evaporated to give a crude product. The crude product was purified by reverse phase chromatography to give A-1-10-1-4 as a pink solid (66 mg, 160.27 µmol, yield: 42.1%, purity: 95%).
¹H NMR (400 MHz, DMSO-*d₆*) δ 12.00 (s, 1H), 7.68 (dt, *J =* 8.9, 2.4 Hz, 1H), 7.54 (ddd, *J =* 8.7, 6.1, 1.3 Hz, 1H), 7.41 (tdd, *J* = 8.5, 3.6, 2.5 Hz, 1H), 7.33 (ddt, *J* = 4.8, 3.5, 2.0 Hz, 1H), 7.26-7.11 (m, 1H), 7.01-6.91 (m, 2H), 6.88 (ddd, *J* = 8.9, 3.7, 2.4 Hz, 1H), 6.37-6.23 (m, 2H), 5.76 (p, *J =* 6.5 Hz, 1H), 1.58 (dd, *J =* 6.6, 1.3 Hz, 3H).
MS-ESI: [M+H]⁺ = 412.0

### Preparation Example (III) 19: Compound A-1-10-1-5

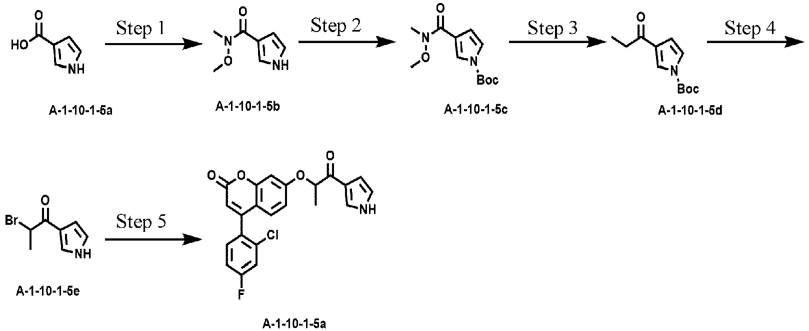

### Step 1

In a dry 50 mL three-necked flask, compound A-1-10-1-5a (1.00 g, 9.00 mmol) and N-methoxymethanamine (1.13 g, 11.70 mmol, hydrochloride salt) were dissolved in N,N-dimethylformamide (15 mL). 2-(7-Azabenzotriazole)-*N,N,N',N*'-tetramethyluronium hexafluorophosphate (4.42 g, 11.70 mmol) and N,N-diisopropylethyl amine (3.49 g, 27.00 mmol) were successively added, and the temperature was slowly raised to 60°C overnight. Water was added to quench the reaction, and the mixture was extracted with ethyl acetate. The organic phases were combined, dried, filtered, and rotary evaporated to give a crude product. The crude product was purified by reverse phase chromatography to give A-1-10-1-5b as a white solid (1.20 g, 4.67 mmol, yield: 51.89%, purity: 60%).
MS-ESI: [M+H]⁺= 155.1

### Step 2

In a dry 50 mL three-necked flask, compound A-1-10-1-5b (1.10 g, 4.28 mmol) was dissolved in dichloromethane (20 mL). Di-tert-butyl dicarbonate (1.39 g, 6.42 mmol), triethylamine (650 mg, 6.42 mmol) and 4-dimethylaminopyridine (52 mg, 428.11 µmol) were successively added thereto. The reaction was carried out at room temperature for 2 h. The solvent was rotary evaporated, and the residue was purified by normal phase chromatography to give A-1-10-1-5c as a white oil (410 mg, 1.61 mmol, yield: 37.66%).
MS-ESI: [M+H]⁺= 255.0

### Step 3

In a dry 50 mL three-necked flask, compound A-1-10-1-5c (410 mg, 1.61 mmol) was dissolved in tetrahydrofuran (5 mL). Ethylmagnesium bromide (2 M, 2.42 mL) was added under an ice bath, and then the temperature was raised to room temperature. The reaction was carried out for 2 h and then quenched with saturated ammonium chloride. The mixture was extracted with ethyl acetate. The organic phases were combined, dried, filtered, rotary evaporated, and purified by normal phase chromatography to give A-1-10-1-5d as a colorless oil (270 mg, 1.21 mmol, yield: 75.00%). MS-ESI: [M+H]⁺= 224.4

### Step 4

In a dry 50 mL three-necked flask, compound A-1-10-1-5d (352 mg, 1.58 mmol) was dissolved in chloroform (5 mL) and ethyl acetate (5 mL). Copper bromide (703 mg, 3.15 mmol) was added thereto. The system was heated to 65°C and reacted for 2 h. LCMS showed that the main component was the product. The mixture was filtered and rotary evaporated to give A-1-10-1-5e (318 mg, crude), which was directly used in the next step.
MS-ESI: [M+H]⁺= 201.9

### Step 5

In a dry 50 mL three-necked flask, compound A-1-10-1-5e (318 mg, 1.57 mmol) and A1-1-1c (458 mg, 1.57 mmol) were dissolved in acetonitrile (10 mL). Potassium carbonate (434 mg, 3.15 mmol) was added thereto. The reaction was carried out at 45°C for 10 h. The mixture was filtered and rotary evaporated. The resulting crude product was subjected to reverse phase chromatography and high-performance liquid chromatography to give A-1-10-1-5c (60 mg, 144.97 µmol, yield: 9.23%, purity: 99.5%).
MS-ESI: [M+H]⁺= 412.0
¹H NMR (400 MHz, DMSO-*d₆*) δ 11.67 (s, 1H) , 7.86 (tt, *J =* 3.4, 1.6 Hz, 1H), 7.68 (dt, *J =* 8.9, 2.3 Hz, 1H), 7.54 (ddd, *J* = 8.6, 6.1, 1.2 Hz, 1H), 7.41 (tdd, *J =* 8.5, 3.7, 2.5 Hz, 1H), 7.00 - 6.78 (m, 4H), 6.56 (q, *J =* 2.3 Hz, 1H), 6.29 (d, *J =* 1.4 Hz, 1H), 5.77-5.63 (m, 1H), 1.55 (dd, *J =* 6.6, 1.1 Hz, 3H).

### Preparation Example (III) 20: Compound A-2-2-1-1

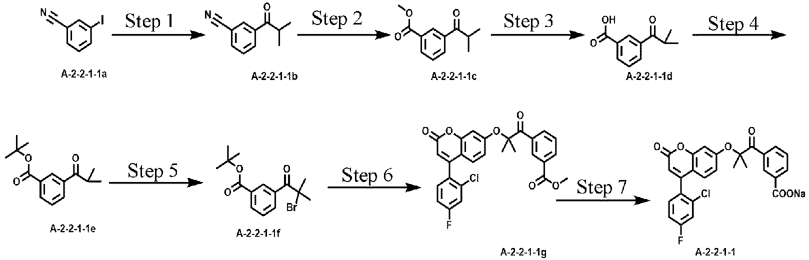

### Step 1

In a dry 50 mL three-necked flask, tetrahydrofuran (10 mL) and pentamethylene magnesium bromide (1.23 g, 4.4 mmol) were added. Copper cyanide di(lithium chloride) complex solution (589 mg, 4.4 mL, 4.4 mmol) was added dropwise to the three-necked flask at -78°C. After reacting for 0.5 h, A-2-2-1-1a (1.00 g, 4.37 mmol) was added at -78°C. The reaction was allowed to proceed at room temperature for 1 h. Then, isobutyryl chloride (698 mg, 6.55 mmol) was added. The reaction was carried out for 0.5 h before sending a sample for monitoring. After completion of the reaction, the reaction was quenched with saturated ammonium chloride. The mixture was extracted with EA three times, washed with saturated brine once, dried over anhydrous sodium sulfate, filtered, and rotary evaporated to give a crude product. The crude product was purified by a silica gel column (petroleum ether:ethyl acetate = 10:1) to give A-2-2-1-1b (560 mg, 2.91 mmol, yield: 66.64%).
MS-ESI: [M+H]⁺= 174.3

### Step 2

In a dry 50 mL three-necked flask, A-2-2-1-1b (560 mg, 2.91 mmol) was added to the reaction flask and dissolved in methanol (10 mL). Concentrated sulfuric acid (2.1 mL) was added, and the temperature was raised to 70°C. The reaction was carried out for 2 h before sending a sample for monitoring. After completion of the reaction, 1 N sodium hydroxide solution was added to adjust the pH to neutral. The mixture was poured into water, extracted with dichloromethane three times, washed with saturated brine once, dried over anhydrous sodium sulfate, filtered, and rotary evaporated to give the crude product A-2-2-1-1c (471 mg, crude).
MS-ESI: [M+H]⁺ = 207.3

### Step 3

In a dry 50 mL three-necked flask, A-2-2-1-1c (400 mg, crude) was added to the reaction flask and dissolved in tetrahydrofuran (5 mL). Then, lithium hydroxide (93 mg, 0.29 mmol), methanol (4 mL) and water (2 mL) were added. The reaction was carried out for 2 h before sending a sample for monitoring. After completion of the reaction, HCl was added to adjust pH. The mixture was poured into water, extracted with dichloromethane three times, washed with saturated brine once, dried over anhydrous sodium sulfate, filtered, and rotary evaporated to give the crude product A-2-2-1-1d (364 mg, crude).
MS-ESI: [M+H]⁺= 193.3

### Step 4

In a dry 50 mL three-necked flask, compound A-2-2-1-1d (364 mg, crude) was added to the reaction flask and dissolved in dichloromethane (5 mL). Then, di-tert-butyl dicarbonate (455 mg, 2.08 mmol) and 4-dimethylaminopyridine (231 mg, 1.89 mmol) were added. The reaction was carried out for 2 h before sending a sample for monitoring. After completion of the reaction, the mixture was poured into water, extracted with dichloromethane three times, washed with saturated brine once, dried over anhydrous sodium sulfate, filtered, and rotary evaporated to give the crude product A-2-2-1-1e (520 mg, crude).
MS-ESI: [M+H]⁺= 249.1

### Step 5

In a dry 50 mL three-necked flask, A-2-2-1-1e (520 mg, crude) was added to the reaction flask and dissolved in dichloromethane (5 mL). Hydrobromic acid (35 mg, 0.20 mmol) and bromine (397 mg, 2.51 mmol) were added at 0°C. The reaction was carried out at room temperature for 2 h before sending a sample for monitoring. After completion of the reaction, the mixture was concentrated and rotary evaporated to give the crude product A-2-2-1-1f (635 mg, crude).
MS-ESI: [M+H]⁺ = 327.3

### Step 6

In a dry 50 mL three-necked flask, compound A-2-2-1-1f (600 mg, crude) was added to the reaction flask and dissolved in N,N-dimethylformamide (10 mL). Then, A1-1-1c (596 mg, 2.02 mmol) and cesium carbonate (1.49 g, 4.58 mmol) were added. The reaction was carried out for 16 h before sending a sample for monitoring. After completion of the reaction, the mixture was poured into water, extracted with EA three times, washed with saturated brine twice, dried over anhydrous sodium sulfate, filtered, and rotary evaporated to give a crude product. The crude product was purified by a silica gel column (DCM:MeOH = 10:1) to give A-2-2-1-1g (110 mg, 0.20 mmol, yield: 11.14%).
MS-ESI: [M+H]⁺= 495.1

### Step 7

In a dry 50 mL three-necked flask, compound A-2-2-1-1g (80 mg, 0.15 mmol) was added to the reaction flask and dissolved in dichloromethane (2.5 mL). Then, TFA (2.5 mL) was added. The reaction was carried out at room temperature for 2 h before sending a sample for monitoring. After completion of the reaction, the mixture was concentrated and rotary evaporated to give a crude product. The crude product was purified by MPLC to give compound A-2-2-1-1 (52 mg, 0.10 mmol, yield: 66.6%).
MS-ESI: [M+H]⁺= 481.2
¹H NMR (400 MHz, DMSO-*d₆*) δ 13.29 (s, 1H), 8.71 (t, *J =* 1.8 Hz, 1H), 8.36 (d, *J =* 7.9 Hz, 1H), 8.12 (dt, *J* = 7.8, 1.4 Hz, 1H), 7.74-7.56 (m, 2H), 7.58-7.34 (m, 2H), 6.89 (d, *J* = 8.8 Hz, 1H), 6.82-6.66 (m, 2H), 6.30 (s, 1H), 1.76 (s, 6H).

### Preparation Example (III) 21: Compound A-1-2-1-83

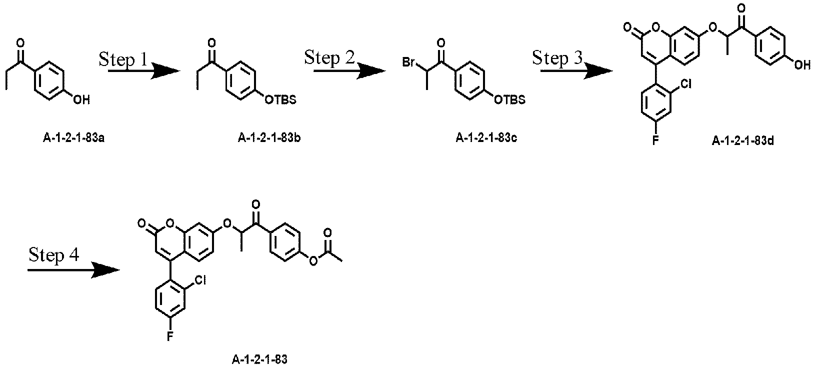

### Step 1

In a 200 mL single-necked flask, A-1-2-1-83a (1.50 g, 9.99 mmol), N,N-dimethylformamide (20 mL), imidazole (2.04 g, 30 mmol) and tert-butyldimethylsilyl chloride (3.01 g, 19.98 mmol) were successively added and then stirred at room temperature for 6 h. After TLC monitoring showed that the reaction was complete, the reaction solution was diluted with water and extracted with ethyl acetate (100 mL × 3). The organic phases were combined, washed with saturated sodium chloride solution, dried over anhydrous sodium sulfate, filtered, and rotary evaporated. The crude product was purified by flash column chromatography to give compound A-1-2-1-83b (2.00 g, 7.56 mmol, yield: 76%).
MS-ESI: [M+H]⁺ = 265.2

### Step 2

In a 100 mL single-necked flask, compound A-1-2-1-83b (500 mg, 1.89 mmol), tetrahydrofuran (10 mL), pyrrolidone hydrotribromide (1.23 g, 3.78 mmol) and 2-pyrrolidone (322 mg, 3.78 mmol) were successively added. After that, the reaction was carried out at 60°C for 6 h. After TLC monitoring showed that the reaction was complete, the reaction solution was diluted with water and extracted with ethyl acetate (50 mL × 3). The organic layers were combined, washed with saturated sodium chloride solution, dried over anhydrous sodium sulfate, filtered, and rotary evaporated. The crude product was purified by flash column chromatography to give compound A-1-2-1-83c (400 mg, 1.17 mmol, yield: 62%).
MS-ESI: [M+H]⁺ = 343.1

### Step 3

In a 100 mL single-necked flask, compound A-1-2-1-83c (380 mg, 1.11 mmol), intermediate A1-1-1c (355 mg, 1.22 mmol), tetrabutylammonium iodide (820 mg, 2.22 mmol), potassium carbonate (307 mg, 2.22 mmol) and N,N-dimethylformamide (10 mL) were successively added. After that, the reaction was carried out at room temperature overnight. After LCMS monitoring showed that the reaction was complete, the reaction solution was diluted with water and extracted with ethyl acetate (50 mL × 3). The organic layers were combined, washed with saturated sodium chloride solution, dried over anhydrous sodium sulfate, filtered, and rotary evaporated. The crude product was purified by flash column chromatography to give compound A-1-2-1-83d (110 mg, 0.25 mmol, yield: 23%).
MS-ESI: [M+H]⁺ = 439.1
¹H NMR (400 MHz, DMSO-*d₆*) δ 10.76 (d, *J =* 1.7 Hz, 1H), 7.97 (dd, *J =* 8.8, 1.3 Hz, 2H), 7.68 (dt, *J* = 8.9, 2.6 Hz, 1H), 7.54 (dd, *J =* 8.6, 6.1 Hz, 1H), 7.41 (tdd, *J =* 8.5, 4.4, 2.5 Hz, 1H), 6.98 - 6.89 (m, 4H), 6.84 (ddd, *J =* 9.1, 7.0, 2.5 Hz, 1H), 6.29 (d, *J =* 1.1 Hz, 1H), 6.12 (p, *J =* 6.7 Hz, 1H), 1.54 (d*, J =* 6.7 Hz, 3H).

### Step 4

In a 50 mL single-necked flask, intermediate A-1-2-1-83d (40 mg, 0.09 mmol) and N,N-diisopropylethyl amine (24 mg, 0.18 mmol) were dissolved in dichloromethane (2 mL). Acetyl chloride (11 mg, 0.14 mmol) was slowly added dropwise to the solution at room temperature. After that, the reaction was carried out at room temperature for 2 h. After LCMS monitoring showed that the reaction was complete, the reaction solution was diluted with dichloromethane (50 mL) and washed with saturated sodium chloride solution. The organic layer was dried over anhydrous sodium sulfate, filtered, and rotary evaporated. The crude product was purified by prep-TLC to give compound A-1-2-1-83 (20 mg, 0.04 mmol, yield: 44%).
MS-ESI: [M+H]⁺ = 481.1
¹H NMR (400 MHz, Chloroform-d) δ 8.10 (d, *J =* 8.8 Hz, 2H), 7.35 - 7.26 (m, 2H), 7.26 - 7.21 (m, 2H), 7.12 (tt, *J =* 8.2, 2.8 Hz, 1H), 6.94 (dd, *J =* 8.8, 3.9 Hz, 1H), 6.85 - 6.68 (m, 2H), 6.17 (s, 1H), *5.55* (q, *J =* 6.8 Hz, 1H), 2.34 (s, 3H), 1.76 (d, *J =* 6.8 Hz, 3H).

### Preparation Example (III) 22: Compound A-1-2-1-89

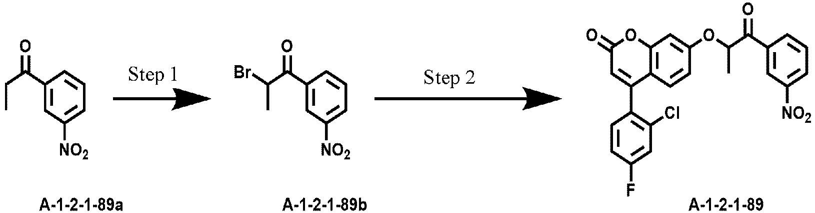

In a 100 mL single-necked flask, A-1-2-1-89a (360 mg, 2 mmol), tetrahydrofuran (10 mL), pyrrolidone hydrotribromide (1.30 g, 4 mmol) and 2-pyrrolidone (340 mg, 4 mmol) were successively added. After that, the reaction was carried out at 60°C for 6 h. After TLC monitoring showed that the reaction was complete, the reaction solution was diluted with water and extracted with ethyl acetate (50 mL × 3). The organic layers were combined, washed with saturated sodium chloride solution, dried over anhydrous sodium sulfate, filtered, and rotary evaporated. The crude product was purified by flash column chromatography to give compound A-1-2-1-89b (400 mg, 1.55 mmol, yield: 77%).
MS-ESI: [M-H]⁻ = 256.0

### Step 2

In a 100 mL single-necked flask, compound A-1-2-1-89b (380 mg, 1.47 mmol), A1-1-1c (470 mg, 1.62 mmol), tetrabutylammonium iodide (1.09 g, 2.94 mmol), potassium carbonate (406 mg, 2.94 mmol) and N,N-dimethylformamide (10 mL) were successively added. After that, the reaction was carried out at room temperature overnight. After LCMS monitoring showed that the reaction was complete, the reaction solution was diluted with water and extracted with ethyl acetate (50 mL × 3). The organic layers were combined, washed with saturated sodium chloride solution, dried over anhydrous sodium sulfate, filtered, and rotary evaporated. The crude product was purified by flash column chromatography to give compound A-1-2-1-89 (140 mg, 0.30 mmol, yield: 20%).
MS-ESI: [M+H]⁺ = 468.1
¹H NMR (400 MHz, Chloroform-d) δ 8.91 (q, *J =* 1.9 Hz, 1H), 8.47 (ddd, *J =* 8.2, 2.3, 1.0 Hz, 1H), 8.38 (dd, *J* = 7.8, 1.4 Hz, 1H), 7.72 (t, *J =* 8.0 Hz, 1H), 7.32 - 7.23 (m, 2H), 7.13 (tt, *J =* 7.8, 2.3 Hz, 1H), 6.97 (dd, *J =* 8.8, 2.6 Hz, 1H), 6.85 - 6.74 (m, 2H), 6.19 (d, *J =* 1.0 Hz, 1H), 5.55 (q, *J* = 6.9 Hz, 1H), 1.81 (d*, J =* 6.8 Hz, 3H).

### Preparation Example (III) 23: Compound A-1-10-1-39

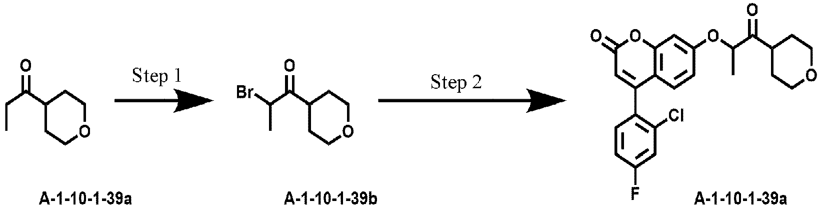

### Step 1

In a 50 mL single-necked flask, A-1-10-1-39a (150 mg, 1.05 mmol), tetrahydrofuran (5 mL), pyrrolidone hydrotribromide (684 mg, 2.10 mmol) and 2-pyrrolidone (179 mg, 2.10 mmol) were successively added. After that, the reaction was carried out at 60°C for 6 h. After TLC monitoring showed that the reaction was complete, the reaction solution was diluted with water and extracted with ethyl acetate (50 mL × 3). The organic layers were combined, washed with saturated sodium chloride solution, dried over anhydrous sodium sulfate, filtered, and rotary evaporated. The crude product was purified by flash column chromatography to give compound A-1-10-1-39b (400 mg, 1.55 mmol, yield: 77%).

¹H NMR (400 MHz, Chloroform-d) δ 4.55 (q, *J* = 6.7 Hz, 1H), 4.02 (dtd, *J* = 11.5, 4.5, 2.3 Hz, 2H), 3.46 (td, *J =* 11.0, 2.4 Hz, 2H), 3.09 (tt, *J =* 11.4, 3.9 Hz, 1H), 1.96 - 1.62 (m, 4H), 1.75 (d, *J* = 6.8 Hz, 3H).

### Step 2

In a 50 mL single-necked flask, A-1-10-1-39b (190.0 mg, 0.86 mmol), intermediate A1-1-1c (225.0 mg, 0.77 mmol), tetrabutylammonium iodide (635.0 mg, 1.72 mmol), potassium carbonate (238.0 mg, 1.72 mmol) and N,N-dimethylformamide (5 mL) were successively added. After that, the reaction was carried out at room temperature overnight. After LCMS monitoring showed that the reaction was complete, the reaction solution was diluted with water and extracted with ethyl acetate (50 mL × 3). The organic layers were combined, washed with saturated sodium chloride solution, dried over anhydrous sodium sulfate, filtered, and rotary evaporated. The crude product was purified by prep-TLC to give compound A-1-10-1-39 (110.0 mg, 0.26 mmol, yield: 30%).
MS-ESI: [M+H]⁺ = 431.1
¹H NMR (400 MHz, Chloroform-d) δ 7.33 (dd, *J =* 8.5, 2.7 Hz, 2H), 7.17 (td, *J =* 8.3, 2.6 Hz, 1H), 7.01 (d, *J =* 8.7 Hz, 1H), 6.86 - 6.73 (m, 2H), 6.24 (s, 1H), 4.86 (q, *J =* 6.8 Hz, 1H), 4.07 - 3.94 (m, 2H), 3.45 (ddt, *J =* 25.8, 13.8, 7.5 Hz, 2H), 3.09 - 2.98 (m, 1H), 1.89-1.68 (m, 4H), 1.59 (d, *J* = 6.8 Hz, 3H).

### Preparation Example (III) 24: Compound A-1-1-1-15

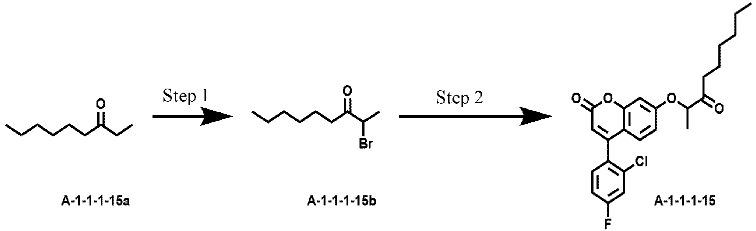

In a 50 mL single-necked flask, A-1-1-1-15a (450.0 mg, 3.16 mmol), tetrahydrofuran (5 mL), pyrrolidone hydrotribromide (1.03 g, 3.16 mmol) and 2-pyrrolidone (269.0 mg, 3.16 mmol) were successively added. After that, the reaction was carried out at room temperature for 4 h. After TLC monitoring showed that the reaction was complete, the reaction solution was diluted with water and extracted with ethyl acetate (50 mL × 3). The organic layers were combined, washed with saturated sodium chloride solution, dried over anhydrous sodium sulfate, filtered, and rotary evaporated to give the crude product A-1-1-1-15b (500.0 mg), which was directly used in the next step.

### Step 2

In a 100 mL single-necked flask, intermediate A-1-1-1-15a (500.0 mg, 2.26 mmol, crude), intermediate A-1-1-1c (657.0 mg, 2.26 mmol), tetrabutylammonium iodide (1.25 g, 3.39 mmol), potassium carbonate (625.0 mg, 4.52 mmol) and N,N-dimethylformamide (10 mL) were successively added. After that, the reaction was carried out at room temperature for 3 h. After LCMS monitoring showed that the reaction was complete, the reaction solution was diluted with water and extracted with ethyl acetate (50 mL × 3). The organic layers were combined, washed with saturated sodium chloride solution, dried over anhydrous sodium sulfate, filtered, and rotary evaporated. The crude product was purified by flash column chromatography to give compound A-1-1-1-15 (110.0 mg, 0.26 mmol, yield: 11%).

MS-ESI: [M-H]⁻ = 429.2.

¹H NMR (400 MHz, Chloroform-d) δ 7.32 (dt, *J =* 8.7, 2.7 Hz, 2H), 7.17 (td, *J =* 8.2, 2.4 Hz, 1H), 7.00 (d, *J =* 8.7 Hz, 1H), 6.86 - 6.69 (m, 2H), 6.22 (s, 1H), 4.74 (q, *J =* 6.8 Hz, 1H), 2.65 (dt, *J =* 18.5, 7.4 Hz, 1H), 2.45 (dt, *J =* 17.9, 7.3 Hz, 1H), 1.57 (t, *J =* 6.1 Hz, 5H), 1.31 - 1.19 (m, 6H), 0.88 (t, *J =* 6.6 Hz, 3H).

### Preparation Example (III) 25: Compound A-1-2-1-73

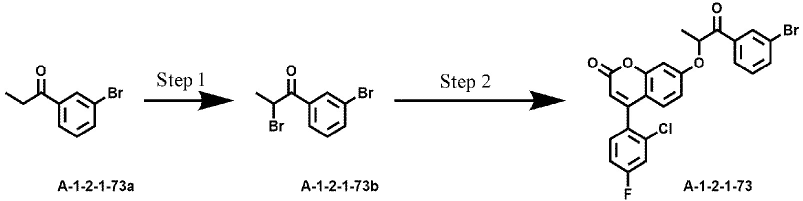

### Step 1

In a 100 mL single-necked flask, compound A-1-2-1-73a (213.0 mg, 1 mmol), tetrahydrofuran (5 mL), pyrrolidone hydrotribromide (652.0 mg, 2 mmol) and 2-pyrrolidone (170.0 mg, 2 mmol) were successively added. After that, the reaction was carried out at 60°C for 2 h. After TLC monitoring showed that the reaction was complete, the reaction solution was diluted with water and extracted with ethyl acetate (50 mL × 3). The organic layers were combined, washed with saturated sodium chloride solution, dried over anhydrous sodium sulfate, filtered, and rotary evaporated. The crude product was purified by flash column chromatography to give compound A-1-2-1-73b (250.0 mg, 0.86 mmol, yield: 86%).

MS-ESI: [M+H]⁺ = 292.9.

### Step 2

In a 100 mL single-necked flask, compound A-1-2-1-73b (250.0 mg, 0.86 mmol), intermediate A1-1-1c (165.0 mg, 0.57 mmol), tetrabutylammonium iodide (211.0 mg, 0.57 mmol), potassium carbonate (158.0 mg, 1.14 mmol) and *N*,*N*-dimethylformamide (10 mL) were successively added. After that, the reaction was carried out at room temperature for 3 h. After LCMS monitoring showed that the reaction was complete, the reaction solution was diluted with water and extracted with ethyl acetate (50 mL × 3). The organic layers were combined, washed with saturated sodium chloride solution, dried over anhydrous sodium sulfate, filtered, and rotary evaporated. The crude product was purified by flash column chromatography to give compound A-1-2-1-73 (226.0 mg, 0.45 mmol, yield: 52%).
MS-ESI: [M+H]⁺= 501.0
¹H NMR (400 MHz, DMSO-*d₆*) δ 8.23 *(d, J =* 1.9 Hz, 1H), 8.08 (dd, *J* = 8.0, 1.5 Hz, 1H), 7.93 (dd, *J=* 7.8, 2.0 Hz, 1H), 7.70 (dt, *J =* 8.9, 2.4 Hz, 1H), 7.60 - 7.53 (m, 2H), 7.48 - 7.37 (m, 1H), 7.09 (dd, *J =* 5.0, 2.4 Hz, 1H), 6.95 (d, *J* = 8.9 Hz, 1H), 6.89 (td, *J =* 8.5, 2.4 Hz, 1H), 6.32 (s, 1H), 6.30 - 6.21 (m, 1H), 1.56 (d, *J =* 6.7 Hz, 3H).

### Preparation Example (III) 26: Compound A-1-2-1-90

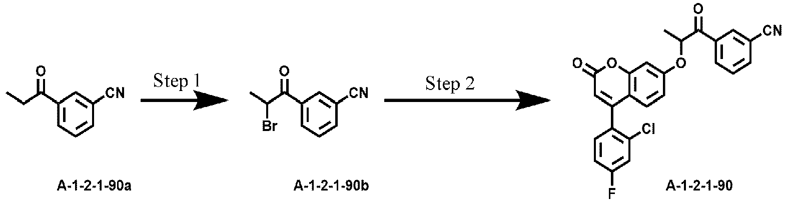

In a 100 mL single-necked flask, compound A-1-2-1-90a (100.0 mg, 0.63 mmol), tetrahydrofuran (5 mL), pyrrolidone hydrotribromide (411.0 mg, 1.26 mmol) and 2-pyrrolidone (107.0 mg, 1.26 mmol) were successively added. After that, the reaction was carried out at 60°C for 2 h. After TLC monitoring showed that the reaction was complete, the reaction solution was diluted with water and extracted with ethyl acetate (50 mL × 3). The organic layers were combined, washed with saturated sodium chloride solution, dried over anhydrous sodium sulfate, filtered, and rotary evaporated. The crude product was purified by flash column chromatography to give compound A-1-2-1-9b (118.0 mg, 0.50 mmol, yield: 79%).
MS-ESI: [M+H]⁺= 237.9

### Step 2

In a 100 mL single-necked flask, compound A-1-2-1-90b (118.0 mg, 0.50 mmol), intermediate A1-1-1c (145.0 mg, 0.50 mmol), tetrabutylammonium iodide (185.0 mg, 0.50 mmol), potassium carbonate (138.0 mg, 1 mmol) and *N*,*N*-dimethylformamide (2.5 mL) were successively added. After that, the reaction was carried out at room temperature for 3 h. After LCMS monitoring showed that the reaction was complete, the reaction solution was diluted with water and extracted with ethyl acetate (50 mL × 3). The organic layers were combined, washed with saturated sodium chloride solution, dried over anhydrous sodium sulfate, filtered, and rotary evaporated. The crude product was purified by flash column chromatography to give compound A-1-2-1-90 (30.5.0 mg, 0.07 mmol, yield: 14%).
MS-ESI: [M+H]⁺ = 448.1
¹H NMR (400 MHz, DMSO-*d₆*) δ 8.58 (q, *J =* 1.6 Hz, 1H), 8.31 (dt, *J =* 8.0, 1.5 Hz, 1H), 8.18 (dt, *J =* 7.7, 1.4 Hz, 1H), 7.80 (t, *J =* 7.8 Hz, 1H), 7.69 (dt, *J =* 8.9, 2.3 Hz, 1H), 7.56 (ddd, *J =* 8.5, 6.1, 2.4 Hz, 1H), 7.42 (tdd, *J =* 8.5, 4.1, 2.6 Hz, 1H), 7.11 (dd, *J =* 3.9, 2.3 Hz, 1H), 6.94 (d, *J =* 8.7 Hz, 1H), 6.92 - 6.87 (m, 1H), 6.32 (d, *J =* 0.9 Hz, 1H), 6.29 (td, *J =* 6.8, 4.0 Hz, 1H), 1.57 (d, *J* = 6.8 Hz, 3H).

### Preparation Example (III) 27: Compound A-1-10-1-3

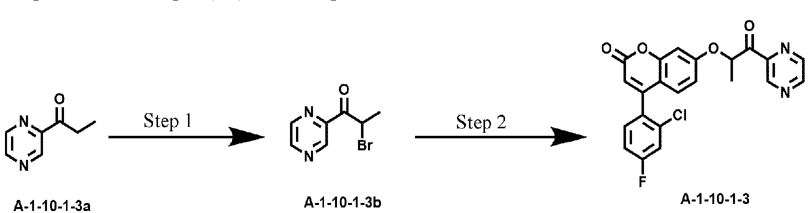

In a 50 mL single-necked flask, A-1-10-1-3a (200.0 mg, 1.47 mmol), tetrahydrofuran (5 mL), pyrrolidone hydrotribromide (951.0 mg, 2.92 mmol) and 2-pyrrolidone (248.0 mg, 2.92 mmol) were successively added. After that, the reaction was carried out at 60°C for 6 h. After TLC monitoring showed that the reaction was complete, the reaction solution was diluted with water and extracted with ethyl acetate (50 mL × 3). The organic layers were combined, washed with saturated sodium chloride solution, dried over anhydrous sodium sulfate, filtered, and rotary evaporated. The crude product was purified by flash column chromatography to give compound A-1-10-1-3b (182.0 mg, 0.85 mmol, yield: 58%).
MS-ESI: [M+H]⁺= 215.0

### Step 2

In a 50 mL single-necked flask, compound A-1-10-1-3b (182.0 mg, 0.85 mmol), intermediate A1-1-1c (163.0 mg, 0.56 mmol), tetrabutylammonium iodide (310.0 mg, 0.84 mmol), potassium carbonate (232.0 mg, 1.68 mmol) and N,N-dimethylformamide (5 mL) were successively added. After that, the reaction was carried out at room temperature overnight. After LCMS monitoring showed that the reaction was complete, the reaction solution was diluted with water and extracted with ethyl acetate (50 mL × 3). The organic layers were combined, washed with saturated sodium chloride solution, dried over anhydrous sodium sulfate, filtered, and rotary evaporated. The crude product was purified by flash column chromatography to give compound A-1-10-1-3 (39.0 mg, 0.09 mmol, yield: 16%).

MS-ESI: [M+H]⁺ = 425.1.

¹H NMR (400 MHz, DMSO-*d₆*) δ 9.17 (s, 1H), 9.00 (d, *J =* 2.4 Hz, 1H), 8.89 (s, 1H), 7.69 (dt, *J* = 8.9, 2.5 Hz, 1H), 7.55 (ddd, *J =* 8.7, 6.2, 2.7 Hz, 1H), 7.46 - 7.38 (m, 1H), 7.00 (dd, *J =* 6.8, 2.2 Hz, 1H), 6.88 (ddd, *J =* 12.3, 11.0, 5.5 Hz, 2H), 6.34 - 6.27 (m, 2H), 1.65 (d, *J =* 6.8 Hz, 3H).

### Preparation Example (III) 28: Compound A-1-10-1-6

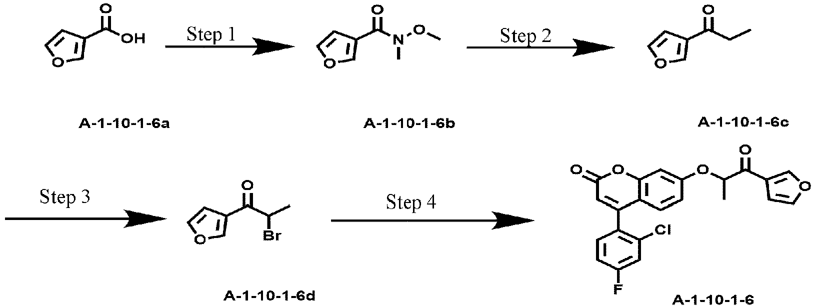

### Step 1

In a 100 mL single-necked flask, A-1-10-1-6a (2.00 g, 17.84 mmol), dichloromethane (50 mL), dimethylhydroxylamine hydrochloride (3.50 g, 35.88 mmol), 1-(3-dimethylaminopropyl) -3-ethylcarbodiimide hydrochloride (5.10 g, 26.60 mmol) and 1-hydroxybenzotriazole (3.60 g, 26.64 mmol) were successively added. After that, the reaction was carried out at 25°C for 16 h. After TLC and LCMS monitoring showed that the reaction was complete, the re action solution was diluted with dichloromethane (100 mL) and washed with saturated brin e. The organic phase was dried over anhydrous sodium sulfate, filtered, and rotary evapora ted. The crude product was purified by flash column chromatography to give compound A -1-10-1-6b (1.80 g, 11.60 mmol, yield: 65%).
MS-ESI: [M+H]⁺ = 156.1

### Step 2

In a 100 mL single-necked flask, compound A-1-10-1-6c (1.80 g, 11.60 mmol) and tetrahydrofuran (20 mL) were added. The system was purged with nitrogen for protection and cooled to -15°C. A solution of ethylmagnesium bromide in tetrahydrofuran (2 M, 11.6 mL, 23.2 mmol) was slowly added dropwise. After that, the mixture was allowed to return to room temperature and reacted for 4 h. After LCMS and TLC monitoring showed that the reaction was complete, saturated aqueous ammonium chloride solution (100 mL) was added to quench the reaction. The mixture was extracted with ethyl acetate (100 mL × 3). The organic layers were combined, washed with saturated sodium chloride solution, dried over anhydrous sodium sulfate, filtered, and rotary evaporated. The crude product was purified by flash column chromatography to give compound A-1-10-1-6c (1.10 g, 8.86 mmol, yield: 76%).
MS-ESI: [M+H]⁺= 125.1

### Step 3

In a 100 mL single-necked flask, compound A-1-10-1-6c (400.0 mg, 3.22 mmol), tetrahydrofuran (10 mL), pyrrolidone hydrotribromide (2.10 g, 6.44 mmol) and 2-pyrrolidone (548.0 mg, 6.44 mmol) were added. After that, the mixture was allowed to return to room temperature and reacted for 6 h. After LCMS and TLC monitoring showed that the reaction was complete, the mixture was diluted with water and extracted with ethyl acetate (50 mL × 3). The organic layers were combined, washed with saturated sodium chloride solution, dried over anhydrous sodium sulfate, filtered, and rotary evaporated. The crude product was purified by flash column chromatography to give compound A-1-10-1-6d (210.0 mg, 1.03 mmol, yield: 32%).
MS-ESI: [M+H]⁺= 203.0

### Step 4

In a 50 mL single-necked flask, compound A-1-10-1-6d (210.0 mg, 1.03 mmol), intermediate A1-1-1c (198.0 mg, 0.68 mmol), tetrabutylammonium iodide (380.0 mg, 1.03 mmol), potassium carbonate (285.0 mg, 2.06 mmol) and *N*,*N*-dimethylformamide (10 mL) were successively added. After that, the reaction was carried out at room temperature overnight. After LCMS and TLC monitoring showed that the reaction was complete, the reaction solution was diluted with water and extracted with ethyl acetate (50 mL × 3). The organic layers were combined, washed with saturated sodium chloride solution, dried over anhydrous sodium sulfate, filtered, and rotary evaporated. The crude product was purified by flash column chromatography to give compound A-1-10-1-6 (43.0 mg, 0.10 mmol, yield: 15%).
MS-ESI: [M+H]⁺ = 413.1
¹H NMR (400 MHz, DMSO-*d₆*) δ 9.17 (s, 1H), 9.00 (d, *J =* 2.4 Hz, 1H), 8.89 (s, 1H), 7.69 (dt, *J* = 8.9, 2.5 Hz, 1H), 7.55 (ddd, *J =* 8.7, 6.2, 2.7 Hz, 1H), 7.46 - 7.38 (m, 1H), 7.00 (dd, *J =* 6.8, 2.2 Hz, 1H), 6.88 (ddd, *J =* 12.3, 11.0, 5.5 Hz, 2H), 6.34 - 6.27 (m, 2H), 1.65 (d*, J =* 6.8 Hz, 3H).

### Preparation Example (III) 29: Compound A-1-2-1-78

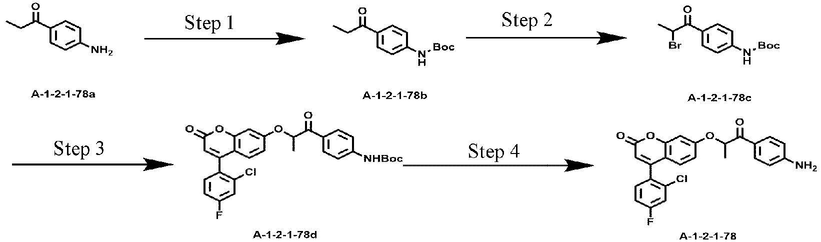

### Step 1

A-1-2-1-78a (1.00 g, 6.70 mmol) and tetrahydrofuran (20 mL) were added to a 100 mL three-necked flask. After thorough stirring, di-tert-butyl dicarbonate (1.61 g, 7.38 mmol) and 4-dimethylaminopyridine (0.08 g, 0.67 mmol) were successively added to the reaction system. The reaction was carried out at room temperature overnight. After LCMS and TLC monitoring showed that the reaction was complete, the mixture was diluted with water and extracted with ethyl acetate (50 mL × 3). The organic phases were combined, washed successively with saturated aqueous sodium bicarbonate solution and saturated brine, dried over anhydrous sodium sulfate, and concentrated under reduced pressure to give compound A-1-2-1-78b (0.56 g, 2.25 mmol, yield: 34%).
MS-ESI: [M+H]⁺ = 250.1

### Step 2

A-1-2-1-78b (0.40 g, 1.60 mmol) and tetrahydrofuran (20 mL) were added to a 100 mL three-necked flask. After sufficient dissolution, pyrrolidone (0.16 g, 1.92 mmol) and pyrrolidone hydrotribromide (0.78 g, 2.40 mmol) were successively added to the reaction system, and the temperature was raised to 50°C. The reaction was carried out for 6 h. After LCMS and TLC monitoring showed that the reaction was complete, the mixture was diluted with water and extracted with ethyl acetate (50 mL × 3). The organic phases were combined, washed with saturated brine, dried over anhydrous sodium sulfate, concentrated under reduced pressure, and purified to give compound A-1-2-1-78c (0.32 g, 0.97 mmol, yield: 61%).
MS-ESI: [M+H]⁺ = 328.1

### Step 3

Compounds A-1-2-1-78c (0.20 g, 0.61 mmol) and A1-1-1c (0.16 g, 0.55 mmol) were added to a 50 mL three-necked flask. Then, acetone (10 mL) was added. After sufficient dissolution, potassium carbonate (0.17 g, 1.22 mmol) and tetrabutylammonium iodide (0.23 g, 0.61 mmol) were successively added. The reaction was carried out at room temperature for 3 h. After TLC and LCMS monitoring showed that the reaction was complete, the mixture was diluted with water and extracted with ethyl acetate (50 mL × 3). The organic phases were combined, washed with saturated brine, dried over anhydrous sodium sulfate, and concentrated under reduced pressure. The crude product was purified by flash column chromatography to give compound A-1-2-1-78d (0.16 g, 0.30 mmol, yield: 49%).
MS-ESI: [M+H]⁺ = 538.1

### Step 4

Compound A-1-2-1-78d (0.10 g, 0.19 mmol) and dichloromethane (3 mL) were added to a 50 mL three-necked flask. After thorough stirring, trifluoroacetic acid (3 mL) was added dropwise to the solution. The reaction was carried out at room temperature for 10 minutes. After TLC and LCMS monitoring showed that the reaction was complete, the reaction was quenched with saturated sodium bicarbonate solution (20 mL). The mixture was diluted with water and extracted with ethyl acetate (50 mL x 3). The organic phases were combined, washed with saturated brine, dried over anhydrous sodium sulfate, and concentrated under reduced pressure. The crude product was purified by flash column chromatography to give compound A-1-2-1-78 (0.05 g, 0.11 mmol, yield: 58%).
MS-ESI: [M+H]⁺ = 438.2
¹H NMR (400 MHz, DMSO-*d₆*) δ 7.81 (d, *J =* 8.6 Hz, 2H), 7.73 - 7.64 (m, 1H), 7.55 (dd, *J =* 8.5, 6.2 Hz, 1H), 7.43 (m, *J =* 4.3 Hz, 1H), 6.92 (d, *J =* 8.9 Hz, 1H), 6.89 (dd, *J =* 5.3, 2.4 Hz, 1H), 6.86 - 6.80 (m, 1H), 6.61 (d, *J =* 8.7 Hz, 2H), 6.30 (s, 3H), 6.06 - 5.93 (m, 1H), 1.54 (d, *J =* 6.6 Hz, 3H).

### Preparation Example (III) 30: Compound A-1-2-1-72

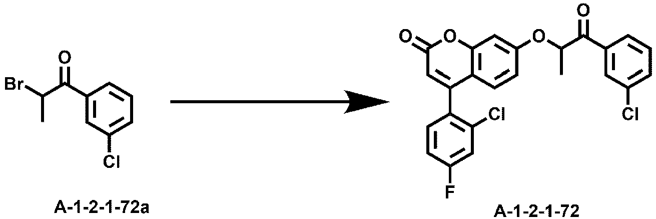

Compounds A-1-2-1-72a (0.50 g, 2.02 mmol) and A1-1-1c (0.53 g, 1.82 mmol) were added to a 50 mL three-necked flask. Then, acetone (10 mL) was added. After sufficient dissolution, potassium carbonate (0.56 g, 4.04 mmol) and tetrabutylammonium iodide (0.75 g, 2.02 mmol) were successively added. The reaction was carried out at room temperature for 3 h. After TLC and LCMS monitoring showed that the reaction was complete, the mixture was diluted with water and extracted with ethyl acetate (50 mL × 3). The organic phases were combined, washed with saturated brine, dried over anhydrous sodium sulfate, and concentrated under reduced pressure. The crude product was purified by flash column chromatography to give compound A-1-2-1-72 (0.16 g, 0.35 mmol, yield: 20%).
MS-ESI: [M+H]⁺ = 457.1
¹H NMR (400 MHz, DMSO-d6) δ 8.10 (s, 1H), 8.03 (d, *J =* 7.7 Hz, 1H), 7.79 (d, *J =* 7.9 Hz, 1H), 7.69 (dd, *J =* 8.9, 2.1 Hz, 1H), 7.63 (t, *J =* 7.9 Hz, 1H), 7.55 (t, *J =* 7.3 Hz, 1H), 7.46 - 7.37 (m, 1H), 7.08 (dd, *J =* 4.5, 2.3 Hz, 1H), 6.93 (t, *J* = 7.9 Hz, 1H), 6.88 (t, 1H), 6.31 (s, 1H), 6.27 (dd, *J* = 9.2, 4.2 Hz, 1H), 1.55 (d, *J =* 6.7 Hz, 3H).

### Preparation Example (III) 31: Compound A-1-2-1-84

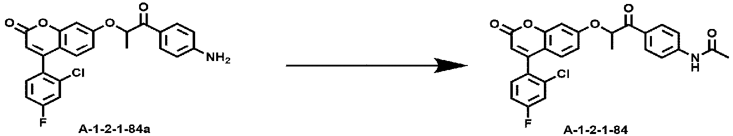

Compound A-1-2-1-84a (0.10 g, 0.23 mmol) and tetrahydrofuran (3 mL) were added to a 50 mL three-necked flask. After sufficient dissolution, acetic anhydride (0.03 g, 0.28 mmol) was added dropwise to the reaction solution. The reaction was carried out at room temperature overnight. After TLC and LCMS monitoring showed that the reaction was complete, ethyl acetate (30 mL) was added to the reaction solution, and the mixture was washed with aqueous hydrochloric acid solution (1.0 M). The aqueous phase was extracted with ethyl acetate (30 mL × 2). The organic phases were combined, washed successively with saturated aqueous sodium bicarbonate solution and saturated brine, and concentrated under reduced pressure. The crude product was purified by flash column chromatography to give compound A-1-2-1-84 (0.06 g, 0.13 mmol, yield: 57%).
MS-ESI: [M+H]⁺ = 480.2
¹H NMR (400 MHz, DMSO-d6) δ 10.37 (s, 1H), 8.06 (d, *J =* 8.4 Hz, 2H), 7.77 (d, *J =* 8.7 Hz, 2H), 7.69 (d, *J =* 8.9 Hz, 1H), 7.59 - 7.51 (m, 1H), 7.47 - 7.35 (m, 1H), 6.98 (dd, *J =* 5.9, 2.1 Hz, 1H), 6.93 (d, *J =* 8.9 Hz, 1H), 6.89 - 6.81 (m, 1H), 6.31 (s, 1H), 6.16 (t, *J =* 6.7 Hz, 1H), 2.10 (s, 3H), 1.57 (d, *J* = 6.5 Hz, 3H)_{∘}

### Preparation Example (III) 32: Compound A-1-2-1-79

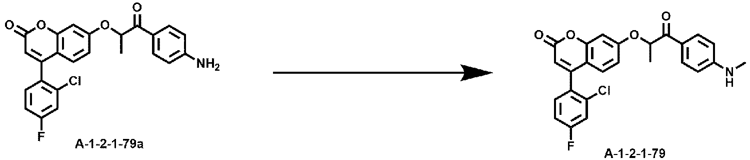

Compound A-1-2-1-79a (0.50 g, 1.14 mmol) and acetone (10 mL) were added to a 50 mL three-necked flask. After sufficient dissolution, iodomethane (1.62 g, 11.40 mmol) and sodium carbonate (0.36 g, 3.42 mmol) were added to the reaction solution. The reaction was carried out at room temperature for 24 h. After TLC and LCMS monitoring showed that the reaction was complete, the mixture was diluted with water and extracted with ethyl acetate (50 mL × 3). The organic phases were combined, washed with saturated brine, dried over anhydrous sodium sulfate, and concentrated under reduced pressure. The crude product was purified by flash column chromatography to give compound P06 (0.13 g, 0.29 mmol, yield: 25%).
MS-ESI: [M+H]⁺ = 452.3
¹H NMR (400 MHz, DMSO-d6) δ 7.87 (d, *J =* 8.5 Hz, 2H), 7.69 (dt, *J =* 8.8, 2.6 Hz, 1H), 7.54 (dd, *J =* 8.5, 6.2 Hz, 1H), 7.46 - 7.37 (m, 1H), 6.92 (d, *J =* 8.9 Hz, 1H), 6.88 (dd, *J =* 5.2, 2.4 Hz, 1H), 6.86 - 6.80 (m, 1H), 6.60 (d, *J =* 8.9 Hz, 2H), 6.29 (s, 1H), 6.02 (t, *J =* 6.9 Hz, 1H), 2.76 (s, 3H), 1.54 (d, *J =* 6.5 Hz, 3H).

### Preparation Example (III) 33: Compound A-1-2-1-80

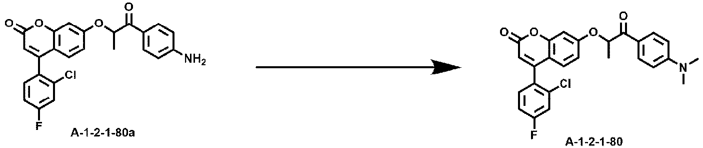

Compound A-1-2-1-80a (0.50 g, 1.14 mmol) and acetone (10 mL) were added to a 50 mL three-necked flask. After sufficient dissolution, iodomethane (1.62 g, 11.40 mmol) and sodium carbonate (0.36 g, 3.42 mmol) were added to the reaction solution. The reaction was carried out at room temperature for 24 h. After TLC and LCMS monitoring showed that the reaction was complete, the mixture was diluted with water and extracted with ethyl acetate (50 mL × 3). The organic phases were combined, washed with saturated brine, dried over anhydrous sodium sulfate, and concentrated under reduced pressure. The crude product was purified by flash column chromatography to give compound A-1-2-1-80 (0.08 g, 0.17 mmol, yield: 15%).
MS-ESI: [M+H]⁺ = 466.2
¹H NMR (400 MHz, DMSO-d6) δ 7.93 (d, *J =* 8.2 Hz, 2H), 7.69 (d, *J =* 8.9 Hz, 1H), 7.54 (dd, *J* = 8.5, 6.2 Hz, 1H), 7.46 - 7.35 (m, 1H), 6.92 (d, *J =* 8.9 Hz, 1H), 6.89 (dd, *J =* 5.4, 2.4 Hz, 1H), 6.86 - 6.81 (m, 1H), 6.77 (d*, J =* 9.0 Hz, 2H), 6.29 (s, 1H), 6.05 (p, *J =* 6.6 Hz, 1H), 3.05 (s, 6H), 1.54 (d, *J =* 6.5 Hz, 3H).

### Preparation Example (III) 34: Compound A-1-1-1-13

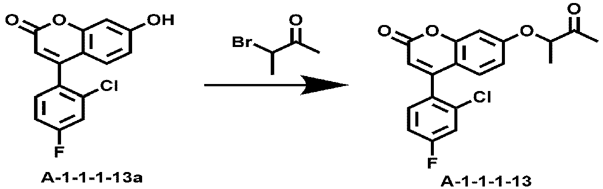

Compound 3-bromo-2-butanone (0.20 g, 1.32 mmol) and A1-1-1c (0.35 g, 1.19 mmol) were added to a 50 mL three-necked flask. Then, acetone (10 mL) was added. After sufficient dissolution, potassium carbonate (0.36 g, 2.64 mmol) and tetrabutylammonium iodide (0.48 g, 1.32 mmol) were successively added. The reaction was carried out at room temperature for 3 h. After TLC and LCMS monitoring showed that the reaction was complete, the mixture was diluted with water and extracted with ethyl acetate (50 mL × 3). The organic phases were combined, washed with saturated brine, dried over anhydrous sodium sulfate, and concentrated under reduced pressure. The crude product was purified by flash column chromatography to give compound A-1-1-1-13 (0.26 g, 0.72 mmol, yield: 62%).
MS-ESI: [M+H]⁺ = 361.1
¹H NMR (400 MHz, DMSO-*d₆*) δ 7.75 - 7.69 (m, 1H), 7.61 - 7.54 (m, 1H), 7.48 - 7.41 (m, 1H), 7.04 (dd, *J =* 6.5, 2.4 Hz, 1H), 6.95 (d, *J =* 8.9 Hz, 1H), 6.91 - 6.86 (m, 1H), 6.33 (s, 1H), 5.25 - 5.14 (m, 1H), 2.23 (d, *J =* 1.2 Hz, 3H), 1.49 (d, *J =* 6.8 Hz, 3H).

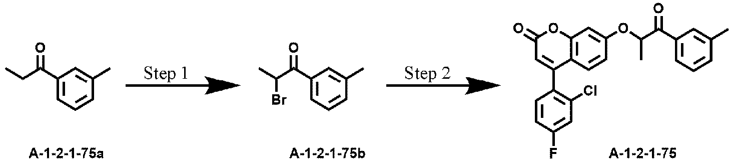

### Preparation Example (III) 35: Compound A-1-2-1-75

### Step 1

A-1-2-1-75a (0.50 g, 3.37 mmol) and tetrahydrofuran (15 mL) were added to a 50 mL three-necked flask. After sufficient dissolution, pyrrolidone (0.34 g, 4 mmol) and pyrrolidone hydrotribromide (1.65 g, 5.06 mmol) were successively added to the reaction system, and the temperature was raised to 50 °C. The reaction was carried out for 6 h. After LCMS monitoring showed that the reaction was complete, the mixture was diluted with water and extracted with ethyl acetate (100 mL × 3). The organic phases were combined, washed with saturated brine, dried over anhydrous sodium sulfate, concentrated under reduced pressure, and purified to give compound A-1-2-1-75b (0.71 g, 3.13 mmol, yield: 93%).

### Step 2

Compounds A-1-2-1-75b (0.15 g, 0.66 mmol) and A1-1-1c (0.17 g, 0.59 mmol) were added to a 50 mL three-necked flask. Then, acetone (10 mL) was added. After sufficient dissolution, potassium carbonate (0.18 g, 1.32 mmol) and tetrabutylammonium iodide (0.24 g, 0.66 mmol) were successively added. The reaction was carried out at room temperature for 3 h. After TLC and LCMS monitoring showed that the reaction was complete, the mixture was diluted with water and extracted with ethyl acetate (50 mL × 3). The organic phases were combined, washed with saturated brine, dried over anhydrous sodium sulfate, and concentrated under reduced pressure. The crude product was purified by flash column chromatography to give compound A-1-2-1-75 (0.12 g, 0.27 mmol, yield: 47%).
MS-ESI: [M-H]⁻ = 435.1
¹H NMR (400 MHz, DMSO-*d₆*) δ 7.93 (d, *J =* 7.3 Hz, 2H), 7.73 (dt, *J =* 8.9, 2.6 Hz, 1H), 7.61 - 7.55 (m, 2H), 7.53 (d, *J =* 7.9 Hz, 1H), 7.50 - 7.42 (m, 1H), 7.04 (dd, *J =* 6.9, 2.4 Hz, 1H), 6.98 (d, *J* = 8.9 Hz, 1H), 6.94 - 6.87 (m, 1H), 6.35 (d, *J =* 1.2 Hz, 1H), 6.31 - 6.23 (m, 1H), 2.44 (s, 3H), 1.60 (d, *J =* 6.4 Hz, 3H).

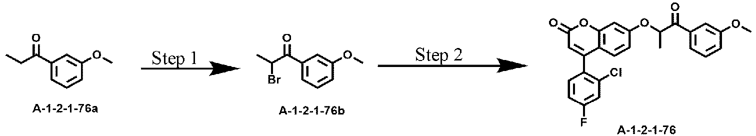

### Preparation Example (III) 36: Compound A-1-2-1-76

### Step 1

A-1-2-1-76a (0.20 g, 1.22 mmol) and tetrahydrofuran (10 mL) were added to a 50 mL three-necked flask. After sufficient dissolution, pyrrolidone (0.12 g, 1.46 mmol) and pyrrolidone hydrotribromide (0.59 g, 1.83 mmol) were successively added to the reaction system, and the temperature was raised to 50 °C. The reaction was carried out for 6 h. After LCMS monitoring showed that the reaction was complete, the mixture was diluted with water and extracted with ethyl acetate (100 mL × 3). The organic phases were combined, washed with saturated brine, dried over anhydrous sodium sulfate, concentrated under reduced pressure, and purified to give compound A-1-2-1-76b (0.22 g, 0.90 mmol, yield: 74%).

### Step 2

Compounds A-1-2-1-76b (0.20 g, 0.82 mmol) and A1-1-1c (0.22 g, 0.74 mmol) were added to a 50 mL three-necked flask. Then, acetone (10 mL) was added. After sufficient dissolution, potassium carbonate (0.23 g, 1.64 mmol) and tetrabutylammonium iodide (0.30 g, 0.82 mmol) were successively added. The reaction was carried out at room temperature for 3 h. After TLC and LCMS monitoring showed that the reaction was complete, the mixture was diluted with water and extracted with ethyl acetate (50 mL × 3). The organic phases were combined, washed with saturated brine, dried over anhydrous sodium sulfate, and concentrated under reduced pressure. The crude product was purified by flash column chromatography to give compound A-1-2-1-76 (0.16 g, 0.35 mmol, yield: 46%).
MS-ESI: [M+H]⁺ = 453.2
¹H NMR (400 MHz, DMSO-*d₆*) δ 7.74 - 7.65 (m, 2H), 7.59 - 7.47 (m, 3H), 7.47 - 7.36 (m, 1H), 7.30 (dd, *J* = 8.3, 2.0 Hz, 1H), 7.02 (dd, *J =* 6.8, 2.2 Hz, 1H), 6.94 (d, *J =* 8.9 Hz, 1H), 6.88 (td, *J* = 8.6, 2.3 Hz, 1H), 6.31 (s, 1H), 6.27 (dd, *J =* 13.2, 6.6 Hz, 1H), 3.83 (d, *J =* 5.5 Hz, 3H), 1.56 (d, *J =* 6.7 Hz, 3H).

### Preparation Example (III) 37: Compound A-1-2-1-70

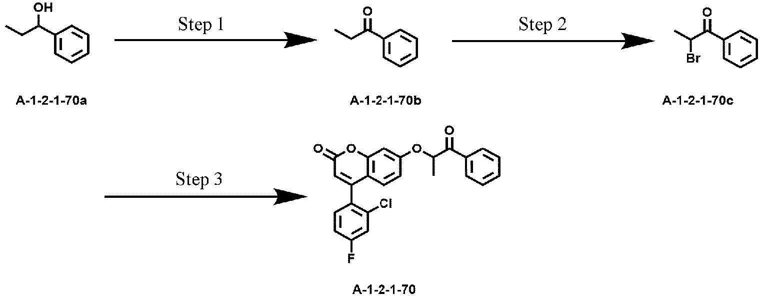

### Step 1

A-1-2-1-70a (0.30 g, 2.20 mmol) and dichloromethane (15 mL) were added to a 50 mL three-necked flask. After sufficient dissolution, Dess-Martin periodinane (1.40 g, 3.30 mmol) was slowly added to the reaction system. The reaction was carried out at room temperature for 1 h. After TLC and LCMS monitoring showed that the reaction was complete, the reaction solution was diluted with ethyl acetate (30 mL) and then filtered. The filtrate was washed with water once. The aqueous phase was extracted with ethyl acetate (30 mL × 3). The organic phases were combined, washed with saturated brine, dried over anhydrous sodium sulfate, and concentrated under reduced pressure to give compound A-1-2-1-70b (0.23 g, 1.71 mmol, yield: 78%).

### Step 2

A-1-2-1-70b (0.23 g, 1.71 mmol) and tetrahydrofuran (10 mL) were added to a 50 mL three-necked flask. After sufficient dissolution, pyrrolidone (0.17 g, 2.05 mmol) and pyrrolidone hydrotribromide (0.83 g, 2.56 mmol) were successively added to the reaction system, and the temperature was raised to 50°C. The reaction was carried out for 6 h. After LCMS monitoring showed that the reaction was complete, the mixture was diluted with water and extracted with ethyl acetate (100 mL × 3). The organic phases were combined, washed with saturated brine, dried over anhydrous sodium sulfate, concentrated under reduced pressure, and purified to give compound A-1-2-1-70c (0.20 g, 0.94 mmol, yield: 55%).

### Step 3

Compounds A-1-2-1-70c (0.10 g, 0.47 mmol) and A1-1-1c (0.12 g, 0.42 mmol) were added to a 50 mL three-necked flask. Then, acetone (10 mL) was added. After sufficient dissolution, potassium carbonate (0.13 g, 0.94 mmol) and tetrabutylammonium iodide (0.17 g, 0.47 mmol) were successively added. The reaction was carried out at room temperature for 3 h. After TLC and LCMS monitoring showed that the reaction was complete, the mixture was diluted with water and extracted with ethyl acetate (50 mL × 3). The organic phases were combined, washed with saturated brine, dried over anhydrous sodium sulfate, and concentrated under reduced pressure. The crude product was purified by flash column chromatography to give compound A-1-2-1-70 (0.11 g, 0.26 mmol, yield: 56%).
MS-ESI: [M-H]⁻ = 421.1
¹H NMR (400 MHz, DMSO-*d₆*) δ 8.09 (d, *J =* 7.8 Hz, 2H), 7.71 (dd, *J =* 16.6, 8.4 Hz, 2H), 7.64 - 7.52 (m, 3H), 7.43 (dt, *J* = 6.7, 4.2 Hz, 1H), 7.03 (dd, *J* = 6.0, 1.8 Hz, 1H), 6.95 (d, *J* = 8.9 Hz, 1H), 6.91 - 6.84 (m, 1H), 6.31 (s, 1H), 6.25 (dd, *J =* 13.0, 6.5 Hz, 1H), 1.58 (d*, J =* 6.6 Hz, 3H).

### Preparation Example (III) 38: Compound A-1-11-1-10

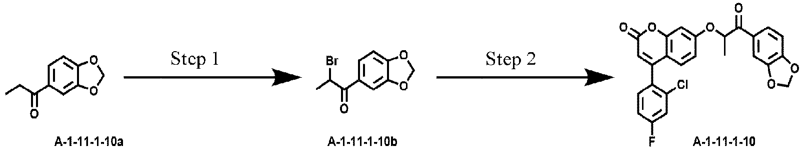

### Step 1

A-1-11-1-10a (0.50 g, 2.81 mmol) and tetrahydrofuran (15 mL) were added to a 50 mL three-necked flask. After sufficient dissolution, pyrrolidone (0.29 g, 3.37 mmol) and pyrrolidone hydrotribromide (1.37 g, 4.22 mmol) were successively added to the reaction system, and the temperature was raised to 50 °C. The reaction was carried out for 6 h. After LCMS monitoring showed that the reaction was complete, the mixture was diluted with water and extracted with ethyl acetate (100 mL × 3). The organic phases were combined, washed with saturated brine, dried over anhydrous sodium sulfate, concentrated under reduced pressure, and purified to give compound A-1-11-1-10b (0.42 g, 1.63 mmol, yield: 58%).

### Step 2

Compounds A-1-11-1-10b (0.20 g, 0.78 mmol) and A1-1-1c (0.20 g, 0.70 mmol) were added to a 50 mL three-necked flask. Then, acetone (10 mL) was added. After sufficient dissolution, potassium carbonate (0.22 g, 1.56 mmol) and tetrabutylammonium iodide (0.29 g, 0.78 mmol) were successively added. The reaction was carried out at room temperature for 3 h. After TLC and LCMS monitoring showed that the reaction was complete, the mixture was diluted with water and extracted with ethyl acetate (50 mL × 3). The organic phases were combined, washed with saturated brine, dried over anhydrous sodium sulfate, and concentrated under reduced pressure. The crude product was purified by flash column chromatography to give compound A-1-11-1-10 (0.18 g, 0.39 mmol, yield: 50%).
MS-ESI: [M-H]⁻= 465.1
¹H NMR (400 MHz, DMSO-*d₆*) δ 7.77 (d, *J =* 8.2 Hz, 1H), 7.69 (d, *J =* 8.8 Hz, 1H), 7.57 (s, 1H), 7.54 (d, *J=* 6.6 Hz, 1H), 7.43 (dd, *J =* 10.5, 6.5 Hz, 1H), 7.12 (d, *J =* 8.2 Hz, 1H), 7.00 - 6.94 (m, 1H), 6.93 (s, 1H), 6.86 (t, *J =* 8.1 Hz, 1H), 6.31 (s, 1H), 6.21 - 6.13 (m, 3H), 1.55 *(d, J =* 6.6 Hz, 3H).

### Preparation Example (III) 39: Compound A-1-2-1-71

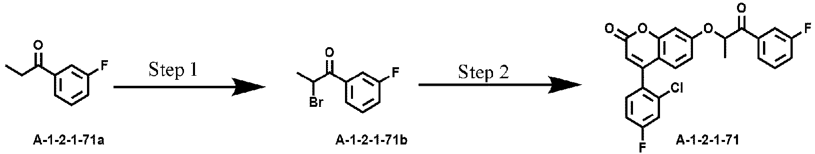

### Step 1

A-1-2-1-71a (1.00 g, 6.57 mmol) and tetrahydrofuran (20 mL) were added to a 100 mL three-necked flask. After sufficient dissolution, pyrrolidone (0.67 g, 7.88 mmol) and pyrrolidone hydrotribromide (3.21 g, 9.86 mmol) were successively added to the reaction system, and the temperature was raised to 50 °C. The reaction was carried out for 6 h. After LCMS monitoring showed that the reaction was complete, the mixture was diluted with water and extracted with ethyl acetate (100 mL × 3). The organic phases were combined, washed with saturated brine, dried over anhydrous sodium sulfate, and concentrated under reduced pressure to give compound A-1-2-1-71b (0.90 g, 3.90 mmol, yield: 59%).

### Step 2

Compounds A-1-2-1-71b (0.20 g, 0.87 mmol) and A1-1-1c (0.23 g, 0.78 mmol) were added to a 50 mL three-necked flask. Then, acetone (10 mL) was added. After sufficient dissolution, potassium carbonate (0.24 g, 1.74 mmol) and tetrabutylammonium iodide (0.32 g, 0.87 mmol) were successively added. The reaction was carried out at room temperature for 3 h. After TLC and LCMS monitoring showed that the reaction was complete, the mixture was diluted with water and extracted with ethyl acetate (50 mL × 3). The organic phases were combined, washed with saturated brine, dried over anhydrous sodium sulfate, and concentrated under reduced pressure. The crude product was purified by flash column chromatography to give compound A-1-2-1-71 (0.12 g, 0.27 mmol, yield: 31%).
MS-ESI: [M+H]⁺ = 441.1
¹H NMR (400 MHz, DMSO-*d₆*) δ 7.93 (d, *J =* 7.7 Hz, 1H), 7.89 (d, *J =* 9.8 Hz, 1H), 7.73 - 7.51 (m, 4H), 7.47 - 7.38 (m, 1H), 7.06 (dd, *J =* 4.8, 2.3 Hz, 1H), 6.94 (t, *J =* 8.9 Hz, 1H), 6.92 - 6.85 (t, 1H), 6.31 (s, 1H), 6.27 (dt*, J =* 11.9, 6.0 Hz, 1H), 1.56 (d*, J =* 6.6 Hz, 3H).

### Preparation Example (III) 40: Compound A-1-2-1-88

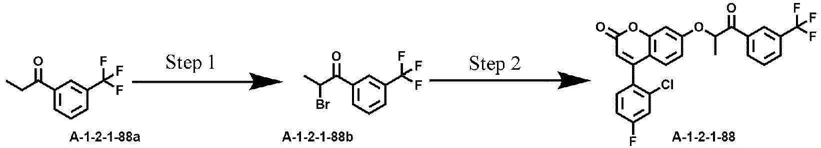

### Step 1

A-1-2-1-88a (1.00 g, 4.95 mmol) and tetrahydrofuran (20 mL) were added to a 100 mL three-necked flask. After sufficient dissolution, pyrrolidone (0.51 g, 5.94 mmol) and pyrrolidone hydrotribromide (2.42 g, 7.43 mmol) were successively added to the reaction system, and the temperature was raised to 50 °C. The reaction was carried out for 6 h. After LCMS monitoring showed that the reaction was complete, the mixture was diluted with water and extracted with ethyl acetate (100 mL × 3). The organic phases were combined, washed with saturated brine, dried over anhydrous sodium sulfate, and concentrated under reduced pressure to give compound A-1-2-1-88b (0.90 g, 3.20 mmol, yield: 65%).

### Step 2

Compounds A-1-2-1-88b (0.30 g, 1.07 mmol) and A1-1-1c (0.28 g, 0.96 mmol) were added to a 50 mL three-necked flask. Then, acetone (10 mL) was added. After sufficient dissolution, potassium carbonate (0.30 g, 2.14 mmol) and tetrabutylammonium iodide (0.39g, 1.07 mmol) were successively added, and the system was heated to 60°C. After TLC and LCMS monitoring showed that the reaction was complete, the mixture was diluted with water and extracted with ethyl acetate (50 mL × 3). The organic phases were combined, washed with saturated brine, dried over anhydrous sodium sulfate, and concentrated under reduced pressure. The crude product was purified by flash column chromatography to give compound A-1-2-1-88 (0.40 g, 0.81 mmol, yield: 76%).
MS-ESI: [M+H]⁺ = 491.1
¹H NMR (400 MHz, DMSO-*d₆*) δ 7.91 (dd, *J =* 15.0, 8.7 Hz, 2H), 7.73 - 7.51 (m, 4H), 7.47 - 7.38 (m, 1H), 7.06 (dd, *J =* 4.8, 2.3 Hz, 1H), 6.90 (ddd, *J =* 12.8, 11.3, 5.7 Hz, 2H), 6.31 (s, 1H), 6.27 (dt, *J=* 11.9, 6.0 Hz, 1H), 1.56 (d, *J =* 6.6 Hz, 3H).

### Preparation Example (III) 41: Compound A-1-7-1-1

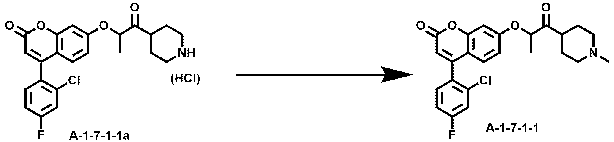

A-1-7-1-1a (0.15 g, 0.32 mmol), formic acid (5 mL) and 37% formaldehyde (5 mL) were added to a 50 mL reaction flask. The system was heated to 80°C and stirred. After TLC and LCMS monitoring showed that the reaction was complete, the mixture was diluted with water and extracted with dichloromethane (50 mL × 3). The organic phases were combined, washed with saturated brine, dried over anhydrous sodium sulfate, and concentrated under reduced pressure. The crude product was purified by flash column chromatography to give compound A-1-7-1-1 (0.13 g, 0.29 mmol, yield: 91%).
MS-ESI: [M+H]⁺= 444.1
¹H NMR (400 MHz, DMSO-*d₆*) δ 7.72 (d, *J =* 8.8 Hz, 1H), 7.57 (dd, *J =* 8.6, 6.1 Hz, 1H), 7.49 - 7.40 (m, 1H), 7.03 (dd, *J =* 5.1, 2.4 Hz, 1H), 6.96 (dd, *J =* 8.8, 1.4 Hz, 1H), 6.91 - 6.83 (m, 1H), 6.34 (s, 1H), 5.48 - 5.28 (m, 1H), 2.99 - 2.71 (m, 3H), 2.24 (s, 3H), 1.99 (dd, *J =* 38.5, 26.3 Hz, 3H), 1.73 - 1.51 (m, 3H), 1.49 (d*, J =* 6.8 Hz, 3H).

### Preparation Example (III) 42: Compound A-1-7-1-4

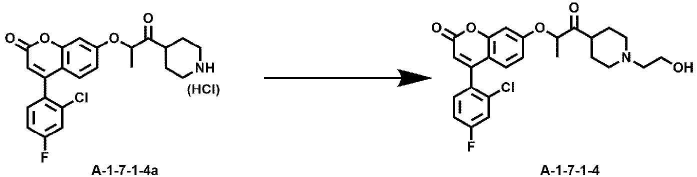

A-1-7-1-4a (0.15 g, 0.32 mmol), 2-chloroethanol (0.14 g, 1.12 mmol), triethylamine (2 mL) and dioxane (10 mL) were added to a 50 mL three-necked flask and stirred at room temperature. After TLC and LCMS monitoring showed that the reaction was complete, the mixture was diluted with water and extracted with dichloromethane (50 mL × 3). The organic phases were combined, washed with saturated brine, dried over anhydrous sodium sulfate, and concentrated under reduced pressure. The crude product was purified by flash column chromatography to give compound A-1-7-1-4 (0.12 g, 0.25 mmol, yield: 78.5%).
MS-ESI: [M+H]⁺ = 474.1
¹H NMR (400 MHz, DMSO-*d₆*) δ 7.71 (d, *J =* 8.8 Hz, 1H), 7.57 (t, 1H), 7.44 (t, *J =* 8.4 Hz, 1H), 7.00 (dd, *J =* 5.6, 2.4 Hz, 1H), 6.95 (dd, *J =* 8.9, 1.8 Hz, 1H), 6.88 - 6.82 (m, 1H), 6.32 (s, 1H), 5.44 - 5.29 (m, 1H), 4.35 (t, *J =* 5.4 Hz, 1H), 3.46 (dd, *J =* 11.7, 6.1 Hz, 2H), 2.96 - 2.61 (m, 4H), 2.35 (t, *J* = 6.3 Hz, 2H), 1.96 (dt, *J =* 20.8, 11.6 Hz, 4H), 1.46 (d, *J =* 10.8 Hz, 3H).

### Preparation Example (III) 43: Compound A-1-7-1-3

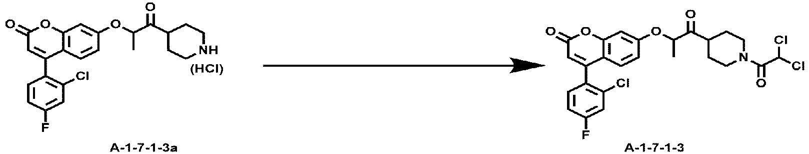

In a dry 50 mL three-necked flask, A-1-7-1-3a (0.15 g, 0.32 mmol), 2,2-dichloroacetic acid (0.10 g, 0.80 mmol), 2-(7-azabenzotriazol-1-yl)-*N,N,N',N'*-tetramethyluronium hexafluorophosphate (0.14 g, 0.38 mmol), triethylamine (1 mL) and dichloromethane (20 mL) were added to a 100 mL three-necked flask and stirred at room temperature. After TLC and LCMS monitoring showed that the reaction was complete, the mixture was diluted with water and extracted with dichloromethane (50 mL × 3). The organic phases were combined, washed with saturated brine, dried over anhydrous sodium sulfate, and concentrated under reduced pressure. The crude product was purified by flash column chromatography to give compound A-1-7-1-3 (0.13 g, 0.24 mmol, yield: 74%).
MS-ESI: [M+H]⁺= 540.1
¹H NMR (400 MHz, CD₃OD) δ 7.50 - 7.42 (m, 2H), 7.29 (td, *J =* 8.1, 1.9 Hz, 1H), 7.04 (d, *J =* 8.8 Hz, 1H), 6.99 - 6.95 (m, 1H), 6.88 (t, *J =* 5.7 Hz, 2H), 6.23 (s, 1H), 5.20 (td, *J =* 6.8, 4.1 Hz, 1H), 4.50 - 4.33 (m, 1H), 4.09 (dd, *J =* 23.4, 13.9 Hz, 1H), 3.22 (ddd, *J =* 14.2, 12.4, 3.0 Hz, 2H), 3.01 - 2.77 (m, 1H), 2.06 (d, *J =* 13.0 Hz, 1H), 1.81 - 1.46 (m, 6H).

### Preparation Example (III) 44: Compound A-1-10-1-7

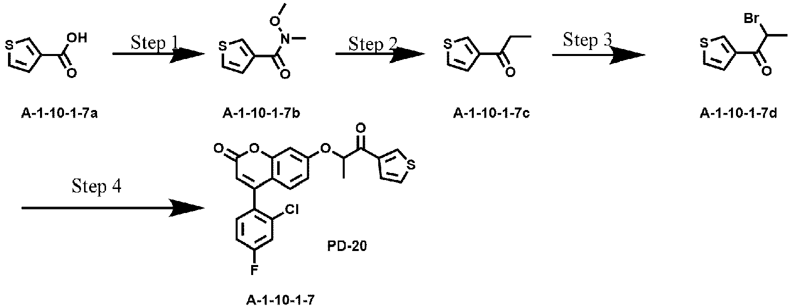

### Step 1

In a dry 50 mL three-necked flask, A-1-10-1-7a (2.56 g, 19.96 mmol), 2-(7-azabenzotriazol-1-yl)-*N,N,N',N'*-tetramethyluronium hexafluorophosphate (9.11 g, 23.95 mmol), triethylamine (5.05 g, 49.90 mmol) and methoxamine hydrochloride (2.14 g, 21.96 mmol) were added to dichloromethane (80 mL) and stirred at room temperature overnight. TLC showed that the starting materials had completely reacted. The reaction solution was washed successively with 1.0 M hydrochloric acid (50 mL), 0.5% aqueous sodium carbonate solution (50 mL) and saturated brine, dried over anhydrous sodium sulfate, and concentrated under reduced pressure. The crude product was purified by flash column chromatography to give compound A-1-10-1-7b (3.24 g, 18.92 mmol, yield: 95%).

### Step 2

In a dry 50 mL three-necked flask, compound A-1-10-1-7b (3.24 g, 18.92 mmol) was added to the 50 mL three-necked flask. Then, tetrahydrofuran (40 mL) was added, and the system was cooled to -10°C. 18 mL of ethylmagnesium bromide (2.0 M) was slowly added dropwise, and the temperature was maintained for half an hour and then slowly raised to room temperature. TLC showed that the starting materials had disappeared. Ethyl acetate (150 mL) and 5% ammonium chloride solution (50 mL) were added. The organic phase was collected, dried over anhydrous sodium sulfate, and concentrated under reduced pressure. The crude product was purified by flash column chromatography to give compound A-1-10-1-7c (1.40 g, 9.99 mmol, yield: 53%).

### Step 3

In a dry 50 mL three-necked flask, A-1-10-1-7c (1.40 g, 9.99 mmol) and pyridinium tribromide (4.79 g, 14.99 mmol) were added to 20 mL of tetrahydrofuran. Under nitrogen protection, the system was heated to 60°C. TLC monitoring showed that the starting materials had disappeared. Ethyl acetate (150 mL) and 5% sodium dithionite solution (50 mL) were added. The organic phase was collected, dried over anhydrous sodium sulfate, and concentrated under reduced pressure. The crude product was purified by flash column chromatography to give compound A-1-10-1-7d (2.00 g, 9.13 mmol, yield: 91%).

### Step 4

In a dry 50 mL three-necked flask, compound A-1-10-1-7d (0.12 g, 0.55 mmol) and A1-1-1c (0.15 g, 0.50 mmol) were added to the 50 mL three-necked flask. Then, acetone (10 mL) was added. After sufficient dissolution, potassium carbonate (0.15 g, 1.10 mmol) and tetrabutylammonium iodide (0.20 g, 0.55 mmol) were successively added. The reaction was carried out at room temperature for 3 h. After TLC and LCMS monitoring showed that the reaction was complete, the mixture was diluted with water and extracted with ethyl acetate (50 mL × 3). The organic phases were combined, washed with saturated brine, dried over anhydrous sodium sulfate, and concentrated under reduced pressure. The crude product was purified by flash column chromatography to give compound A-1-10-1-7 (0.15 g, 0.35 mmol, yield: 64%).
MS-ESI: [M+H]⁺= 429.0
¹H NMR (400 MHz, DMSO-*d₆*) δ 8.80 (td, *J =* 2.9, 1.2 Hz, 1H), 7.77 - 7.67 (m, 2H), 7.60 (d, *J=* 4.5 Hz, 1H), 7.56 (dd, *J =* 8.6, 6.1 Hz, 1H), 7.47 - 7.37 (m, 1H), 7.00 (dd, *J =* 7.5, 2.4 Hz, 1H), 6.96 (d, *J* = 9.3 Hz, 1H), 6.92 - 6.85 (m, 1H), 6.33 (d, *J =* 1.0 Hz, 1H), 6.00 (t, *J =* 6.8 Hz, 1H), 1.60 (dd, *J =* 6.7, 0.9 Hz, 3H).

### Preparation Example (III) 45: Compound A-1-10-1-47

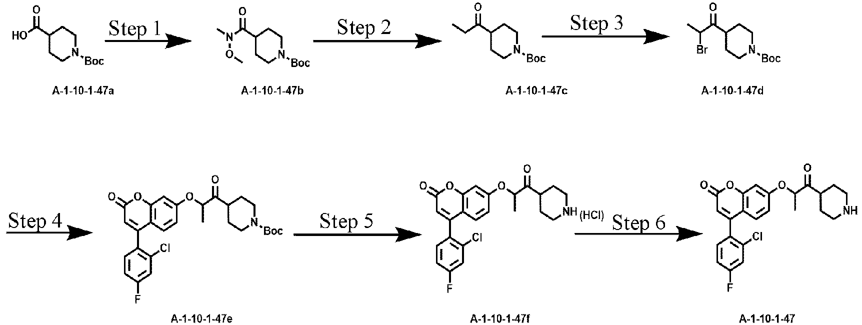

### Step 1

In a dry 50 mL three-necked flask, A-1-10-1-47a (15.00 g, 65.42 mmol), 2-(7-azabenzotriazole)-*N*,*N*,*N'*,*N'*-tetramethyluronium hexafluorophosphate (29.85 g, 78.50 mmol), triethylamine (16.55 g, 163.55 mmol) and methoxymethylamine hydrochloride (7.02 g, 71.96 mmol) were added to 200 mL of dichloromethane and stirred at room temperature overnight. TLC showed that the starting materials had completely reacted. The mixture was washed successively with 1.0 M hydrochloric acid (200 mL), 0.5% aqueous sodium carbonate solution (150 mL) and water, dried over anhydrous sodium sulfate, concentrated, and subjected to column chromatography to give compound A-1-10-1-47b (16.00 g, 58.75 mmol, yield: 90%).

### Step 2

In a dry 50 mL three-necked flask, compound A-1-10-1-47b (16.00 g, 58.75 mmol) was added to the 50 mL three-necked flask. Then, tetrahydrofuran (100 mL) was added, and the system was cooled to -10°C. 50 mL of ethylmagnesium bromide (2.0 M) was slowly added dropwise, and the temperature was maintained for half an hour and then slowly raised to room temperature. TLC showed that the starting materials had disappeared. 50 mL of ethyl acetate and 5% ammonium chloride solution (50 mL) were added. The organic phase was collected, dried over anhydrous sodium sulfate, and concentrated under reduced pressure. The crude product was purified by flash column chromatography to give A-1-10-1-47c (10.00 g, 41.44 mmol, yield: 71%).

### Step 3

In a dry 50 mL three-necked flask, A-1-10-1-47c (10.00 g, 41.44 mmol) and pyridinium tribromide (19.88 g, 62.16 mmol) were added to 150 mL of tetrahydrofuran. Under nitrogen protection, the system was heated to 60°C. TLC monitoring showed that the starting materials had disappeared. Ethyl acetate (100 mL) and 5% sodium dithionite solution (100 mL) were added. The organic phase was collected, dried over anhydrous sodium sulfate, and concentrated under reduced pressure. The crude product was purified by flash column chromatography to give A-1-10-1-47d (6.73 g, 21.02 mmol, yield: 51%).

### Step 4

In a dry 50 mL three-necked flask, compound A-1-10-1-47d (6.73 g, 21.02 mmol) and A1-1-1c (5.49 g, 18.92 mmol) were added to a 100 mL three-necked flask. Then, acetone (50 mL) was added. After sufficient dissolution, potassium carbonate (5.81 g, 42.04 mmol) and tetrabutylammonium iodide (7.76 g, 21.02 mmol) were successively added. The reaction was carried out at room temperature for 3 h. After TLC and LCMS monitoring showed that the reaction was complete, the mixture was diluted with water and extracted with ethyl acetate (100 mL × 3). The organic phases were combined, washed with saturated brine, dried over anhydrous sodium sulfate, and concentrated under reduced pressure. The crude product was purified by flash column chromatography to give compound A-1-10-1-47e (2.68 g, 5.06 mmol, yield: 24%).

### Step 5

In a dry 50 mL three-necked flask, compound A-1-10-1-47e (2.68 g, 5.06 mmol) and ethyl acetate (50 mL) were added to a 100 mL three-necked flask. Then, 10 mL of hydrogen chloride dioxane solution (4.0 M) was added, and the system was heated to 50°C. After TLC monitoring showed that the reaction was complete, the system was cooled to 0°C and filtered to give A-1-10-1-47f hydrochloride (2.00 g, 4.66 mmol, yield: 85%).
MS-ESI: [M+H]⁺= 430.1

### Step 6

In a dry 50 mL three-necked flask, compound A-1-10-1-47f (0.10 g, 0.22 mmol), potassium carbonate (0.15 g, 1.1 mmol) and dichloromethane (5 mL) were added to the 50 mL three-necked flask and stirred at room temperature for 2 h. The mixture was filtered and concentrated. The crude product was purified by flash column chromatography to give compound A-1-10-1-47 (0.08 g, 0.19 mmol, yield: 87%).
MS-ESI: [M+H]⁺= 430.1
¹H NMR (400 MHz, DMSO-*d₆*) δ 7.71 (dd, *J =* 8.9, 2.2 Hz, 1H), 7.56 (dd, *J =* 8.6, 6.1 Hz, 1H), 7.44 (t, *J =* 9.1, 7.9 Hz, 1H), 7.02 (dd, *J =* 6.0, 2.4 Hz, 1H), 6.95 (d, *J =* 9.0 Hz, 1H), 6.87 (ddd, *J* = 8.9, 6.5, 2.4 Hz, 1H), 6.33 (s, 1H), 5.40 (qd, *J =* 6.6, 2.9 Hz, 1H), 3.22 - 2.95 (m, 4H), 2.82 - 2.67 (m, 2H), 2.01 (d, 1H), 1.69 (d, *J =* 12.1 Hz, 1H), 1.61 - 1.50 (m, 2H), 1.48 (d, *J* = 6.7 Hz, 3H).

### Preparation Example (III) 46: Compound A-1-2-1-104

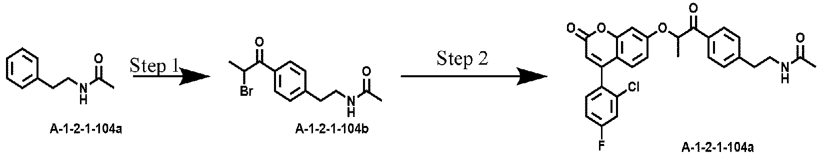

### Step 1

In a 100 mL single-necked flask, A-1-2-1-104a (800.0 mg, 4.90 mmol) and 2-bromopropionyl chloride (1.68 g, 9.80 mmol) were dissolved in dichloromethane (10 mL). Anhydrous aluminum chloride (1.96 g, 14.70 mmol) was slowly added to the solution at 0°C. After that, the reaction was carried out at 40°C for 1 h. After LCMS monitoring showed that the reaction was complete, the reaction solution was quenched with water (100 mL) and extracted with ethyl acetate (50 mL × 3). The organic layers were combined, washed with saturated sodium chloride solution, dried over anhydrous sodium sulfate, filtered, and rotary evaporated. The crude product was purified by flash column chromatography to give compound A-1-2-1-104b (700.0 mg, 2.35 mmol, yield: 48%).
MS-ESI: [M+H]⁺ = 298.0

### Step 2

In a 100 mL single-necked flask, intermediate A-1-2-1-104b (650.0 mg, 2.18 mmol), intermediate A1-1-1c (506.0 mg, 1.74 mmol), tetrabutylammonium iodide (1.21 g, 3.27 mmol), potassium carbonate (603.0 mg, 4.36 mmol) and *N*,*N*-dimethylformamide (10 mL) were successively added. After that, the reaction was carried out at room temperature for 3 h. After LCMS monitoring showed that the reaction was complete, the reaction solution was diluted with water and extracted with ethyl acetate (50 mL × 3). The organic layers were combined, washed with saturated sodium chloride solution, dried over anhydrous sodium sulfate, filtered, and rotary evaporated. The crude product was purified by flash column chromatography to give compound A-1-2-1-104 (200.0 mg, 0.39 mmol, yield: 18%).
MS-ESI: [M+H]⁺508.1
¹H NMR (400 MHz, DMSO-*d₆*) δ 8.03 (d, *J =* 7.8 Hz, 2H), 7.95 (t, *J =* 5.7 Hz, 1H), 7.68 (ddd, *J=* 10.6, 5.2, 2.7 Hz, 1H), 7.54 (dd, *J =* 8.5, 5.9 Hz, 1H), 7.42 (d, *J =* 8.3 Hz, 3H), 6.98 (dd, *J =* 6.6, 2.4 Hz, 1H), 6.93 (d, *J =* 8.9 Hz, 1H), 6.90 - 6.84 (m, 1H), 6.30 (q, *J =* 1.2 Hz, 1H), 6.23 (qd, *J =* 6.7, 4.6 Hz, 1H), 3.33 - 3.26 (m, 2H), 2.80 (t, *J =* 7.2 Hz, 2H), 1.78 (s, 3H), 1.56 (d, *J =* 6.6 Hz, 3H).

### Preparation Example (III) 47: Compound A-1-2-1-98

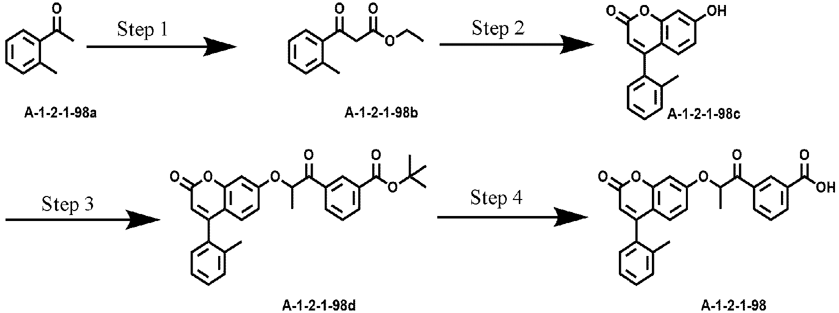

### Step 1

In a dry 250 mL three-necked flask, diethyl carbonate (17.61 g, 149.04 mmol) was added and dissolved in tetrahydrofuran (50 mL), and the system was purged with nitrogen. The reactor was cooled to 0°C in a low-temperature reaction bath. Sodium hydride (2.98 g, 74.52 mmol) was added in batches, and the reaction was carried out for 1 h. Then, A-1-2-1-98a (5.00 g, 37.26 mmol) was added to the flask. After that, the temperature was slowly raised to room temperature, and the reaction was carried out for 2 h (preferably 2-4 h). After TLC showed that the reaction was complete, the reaction was quenched with 1 M hydrochloric acid solution at 0°C to pH = 1, while controlling the temperature not to exceed 10°C. The reaction solution was extracted with ethyl acetate (100 mL × 3), and the layers were separated. The organic phases were combined, washed with saturated sodium chloride solution (200 mL), dried over anhydrous sodium sulfate, and rotary evaporated to give the crude intermediate A-1-2-1-98b (6.00 g, 33.94 mmol, yield: 54.7%).

### Step 2

In a dry 250 mL three-necked flask, A-1-2-1-98b (6.00 g, 20.36 mmol) and resorcinol (2.24 g, 20.36 mmol) were added and dissolved in methanesulfonic acid (30 mL). The temperature was raised to 50°C, and the reaction was carried out for 4 h (preferably 4-8 h). After LCMS showed that the reaction was complete, the reaction solution was slowly added to ethanol (100 mL) while controlling the quenching temperature. After that, the reaction solution was added to water (100 mL) and extracted with ethyl acetate (200 mL × 3). The layers were separated. The organic phases were combined, washed with saturated sodium chloride solution (500 mL), dried over anhydrous sodium sulfate, and rotary evaporated to give the crude intermediate A-1-2-1-98c (5.50 g, 21.80 mmol, yield: 91%).
MS-ESI: [M+H]⁺ = 253.2
¹H NMR (400 MHz, DMSO-*d₆*) δ 7.41 - 7.31 (m, 3H), 7.21 (d, *J =* 7.7 Hz, 1H), 6.83 - 6.77 (m, 2H), 6.72 (dd, *J =* 8.7, 2.2 Hz, 1H), 6.07 (d, *J =* 1.9 Hz, 1H), 2.09 (s, 3H).

### Step 3

In a dry 100 mL three-necked flask, A-1-2-1-98c (1.61 g, 6.39 mmol), A-1-2-1-1d (2.00 g, 6.39 mmol), potassium carbonate (1.77 g, 12.78 mmol) and tetrabutylammonium iodide (2.36 g, 6.39 mmol) were successively added, dissolved in acetone (30 mL), and stirred at room temperature for 8 h (preferably 8-12 h). After LCMS showed that the reaction was complete, the insolubles were filtered, and the solvent was rotary evaporated. Water (30 mL) was added. The mixture was extracted with ethyl acetate (15 mL × 3), and the layers were separated. The organic phases were combined, washed with saturated sodium chloride solution (20 mL), dried over anhydrous sodium sulfate, and purified by silica gel column chromatography (PE:EA = 5:1) to give the intermediate A-1-2-1-98d (0.50 g, 1.03 mmol, yield: 16.20%).
MS-ESI: [M-^{t}Bu]⁺= 429.2

### Step 4

In a dry 100 mL three-necked flask, A-1-2-1-98d (0.50 g, 1.03 mmol) was added and dissolved in dichloromethane (10 mL). Trifluoroacetic acid (10 mL) was added and stirred for 4 h (preferably, the reaction time was 4-8 h). After LCMS showed that the reaction was complete, the solvent was rotary evaporated. The residue was purified by silica gel column chromatography (PE:EA = 2:1) to give the product A-1-2-1-98 (335.30 mg, 0.78 mmol, yield: 75.0%).
MS-ESI: [M-H]⁻= 427.2
¹H NMR (400 MHz, Chloroform-d) δ 8.79 (t, *J =* 1.7 Hz, 1H), 8.36 (dq, *J =* 7.8, 1.5 Hz, 1H), 8.29 (dq, *J* = 7.9, 1.5 Hz, 1H), 7.65 (td, *J =* 7.8, 1.8 Hz, 1H), 7.37 (td, *J =* 7.5, 1.5 Hz, 1H), 7.32 - 7.27 (m, 2H), 7.15 - 7.10 (m, 1H), 6.94 (dd, *J* = 8.7, 0.9 Hz, 1H), 6.87 - 6.60 (m, 2H), 6.15 (s, 1H), 5.63 (dd, *J* = 6.9, 4.4 Hz, 1H), 2.13 (s, 3H), 1.79 (d*, J =* 6.9 Hz, 3H).

### Preparation Example (III) 48: Compound A-1-2-1-99

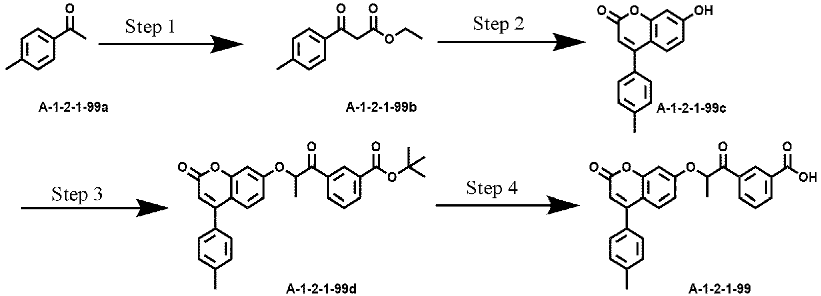

### Step 1

In a dry 250 mL three-necked flask, diethyl carbonate (17.61 g, 149.04 mmol) was added and dissolved in tetrahydrofuran (50 mL), and the system was purged with nitrogen. The reactor was cooled to 0°C in a low-temperature reaction bath. Sodium hydride (2.98 g, 74.52 mmol) was added in batches, and the reaction was carried out for 1 h. Then, A-1-2-1-99a (5.00 g, 37.26 mmol) was added to the flask. After that, the temperature was slowly raised to room temperature, and the reaction was carried out for 2 h (preferably 2-4 h). After TLC showed that the reaction was complete, the reaction was quenched with 1 M hydrochloric acid solution at 0°C to pH = 1, while controlling the temperature not to exceed 10°C. The reaction solution was extracted with ethyl acetate (100 mL × 3), and the layers were separated. The organic phases were combined, washed with saturated sodium chloride solution (200 mL), dried over anhydrous sodium sulfate, and rotary evaporated to give the crude intermediate A-1-2-1-99b (7.00 g, 33.94 mmol, yield: 91.1%).

### Step 2

In a dry 100 mL three-necked flask, A-1-2-1-99b (6.00 g, 33.94 mmol) and resorcinol (3.74 g, 19.98 mmol) were added and dissolved in methanesulfonic acid (30 mL). The temperature was raised to 50°C, and the reaction was carried out for 4 h (preferably 4-8 h). After LCMS showed that the reaction was complete, the reaction solution was slowly added to ethanol (100 mL) while controlling the quenching temperature. After that, the reaction solution was added to water (100 mL) and extracted with ethyl acetate (200 mL × 3). The layers were separated. The organic phases were combined, washed with saturated sodium chloride solution (500 mL), dried over anhydrous sodium sulfate, and rotary evaporated to give the crude intermediate A-1-2-1-99c (5.50 g, 11.71 mmol, yield: 54.6%).
MS-ESI: [M+H]⁺ = 253.2

### Step 3

In a dry 100 mL three-necked flask, A-1-2-1-99c (1.61 g, 6.39 mmol), A-1-2-1-98c (2.00 g, 6.39 mmol), potassium carbonate (1.77 g, 12.78 mmol) and tetrabutylammonium iodide (2.36 g, 6.39 mmol) were successively added, dissolved in acetone (30 mL), and stirred at room temperature for 8 h (preferably 8-12 h). After LCMS showed that the reaction was complete, the insolubles were filtered, and the solvent was rotary evaporated. Water (30 mL) was added. The mixture was extracted with ethyl acetate (15 mL × 3), and the layers were separated. The organic phases were combined, washed with saturated sodium chloride solution (20 mL), dried over anhydrous sodium sulfate, and purified by silica gel column chromatography (PE:EA = 5:1) to give the intermediate A-1-2-1-99d (0.35 g, 0.72 mmol, yield: 11.31%).
MS-ESI: [M-^{t}Bu]⁺= 429.2

### Step 4

In a dry 50 mL three-necked flask, A-1-2-1-99d (0.35 g, 0.72 mmol) was added and dissolved in dichloromethane (10 mL). Trifluoroacetic acid (10 mL) was added and stirred for 4 h (preferably, the reaction time was 4-8 h). After LCMS showed that the reaction was complete, the solvent was rotary evaporated. The residue was purified by silica gel column chromatography (PE:EA = 2:1) to give the product A-1-2-1-99 (238.60 mg, 0.56 mmol, yield: 74.0%).
MS-ESI: [M-H]⁻= 427.2
¹H NMR (400 MHz, Chloroform-d) δ 8.80 (t, *J =* 1.8 Hz, 1H), 8.36 (dt, *J =* 7.7, 1.4 Hz, 1H), 8.30 (dt, *J =* 7.9, 1.5 Hz, 1H), 7.65 (t, *J =* 7.8 Hz, 1H), 7.41 (d, *J =* 8.7 Hz, 1H), 7.30 (s, 4H), 6.86 - 6.76 (m, 2H), 6.20 (s, 1H), 5.64 (d, *J =* 6.9 Hz, 1H), 2.43 (s, 3H), 1.80 (d, *J =* 6.9 Hz, 3H).

### Preparation Example (III) 49: Compound A-1-2-1-100

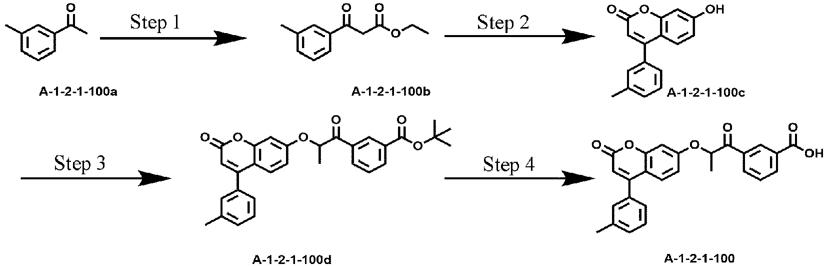

### Step 1

In a dry 100 mL three-necked flask, diethyl carbonate (8.80 g, 74.52 mmol) was added and dissolved in tetrahydrofuran (50 mL), and the system was purged with nitrogen. The reactor was cooled to 0°C in a low-temperature reaction bath. Sodium hydride (1.49 g, 37.26 mmol) was added in batches, and the reaction was carried out for 1 h. Then, A-1-2-1-100a (2.50 g, 18.63 mmol) was added to the flask. After that, the temperature was slowly raised to room temperature, and the reaction was carried out for 2 h (preferably 2-4 h). After TLC showed that the reaction was complete, the reaction was quenched with 1 M hydrochloric acid solution at 0°C to pH = 1, while controlling the temperature not to exceed 10°C. The reaction solution was extracted with ethyl acetate (100 mL × 3), and the layers were separated. The organic phases were combined, washed with saturated sodium chloride solution (200 mL), dried over anhydrous sodium sulfate, and rotary evaporated to give the crude intermediate A-1-2-1-100b (3.00 g, 33.94 mmol, yield: 78.1%).

### Step 2

In a dry 100 mL three-necked flask, A-1-2-1-100b (3.00 g, 14.55 mmol) and resorcinol (1.60 g, 14.55 mmol) were added and dissolved in methanesulfonic acid (30 mL). The temperature was raised to 50°C, and the reaction was carried out for 4 h (preferably 4-8 h). After LCMS showed that the reaction was complete, the reaction solution was slowly added to ethanol (100 mL) while controlling the quenching temperature. After that, the reaction solution was added to water (100 mL) and extracted with ethyl acetate (200 mL × 3). The layers were separated. The organic phases were combined, washed with saturated sodium chloride solution (500 mL), dried over anhydrous sodium sulfate, and rotary evaporated to give the crude intermediate A-1-2-1-100c (3.50 g, 13.88 mmol, yield: 81.1%).
MS-ESI: [M+H]⁺ = 253.2
¹H NMR (400 MHz, DMSO-*d₆*) δ 7.41 - 7.31 (m, 3H), 7.21 (d, *J =* 7.7 Hz, 1H), 6.83 - 6.77 (m, 2H), 6.72 (dd, *J =* 8.7, 2.2 Hz, 1H), 6.07 (d, *J =* 1.9 Hz, 1H), 2.09 (s, 3H).

### Step 3

In a dry 100 mL three-necked flask, A-1-2-1-100c (1.61 g, 6.39 mmol), A-1-2-1-98c (2.00 g, 6.39 mmol), potassium carbonate (1.77 g, 12.78 mmol) and tetrabutylammonium iodide (2.36 g, 6.39 mmol) were successively added, dissolved in acetone (30 mL), and stirred at room temperature for 8 h (preferably 8-12 h). After LCMS showed that the reaction was complete, the insolubles were filtered, and the solvent was rotary evaporated. Water (30 mL) was added. The mixture was extracted with ethyl acetate (15 mL × 3), and the layers were separated. The organic phases were combined, washed with saturated sodium chloride solution (20 mL), dried over anhydrous sodium sulfate, and purified by silica gel column chromatography (PE:EA = 5:1) to give the intermediate A-1-2-1-100d (0.60 g, 1.24 mmol, yield: 19.40%).
MS-ESI: [M-^{t}Bu]⁺= 429.2

### Step 4

In a dry 50 mL three-necked flask, A-1-2-1-100d (0.60 g, 1.24 mmol) was added and dissolved in dichloromethane (10 mL). Trifluoroacetic acid (10 mL) was added and stirred for 4 h (preferably, the reaction time was 4-8 h). After LCMS showed that the reaction was complete, the solvent was rotary evaporated. The residue was purified by silica gel column chromatography (PE:EA = 2:1) to give the product A-1-2-1-100 (331.6.0 mg, 0.77 mmol, yield: 60.0%).
MS-ESI: [M-H]⁻= 427.2
¹H NMR (400 MHz, Chloroform-d) δ 8.80 (t, *J =* 1.8 Hz, 1H), 8.36 (dt, *J =* 7.8, 1.4 Hz, 1H), 8.30 (dt, *J* = 7.9, 1.5 Hz, 1H), 7.65 (t, *J =* 7.8 Hz, 1H), 7.43 - 7.32 (m, 2H), 7.32 - 7.28 (m, 1H), 7.21 - 7.14 (m, 2H), 6.89 - 6.76 (m, 2H), 6.20 (s, 1H), 5.64 (q, *J* = 6.9 Hz, 1H), 2.41 (s, 3H), 1.80 (d, *J* = 6.9 Hz, 3H).

### Preparation Example (III) 50: Compound A-1-2-1-101

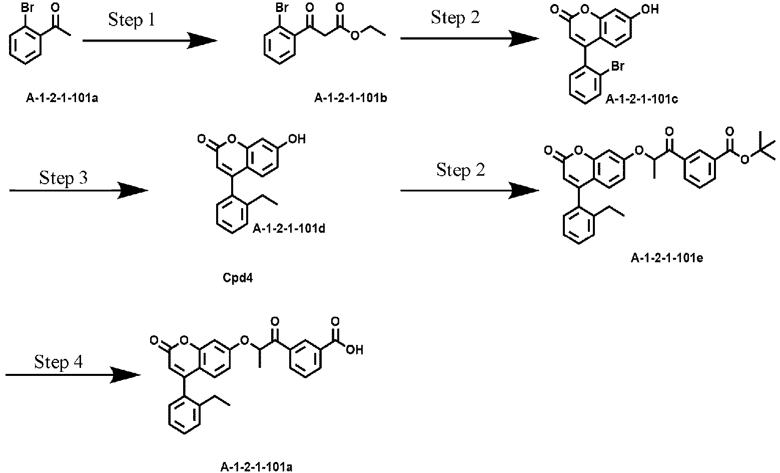

### Step 1

In a dry 100 mL three-necked flask, diethyl carbonate (11.87 g, 100.48 mmol) was added and dissolved in tetrahydrofuran (50 mL), and the system was purged with nitrogen. The reactor was cooled to 0°C in a low-temperature reaction bath. Sodium hydride (2.01 g, 50.24 mmol) was added in batches, and the reaction was carried out for 1 h. Then, A-1-2-1-101a (5.00 g, 25.12 mmol) was added to the flask. After that, the temperature was slowly raised to room temperature, and the reaction was carried out for 2 h (preferably 2-4 h). After TLC showed that the reaction was complete, the reaction was quenched with 1 M hydrochloric acid solution at 0°C to pH = 1, while controlling the temperature not to exceed 10°C. The reaction solution was extracted with ethyl acetate (100 mL × 3), and the layers were separated. The organic phases were combined, washed with saturated sodium chloride solution (200 mL), dried over anhydrous sodium sulfate, and rotary evaporated to give the crude intermediate A-1-2-1-101b (6.50 g, 23.98 mmol, yield: 95.44%).
MS-ESI: [M-H]⁻ = 268.9

### Step 2

In a dry 100 mL three-necked flask, A-1-2-1-101b (6.50 g, 23.98 mmol) and resorcinol (2.64 g, 23.98 mmol) were added and dissolved in methanesulfonic acid (30 mL). The temperature was raised to 50°C, and the reaction was carried out for 4 h (preferably 4-8 h). After LCMS showed that the reaction was complete, the reaction solution was slowly added to ethanol (100 mL) while controlling the quenching temperature. After that, the reaction solution was added to water (100 mL) and extracted with ethyl acetate (200 mL × 3). The layers were separated. The organic phases were combined, washed with saturated sodium chloride solution (500 mL), dried over anhydrous sodium sulfate, and rotary evaporated to give the crude intermediate A-1-2-1-101c (7.20 g, 19.30 mmol, yield: 80.49%).
MS-ESI: [M-H]⁻ = 314.9

### Step 3

In a dry 100 mL three-necked flask, A-1-2-1-101c (2.00 g, 6.31 mmol), ethylboronic acid (2.33 g, 31.55 mmol) and potassium phosphate (4.02 g, 18.93 mmol) were successively added and dissolved in 20 mL of toluene and 4 mL of water. The system was purged with nitrogen. Palladium acetate (0.071 g, 0.32 mmol) and tricyclohexyl phosphorus (0.18 g, 0.63 mmol) were added. The reaction was carried out at 100°C for 6 h (preferably 6-8 h). After LCMS showed that the reaction was complete, the insolubles were filtered, and the solvent was rotary evaporated. Water (20 mL) was added. The mixture was extracted with ethyl acetate (20 mL × 3), and the layers were separated. The organic phases were combined, washed with saturated sodium chloride solution (20 mL), dried over anhydrous sodium sulfate, and purified by silica gel column chromatography (PE:EA = 5:1) to give the intermediate A-1-2-1-101d (1.15 g, 4.31 mmol, yield: 68.48%).
MS-ESI: [M-H]⁻= 265.1

### Step 4

In a dry 100 mL three-necked flask, A-1-2-1-101d (1.15 g, 4.31 mmol) and A1-2-1-98c (1.50 g, 4.79 mmol) were successively added and dissolved in 10 mL of acetone. Tetrabutylammonium iodide (1.77 g, 4.79 mmol) and potassium carbonate (1.32 g, 9.58 mmol) were added, stirred, and dispersed at room temperature for 8 h (preferably 8-12 h). After LCMS showed that the reaction was complete, the insolubles were filtered, and the solvent was rotary evaporated. Water (30 mL) was added. The mixture was extracted with ethyl acetate (20 mL × 3), and the layers were separated. The organic phases were combined, washed with saturated sodium chloride solution (20 mL), dried over anhydrous sodium sulfate, and purified by silica gel column chromatography (PE:EA = 5:1) to give the intermediate A-1-2-1-101e (580.0 mg, 1.16 mmol, yield: 24.29%).
MS-ESI: [M-H]⁻ = 497.2

### Step 5

In a dry 50 mL three-necked flask, A-1-2-1-101e (580.0 mg, 1.16 mmol) was added and dissolved in dichloromethane (10 mL). Trifluoroacetic acid (10 mL) was added and stirred for 4 h (preferably, the reaction time was 4-8 h). After LCMS showed that the reaction was complete, the solvent was rotary evaporated. The residue was purified by silica gel column chromatography (PE:EA = 2:1) to give the product A-1-2-1-101 (336.2.0 mg, 0.76 mmol, yield: 36.27%).

MS-ESI: [M-H]⁻ = 441.2.

¹H NMR (400 MHz, Chloroform-d) δ 8.79 (t, *J =* 1.6 Hz, 1H), 8.36 (dq, *J =* 7.8, 1.6 Hz, 1H), 8.33 - 8.26 (m, 1H), 7.65 (td, *J* = 7.8*,* 2.1 Hz, 1H), 7.41 (td, *J =* 7.5, 1.4 Hz, 1H), 7.35 (dd, *J =* 7.8, 1.4 Hz, 1H), 7.30 - 7.24 (m, 1H), 7.10 (dt, *J =* 7.6, 1.6 Hz, 1H), 6.94 (dd, *J =* 8.8, 1.4 Hz, 1H), 6.81 - 6.72 (m, 2H), 6.17 (s, 1H), 5.66 - 5.53 (m, 1H), 2.54 - 2.27 (m, 2H), 1.79 (d, *J* = 6.8 Hz, 3H), 1.14 - 0.95 (m, 3H).

### Preparation Example (III) 51: Compound A-1-2-1-102

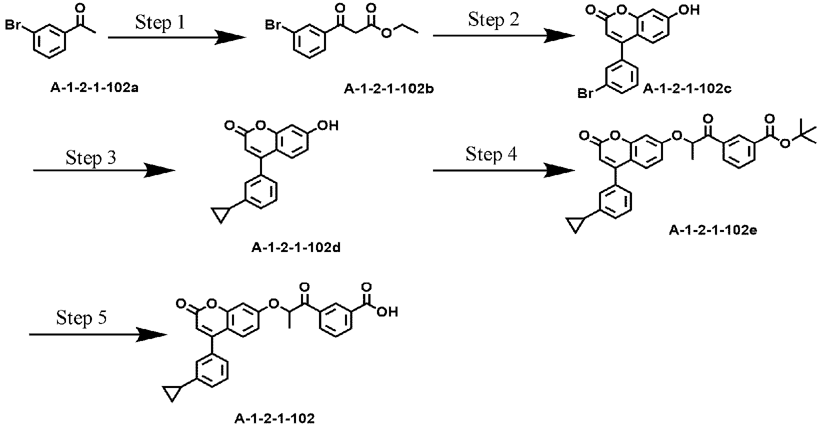

### Step 1

In a dry 100 mL three-necked flask, diethyl carbonate (11.87 g, 100.48 mmol) was added and dissolved in tetrahydrofuran (50 mL), and the system was purged with nitrogen. The reactor was cooled to 0°C in a low-temperature reaction bath. Sodium hydride (2.01 g, 50.24 mmol) was added in batches, and the reaction was carried out for 1 h. Then, A-1-2-1-102a (5.00 g, 25.12 mmol) was added to the flask. After that, the temperature was slowly raised to room temperature, and the reaction was carried out for 2 h (preferably 2-4 h). After TLC showed that the reaction was complete, the reaction was quenched with 1 M hydrochloric acid solution at 0°C to pH = 1, while controlling the temperature not to exceed 10°C. The reaction solution was extracted with ethyl acetate (100 mL × 3), and the layers were separated. The organic phases were combined, washed with saturated sodium chloride solution (200 mL), dried over anhydrous sodium sulfate, and rotary evaporated to give the crude intermediate A-1-2-1-102b (6.50 g, 23.98 mmol, yield 95.44%).
MS-ESI: [M-H]⁻ = 268.9

### Step 2

In a dry 100 mL three-necked flask, A-1-2-1-102b (6.50 g, 23.98 mmol) and resorcinol (2.64 g, 23.98 mmol) were added and dissolved in methanesulfonic acid (30 mL). The temperature was raised to 50°C, and the reaction was carried out for 4 h (preferably 4-8 h). After LCMS showed that the reaction was complete, the reaction solution was slowly added to ethanol (100 mL) while controlling the quenching temperature. After that, the reaction solution was added to water (100 mL) and extracted with ethyl acetate (200 mL × 3). The layers were separated. The organic phases were combined, washed with saturated sodium chloride solution (500 mL), dried over anhydrous sodium sulfate, and rotary evaporated to give the crude intermediate A-1-2-1-102c (7.20 g, 19.30 mmol, yield: 80.49%).
MS-ESI: [M-H]⁻ = 314.9

### Step 3

In a dry 100 mL three-necked flask, A-1-2-1-102c (4.00 g, 12.61 mmol), cyclopropylboronic acid (15.13 g, 7.57 mmol) and potassium phosphate (8.03 g, 37.83 mmol) were successively added and dissolved in 40 mL of toluene and 8 mL of water. The system was purged with nitrogen. Palladium acetate (0.14 g, 0.63 mmol) and tricyclohexyl phosphorus (0.35 g, 1.26 mmol) were added. The reaction was carried out at 100°C for 6 h. After LCMS showed that the reaction was complete, the insolubles were filtered, and the solvent was rotary evaporated. Water (30 mL) was added. The mixture was extracted with ethyl acetate (20 mL × 3), and the layers were separated. The organic phases were combined, washed with saturated sodium chloride solution (20 mL), dried over anhydrous sodium sulfate, and purified by silica gel column chromatography (PE:EA = 5:1) to give the intermediate A-1-2-1-102d (1.75 g, 5.75 mmol, yield: 48.43%).
MS-ESI: [M-H]⁻= 277.0

### Step 4

In a dry 100 mL three-necked flask, tert-butyl 3-(2-bromopropanoyl)benzoate (2.50 g, 7.98 mmol) was dissolved in 20 mL of acetone. Tetrabutylammonium iodide (2.95 g, 7.98 mmol) and potassium carbonate (2.21 g, 15.96 mmol) were added, stirred and dispersed. Finally, A-1-2-1-102d (1.75 g, 6.29 mmol) was added and stirred at room temperature for 8 h (preferably 8-12 h). After LCMS showed that the reaction was complete, the insolubles were filtered, and the solvent was rotary evaporated. Water (30 mL) was added. The mixture was extracted with ethyl acetate (20 mL × 3), and the layers were separated. The organic phases were combined, washed with saturated sodium chloride solution (20 mL), dried over anhydrous sodium sulfate, and purified by silica gel column chromatography (PE:EA = 5:1) to give the intermediate A-1-2-1-102e (1.30 g, 2.55 mmol, yield: 31.90%).
MS-ESI: [M-^{t}Bu]⁺= 455.2

### Step 5

In a dry 100 mL three-necked flask, A-1-2-1-102e (1.30 g, 2.55 mmol) was added and dissolved in dichloromethane (10 mL). Trifluoroacetic acid (10 mL) was added and stirred for 4 h (preferably, the reaction time was 4-8 h). After LCMS showed that the reaction was complete, the solvent was rotary evaporated. The residue was purified by silica gel column chromatography (PE:EA = 2:1) to give the product A-1-2-1-102 (679.3.0 mg, 1.38 mmol, yield: 54.33%).
MS-ESI: [M+H]⁺ = 455.2
¹H NMR (400 MHz, Chloroform-d) δ 8.79 (d, *J* = 1.7 Hz, 1H), 8.36 (dt, *J* = 7.7, 1.4 Hz, 1H), 8.30 (dt, *J* = 7.8, 1.5 Hz, 1H), 7.65 (t, *J =* 7.8 Hz, 1H), 7.42 - 7.29 (m, 2H), 7.19 - 7.12 (m, 2H), 7.09 (t, *J* = 1.8 Hz, 1H), 6.85 - 6.77 (m, 2H), 6.19 (s, 1H), 5.63 (d, *J*= 6.9 Hz, 1H), 1.94 (ddd, *J* = 8.5, 5.1, 3.4 Hz, 1H), 1.80 (d, *J =* 6.8 Hz, 3H), 1.08 - 0.95 (m, 2H), 0.72 (dt, *J =* 6.7, 4.7 Hz, 2H).

### Preparation Example (III) 52: Compound A-1-2-1-103

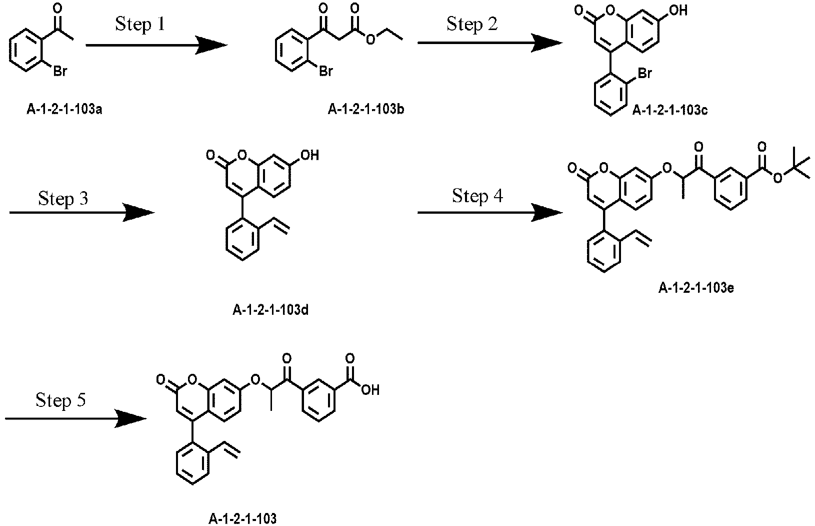

### Step 1

In a dry 100 mL three-necked flask, diethyl carbonate (11.87 g, 100.48 mmol) was added and dissolved in tetrahydrofuran (50 mL), and the system was purged with nitrogen. The reactor was cooled to 0°C in a low-temperature reaction bath. Sodium hydride (2.01 g, 50.24 mmol) was added in batches, and the reaction was carried out for 1 h. Then, A-1-2-1-103a (5.00 g, 25.12 mmol) was added to the flask. After that, the temperature was slowly raised to room temperature, and the reaction was carried out for 2 h (preferably 2-4 h). After TLC showed that the reaction was complete, the reaction was quenched with 1 M hydrochloric acid solution at 0°C to pH = 1, while controlling the temperature not to exceed 10°C. The reaction solution was extracted with ethyl acetate (100 mL × 3), and the layers were separated. The organic phases were combined, washed with saturated sodium chloride solution (200 mL), dried over anhydrous sodium sulfate, and rotary evaporated to give the crude intermediate A-1-2-1-103b (6.50 g, 23.98 mmol, yield: 95.44%).
MS-ESI: [M-H]⁻ = 268.9

### Step 2

In a dry 100 mL three-necked flask, A-1-2-1-103b (6.50 g, 23.98 mmol) and resorcinol (2.64 g, 23.98 mmol) were added and dissolved in methanesulfonic acid (30 mL). The temperature was raised to 50°C, and the reaction was carried out for 4 h (preferably 4-8 h). After LCMS showed that the reaction was complete, the reaction solution was slowly added to ethanol (100 mL) while controlling the quenching temperature. After that, the reaction solution was added to water (100 mL) and extracted with ethyl acetate (200 mL × 3). The layers were separated. The organic phases were combined, washed with saturated sodium chloride solution (500 mL), dried over anhydrous sodium sulfate, and rotary evaporated to give the crude intermediate A-1-2-1-103c (7.20 g, 19.30 mmol, yield: 80.49%).
MS-ESI: [M-H]⁻ = 314.9

### Step 3

In a dry 100 mL three-necked flask, A-1-2-1-103c (2.00 g, 6.31mmol), palladium acetate (0.071 g, 0.32 mmol), triphenylphosphine (0.17 g, 0.63 mmol), cesium carbonate (6.17 g, 18.93 mmol) and potassium vinyltrifluoroborate (1.27 g, 9.46 mmol) were successively added. The system was purged with nitrogen, and the mixture was dissolved in 20 mL of dioxane and 2 mL of water. The reaction was carried out at 100°C for 6 h (preferably 6-8 h). After LCMS showed that the reaction was complete, the insolubles were filtered, and the solvent was rotary evaporated. Water (20 mL) was added. The mixture was extracted with ethyl acetate (20 mL × 3), and the layers were separated. The organic phases were combined, washed with saturated sodium chloride solution (20 mL), dried over anhydrous sodium sulfate, and purified by silica gel column chromatography (PE:EA = 5:1) to give the intermediate A-1-2-1-103d (1.30 g, 4.60 mmol, yield: 54.90%).

### Step 4

In a dry 100 mL three-necked flask, A-1-2-1-103d (1.22 g, 4.60 mmol) and A-1-2-1-1d (1.60 g, 5.11 mmol) were successively added and dissolved in 20 mL of acetone. Tetrabutylammonium iodide (1.89 g, 5.11 mmol) and potassium carbonate (1.41 g, 10.22 mmol) were added, stirred, and dispersed at room temperature for 8 h (preferably 8-12 h). After LCMS showed that the reaction was complete, the insolubles were filtered, and the solvent was rotary evaporated. Water (30 mL) was added. The mixture was extracted with ethyl acetate (20 mL × 3), and the layers were separated. The organic phases were combined, washed with saturated sodium chloride solution (20 mL), dried over anhydrous sodium sulfate, and purified by silica gel column chromatography (PE:EA = 5:1) to give the intermediate A-1-2-1-103e (860.0 mg, 1.73 mmol, yield: 33.90%).
MS-ESI: [M-H]⁻= 495.2

### Step 5

In a dry 50 mL three-necked flask, A-1-2-1-103e (860.0 mg, 1.73 mmol) was added and dissolved in dichloromethane (10 mL). Trifluoroacetic acid (10 mL) was added and stirred for 4 h (preferably, the reaction time was 4-8 h). After LCMS showed that the reaction was complete, the solvent was rotary evaporated. The residue was purified by silica gel column chromatography (PE:EA = 2:1) to give the product A-1-2-1-103 (383.0.0 mg, 0.87 mmol, yield: 41.22%).
MS-ESI: [M-H]⁻ = 439.2
¹H NMR (400 MHz, Chloroform-d) =δ 8.79 (t, *J* = 1.7 Hz, 1H), 8.36 (dq, *J* = 7.8, 1.6 Hz, 1H), 8.32 - 8.26 (m, 1H), 7.72 - 7.63 (m, 2H), 7.45 (td, *J =* 7.7, 1.5 Hz, 1H), 7.36 (tt, *J =* 7.4, 1.0 Hz, 1H), 7.17 (dt, *J =* 7.5, 1.6 Hz, 1H), 7.02 - 6.94 (m, 1H), 6.83 - 6.71 (m, 2H), 6.51 - 6.33 (m, 1H), 6.17 (s, 1H), 5.71 (dd, *J* = 17.3, 1.0 Hz, 1H), 5.65 - 5.57 (m, 1H), 5.18 (dd, *J* = 10.9, 1.0 Hz, 1H), 1.79 (d*, J =* 6.9 Hz, 3H).

### Preparation Example (III) 53: Compound A-1-2-2-2

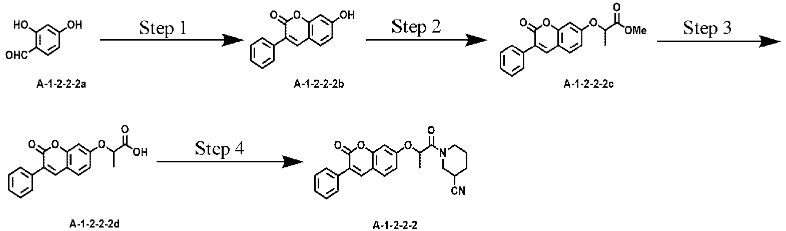

### Step 1

In a dry 100 mL three-necked flask, compound A-1-2-2-2a (5.00 g, 36.2 mmol) and phenylacetyl chloride (6.16 g, 39.8 mmol, 5.31 mL) were dissolved in acetone (360 mL), and potassium carbonate (17.5 g, 126 mmol) was added at 25°C. The mixture was stirred at 60°C for 6 h. The progress of the reaction was monitored by LCMS. After completion of the reaction, the reaction solution was poured into water (300 mL) and extracted with ethyl acetate (200 mL × 3). The organic phases were combined, washed with saturated brine (40 mL), dried over anhydrous sodium sulfate, filtered, and concentrated. The crude product was directly used in the next step. Compound A-1-2-2-2b as a yellow solid (7.26 g, 29.7 mmol, yield: 82.1%, purity: 97.5%) was obtained.
MS-ESI: [M+H]⁺ = 239.1
¹H NMR (400 MHz, DMSO-*d₆*)= δ10.61 (br s, 1H), 8.15 (s, 1H), 7.71 - 7.69 (m, 1H), 7.68 (s, 1H), 7.60 (d, *J =* 8.4 Hz, 1H), 7.46 - 7.41 (m, 2H), 7.40 - 7.35 (m, 1H), 6.82 (dd, *J =* 2.4, 8.8 Hz, 1H), 6.76 (d, *J* = 2.4 Hz, 1H).

### Step 2

In a dry 100 mL three-necked flask, compound A-1-2-2-2b (1.50 g, 6.14 mmol, 97.5%) and methyl 2-bromopropanoate (1.23 g, 7.37 mmol) were dissolved in N,N-dimethylformamide (15 mL), and cesium carbonate (4.00 g, 12.3 mmol) was added at 25°C. The mixture was stirred at 70°C for 2 h. The progress of the reaction was monitored by LCMS. After completion of the reaction, the reaction solution was poured into water (100 mL) and extracted with ethyl acetate (100 mL × 3). The organic phases were combined, washed with saturated brine (150 mL), dried over anhydrous sodium sulfate, filtered, and concentrated. The crude product was directly used in the next step. Compound A-1-2-2-2c as a yellow solid (1.77 g, 5.24 mmol, yield: 85.3%, purity: 96.0%) was obtained.
MS-ESI: [M+H]⁺ = 325.0
¹H NMR( 400 MHz, DMSO-*d₆*)=δ 8.20 (s, 1H), 7.72 - 7.69 (m, 3H), 7.48 - 7.37 (m, 3H), 6.98 (dd, *J =* 2.4, 4.4 Hz, 2H), 5.24 (q, *J* = 6.8 Hz, 1H), 3.70 (s, 3H), 1.56 (d, *J =* 6.8 Hz, 3H).

### Step 3

In a dry 100 mL three-necked flask, compound A-1-2-2-2c (1.77 g, 5.24 mmol, purity: 96.0%) was dissolved in tetrahydrofuran (90 mL) and water (30 mL), and lithium hydroxide monohydrate (219.0 mg, 5.24 mmol, 1.00 eq) was added at 0°C. The mixture was stirred at 0°C for 2 h. The progress of the reaction was monitored by LCMS. After completion of the reaction, the mixture was adjusted to pH = 8 with 1M HCl and then extracted with ethyl acetate (100 mL × 3). The combined organic layer was dried over sodium sulfate, filtered, and concentrated. The residue was purified by preparative HPLC (under HCl conditions) to give compound A-1-2-2-2d as a white solid (200.0 mg, 524 µmol, yield: 10.0%, purity: 81.3%).
MS-ESI: [M+H]⁺ = 311.1

### Step 4

In a dry 100 mL three-necked flask, compound A-1-2-2-2d (200.0 mg, 524 µmol, purity: 81.3%), 3-piperidinecarbonitrile (57.7.0 mg, 524 µmol), 2-(7-azabenzotriazole)-*N,N,N',N'*-tetra methyluronium hexafluorophosphate (239.0 mg, 628 µmol) and *N*,*N*-diisopropylethyl amine (101.0 mg, 786 µmol) were dissolved in *N*,*N*-dimethylformamide (2 mL). The mixture was stirred at 20°C for 16 h. The progress of the reaction was monitored by LCMS. After c ompletion of the reaction, the reaction mixture was poured into water (10 mL) and extract ed with ethyl acetate (10 mL × 3). The combined organic phase was washed with saturate d brine (40 mL), dried over anhydrous sodium sulfate, filtered, and concentrated. The resi due was purified by preparative HPLC (Column: Phenomene* C18 150*25m*10 µm; Mobi le phase: [Water (ammonium bicarbonate)-acetonitrile; B%: 30%-60%, 8 min). The mixture was freeze-dried to give A-1-2-2-2 as a white solid (70.0 mg, 172 µmol, yield: 32.8%, p urity: 99.0%).
MS-ESI: [M+H]⁺ = 403.0

### Preparation Example (III) 54: Compound A-1-2-6-22

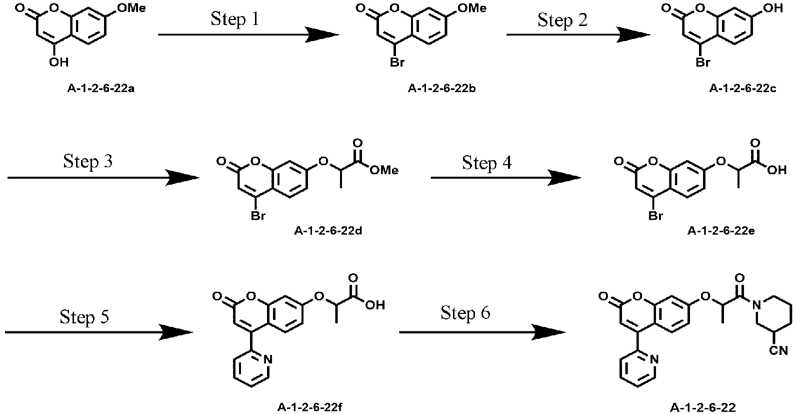

### Step 1

In a dry 100 mL three-necked flask, a solution of compound A-1-2-6-22a (4.50 g, 23.4 mmol, 1.00 eq) in POBr₃ (8.73 g, 30.4 mmol, 3.09 mL, 1.30 eq) was stirred at 130°C for 1 h. The progress of the reaction was monitored by LCMS. After completion of the reaction, the reaction mixture was cooled to 20°C, poured into water (50 mL), and then adjusted to pH = 9 with saturated aqueous sodium bicarbonate solution. The reaction mixture was extracted with ethyl acetate (20 mL × 3). The organic phases were combined, washed with saturated brine (20 mL × 2), dried over anhydrous sodium sulfate, and concentrated to give a crude product. The product was directly used in the next step. Compound A-1-2-6-22b as a yellow solid (3.00 g, 7.47 mmol, yield: 31.9%, purity: 63.5%) was obtained.
MS-ESI: [M+H]⁺ = 254.8

### Step 2

In a dry 100 mL three-necked flask, compound A-1-2-6-22b (3.00 g, 11.8 mmol, 1.00 eq) was added and dissolved in dichloromethane (100 mL). After purging with nitrogen, the reaction temperature was controlled at 20°C. Boron tribromide (8.84 g, 35.3 mmol, 3.40 mL, 3.00 eq) was added and stirred at 20°C for another 1 h. The progress of the reaction was monitored by LCMS. After completion of the reaction, the reaction mixture was poured into water (300 mL) at 20°C, adjusted to pH = 9 with saturated aqueous sodium bicarbonate solution, and extracted with ethyl acetate (50 mL × 3). The organic phases were combined, washed with saturated brine (50 mL × 2), dried over anhydrous sodium sulfate, and concentrated to give a crude product. The crude product was directly used in the next step. Compound A-1-2-6-22 as a yellow solid (2.70 g, 1.04 mmol, yield: 8.83%, purity: 9.27%) was obtained.
MS-ESI: [M+H]⁺= 240.8

### Step 3

In a dry 100 mL three-necked flask, compound a-1-2-6-22c (2.54 g, 10.5 mmol, 1.00 eq), methyl 2-bromopropionate (1.85 g, 11.1 mmol, 1.23 mL, 1.05 eq) and cesium carbonate (5.15 g, 15.8 mmol, 1.5 eq) were dissolved in N,N-dimethylformamide (26 mL). The mixture was stirred at 20°C for 2 h. The progress of the reaction was monitored by LCMS. After completion of the reaction, the reaction mixture was poured into water (100 mL). The reaction mixture was extracted with ethyl acetate (20 mL × 3). The organic phases were combined, washed with saturated brine (20 mL × 2), dried over anhydrous sodium sulfate, and concentrated to give a crude product. The product was directly used in the next step. Compound A-1-2-6-22d as a yellow solid (2.45 g, 4.28 mmol, yield: 40.7%, purity: 57.2%) was obtained.
MS-ESI: [M+H]⁺ = 326.8

### Step 4

In a dry 100 mL three-necked flask, compound A-1-2-6-22d (2.45 g, 7.49 mmol, 1.00 eq) was dissolved in hydrochloric acid solution (25 mL, purity: 37%) and stirred at 20°C for 12 h. The progress of the reaction was monitored by LCMS. After completion of the reaction, the reaction mixture was poured into 1M hydrochloric acid solution (100 mL) at 20°C. The reaction mixture was extracted with ethyl acetate (20 mL × 3). The organic phases were combined, washed with saturated brine (100 mL × 2), dried over anhydrous sodium sulfate, and concentrated to give a crude product. The crude product was directly used in the next step. Compound A-1-2-6-22e as a yellow solid (2.37 g, 4.97 mmol, yield: 66.3%, purity: 65.6%) was obtained.
MS-ESI: [M+H]⁺ = 312.8

### Step 5

In a dry 100 mL three-necked flask, compound a-1-2-6-22e (150.0 mg, 479 µmol, 1.00 eq), tributyl (2-pyridyl)stannane (229.0 mg, 621 µmol), cuprous iodide (45.6.0 mg, 240 µmol) and triphenylphosphine (25.1.0 mg, 95.8 µmol) were added. After purging with nitrogen, tris(dibenzylidene)acetone dipalladium (43.9.0 mg, 47.9 µmol) was added. Finally, dioxane (7 mL) was added as the solvent. The mixture was degassed under vacuum and purged with nitrogen three times again, and then stirred at 50°C for 12 h. The progress of the reaction was monitored by LCMS. After completion of the reaction, the mixture was cooled to 25°C and filtered through a pad of diatomite powder. The filtrate was washed with ethyl acetate (10 mL). Water (30 mL) was added to the filtrate, and the mixture was extracted with ethyl acetate (10 mL). The organic phases were combined, washed with saturated brine (20 mL × 2), dried over anhydrous sodium sulfate, and concentrated to give a crude product. The product was directly used in the next step. Compound A-1-2-6-22f as a yellow oil (530.0 mg, crude) was obtained.
MS-ESI: [M+H]⁺= 311.9

### Step 6

In a dry 100 mL three-necked flask, compound a-1-2-6-6-22f (530.0 mg, 1.70 mmol), 3-pi peridinecarbonitrile (225.0 mg, 2.04 mmol), 2-(7-azabenzotriazole)-*N*,*N*,*N'*,*N'*-tetramethyluroni um hexafluorophosphate (971.0 mg, 2.55 mmol, 1.50 eq) and N,N-diisopropylethyl amine ( 440.0 mg, 3.41 mmol, 593 µL, 2.00 eq) was dissolved in *N*,*N*-diisopropylethyl amine (5 mL) and stirred at 20°C for 1 h. The progress of the reaction was monitored by LCMS. After completion of the reaction, the reaction mixture was poured into water (30 mL) and extracted with ethyl acetate (10 mL × 3). The organic phases were combined, washed wi th saturated brine (20 mL × 2), dried over anhydrous sodium sulfate, and concentrated to give a crude product. The crude product was purified by preparative HPLC (Column: Phe nome ne * C18 250*50 mm*10 µm; Mobile phase: [Water (ammonium bicarbonate)-aceton itrile; B%: 15%-4.6%, 9 minutes) to give A-1-2-6-22 as a yellow solid (84.0 mg, 206 µm ol, yield: 12.1%, purity: 99.0%).
MS-ESI: [M+H]⁺= 404.1
¹H NMR: (400 MHz, DMSO-*d₆*)=δ 8.78 (d, *J =* 4.0 Hz, 1H), 8.11 - 7.94 (m, 1H), 7.78 *(d, J* = 7.6 Hz, 1H), 7.73 - 7.65 (m, 1H), 7.72 - 7.66 (m, 1H), 7.03 - 6.91 (m, 1H), 6.90 - 6.86 (m, 1H), 6.44 (s, 1H), 5.63 - 5.53 (m, 1H), 4.02 - 3.82 (m, 1H), 3.80 - 3.63 (m, 1H), 3.60 - 3.46 (m, 2H), 3.17 - 3.01 (m, 1H), 1.89 *(d, J =* 4.8 Hz, 2H), 1.68 - 1.43 (m, 5 H).

### Preparation Example (III) 55: Compound A-1-2-6-23

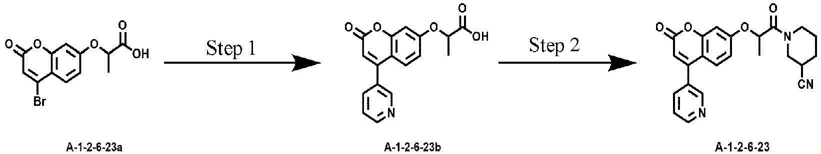

### Step 1

In a dry 100 mL three-necked flask, compound a-1-2-6-23a (600.0 mg, 1.92 mmol) and 3-pyridylboronic acid (283.0 mg, 2.30 mmol) were dissolved in a mixed solution of dioxane (6 mL) and water (2 mL). After purging with nitrogen, [1,1'-bis(diphenylphosphino)ferrocene]dichloropalladium (140.0 mg, 192 µmol) and potassium acetate (376 mg, 3.83 mmol) were added. The mixture was degassed and purged with nitrogen three times again, and then stirred at 90°C for 1 h. The progress of the reaction was monitored by LCMS. After completion of the reaction, the mixture was cooled to 25°C and filtered through a pad of diatomite powder. The filtrate was washed with ethyl acetate (10 mL). Water (30 mL) was added to the filtrate, and the mixture was extracted with ethyl acetate (10 mL). The organic phases were combined, washed with saturated brine (20 mL × 2), dried over anhydrous sodium sulfate, and concentrated to give a crude product. The crude product was directly used in the next step. Compound A-1-2-6-23b as a yellow oil (722.0 mg, 993 µmol, yield: 51.8%, purity: 42.8%) was obtained.
MS-ESI: [M+H]⁺ = 311.8

### Step 2

In a dry 100 mL three-necked flask, compound A-1-2-6-23b (722.0 mg, 2.32 mmol), 3-pip eridinecarbonitrile (306.0 mg, 2.78 mmol), 2-(7-azabenzotriazole)-*N*,*N*,*N'*,*N'*-tetramethyluroniu m hexafluorophosphate (1.32 g, 3.48 mmol) and *N*,*N*-diisopropylethyl amine (600.0 mg, 4. 64 mmol) were dissolved in *N*,*N*-dimethylformamide (8 mL). The mixture was stirred at 2 0°C for 1 h. The progress of the reaction was monitored by LCMS. After completion of t he reaction, the reaction mixture was poured into water (50 mL) and extracted with ethyl acetate (20 mL × 3). The organic phases were combined, washed with saturated brine (30 mL × 2), dried over anhydrous sodium sulfate, and concentrated to give a crude product. The crude product was purified by preparative HPLC (Column: Phenome ne * C18 250* 50 mm*10 µm; Mobile phase: [Water (ammonium bicarbonate)-acetonitrile; B%: 15%-45%, 8 minutes) to give A-1-2-6-23 as an off-white solid (96.60 mg, 233 µmol, yield: 10.0%, purity: 97.1%).
MS-ESI: [M+H]⁺= 404.0
¹H NMR:(400 MHz, DMSO-*d₆*)=δ 8.81 - 8.67 (m, 2H), 7.98 (d, *J =* 8.0 Hz, 1H), 7.62 - 7.59 (m, 1H), 7.36 - 7.19 (m, 1H), 7.08 - 6.92 (m, 1H), 6.88 (d, *J =* 8.8 Hz, 1H), 6.37 (s, 1H), 5.62 - 5.45 (m, 1H), 4.04 - 3.83 (m, 1H), 3.80 - 3.63 (m, 1H), 3.59 - 3.37 (m, 2H), 3.18 - 3.00 (m, 1H), 2.02 - 1.79 (m, 2H), 1.78 - 1.40 (m, 5H).

### Preparation Example (III) 56: Compound A-1-2-6-24

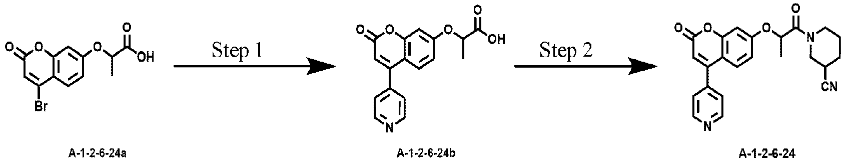

### Step 1

In a dry 100 mL three-necked flask, compound a-1-2-6-24a (400 mg, 1.28 mmol) and 4-pyridylboronic acid (188.0 mg, 1.53 mmol, 1.20 eq) were dissolved in a mixed solution of dioxane (4.00 mL) and water (1.00 mL). After purging with nitrogen, [1,1'-bis(diphenylphosphino)ferrocene]dichloropalladium (93.5 mg, 128 umol) and potassium acetate (251 mg, 2.56 mmol) were added. The mixture was degassed and purged with nitrogen three times again. The mixture was stirred at 90°C for 1 h. The progress of the reaction was monitored by LCMS. After completion of the reaction, the reaction mixture was cooled to 25°C, poured into saturated ammonium chloride solution (20.0 mL) at 0-5°C, and extracted with ethyl acetate (10.0 mL * 3). The organic phases were combined, washed with saturated brine (10.0 mL * 2), dried over anhydrous sodium sulfate, filtered, and concentrated to give a crude product. The crude product was directly used in the next step. Compound A-1-2-6-24b as a yellow oil (250.0 mg, 426 µmol, yield: 33.3%, purity: 53.0%) was obtained.
MS-ESI: [M+H]⁺= 311.9

### Step 2

In a dry 100 mL three-necked flask, compound A-1-2-6-24b (247.0 mg, 793 umol), 3-pipe ridinecarbonitrile (105.0 mg, 952 umol), 2-(7-azabenzotriazole)-N,N,N',N'-tetramethyluronium hexafluorophosphate (453.0 mg, 1.19 mmol) and N,N-diisopropylethyl amine (205.0 mg, 1. 59 mmol) were dissolved in N,N-dimethylformamide (2.50 mL. The mixture was stirred at 20°C for 1 h. The progress of the reaction was monitored by LCMS. After completion o f the reaction, the reaction mixture was poured into ice water (20.0 mL), adjusted to pH = 8 with saturated aqueous sodium bicarbonate solution, and extracted with ethyl acetate ( 10.0 mL * 3). The organic phases were combined, washed with saturated brine (10.0 mL * 2), dried over anhydrous sodium sulfate, and concentrated to give a crude product. The crude product was purified by preparative HPLC (Column: Waters * bridge 150*25 mm 1 0 um; Mobile phase: [Water (ammonium bicarbonate)-acetonitrile; B%: 16%-46%, 8 minute s) to give compound A-1-2-6-24 (31.40 mg, 76.3 umol, yield: 9.61%, purity: 98.0%).
MS-ESI: [M+H]⁺= 404.0.
¹H NMR:(400 MHz, DMSO-d6)δ 8.81 - 8.73 (m, 2H), 7.58 - 7.51 (m, 2H), 7.31 - 7.20 (m, 1H), 7.08 - 6.92 (m, 1H), 6.91 - 6.81 (m, 1H), 6.35 (s, 1H), 5.59 - 5.46 (m, 1H), 4.03 - 3.83 (m, 1H), 3.80 - 3.64 (m, 1H), 3.56 - 3.35 (m, 2H), 3.25 - 3.02 (m, 1H), 1.89 (d, J = 4.8 Hz, 2H), 1.76 - 1.41 (m, 5H).

### Preparation Example (III) 57: Compound A-7-1-1-1

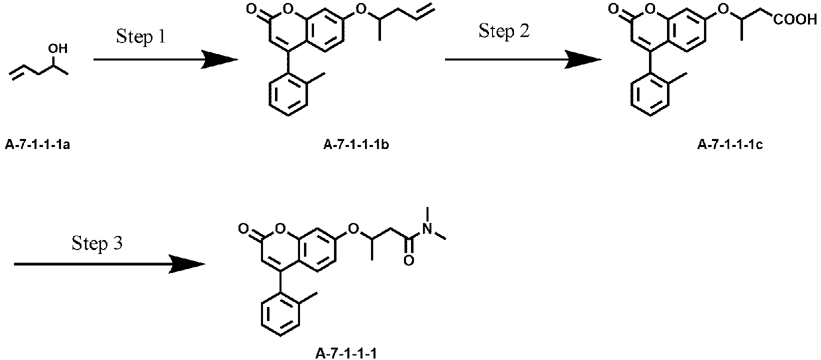

### Step 1

In a dry 100 mL three-necked flask, A-7-1-1-1a (444.0 mg, 5.15 mmol), triphenylphosphine (1.56 g, 5.95 mmol) and A-1-2-1-98c (1.00 g, 3.96 mmol, 1.00 eq) were dissolved in tetrahydrofuran (10 mL). The system was purged with nitrogen. Diisopropyl azodicarboxylate (1.28 g, 6.34 mmol) was added dropwise at 0°C while controlling the reaction temperature not to exceed 5°C. After completion of the dropwise addition, the mixture was stirred at 20°C for 12 h. The progress of the reaction was monitored by LCMS. After completion of the reaction, the reaction mixture was poured into saturated sodium sulfite solution (10 mL), adjusted to pH 9 with saturated aqueous sodium bicarbonate solution, and extracted with dichloromethane (5 mL × 3). The organic phases were combined, washed with saturated brine (5.00 mL × 2), dried over anhydrous sodium sulfate, and concentrated to obtain a crude product. The crude product was purified by column chromatography (silica gel, ethyl acetate:petroleum ether = 1:20 to 1:10) to give the product (petroleum ether:ethyl acetate = 10:1, Rf = 0.60) A-7-1-1-1b as a yellow compound (1.10 g, 3.43 mmol, yield: 86.6%, purity: 100%).
MS-ESI: [M+H]⁺= 321.1
¹H NMR:(400 MHz, DMSO-*d₆*)δ 7.46 - 7.31 (m, 3H), 7.24 (d, *J =* 7.6 Hz, 1H), 7.09 (s, 1H), 6.85 (d, *J* = 1.2 Hz, 2H), 6.16 (s, 1H), 5.84 - 5.79 (m, 1H), 5.19 - 5.02 (m, 2H), 4.72 - 4.67 (m, 1H), 2.48 - 2.31 (m, 2H), 2.11 (s, 3H) 1.27 (d, *J =* 6.0 Hz, 3H).

### Step 2

In a dry 100 mL three-necked flask, sodium periodate (3.30 g, 15.5 mmol) and ruthenium trichloride monohydrate (15.5.0 mg, 68.7 µmol) were dissolved in a mixed solvent of dichloromethane (2 mL), acetonitrile (2 mL) and water (6 mL) and cooled to 0°C. A-7-1-1-1b (1.10 g, 3.43 mmol) dissolved in dichloromethane (2 mL) and acetonitrile (2 mL) was added and stirred at -5-0°C for 3 h. The progress of the reaction was monitored by LCMS. After completion of the reaction, the reaction mixture was poured into saturated sodium sulfite solution (30 mL), adjusted to pH = 9 with saturated aqueous sodium bicarbonate solution, and extracted with dichloromethane (10 mL × 3). The organic phases were combined, washed with saturated brine (30 mL × 2), dried over anhydrous sodium sulfate, and concentrated to give a crude product. The crude product was purified by Pre-HPLC (Column: Phenome ne * Luna C18 200*40 mm*10 µm; Mobile phase: [Water (hydrochloric acid)-acetonitrile]; B%: 40%-70%, 10 minutes) to give compound A-7-1-1-1c (70.0 mg, 203 µmol, yield: 5.92%, purity: 98.2%).
MS-ESI: [M+H]⁺ = 339.0

### Step 3

In a dry 100 mL three-necked flask, compound A-7-1-1-1c (70.0 mg, 207 µmol), dimethylamine (10.30 mg, 228 µmol), 2-(7-azabenzotriazole)-*N,N,N',N'*-tetramethyluronium hexafluorophosphate (118.0 mg, 310 µmol) and N,N-diisopropylethyl amine (80.2 mg, 621 µmol) were dissolved in N,N-dimethylformamide (1 mL) and stirred at 20 °C for 1 h. The progress of the reaction was monitored by LCMS. After completion of the reaction, the reaction mixture was poured into water (20 mL) and extracted with ethyl acetate (10 mL × 3). The organic phases were combined, washed with saturated brine (10 mL × 2), dried over anhydrous sodium sulfate, and concentrated to give a crude product. The crude product was purified by preparative HPLC (Column: Phenome ne * Luna C18 150*2 5 mm*10 µm; Mobile phase: [Water (hydrochloric acid)-acetonitrile]; B%: 43%-72%, 10 minutes) to give A-7-1-1-1 (40.0 mg, 98.8 µmol, yield: 47.7%, purity: 95.8%) as a yellow solid.
MS-ESI: [M+H]⁺ = 366.1
¹H NMR(400 MHz, DMSO-*d₆*)δ 7.48 - 7.30 (m, 3H), 7.23 (d, *J =* 7.6 Hz, 1H), 7.07 - 6.98 (m, 1H), 6.90 - 6.77 (m, 2H), 6.17 (s, 1H), 5.06 - 4.93 (m, 1H), 2.98 (s, 3H), 2.85 - 2.82 (m, 1H), 2.80 (s, 3H), 2.63 - 2.53 (m, 1H), 2.12 (s, 3H), 1.32 (d, *J =* 6.0 Hz, 3H).

### Preparation Example (III) 58: Compound A-7-1-1-2

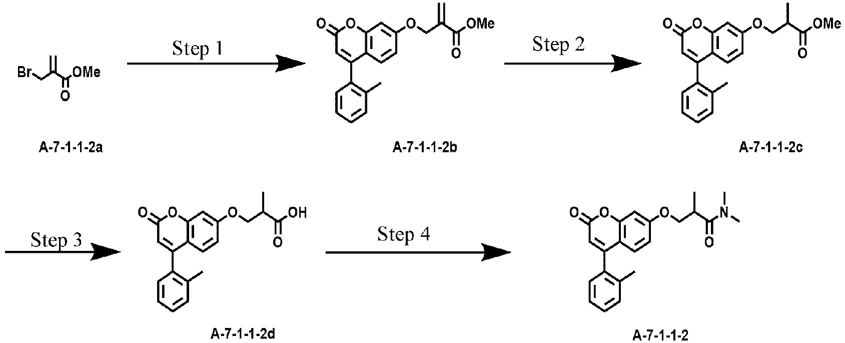

### Step 1

In a dry 100 mL three-necked flask, A-1-2-1-98c (1.00 g, 3.96 mmol), cesium carbonate (2.58 g, 7.93 mmol) and compound A-7-1-1-2a (851.0 mg, 4.76 mmol) were dissolved in *N,N*-dimethylformamide (10 mL). The mixture was stirred at 20°C for 2 h. The progress of the reaction was monitored by LCMS. After completion of the reaction, the reaction mixture was poured into water (50 mL) and extracted with ethyl acetate (10 mL × 3). The organic phases were combined, washed with saturated brine (10 mL × 2), dried over anhydrous sodium sulfate, and concentrated to give a crude product. The crude product was directly used in the next step. Compound A-7-1-1-2b as a yellow oil (1.35 g, 3.68 mmol, yield: 93.0%, purity: 95.6%) was obtained.
MS-ESI: [M+H]⁺= 351.1
¹H NMR(400 MHz, DMSO-*d*₆)δ 7.46 - 7.31 (m, 3H), 7.24 (d, *J* = 7.6 Hz, 1H), 7.15 (d, *J =* 2.0 Hz, 1H), 6.97 - 6.84 (m, 2H), 6.34 (s, 1H), 6.20 (s, 1H), 6.06 (d, *J =* 0.8 Hz, 1H), 4.87 (s, 2H), 3.74 (s, 3H), 2.11 (s, 3H).

### Step 2

In a dry 100 mL three-necked flask, compound A-7-1-1-2b (1.05 g, 3.00 mmol) was dissolved in ethyl acetate (5 mL). The system was purged with nitrogen. A solution of wet palladium on carbon (0.16 g, 30.0 µmol, purity: 10.0%) in ethyl acetate was added. The mixture was degassed under vacuum, purged with nitrogen several times, charged with hydrogen gas (15.0 Psi), and stirred at 20°C for 12 h. The progress of the reaction was monitored by LCMS. After completion of the reaction, the mixture was filtered through diatomite, and the filtrate was concentrated to give the product. The crude product was directly used in the next step. Compound A-7-1-1-2c as a yellow oil (0.85 g, 1.21 mmol, yield: 40.2%, purity: 50.0%) was obtained.
MS-ESI: [M+H]⁺ = 353.1

### Step 3

In a dry 100 mL three-necked flask, compound A-7-1-1-2c (0.85 g, 2.41 mmol) was dissolved in tetrahydrofuran (10 mL), and concentrated hydrochloric acid (10 mL) (37% purity) was added. The mixture was stirred at 20°C for 1 h. The progress of the reaction was monitored by LCMS. After completion of the reaction, the reaction mixture was extracted with ethyl acetate (10 mL × 3). The organic phases were combined, washed with saturated brine (10 mL × 2), dried over anhydrous sodium sulfate, and concentrated to give a crude product. The crude product was purified by preparative HPLC (Column: Phenome ne * Luna C18 250*50 mm*10 µm; Mobile phase: [Water (hydrochloric acid)-acetonitrile]; B%: %-67%, 20 minutes) to give compound A-7-1-1-2d (300.0 mg, 887 µmol, yield: 36.8%, purity: 100%) as a yellow oil.
MS-ESI: [M+H]⁺= 339.0

### Step 4

In a dry 100 mL three-necked flask, compound A-7-1-1-2d (287.0 mg, 848 µmol, 1.00 eq), dimethylamine (42.10 mg, 933 µmol, 47.3 µL), 2-(7-azabenzotriazole)*-N,N,N',N'*-tetramethyl uronium hexafluorophosphate (484.0 mg, 1.27 mmol) and *N,N*-diisopropylethyl amine (329. 0 mg, 2.54 mmol) were dissolved in *N,N*-dimethylformamide (1 mL). The mixture was sti rred at 20°C for 2 h. The progress of the reaction was monitored by LCMS. After compl etion of the reaction, the reaction mixture was poured into water (10 mL) and extracted with ethyl acetate (5.00 mL × 3). The organic phases were combined, washed with saturat ed brine (10 mL × 2), dried over anhydrous sodium sulfate, and concentrated to give a cr ude product. The crude product was purified by Pre-HPLC (Column: Phenome ne * Luna C18 200*40 mm*10 µm; Mobile phase: [Water (hydrochloric acid)-acetonitrile]; B%: 40% -70%, 10 minutes) to give A-7-1-1-2 (207.0 mg, 543 µmol, yield: 64.0%, purity: 95.8%) a s a yellow solid.
MS-ESI: [M+H]⁺ = 366.1
¹H NMR(400 MHz, DMSO-d*₆*)δ 7.46 - 7.31 (m, 3H), 7.24 (d, *J=* 7.2 Hz, 1H), 7.08 (d, *J =* 1.6 Hz, 1H), 6.86 (t, *J =* 1.2 Hz, 2H), 6.18 (s, 1H), 4.26 - 4.22 (m, 1H), 3.99 - 3.96 (m, 1H), 3.68 - 3.55 (m, 1H), 3.05 (s, 3H), 2.83 (s, 3H), 2.10 (s, 3H), 1.08 (d, *J =* 6.8 Hz, 3H).

### Preparation Example (III) 59: Compound A-1-2-1-42

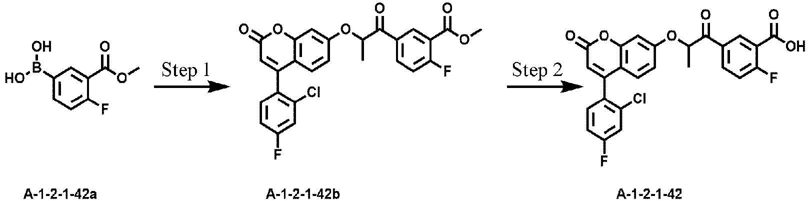

### Step 1

In a dry 50 mL three-necked flask, A-1-2-1-33c (1.00 g, 2.76 mmol) and A-1-2-1-42a (1.09 g, 2.76 mmol) were added. After purging with nitrogen, tetrakis(triphenylphosphine)palladium (79.7 mg, 0.05 mmol) was added and dissolved in 5 mL of dioxane. Finally, dimethyl dicarbonate (462.6 mg, 3.45 mmol) was added. The reaction was carried out at 115 °C for 2 h (preferably 2-4 h). After LCMS analysis showed that the reaction was complete, the mixture was cooled to room temperature and filtered. The filtrate was collected, and 10 mL of water was added. Then, the mixture was extracted with ethyl acetate (10 mL × 3), washed with 20 mL of saturated brine, dried over anhydrous sodium sulfate, and purified by silica gel column chromatography (PE:EA = 5%-30 %) to give the intermediate A-1-2-1-42b (930.0 mg, 1.86 mmol, yield: 67.62 %).
MS-ESI: [M+H]⁺ = 499.1

### Step 2

In a dry 50 mL three-necked flask, A-1-2-1-42a (800.0 mg, 1.60 mmol) was added and dissolved in 5 mL of tetrahydrofuran, and the system was cooled at 0°C, Then, 5 mL of 2 M aqueous sodium hydroxide solution was added. The reaction was carried out for 30 min (preferably 0.5-2 h). After TLC showed that the reaction was complete, the mixture was adjusted to pH = 1 with 1 M aqueous hydrochloric acid solution, extracted with ethyl acetate (5 mL × 3), washed with saturated brine (5 mL × 2), dried over anhydrous sodium sulfate, rotary evaporated, and purified by silica gel column chromatography (DCM:MeOH = 50:1) to give the desired product A-1-2-1-42 (237.0 mg, 0.489 mmol, yield: 30.48%).
MS-ESI: [M-H]⁻= 483.1
¹H NMR (400 MHz, DMSO-*d*₆) δ 8.52 (dd, *J =* 7.0, 2.4 Hz, 1H), 8.37 - 8.32 (m, 1H), 7.68 (dd, *J* = 8.7, 2.1 Hz, 1H), 7.59 - 7.48 (m, 2H), 7.42 (dq, *J =* 8.4, 5.5, 4.4 Hz, 1H), 7.09 (dd, *J =* 4.0, 2.4 Hz, 1H), 6.96 - 6.86 (m, 2H), 6.31 (s, 1H), 6.26 (qd, *J =* 6.7, 4.0 Hz, 1H), 1.58 (d, *J =* 6.7 Hz, 3H).

### Preparation Example (III) 60: Compound A-1-10-1-20

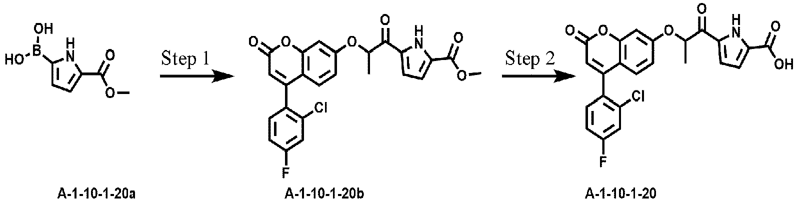

### Step 1

In a dry 50 mL three-necked flask, A-1-2-1-98c (500.0 mg, 1.38 mmol) and A-1-10-1-20a (530.0 mg, 2.48 mmol) were added. After purging with nitrogen, tetrakis(triphenylphosphine)palladium (79.70 mg, 0.07 mmol) was added and dissolved in 5 mL of dioxane solution. Finally, dimethyl dicarbonate (462.6 mg, 3.45 mmol) was added. The reaction was carried out at 115 °C for 2 h (preferably 2-4 h). After LCMS analysis showed that the reaction was complete, the mixture was cooled to room temperature and filtered. The filtrate was collected, and 10 mL of water was added. Then, the mixture was extracted with ethyl acetate (10 mL × 3), washed with 20 mL of saturated brine, dried over anhydrous sodium sulfate, and purified by silica gel column chromatography (PE:EA = 3:1) to give the intermediate A-1-10-1-20b (590.0 mg, 1.26 mmol, yield: 91.10 %).
MS-ESI: [M+H]⁺ = 470.0

### Step 2

In a dry 50 mL three-necked flask, A-1-10-1-20b (400.0 mg, 0.85 mmol) was added and dissolved in 5 mL of tetrahydrofuran, and the system was cooled at 0°C. Then, 2 mL of 2 M aqueous sodium hydroxide solution was added. The reaction was carried out for 30 min (preferably 0.5-2 h). After TLC showed that the reaction was complete, the mixture was adjusted to pH = 1 with 1 M aqueous hydrochloric acid solution, extracted with ethyl acetate (5 mL × 3), washed with saturated brine (5 mL × 2), dried over anhydrous sodium sulfate, rotary evaporated, purified by silica gel column chromatography (DCM:MeOH = 50:1), and then re-separated by a preparative plate (DCM:MeOH = 10%) to give the desired product A-1-10-1-20 (102.0 mg, 0.178 mmol, yield: 22.0%).
MS-ESI: [M-H]⁻= 454.1

### Preparation Example (III) 61: Compound A-1-2-1-45

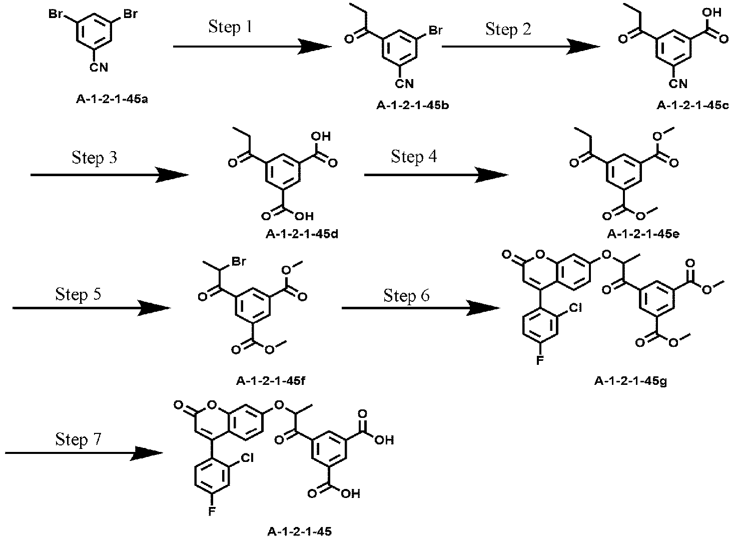

### Step 1:

In a dry 500 mL three-necked flask, A-1-2-1-45a (20.00 g, 76.65 mmol) was added, and the system was purged with nitrogen. After that, anhydrous tetrahydrofuran (200 mL) was added for dissolution, and the system was cooled to 0°C. Isopropylmagnesium chloride (2M, 42.16 mL, 84.32 mmol) was slowly added dropwise while controlling the reaction temperature not to exceed 5°C. After completion of the dropwise addition, stirring was continued at 0°C for 1 h, and N-methoxy-N-methylpropanamide (9.88 mL, 84.32 mmol) diluted with anhydrous tetrahydrofuran (30 mL) was added dropwise. After completion of the dropwise addition, the mixture was transferred to 25°C, and the reaction was allowed to proceed for 2 h. After LCMS showed that the reaction was complete, the system was cooled to 0°C, and the reaction was slowly quenched with saturated NH4Cl solution while controlling the quenching temperature not to exceed 5°C. After quenching, the mixture was extracted with ethyl acetate (100 mL × 3), and the layers were separated. The organic phases were combined, washed with saturated sodium chloride solution (300 mL), dried over anhydrous sodium sulfate, and purified by silica gel column chromatography (PE:EA = 5:1) to give the intermediate A-1-2-1-45b (18.70 g, 70.69 mmol, yield: 85.0%).
MS-ESI: [M+H]⁺ = 237.9
¹H NMR (400 MHz, Chloroform-d) δ 8.29 (t, *J =* 1.7 Hz, 1 H), 8.15 (d, *J =* 1.7 Hz, 1 H), 7.96 (t, *J* = 1.7 Hz, 1 H), 3.10 - 2.89 (m, 2 H), 1.24 (td, *J =* 7.2, 1.6 Hz, 3 H).

### Step 2

In a dry 500 mL three-necked flask, palladium acetate (0.48 g, 2.12 mmol) and 4,5-bis(diphenylphosphino)-9,9-dimethylxanthene (1.23 g, 2.12 mmol) were added, and the system was purged with nitrogen. Then, A-1-2-1-45b (18.70 g, 70.69 mmol) and dicyclohexyl carbodiimide (2.92 g, 14.14 mmol) were added to the flask and dissolved in N,N-dimethylformamide (200 mL). The system was thoroughly purged with nitrogen again. After that, formic acid (18.67 mL, 494.83 mmol) and triethylamine (19.65 mL, 141.38 mmol) were successively and slowly added to the flask, and the temperature was raised to 80°C (preferably 80-100°C). The mixture was stirred for 4 h (preferably 4-8 h). After LCMS showed that the reaction was complete, the system was cooled to room temperature, and water (200 mL) was added to quench the reaction. The reaction solution was filtered through diatomite to remove insolubles, and then extracted with methyl tert-butyl ether (150 mL × 3). The layers were separated. The organic phases were combined, washed with saturated sodium chloride solution (500 mL), dried over anhydrous sodium sulfate, and purified by silica gel column chromatography (DCM:MeOH = 15:1) to give the intermediate A-1-2-1-45c (9.50 g, 46.75 mmol, yield: 59.5%).
MS-ESI: [M-H]⁻= 202.1
¹H NMR (400 MHz, DMSO-*d*₆) δ 8.63 (d, *J* = 5.0 Hz, 2 H), 8.51 (s, 1 H), 3.15 (q, *J =* 7.0 Hz, 2 H), 1.10 (t, *J* = 7.1 Hz, 3 H).

### Step 3

In a dry 100 mL three-necked flask, A-1-2-1-45c (9.50 g, 46.75 mmol) was added, and the system was purged with nitrogen. Then, 20 mL of 3.3 M potassium hydroxide solution and 30 mL of methanol were added for dissolution, and the temperature was raised to 80°C (preferably 60-80°C). The mixture was stirred for 2 h (preferably 2-4 h). After LCMS showed that the reaction was complete, the mixture was cooled to room temperature and filtered. The filtrate was collected, adjusted to pH 1 with 2 M hydrochloric acid solution, and extracted with ethyl acetate (50 mL × 3). The layers were separated. The organic phases were combined, washed with saturated sodium chloride solution (100 mL), dried over anhydrous sodium sulfate, and purified by silica gel column chromatography (DCM:MeOH = 15:1, 1:1000 AcOH) to give the intermediate A-1-2-1-45d (4.30 g, 19.35 mmol, yield: 48.47%).
MS-ESI: [M-H]⁻= 221.1
¹H NMR (400 MHz, DMSO-*d*₆) δ 8.75 - 8.61 (m, 3 H), 5.01 (t, *J =* 6.5 Hz, 2 H), 1.32 (t, *J* = 6.2 Hz, 3 H).

### Step 4

In a dry 100 mL three-necked flask, A-1-2-1-45d (4.30 g, 19.35 mmol) was added, and the system was purged with nitrogen. Then, N,N-dimethylformamide (40 mL) was added for dissolution, and the system was cooled to 0°C. Potassium carbonate (10.70 g, 77.4 mmol) was added in batches, and then stirring was continued at 0°C for 1 h. Iodomethane (4.82 mL, 77.4 mmol) was added dropwise. After completion of the dropwise addition, stirring was continued for 15 min, and then the temperature was raised to 25°C. The reaction is carried out for 2 h (preferably 2-4 h). After TLC showed that the reaction was complete, water (50 mL) was added to quench the reaction. The reaction solution was extracted with ethyl acetate (30 mL × 3), washed with saturated brine (100 mL), dried over anhydrous sodium sulfate, and purified by silica gel column chromatography (PE:EA = 2:1) to give the intermediate A-1-2-1-45e (1.20 g, 4.80 mmol, yield: 25.0%).

¹H NMR (400 MHz, DMSO-*d₆*) δ 8.63 (dd, *J* = 7.6, 1.7 Hz, 3 H), 3.93 (s, 6 H), 3.17 (q, *J=* 7.0 Hz, 2 H), 1.11 (t, *J =* 7.0 Hz, 3 H).

### Step 5

In a dry 50 mL three-necked flask, A-1-2-1-45e (1.20 g, 4.80 mmol) was added, and the system was purged with nitrogen. After that, anhydrous tetrahydrofuran (10 mL) was added for dissolution, and the system was cooled to 0°C. Pyridinium tribromide (1.84 g, 5.76 mmol) was added in batches, and then the temperature was raised to 25°C. The reaction was carried out for 6 h (preferably 6-8 h). After LCMS showed that the reaction was complete, the reaction solution was filtered. The filtrate was collected, diluted with water (20 mL), and extracted with ethyl acetate (20 mL × 3). The layers were separated. The organic phases were combined, washed successively with saturated sodium bicarbonate solution (50 mL) and saturated sodium chloride solution (50 mL), dried over anhydrous sodium sulfate, and purified by silica gel column chromatography (PE:EA = 2:1) to give the intermediate A-1-2-1-45f (1.20 g, 3.65 mmol, yield: 76.03%).

### Step 6

In a dry 50 mL three-necked flask, A-1-2-1-45f (1.20 g, 3.65 mmol), A1-1-1c (1.06 g, 3.65 mmol), potassium carbonate (1.01 g, 7.30 mmol) and tetrabutylammonium iodide (1.35 g, 3.65 mmol) were successively added and dissolved in acetone (10 mL). The mixture was stirred at room temperature overnight. After LCMS showed that the reaction was complete, the mixture was diluted with water (20 mL) and extracted with ethyl acetate (20 mL × 3). The layers were separated. The organic phases were combined, washed with saturated sodium chloride solution (50 mL), dried over anhydrous sodium sulfate, and purified by silica gel column chromatography (PE:EA = 2:1) to give the intermediate A-1-2-1-45g (0.98 g, 1.82 mmol, yield: 49.88%).
MS-ESI: [M+H]⁺ = 539.0

### Step 7

In a dry 50 mL three-necked flask, A-1-2-1-45g (0.20 g, 0.37 mmol) was added and dissolved in 4 mL of tetrahydrofuran, and the system was cooled to 0°C. Then, 2 M sodium hydroxide solution (2 mL) was added dropwise and stirred for 1 h (preferably 1-2 h). After LCMS showed that the reaction was complete, the mixture was diluted with water (10 mL), and 2 M hydrochloric acid solution was added at 0°C to quench the reaction and adjust the pH to 1. The mixture was extracted with ethyl acetate (5 mL × 3), and the layers were separated. The organic phases were combined, washed with saturated sodium chloride solution (10 mL), dried over anhydrous sodium sulfate, and purified by silica gel column chromatography (DCM:MeOH = 15:1, 1:1000 AcOH) to give the final product A-1-2-1-45 (42.60 mg, 0.08 mmol, yield: 21.1%).
MS-ESI: [M-H]⁻= 509.0
¹H NMR (400 MHz, DMSO-*d*₆) δ 8.67 (d, *J* = 1.7 Hz, 2H), 8.63 (t, *J* = 1.6 Hz, 1H), 7.64 - 7.61 (m, 1H), 7.51 - 7.47 (m, 1H), 7.39 - 7.33 (m, 1H), 7.10 - 7.09 (m, 1H), 6.89 - 6.82 (m, 2H), 6.33 - 6.26 (m, 1H), 6.25 (s, 1H), 1.54 (d, *J =* 6.8 Hz, 3H).

### Preparation Example (III) 62: Compound A-1-2-1-105

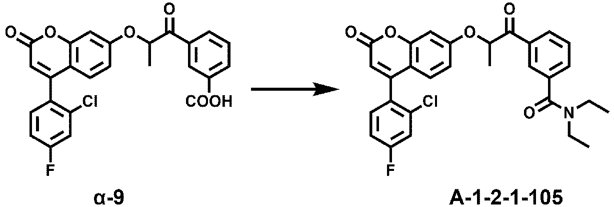

### Step 1

In a dry 50 mL three-necked flask, compound α-9 (500.0 mg, 1.07 mmol) was dissolved in N,N-dimethylformamide (5 mL). Then, 1-(3-dimethylaminopropyl)-3-ethylcarbodiimide hydrochloride (308.0 mg, 1.60 mmol) and 1-hydroxybenzotriazole (217.0 mg, 1.60 mmol) were added and stirred at room temperature for 1 h. Then, triethylamine (325.0 mg, 3.21 mmol) was added dropwise. The reaction was carried out at room temperature for 2 h, and the progress of the reaction was monitored by TLC. After TLC showed that the reaction was complete, water (10 mL) was added to quench the reaction. Then, ethyl acetate (10 mL) was added for extraction, and the layers were separated. The aqueous phase was further extracted with ethyl acetate (10 mL × 2). The combined organic phase was collected, washed with saturated aqueous sodium chloride solution (30 mL), and dried over anhydrous sodium sulfate. The resulting crude product was purified by silica gel column chromatography (PE:EA = 3:1) to give pure compound A-1-2-1-105 (80.0 mg, 0.153 mmol, yield: 14.3%).
MS-ESI: [M+H]⁺ = 522.2
¹H NMR (400 MHz, Chloroform-d) δ 8.12 - 8.03 (m, 2H), 7.64 (dq, *J =* 7.6, 1.5 Hz, 1H), 7.56 (t, *J =* 7.7 Hz, 1H), 7.34 - 7.23 (m, 2H), 7.13 (tt, *J =* 8.3, 2.8 Hz, 1H), 6.95 (dd, *J =* 8.8, 3.9 Hz, 1H), 6.83 - 6.71 (m, 2H), 6.17 (d, *J =* 1.3 Hz, 1H), 5.61 (qd, *J =* 6.9, 2.8 Hz, 1H), 3.60 - 3.52 (m, 2H), 3.26 (s, 2H), 1.76 (d, *J =* 6.8 Hz, 3H), 1.32 - 1.14(m, 6H).

### Preparation Example (III) 63: Compound A-1-2-1-106

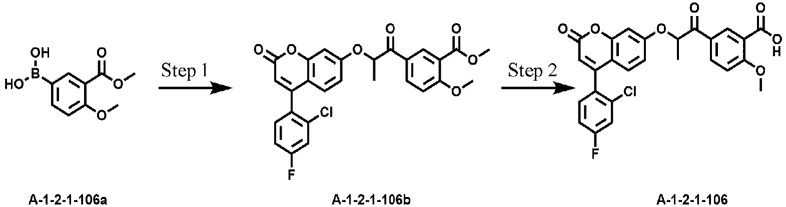

### Step 1

In a dry 50 mL three-necked flask, A-1-2-1-33c (500.0 mg, 1.38 mmol) and A-1-2-1-106a (579.6 mg, 2.76 mmol) were added. After purging with nitrogen, tetrakis(triphenylphosphine)palladium (79.7 mg, 0.05 mmol) was added and dissolved in 5 mL of dioxane solution. Finally, dimethyl dicarbonate (462.6 mg, 3.45 mmol) was added. The reaction was carried out at 115 °C for 2 h (preferably 2-4 h). After LCMS analysis showed that the reaction was complete, the mixture was cooled to room temperature and filtered. The filtrate was collected, and 10 mL of water was added. Then, the mixture was extracted with ethyl acetate (10 mL × 3), washed with 20 mL of saturated brine, dried over anhydrous sodium sulfate, and purified by silica gel column chromatography (PE:EA = 3:1) to give the intermediate A-1-2-1-106b (704.0 mg, 1.26 mmol, yield: 93.02%).
MS-ESI: [M+H]⁺ = 511.0

### Step 2

In a dry 50 mL three-necked flask, A-1-2-1-106b (712.0 mg, 1.39 mmol) was added and dissolved in 5 mL of tetrahydrofuran, and the system was cooled at 0°C. Then, 5 mL of 2 M aqueous sodium hydroxide solution was added. The reaction was carried out for 30 min (preferably 0.5-2 h). After TLC showed that the reaction was complete, the mixture was adjusted to pH = 1 with 1M aqueous hydrochloric acid solution, extracted with ethyl acetate (5 mL × 3), washed with saturated brine (5 mL × 2), dried over anhydrous sodium sulfate, rotary evaporated, purified by silica gel column chromatography (DCM:MeOH = 50:1), and then re-separated by a preparative plate (DCM:MeOH = 10: 1) to give the desired product A-1-2-1-106 (312.0 mg, 0.628 mmol, yield: 45.1%).
MS-ESI: [M-H]⁻ = 495.1
¹H NMR (400 MHz, Chloroform-d) δ 8.86 (t, *J =* 2.4 Hz, 1H), 8.30 (dt, *J =* 9.0, 2.3 Hz, 1H), 7.31 - 7.23 (m, 2H), 7.19 (d, *J =* 8.9 Hz, 1H), 7.12 (tt, *J =* 8.3, 2.4 Hz, 1H), 6.94 (dd, *J =* 8.8, 2.9 Hz, 1H), 6.84 - 6.70 (m, 2H), 6.17 (s, 1H), 5.60 (q, *J =* 6.8 Hz, 1H), 4.16 (s, 3H), 1.77 (d, *J =* 6.9 Hz, 3H).

### Preparation Example (III) 64: Compound A-1-2-1-49

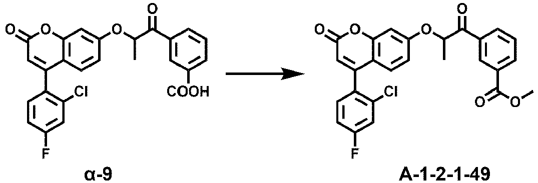

In a dry 50 mL three-necked flask, compound α-9 (500.0 mg, 1.07 mmol) and potassium carbonate (295.770 mg, 2.14 mmol) were dissolved in N,N-dimethylformamide (5 mL), and the suspension was stirred. Then, iodomethane (227.81.0 mg, 1.60 mmol) was added dropwise. The reaction was carried out at room temperature for 2 h, and the progress of the reaction was monitored by TLC. After TLC showed that the reaction was complete, water (10 mL) was added to quench the reaction. Then, ethyl acetate (10 mL) was added for extraction, and the layers were separated. The aqueous phase was further extracted with ethyl acetate (10 mL × 2). The combined organic phase was collected, washed with saturated aqueous sodium chloride solution (30 mL), and dried over anhydrous sodium sulfate. The resulting crude product was purified by silica gel column chromatography to give pure compound A-1-2-1-49 (305.0 mg, 0.634 mmol, yield: 58.3%).
MS-ESI: [M+H]⁺= 481.0
¹H NMR (400 MHz, DMSO-*d*₆) δ 8.54 (t, *J =* 1.8 Hz, 1H), 8.36 (dq, *J =* 7.9, 1.5 Hz, 1H), 8.23 (dt, *J =* 7.8, 1.4 Hz, 1H), 7.74 (t, *J =* 7.8 Hz, 1H), 7.65 (dt, *J =* 8.8, 2.5 Hz, 1H), 7.53 (ddd, *J =* 8.3, 6.1, 1.9 Hz, 1H), 7.44 - 7.36 (m, 1H), 7.09 (dd, *J =* 5.7, 2.3 Hz, 1H), 6.96 - 6.85 (m, 2H), 6.29 (t, *J*= 2.0 Hz, 2H), 3.88 (s, 3H), 1.59 (d, *J =* 6.6, 3H).

### Preparation Example (III) 65: Compound A-1-7-1-2

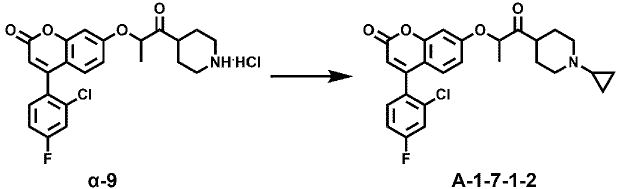

In a dry 50 mL three-necked flask, compound α-9 (250.0 mg, 0.54 mmol) was dissolved in dichloroethane (5 mL). Then, cyclopropylboronic acid (93.0 mg, 1.08 mmol), bipyridine (169.0 mg, 1.08 mmol), copper acetate (196.0 mg, 1.08 mmol) and sodium carbonate (172.0 mg, 1.62 mmol) were successively added. The reaction was carried out at 80°C for 4 h, and the progress of the reaction was monitored by TLC. After TLC showed that the reaction was complete, water (10 mL) was added to quench the reaction. Then, ethyl acetate (10 mL) was added for extraction, and the layers were separated. The aqueous phase was further extracted with ethyl acetate (10 mL × 2). The combined organic phase was collected, washed with saturated aqueous sodium chloride solution (20 mL), and dried over anhydrous sodium sulfate. The resulting crude product was purified by silica gel column chromatography to give pure compound A-1-7-1-2 (45.3 mg, 0.096 mmol, yield: 18.0%).
MS-ESI: [M+H]⁺ = 470.2

### Preparation Example (III) 66: Compound A-1-2-7-3

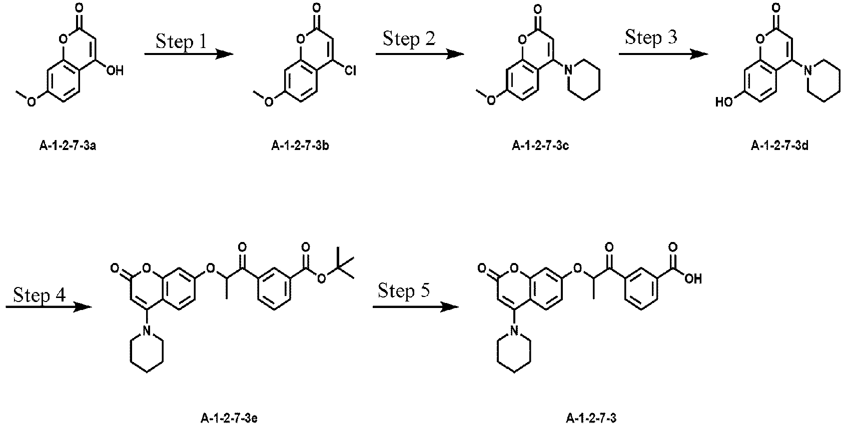

### Step 1

In a dry 50 mL three-necked flask, compound A-1-2-7-3a (1.00 g, 5.20 mmol) was dissolved in phosphorus oxychloride (16.80 g, 109.57 mmol). Then, triethylamine (0.79 g, 7.80 mmol) was slowly added dropwise to the reaction solution. After completion of the dropwise addition, the reaction was carried out at 110°C for 2 h to give a yellow and clear reaction solution. After completion of the reaction, the reaction solution was slowly poured into an ice-water mixture and continuously stirred to completely quench the reaction. Then, the mixture was extracted with dichloromethane, dried, rotary evaporated, and purified to give the desired product A-1-2-7-3b (0.50 g, 45.62%) as a white solid, which was directly used in the next step.

### Step 2

In a dry 50 mL three-necked flask, compound A-1-2-7-3b (0.40 g, 1.90 mmol) and hexahydropyridine (0.24 g, 2.85 mmol) were dissolved in N,N-dimethylformamide (10 mL). Then, triethylamine (0.58 g, 5.70 mmol) was added. The reaction was carried out at room temperature for 16 h to give a yellow suspension. The TLC spot plate showed that the starting materials had completely reacted and a new spot had appeared. LCMS showed that the main peak was the desired peak. The reaction was then subjected to a work-up. The reaction solution was poured into ethyl acetate (50 mL) and then washed and extracted with saturated brine (40 mL × 3). The organic phases were combined, collected, dried, concentrated, and then subjected to column purification (PE:EA = 3:1) to give the desired product A-1-2-7-3c (0.40 g, purity: 93%) as a pale yellow solid.
MS-ESI: [M+H]⁺ = 260.2

### Step 3

In a dry 50 mL three-necked flask, compound A-1-2-7-3c (0.40 g, 1.54 mmol) was dissolved in toluene (15 mL). Then, aluminum trichloride (0.3 g, 1.85 mmol) was added to the reaction solution. After the reaction was purged with nitrogen for protection, the mixture was refluxed at 115°C for 3 h to give a black reaction solution. The TLC spot plate showed that the starting materials had completely reacted and a new spot had appeared. The reaction solution was cooled to room temperature, and methanol was added and stirred for 10 min. Then, the reaction solution was directly rotary evaporated, mixed with an adsorbent, and subjected to column purification (PE:EA = 2:1) to give the desired product A-1-2-7-3d (0.30 g, purity: 96.9%) as a gray solid.
MS-ESI: [M+H]⁺ = 246.2

### Step 4

In a dry 50 mL three-necked flask, compound A-1-2-7-3d (0.30 g, 1.22 mmol), A-1-2-1-1d (0.46 g, 1.46 mmol) and potassium carbonate (0.42 g, 3.05 mmol) were dissolved in N,N-dimethylformamide (10 mL). Then, the reaction was carried out at room temperature for 16 h to give a brown turbid solution. TLC showed that a new spot had appeared and the starting materials had completely reacted. The reaction was then subjected to a work-up. The reaction solution was poured into ethyl acetate (50 mL) and then washed with saturated brine three times. The organic phase was collected, dried, concentrated, and then subjected to column purification (PE:EA = 3:1) to give the desired product A-1-2-7-3e as a yellow solid (80.0 mg, yield: 13.70%).

### Step 5

In a 50 mL eggplant-shaped flask, A-1-2-7-3e (0.10 g, 0.21 mmol) was added and dissolved in dichloromethane (20 mL). Then, trifluoroacetic acid (2 mL) was added. The reaction was carried out at room temperature for 1 h. TLC monitoring showed that the reaction was complete. The reaction was then subjected to a work-up. The reaction solution was adjusted to pH 5-6 with aqueous sodium bicarbonate solution and then washed with 50 mL of saturated brine and 100 mL of ethyl acetate. The organic phase was collected, dried, concentrated, and subjected to column purification (DCM:MeOH = 25:1) followed by (PE:EA = 2:1) to give the desired product A-1-2-7-3 (20.9 mg, purity: 89.62%) as a white solid.
MS-ESI: [M+H]⁺ = 422.3

### Preparation Example (III) 67: Compound A-1-2-3-2

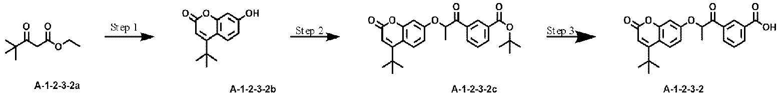

### Step 1

In a dry 50 mL three-necked flask, compound A-1-2-3-2a (2.00 g, 11.61 mmol) and resorcinol (1.41 g, 12.77 mmol) were weighed into the reaction flask and then dissolved in methanesulfonic acid (20 mL). The reaction was carried out at 50°C for 3 h to give a black reaction solution. The TLC spot plate showed that the starting materials had completely reacted. The reaction was then subjected to a work-up. The reaction solution was returned to room temperature, and 30 mL of ethanol was added. Solid sodium bicarbonate was added to adjust the pH to 6-7. Then, 100 mL of water was added. The mixture was extracted with ethyl acetate (100 mL × 2). The organic phases were combined, dried, concentrated to give a crude product, and subjected to column purification (PE:EA = 2:1) to give the desired product A-1-2-3-2b (1.00 g, yield: 39.45%, purity: 100%) as a pale yellow solid.

¹H NMR (400 MHz, Chloroform-d) δ: 8.06 (d, *J* = 8.8 Hz, 1H), 7.00 (dd, *J* = 8.8, 2.3 Hz, 1H), 6.96 (d, *J =* 2.2 Hz, 1H), 6.26 (s, 1H), 1.34 (s, 9H).

### Step 2

In a dry 50 mL three-necked flask, compounds A-1-2-3-2b (1.00 g, 4.58 mmol), A-1-2-1-1d (1.72 g, 5.50 mmol) and potassium carbonate (1.58 g, 11.45 mmol) were dissolved in acetone (30 mL). Then, the reaction was carried out at room temperature to give a yellow turbid solution. TLC showed that a new spot had appeared and the starting materials had completely reacted. The reaction was then subjected to a work-up. The reaction solution was poured into ethyl acetate (50 mL) and then washed with saturated brine three times. The organic phase was collected, dried, concentrated, and then subjected to column purification (PE:EA = 3:1) to give the desired product A-1-2-3-2c (1.80 g, yield: 87.20%) as a yellow solid.

¹H NMR (400 MHz, Chloroform-d) δ: 8.68 (s, 1H), 8.24 (d, *J =* 7.6 Hz, 2H), 8.08 (d, *J* = 8.8 Hz, 1H), 7.58 (t, *J =* 7.6 Hz, 1H), 6.96 (dd, *J =* 8.8, 2.4 Hz, 1H), 6.84 (t, *J =* 5.2 Hz, 1H), 6.22 (d, *J* = 11.2 Hz, 1H), 5.62 (q, *J* = 6.8 Hz, 1H), 1.80 (t, *J =* 9.2 Hz, 3H), 1.64 - 1.55 (m, 9H), 1.35 - 1.27 (m, 9H).

### Step 3

In a 50 mL eggplant-shaped flask, A-1-2-3-2c (1.8 g, 4.0 mmol) was added and dissolved in dichloromethane (20 mL). Then, trifluoroacetic acid (2 mL) was added. The reaction was carried out at room temperature for 1 h. TLC monitoring showed that the reaction was complete. The reaction was then subjected to a work-up. The reaction solution was adjusted to pH ~ 5-6 with aqueous sodium bicarbonate solution and then washed with ethyl acetate (100 mL) and saturated brine (60 mL). The organic phase was collected, dried, and subjected to column purification (DCM:MeOH 0%-4%) to give the desired product. The product was added to 2 mL of acetonitrile and 20 mL of water and then freeze-dried to give the desired product A-1-2-3-2 (409.8 mg, purity: 98.1%) as a white powder.
MS-ESI: [M+H]⁺= 395.3
¹H NMR (400 MHz, DMSO-*d₆*) δ 8.55 (s, 1H), 8.29 (dd, *J =* 3.6 7.8 Hz, 2H), 7.90 (d, *J =* 8.8 Hz, 1H), 7.74 (t, *J =* 7.8 Hz, 1H), 7.16 (t, *J =* 6.8 Hz, 1H), 7.05 (dt, *J =* 17.2, 8.8 Hz, 1H), 6.33 (q, *J =* 6.8 Hz, 1H), 6.13 (s, 1H), 1.61 (d, *J =* 6.8 Hz, 3H), 1.35 - 1.22 (m, 9H).

### Preparation Example (III) 68: Compound A-1-2-1-5

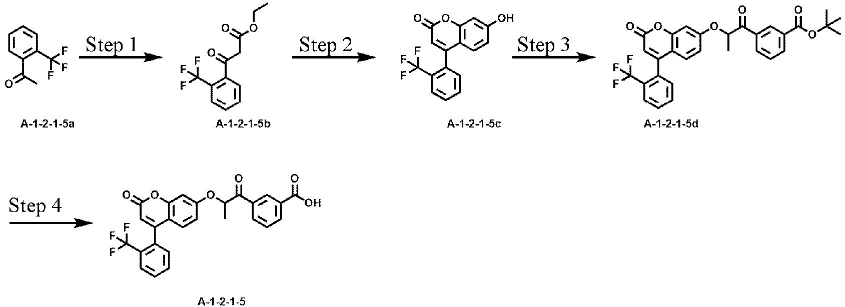

### Step 1

In a 50 mL three-necked flask, diethyl carbonate (6.28 g, 53.16 mmol) was added and dissolved in tetrahydrofuran (30 mL), and sodium hydride (1.06 g, 26.58 mmol) was added at 0°C. Then, the reaction was carried out at 0°C under nitrogen protection for 1 h to give a pale yellow emulsion. Then, A-1-2-1-5a (2.50 g, 133.29 mmol) was slowly added to the reaction solution and transferred to room temperature for 1 h. Then, the reaction was carried out at 55°C for 6 h to give a brown reaction solution. TLC showed that the reaction was complete. The reaction was then subjected to a work-up. 1M aqueous hydrochloric acid solution (50 mL) was added to the reaction solution. The mixture was extracted with 50 mL of ethyl acetate twice and then washed with saturated brine. Then, the organic phase was collected, dried, and concentrated to give the crude product A-1-2-1-5b (3.50 g, crude) as a brown oil, which was directly used in the next step.
MS-ESI: [M-H]⁻ = 259.1

### Step 2

In a 50 mL three-necked flask, compound A-1-2-1-5b (3.50 g, 13.45 mmol) and resorcinol (1.63 g, 14.79 mmol) were dissolved in 30 mL of methanesulfonic acid. Then, the reaction was carried out at 55°C for 3 h to give a black reaction solution. TLC showed that the reaction was complete. The reaction was then subjected to a work-up. The reaction solution was poured into an ice-cold aqueous ethanol solution (30 mL) and stirred for 1 h. Then, ethyl acetate (100 mL) was added, and the mixture was washed with saturated brine (80 mL × 2). The organic phase was collected, dried, and concentrated to give a crude product. The crude product was then mixed with an adsorbent and subjected to column purification (PE:EA = 3:1) to give the desired product A-1-2-1-5c (0.80 g, purity: 95.8%) as a yellow oil.
MS-ESI: [M+H]⁺ = 307.1

### Step 3

In a 50 mL three-necked flask, the reactants A-1-2-1-5c (0.80 g, 2.61 mmol) and A-1-2-1-1d (0.82 g, 2.61 mmol) were dissolved in acetone (25 mL). Then, potassium carbonate (0.9 g, 6.52 mmol) was added. The reaction was carried out at room temperature for 16 h to give a brown turbid solution. LCMS showed the formation of the desired product. The reaction was then subjected to a work-up. The reaction solution was directly rotary evaporated and then subjected to column purification (PE:EA = 3:1) to give the desired product A-1-2-1-5d (0.80 g, purity: 77.39%) as a yellow solid.
MS-ESI: [M-^{t}Bu]⁻ = 481.0

### Step 4

In a 50 mL three-necked flask, compound A-1-2-1-5d (0.80 g, 1.49 mmol, ) was dissolved in dichloromethane (20 mL). Then, trifluoroacetic acid (5 mL) was added. The reaction was carried out at room temperature for 1 h to give a yellow reaction solution. TLC monitoring showed that the reaction was complete. The reaction was then subjected to a work-up. Aqueous sodium bicarbonate solution was added to the reaction solution to pH ~ 5-6. Then, the mixture was extracted with ethyl acetate (100 mL) and saturated brine (50 mL). The organic phase was collected, dried, rotary evaporated, and then subjected to column purification (DCM:MeOH = 50:1) to give the desired product A-1-2-1-5 (325.6 mg, purity: 98.3%) as a white solid.
MS-ESI: [M-H]⁻ = 481.0
¹H NMR (400 MHz, Chloroform-d) δ 8.79 (s, 1H), 8.32 (dd, *J =* 16.4, 7.6 Hz, 2H), 8.16 (d, *J =* 8.8 Hz, 1H), 7.84 (d, *J =* 7.2 Hz, 1H), 7.74 - 7.57 (m, 4H), 7.04 (dd, *J =* 8.8, 2.4 Hz, 1H), 6.83 (d, *J* = 2.4 Hz, 1H), 6.46 (s, 1H), 5.64 (dd*, J =* 13.8, 6.8 Hz, 1H), 1.83 (d, *J =* 6.8 Hz, 3H).

### The following are Experimental Examples.

### Experimental Example 1: Determination of Cell Activity

To examine the effect of compounds on cell viability, ATP was directly quantified using the CellTiter-Glo Luminescent Cell Viability Assay (Promega) to determine the number of viable cells.

Cell density was selected based on optimal signal intensity and growth curve for the inoculation of A2780 cells. The cells were incubated at 37°C and 5% CO2 overnight. Cells were treated with blank group, DMSO group and compound group, respectively, at 37°C and 5% CO2 for 7 days. The prepared solutions were added to cell wells according to the instructions of CellTiter-Glo (Promega), and shaken at a speed of 400g for 2 min. Then, the solutions were centrifuged at 1000g for 2 min and incubated at rest for 30 min to ensure a complete reaction. Fluorescence readings were taken using a multi-label microplate reader (Perkin Elmer) to calculate IC₅₀ values.

The results of the cell activities after treatment with the compounds are shown in Table I-1. The results show that the compounds of the present invention can effectively inhibit the activity of POLRMT and thus have a good inhibitory effect on the proliferation of A2780 cells, among which some have better activity than IMT1B (purchased from MedChemExpress, CAS 2304621-06-3).

**Table I-1: Determination of the cell activity of compounds**

| **Compounds** | **IC50** | **Compounds** | **IC50** |
|---|---|---|---|
| IMT1B | (++) | A-1-2-1-79 | (++) |
| α-1 | (++) | A-1-2-1-80 | (++) |
| α-2 | (++) | A-1-2-1-82 | (+++) |
| α-3 | (++) | A-1-2-1-83 | (+++) |
| α-4 | (+++) | A-1-2-1-84 | (+++) |
| α-5 | (+++) | A-1-2-1-89 | (+) |
| α-6 | (+++) | A-1-2-1-90 | (++) |
| α-7 | (++) | A-1-2-1-93 | (++) |
| α-8 | (++) | A-1-2-1-94 | (+++) |
| α-9 | (+++) | A-1-2-1-98 | (+++) |
| α-10 | (+++) | A-1-2-1-99 | (+) |
| α-11 | (++) | A-1-2-1-100 | (+) |
| α-12 | (+) | A-1-2-1-101 | (+) |
| α-13 | (++) | A-1-2-1-103 | (+) |
| A1-1-1 | (+) | A-1-2-1-104 | (+++) |
| A1-1-5 | (++) | A-1-2-6-2 | (+) |
| A2-1-1 | (+) | A-1-7-1-1 | (+) |
| A2-1-5 | (++) | A-1-7-1-2 | (+) |
| A-1-2-1-1 | (+) | A-1-7-1-3 | (++) |
| A-1-2-1-2 | (++) | A-1-7-1-4 | (+) |
| A-1-2-1-3 | (++) | A-1-8-1-1 | (+) |
| A-1-2-1-8 | (+) | A-1-8-1-2 | (+) |
| A-1-2-1-35 | (++) | A-1-10-1-4 | (+) |
| A-1-2-1-36 | (++) | A-1-10-1-5 | (+++) |
| A-1-2-1-40 | (++) | A-1-10-1-6 | (++) |
| A-1-2-1-70 | (++) | A-1-10-1-7 | (++) |
| A-1-2-1-71 | (++) | A-1-10-1-35 | (+) |
| A-1-2-1-72 | (+) | A-1-10-1-39 | (+++) |
| A-1-2-1-73 | (+) | A-1-11-1-1 | (++) |
| A-1-2-1-75 | (++) | A-1-11-1-10 | (+) |
| A-1-2-1-76 | (+) | A-1-11-1-13 | (+) |
| A-1-2-1-78 | (+++) | A-1-12-1-27 | (+) |
| A-1-2-6-23 | (-) | A-1-2-6-24 | (-) |
| A-1-2-6-25 | (-) | A-1-2-2-2 | (-) |

| | | | |
|---|---|---|---|
| (+++): IC₅₀ ≤ 20 nM; (++): 20 nM ≤ IC₅₀ ≤ 100 nM; (+): 100 nM ≤ IC₅₀ ≤ 1 µM; (-): IC₅₀ ≥ 1 µM; | | | |

### Experimental Example 2: Screening of Compound Selectivity by Real-time Fluorescent Quantitative PCR

Tumor cells A549 were plated at an appropriate density and incubated overnight. The cells were treated with compounds or DMSO for 72 h, and total cellular RNA was extracted according to the instructions of TaqMan^{™} Fast Advanced Cells-to-CT^{™} Kit (Invitrogen # A35377). Briefly, the cells were washed with PBS twice. Supernatants were discarded, and 50 µL/well of lysis buffer was added. The cells were incubated at room temperature for 5 min. Then, 5 µL of stop buffer was added. The mixture was left to stand for 2 min and then placed on ice in preparation for reverse transcription and extraction of cDNA. QPCR was carried out according to the requirements of TaqMan^{®} Fast Advanced Master Mix (Invitrogen). Eukaryotes have three different nuclear RNA polymerases, namely RNA polymerase I (RNA pol I), RNA polymerase II (RNA pol II) and RNA polymerase III (RNA pol III). To eliminate the effect of the compounds on the inhibition of the activity of other RNA polymerases, we selected 18S rRNA, 28S rRNA; β-actin; 5S rRNA; ND1, CYTB and COX1 transcriptionally regulated by RNA Pol I, II, III and POLRMT. Whether the mRNA levels of these genes were inhibited was detected. The qPCR results were analyzed by the ΔΔCt method and statistically analyzed by Graphpad8.0. The significant differences were statistically analyzed by T-Test.

Table I-2 shows the expression of genes regulated by different polymerases. The results show that the compounds of the present invention, similar to IMT1B, do not inhibit the activities of RNA polymerase I, II and III, have good RNA polymerase selectivity, and can effectively avoid off-target effects.

**Table I-2: Expression of genes regulated by different polymerases**

| **Compounds** | **RNA pol I (18S, 28S)** | **RNA pol II (β-actin)** | **RNA pol III (5S)** | **POLRMT (ND1, CYTB, COX1)** |
|---|---|---|---|---|
| **IMTIB** | (-) | (-) | (-) | (++) |
| **A1-1-5** | (-) | (-) | (-) | (++) |
| **α-1** | (-) | (-) | (-) | (++) |
| **α-2** | (-) | (-) | (-) | (++) |
| **α*-*3** | (-) | (-) | (-) | (++) |
| **0.1% DMSO** | (-) | (-) | (-) | (-) |

| | | | | |
|---|---|---|---|---|
| (+) represents P < 0.05; (++) represents P < 0.01; (-) represents P ≥ 0.05. | | | | |

### Experimental Example 3: Pharmacokinetic Assay

The pharmacokinetic assay of compounds was carried out in CD1 mice aged 6-8 weeks with IMT1B as a positive control. The compounds and the positive control were dissolved in 30% aqueous hydroxypropyl-β-cyclodextrin solution containing 25% PEG400 and administered via intravenous injection (1 mg/kg) or intragastric administration (5 mg/kg). Blood samples were collected at 0.033, 0.083, 0.25, 0.5, 1, 2, 4, 8, 24 h and 0.083, 0.25, 0.5, 1, 2, 4, 8, 24 h respectively after administration.

The concentration of the compounds in plasma samples was analyzed using the LC-MS/MS method. Pharmacokinetic calculations were performed using WinNonlin (PhoenixTM, version 8.3) or other similar software. The following pharmacokinetic parameters were calculated from the plasma concentration vs. time data: for intravenous administration: T_{1/2}, C₀, AUCₗₐₛₜ, AUC_{inf}, MRT_{inf}, Cl, Vss, regression coefficient, etc.; for oral administration: T_{1/2}, Cₘₐₓ, Tₘₐₓ, AUCₗₐₛₜ, AUC_{inf}, bioavailability, etc. The above parameter data were statistically calculated and described using descriptive statistics such as mean value and standard deviation.

Table I-3 shows the T_{1/2} data of some compounds for oral administration, and Table I-4 shows the main pharmacokinetic parameters of IMT1B and some compounds. The results show that the examples of the present invention exhibit good metabolic properties, and T_{1/2} for oral administration is generally superior to IMT1B. Moreover, the compounds A1-1-5, A2-1-5 and A-1-2-1-93 are significantly superior to IMT1B in terms of maximum plasma concentration Cₘₐₓ, plasma exposure AUC and bioavailability F.

**Table I-3: T_{1/2} (h) data of some compounds for oral administration**

| **Compounds** | **T_{1/2} (h)** |
|---|---|
| **IMT1B** | 2.445 |
| **A2-1-5** | 3.985 |
| **A1-1-5** | 3.158 |
| **α-1** | 4.210 |
| **α-7** | 4.260 |
| **α-8** | 7.91 |
| **α-9** | 4.08 |
| **α-10** | 4.286 |
| **A1-4-5** | 9.733 |
| **α-13** | 6.914 |
| **α-11** | 5.266 |
| **A-1-2-1-93** | 11.706 |
| **A-1-2-1-40** | 3.574 |
| **A-1-2-1-94** | 4.569 |
| **A-1-10-1-4** | 3.756 |
| **A-1-7-1-2** | 3.778 |
| **A1-1-9** | 3.893 |
| **A2-1-1** | 3.536 |

**Table I-4: Main pharmacokinetic parameters of some compounds**

| **Compound s** | **T_{1/2} (h)** | **Cₘₐₓ (ng/mL)** | **AUCₗₐₛₜ (h*ng/mL)** | **AUC_{Inf} (h*ng/mL)** | **F (%)** |
|---|---|---|---|---|---|
| **IMT1B** | 2.445 | 7300 | 24389.836 | 24420.076 | 80.236 |
| **α-9** | 4.08 | 9980 | 82056 | 83439 | 121 |
| **A2-1-5** | 3.985 | 1062 | 9738.987 | 9867.756 | 137.555 |
| **A1-1-5** | 3.158 | 44100 | 177976.233 | 178600.932 | 85.846 |
| **A-1-2-1-93** | 11.706 | 8963.333 | 154622.828 | 211495.958 | 102.733 |

### Experimental Example 4: In Vivo Pharmacodynamic Test

The efficacy of compounds was evaluated using an orthotopic mouse model of leukemia. MOLM-13-luc tumor cells were cultured in RPMI-1640 medium (Vivacell, C3010-0500) containing 20% fetal bovine serum (Trans, Fs201-02), 1% penicillin-streptomycin mixture (Solarbio, P1400) and 0.5 µg/mL puromycin (Selleckchem, S7417) in an incubator at 37°C with 5% CO2. The medium was replaced every 2 to 352 days. After the cell confluence reached 80%-90%, the cells were subcultured into separate flasks. The tumor cells in the logarithmic growth phase were used for the inoculation of tumors in vivo. Approximately 7 days after the inoculation of the tumor cells, according to the body weights of the animals and optical signal intensity at tumor sites, the animals were randomly divided into 4 groups, namely solvent control group [5% TPGS in PH 8 buffer (50 mM potassium dihydrogen phosphate buffer), BID, intragastric administration], IMT1B (50 mpk, BID, intragastric administration), A1-1-5 (25 mpk, BID, intragastric administration) and A1-1-5 (50 mpk, BID, intragastric administration), with 10 animals in each group. Tumor growth was monitored, and behavioral changes in the animals were also monitored.

During the experiment, all the animals were weighed twice a week. The percentage of body weight change (BW change, %) was calculated using the following formula: BW change = (BW Day X / BW Day 0) × 100, where BW Day X is the body weight of the animals on the day of administration, and BW Day 0 is the body weight of the animals on the day of grouping. The administration was stopped if the body weight of the animals decreased by more than 15% of the body weight on the day of grouping.

Mice were imaged twice a week according to their status using the small animal living imaging system IVIS Lumina III (Perkin Elmer). The signal intensity of the bioluminescence imaging (BLI, unit: photons/s) at tumor cell inoculation sites in mice was monitored, which served as the main indicator for evaluating tumor growth and the efficacy of drugs. The specific operation procedures were as follows.

After the mice were intraperitoneally injected with D-luciferin (manufactured by Perkin Elmer, 15 mg/mL solution, injected at 5 µL/g according to the body weight of experimental animals), the animals were subjected to inhalation anesthesia using 1%-2% isoflurane. Approximately 10 minutes after the injection of D-luciferin, the animals were imaged using IVIS Lumina III. The imaging results were analyzed and processed using the Living Image software (Perkin Elmer), and the optical signal intensity in the ROI (regions of interest) of the whole body of each animal was calculated. The animals in the control group died. The administration and imaging were continued for the animals in other groups. At the endpoint of the experiment, the median survival times of all groups throughout the experiment were calculated, and the curves were plotted.

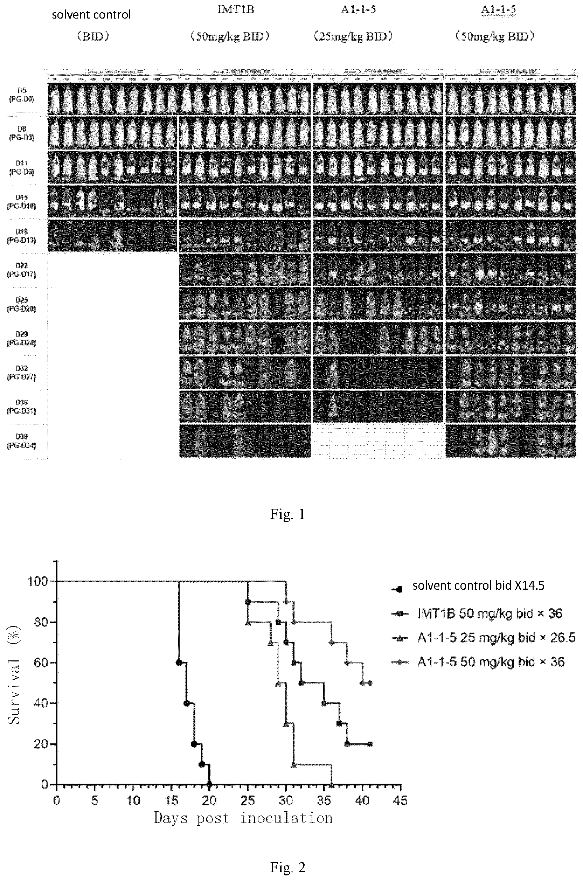

FIGs. 1 and 2 show BLI imaging results and survival curves of the model. The results indicate that compound A1-1-5 exhibits a good tumor inhibitory effect in the pharmacodynamic experiment of the MOLM13 orthotopic hematological tumor model, with A1-1-5 being effective at a relatively low dose. At the same dose, A1-1-5 was significantly superior to IMT1B in terms of tumor inhibition and median survival time, demonstrating better efficacy. During the survival period of animals, there was no significant difference in the body weight change between the administration group and the control group.

## Claims

1. A benzopyrone-based compound represented by formula (I), or stereoisomer thereof, or salt thereof, or prodrug thereof, or deuteride thereof, or hydrate thereof, or solvate thereof, 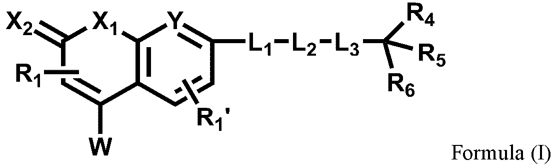
wherein X₁ and X₂ are each independently selected from the group consisting of O or NH group; Y is selected from the group consisting of CR group or N, wherein R is H or C₁-C₃ alkyl; R₁ and R₁' may be identical or different and are each independently selected from the group consisting of hydrogen, alkyl, cycloalkyl, aryl, heterocyclyl or heteroaryl;
L₁ and L₃ are each independently selected from the group consisting of -O-, -S-, -NR₃-, -CR₂R₃-, -CR₂(R₃), -CO-, -SO- or -SO₂- groups, L₂ is a direct linkage, -CR₂R₃-, -CR₂(R₃)- or -C(R₂R₃)-, wherein R₂ is selected from the group consisting of H or C₁-C₄ linear alkyl; R₃ is selected from the group consisting of H or C₁-C₄ linear alkyl; or R₂ and R₃ in a group of -CR₂(R₃) together form an oxo group;
R₄, R₅ and R₆ are each independently selected from the group consisting of H, halogen, -C-OR₈(R₉), -CR₈(R₉), -CN, -NO₂, -OR₈, -NR₈R₉, -SR₈, -COR₈, -SOR₈, -SO₂R₈, -NR₈COR₉, -CONR₈R₉, -OCOR₈, -COOR₈, -OCONR₈R₉, -NR₈CONR₉R₁₀, -NR₈COOR₉, -NR₈SO₂R₉, -SO₂NR₈R₉, -OSO₂R₈, -SO₃R₈, linear alkyl, linear heteroalkyl, cycloalkyl, heterocycloalkyl, alkenyl, alkynyl, aryl or heteroaryl, or aromatic fused heterocyclyl, wherein R₈, R₉ and R₁₀ are each independently selected from the group consisting of H, linear alkyl, linear heteroalkyl, cycloalkyl, heterocycloalkyl, aryl or heteroaryl; wherein the linear alkyl, linear heteroalkyl, cycloalkyl, heterocycloalkyl, aryl or heteroaryl may be independently substituted with one or more R₁₁ selected from the group consisting of halogen, cyano, hydroxyl, mercapto, ether group, nitro, alkoxy, amino, amine group, carboxyl, sulfonic acid group, ester group, acyloxy, amide, sulfonic acid ester group, sulfonamido, linear alkyl, linear heteroalkyl, cycloalkyl, heterocycloalkyl, aryl or heteroaryl, or aromatic fused heterocyclyl, wherein each of the linear alkyl, linear heteroalkyl, cycloalkyl, heterocycloalkyl, aryl or heteroaryl, or aromatic fused heterocyclyl may be independently substituted with one or more selected from the group consisting of halogen, cyano, hydroxyl, mercapto, ether group, nitro, alkoxy, amino, amine group, carboxyl, sulfonic acid group, ester group, amide, sulfonic acid ester group, sulfonamido, alkyl or haloalkyl; or R₄ and R₅ together form an oxo group;
or, any two or three of R₄, R₅ and R₆ together with the carbon atom to which L₃ is attached form a cyclic group A to which L₃ is attached: 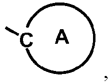 wherein the cyclic group A is any one selected from the group consisting of the following cyclic groups: aromatic ring group, saturated or unsaturated cycloalkyl, monoheterocyclyl, fused heterocyclyl, benzoheterocyclyl, spirocyclic group, bridged cyclic group and their cyclic groups substituted with R₁₁;
W is selected from the group consisting of hydrogen, H, alkyl, -CR₈ₐR₉ₐR₁₀ₐ, -NR₈ₐR₉ₐ, -OR₁₀ₐ, or a carbon atom-containing cyclic group B1 that may be substituted with one or two or more R₇ and is attached to a benzopyrone ring through the carbon atom: 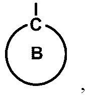 or a nitrogen atom-containing cyclic group B2 that is attached to a benzopyrone ring through the nitrogen atom: 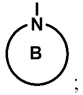 here, R₈ₐ, R₉ₐ and R₁₀ₐ are each independently selected from the group consisting of H, halogen, alkyl, cyano, hydroxyl, mercapto, ether group, nitro, alkoxy, amino, amine group, carboxyl, sulfonic acid group, ester group, acyloxy, amide, sulfonic acid ester group, sulfonamido, cycloalkyl, heterocycloalkyl, aryl or heteroaryl, or aromatic fused heterocyclyl; the cyclic group B1 and the cyclic group B2 are each independently selected from the group consisting of cycloalkyl, heterocycloalkyl, aryl or heteroaryl;
wherein R₇ is selected from the group consisting of H, halogen, -CN, -NO₂, -OR₈, -NR₈R₉, -SR₈, -COR₈, -SOR₈. -SO₂R₈, -NR₈COR₉. -CONR₈R₉, -OCOR₈, -COOR₈, -OCONR₈R₉, -NR₈CONR₉R₁₀, -NR₈COOR₉, -NR₈SO₂R₉, -SO₂NR₈R₉, -OSO₂R₈, -SO₃R₈, linear alkyl, linear heteroalkyl, cycloalkyl, heterocycloalkyl, aryl or heteroaryl, or aromatic fused heterocyclyl, preferably halogen, alkyl, cyano, hydroxyl, mercapto, ether group, nitro, alkoxy, amino, amine group, carboxyl, sulfonic acid group, ester group, amide, acyloxy, sulfonic acid ester group, sulfonamido, cycloalkyl, heterocycloalkyl, aryl or heteroaryl; wherein each of the linear alkyl, linear heteroalkyl, cycloalkyl, heterocycloalkyl, aryl or heteroaryl, or aromatic fused heterocyclyl is independently substituted with one or two or more R₁₁, and R₈, R₉ and R₁₀ have the same meaning as R₈, R₉ and R₁₀ present in R₄, R₅ and R₆; and
formula (I) does not comprise the following compound:

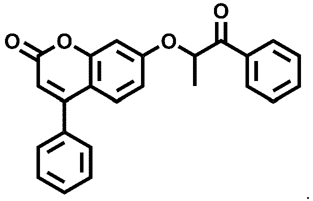

2. The compound, or stereoisomer thereof, or salt thereof, or prodrug thereof, or deuteride thereof, or hydrate thereof, or solvate thereof according to claim 1, wherein the X₁ is -O- group, X₂ is -O-group, Y is -CR- group, R₁ and R₁' are both hydrogen;
or, X₁ is -NH- group, X₂ is -O- group, Y is -CR- group, R₁ and R₁' are both hydrogen;
or, X₁ is -O- group, X₂ is -NH- group, Y is -CR- group, R₁ and R₁' are both hydrogen;
or, X₁ is -O- group, X₂ is -O- group, Y is -N- group, R₁ and R₁' are both hydrogen.

3. The compound, or stereoisomer thereof, or salt thereof, or prodrug thereof, or deuteride thereof, or hydrate thereof, or solvate thereof according to claim 1 or 2, wherein the -L₁-L₂-L₃- as a whole is any one selected from the group consisting of -O-CR₂(R₃)-CO-, -O-CR₂R₃-CO-, -CR₂(R₃)-CR₂(R₃)-CO-, -NR₃-CR₂(R₃)-CO-, -S-CR₂(R₃)-CO-, -SO₂-CR₂(R₅)-CO-, -O-CR₂(R₃)-CR₂R₃-, -O-CR₂(R₃)-SO₂- and -CR₂(R₃)-NR₃-CO-;
wherein the R₂ is preferably one of H, methyl and ethyl, more preferably methyl; R₃ is preferably methyl or H, more preferably H.

4. The compound, or stereoisomer thereof, or salt thereof, or prodrug thereof, or deuteride thereof, or hydrate thereof, or solvate thereof according to any one of claims 1 to 3, wherein the -L₁-L₂-L₃-as a whole is any one selected from the group consisting of -O-CH(CH₃)-CO-, -O-CR₂(R₃)-CO-, -CR₂R₃-CH(CH₃)-CO-, -NR₃-CH(CH₃)-CO-, -S-CH(CH₃)-CO-, -SO₂-CH(CH₃)-CO-, -O-CR₂(R₃)-CR₂(R₃)-, -O-CH(CH₃)-SO₂- and -CR₂(R₃)-NR₃-CO-.

5. The compound, or stereoisomer thereof, or salt thereof, or prodrug thereof, or deuteride thereof, or hydrate thereof, or solvate thereof according to any one of claims 1 to 4, wherein the cyclic group A is any one selected from the group consisting of the following cyclic groups: C₃-C₁₂ cycloalkyl, C₃-C₁₂ heterocycloalkyl, C₅-C₁₂ aryl and C₅-C₁₂ heteroaryl, preferably C₃-C₁₂ cycloalkyl, phenyl, naphthyl, anthranyl, phenanthryl, anthraquinonyl, furyl, pyrrolyl, thienyl, pyrazolyl, imidazolyl, oxazolyl, thiazolyl, isothiazolyl, pyridyl, pyranyl, thiopyranyl, pyridazinyl, pyrimidinyl, pyrazinyl, piperazinyl, indolyl, benzimidazolyl, carbazolyl, thiazolinyl, quinolinyl, isoquinolinyl, purinyl, acridinyl, phenazinyl and phenothiazinyl;
more preferably, the cyclic group A is any one selected from the group consisting of the following cyclic groups: 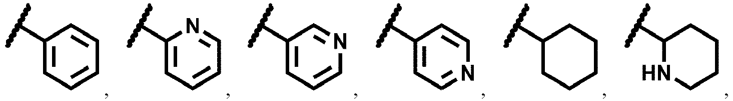 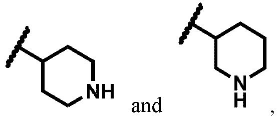 wherein these cyclic groups A may be substituted with one or two or more groups selected from the group consisting of halogen, cyano, hydroxyl, mercapto, ether group, nitro, alkoxy, amino, amine group, carboxyl, sulfonic acid group, ester group, amide, sulfonic acid ester group, sulfonamido, linear alkyl, linear heteroalkyl, cycloalkyl, heterocycloalkyl, aryl and heteroaryl.

6. The compound, or stereoisomer thereof, or salt thereof, or prodrug thereof, or deuteride thereof, or hydrate thereof, or solvate thereof according to any one of claims 1 to 5, wherein the R₄, R₅ and R₆ are each independently selected from the group consisting of -CR₈(R₉), R₈ is H, linear alkyl, cyano or cycloalkyl, and R₉ is linear alkyl, linear heteroalkyl, cycloalkyl, heterocycloalkyl, aryl or heteroaryl substituted with halogen, cyano, hydroxyl, mercapto, ether group, nitro, alkoxy, amino, amine group, carboxyl, sulfonic acid group, ester group, acyl, amide, sulfonic acid ester group and sulfonamido, where R₉ is attached to the carbon atom to which L₃ is attached through the oxygen atom, nitrogen atom or carbon atom on the substituent.

7. The compound, or stereoisomer thereof, or salt thereof, or prodrug thereof, or deuteride thereof, or hydrate thereof, or solvate thereof according to any one of claims 1 to 6, wherein the substituted cyclic group B1 and the substituted cyclic group B2 are each independently selected from the group consisting of halocycloalkyl, haloheterocycloalkyl, haloaryl and haloheteroaryl;
further preferably, the substituted cyclic group B1 and the substituted cyclic group B2 are each independently 2-chloro-4-fluorophenyl, chlorophenyl, methyl-substituted phenyl, amide-substituted phenyl, amino-substituted phenyl or phenylalkyl.

8. The compound, or stereoisomer thereof, or salt thereof, or prodrug thereof, or deuteride thereof, or hydrate thereof, or solvate thereof according to any one of claims 1 to 7, wherein the cyclic group B1 is C₅-C₁₀ aryl, C₅-C₁₀ heteroaryl, C₃-C₁₀ cycloalkyl or C₃-C₁₀ heterocycloalkyl.

9. The compound, or stereoisomer thereof, or salt thereof, or prodrug thereof, or deuteride thereof, or hydrate thereof, or solvate thereof according to any one of claims 1 to 8, wherein the compound of formula (I) is selected from the group consisting of compounds represented by any one of the following structural formulas: 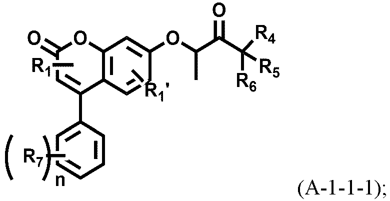 wherein the R₇ is preferably any one or two or more of F, Cl, Br, I, OH, OR₂, C₁₋₃ alkyl, sulfonic acid group, nitro, amino and amine group; R₁ and R₁' are independently selected from the group consisting of hydrogen, C₁₋₄ alkyl, C₁₋₄ alkoxy; wherein R₂ is the same as defined in claim 1; n is an integer from 0 to 5; 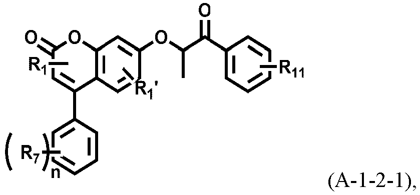 wherein the R₇ is preferably any one or two or more of F, Cl, Br, I, OH, OR₂ₐ, C₁₋₃ alkyl, sulfonic acid group, nitro, amino or amine group; R₁₁ is preferably any one or two or more of F, Cl, Br, I, C₁₋₄ alkoxy, acyl, or C₁₋₃ alkyl, sulfonic acid group, nitro, amino, cyano, amine group, -C₀-₄ alkyl-COOH, ester group, acyloxy, ether group, amide, aminoacyl, cycloalkyl-substituted aminoacyl, or aralkyl-substituted aminoacyl, carboxyl-substituted C₁₋₃ alkoxy, carboxyl-substituted amine group, cycloalkyl-substituted C₁₋₃ alkoxy, acyloxy-containing C₃₋₅ cycloalkyl or 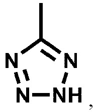 wherein R₁₁ may form a benzene fused azacyclic or oxacyclic structure with the benzene ring to which it is attached; R₁ and R₁' are independently selected from the group consisting of hydrogen, C₁₋₄ alkyl, C₁₋₄ alkyloxy; R₂ₐ is selected from the group consisting of C₁-C₄ alkyl, C₁-C₄ alkenyl, C₁-C₂ alkoxy-substituted C₁-C₄ alkyl, C₁-C₄ alkyl-substituted formyl; n is an integer from 0 to 5; 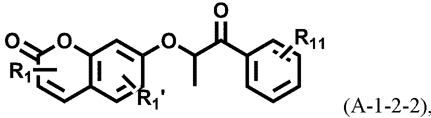 wherein R₁₁ is preferably -C₀₋₄ alkyl-COOH; R₁ and R₁' are independently selected from the group consisting of hydrogen, C₁₋₄ alkyl, C₁₋₄ alkoxy, C₁₋₄ alkylphenyl; 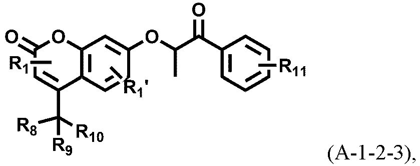 wherein 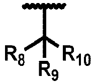 is preferably C₁₋₄ alkyl and C₁₋₄ alkylphenyl, more preferably methyl, isobutyl or benzyl; R₁₁ is preferably -C₀₋₄ alkyl-COOH; R₁ and R₁' are independently selected from the group consisting of hydrogen, C₁₋₄ alkyl, C₁₋₄ alkoxy, C₁₋₄ alkylphenyl; 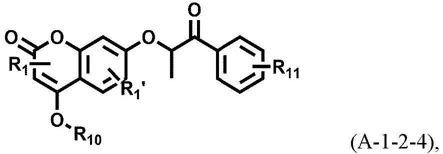 wherein R₁₀ is preferably C₁₋₄ alkyl, hydroxyl-substituted C₁₋₄ alkyl, C₁₋₄ alkoxy or hydroxyl-substituted C₁₋₄ alkoxy; R₁₁ is preferably -C₀-C₄ alkyl-COOH; R₁ and R₁' are independently selected from the group consisting of hydrogen, C₁₋₄ alkyl, C₁₋₄ alkoxy, C₁₋₄ alkylphenyl; 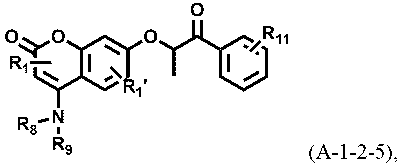 wherein R₈ and R₉ are independently selected from the group consisting of hydrogen or C₁₋₄ alkyl; R₁₁ is preferably -C₀-C₄ alkyl-COOH; R₁ and R₁' are independently selected from the group consisting of hydrogen, C₁₋₄ alkyl, C₁₋₄ alkoxy, C₁₋₄ alkylphenyl; 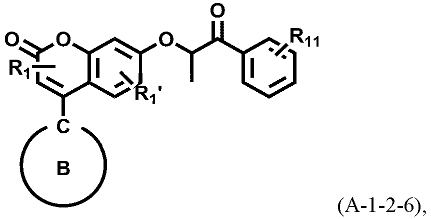 wherein the cyclic group, i.e., cyclic group B1, is preferably selected from the group consisting of C₃₋₁₂ cycloalkyl, 4- to 10-membered heterocyclyl or 5- to 10-membered aryl; R₁₁ is preferably -C₀-C₄ alkyl-COOH; the heterocyclyl is further preferably any one of furyl, pyrrolyl, thienyl, pyrazolyl, imidazolyl, oxazolyl, thiazolyl, isothiazolyl, pyridyl, pyranyl, thiopyranyl, pyridazinyl, pyrimidinyl, pyrazinyl, piperazinyl, indolyl, benzimidazolyl, carbazolyl, thiazolinyl, quinolinyl, isoquinolinyl, purinyl, acridinyl, phenazinyl and phenothiazinyl; R₁ and R₁' are both preferably hydrogen, C₁₋₄ alkyl, C₁₋₄ alkoxy, C₁₋₄ alkylphenyl; the cyclic group B1 is optionally substituted with one or two or more of hydrogen, halogen, hydroxyl, C₁₋₆ alkyl, C₁₋₆ alkyl-substituted hydroxyl, amino and amine group, or is unsubstituted; 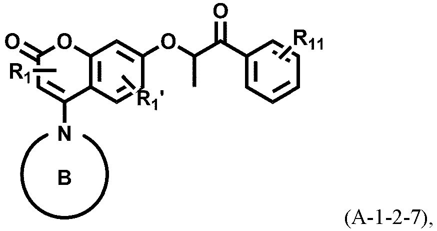 wherein the cyclic group, i.e., cyclic group B2, is preferably selected from the group consisting of C₃₋₁₂ cycloalkyl, 4- to 10-membered heterocyclyl or 5- to 10-membered aryl; R₁₁ is preferably -C₀₋₄ alkylcarboxyl; the heterocyclyl is further preferably any one of furyl, pyrrolyl, thienyl, pyrazolyl, imidazolyl, oxazolyl, thiazolyl, isothiazolyl, pyridyl, pyranyl, thiopyranyl, pyridazinyl, pyrimidinyl, pyrazinyl, piperazinyl, indolyl, benzimidazolyl, carbazolyl, thiazolinyl, quinolinyl, isoquinolinyl, purinyl, acridinyl, phenazinyl and phenothiazinyl; R₁ and R₁' are both preferably hydrogen; the cyclic group B2 is optionally substituted with one or two or more of hydrogen, halogen, hydroxyl, C₁₋₆ alkyl, C₁₋₆ alkyl-substituted hydroxyl, amino and amine group, or is unsubstituted; 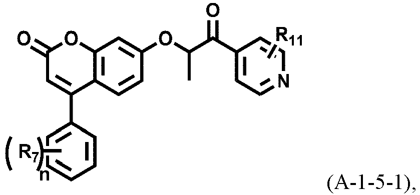 wherein the R₇ is preferably any one or two or more of F, Cl, Br, I, OH, OR₂ₐ, C₁₋₆ alkyl, sulfonic acid group, nitro, amino and amine group, and more preferably, the benzene ring in the structural formula is substituted with both chlorine and fluorine; R₁₁ is preferably any one or two or more of F, Cl, Br, I, oxo, C₁₋₃ alkoxy or alkyl, cycloalkyl, sulfonic acid group, nitro, amino, cyano, amine group, -C₀₋₄ alkyl-carboxyl, ester group, acyloxy and ether group; R₁ and R₁' are independently selected from the group consisting of hydrogen, C₁₄ alkyl, C₁₋₄ alkoxy, C₁₋₄ alkylphenyl; n is an integer from 0 to 5; R₂ₐ is selected from the group consisting of C₁-C₄ alkyl, C₁-C₄ alkenyl, C₁-C₂ alkoxy-substituted C₁-C₄ alkyl, C₁-C₄ alkyl-substituted formyl; 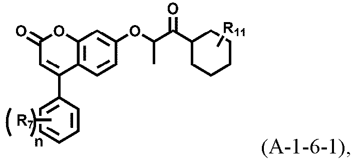 wherein the R₇ is preferably any one or two or more of F, Cl, Br, I, OH, OR₂ₐ, C₁₋₆ alkyl, sulfonic acid group, nitro, amino or amine group, and more preferably, the benzene ring in the structural formula is substituted with both chlorine and fluorine; R₁₁ is preferably any one or two or more of F, Cl, Br, I, oxo, C₁₋₆ alkoxy, C₁₋₆ alkyl, cycloalkyl, sulfonic acid group, nitro, amino, cyano, amine group or -C₀-₄ alkyl-carboxyl; R₁ and R₁' are independently selected from the group consisting of hydrogen, C₁₋₄ alkyl, C₁₋₄ alkoxy, C₁₋₄ alkylphenyl; n is an integer from 0 to 5; R₂ₐ is selected from the group consisting of C₁-C₄ alkyl, C₁-C₄ alkenyl, C₁-C₂ alkoxy-substituted C₁-C₄ alkyl, C₁-C₄ alkyl-substituted formyl; 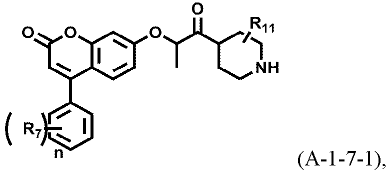 wherein the R₇ is preferably any one or two or more of F, Cl, Br, I, OH, OR₂ₐ, C₁₋₆ alkyl, sulfonic acid group, nitro, amino or amine group, and more preferably, the benzene ring in the structural formula is substituted with both chlorine and fluorine; R₁₁ is preferably any one or two or more of F, Cl, Br, I, oxo, C₁₋₆ alkoxy, C₁₋₆ alkyl, C₃₋₁₀ cycloalkyl, sulfonic acid group, hydroxyl, hydroxyl-substituted C₁₋₆ alkyl, nitro, amino, cyano, amine group or -C₀-₄ alkylcarboxyl; n is an integer from 0 to 5; R₂ₐ is selected from the group consisting of C₁-C₄ alkyl, C₁-C₄ alkenyl, C₁-C₂ alkoxy-substituted C₁-C₄ alkyl, C₁-C₄ alkyl-substituted formyl; 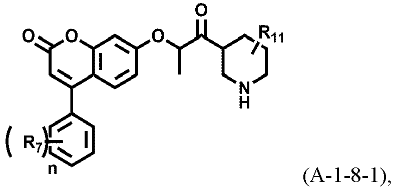 wherein the R₇ is preferably any one or two or more of F, Cl, Br, I, OH, OR₂ₐ, C₁₋₆ alkyl, C₃₋₁₀ cycloalkyl, sulfonic acid group, nitro, amino or amine group, and more preferably, the benzene ring in the structural formula is substituted with both chlorine and fluorine; R₁₁ is preferably any one or two or more of F, Cl, Br, I, oxo, C₁₋₃ alkoxy or alkyl, cycloalkyl, sulfonic acid group, hydroxyl, nitro, amino, cyano, amine group, -C₀₋₄ alkylcarboxyl; n is an integer from 0 to 5; R₂ₐ is selected from the group consisting of C₁-C₄ alkyl, C₁-C₄ alkenyl, C₁-C₂ alkoxy-substituted C₁-C₄ alkyl, C₁-C₄ alkyl-substituted formyl; 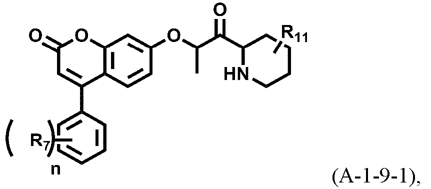 wherein the R₇ is preferably any one or two or more of F, Cl, Br, I, OH, OR₂ₐ, or C₁₋₃ alkyl, sulfonic acid group, nitro, amino and amine group, and more preferably, the benzene ring in the structural formula is substituted with both chlorine and fluorine; R₁₁ may be hydrogen, or any one or two or more of F, Cl, Br, I, oxo, C₁₋₆ alkoxy, C₁₋₆ alkyl, C₃₋₁₀ cycloalkyl, sulfonic acid group, hydroxyl, nitro, amino, cyano, amine group and -C₀₋₄ alkylcarboxyl; n is an integer from 0 to 5; R₂ₐ is selected from the group consisting of C₁-C₄ alkyl, C₁-C₄ alkenyl, C₁-C₂ alkoxy-substituted C₁-C₄ alkyl, C₁-C₄ alkyl-substituted formyl; 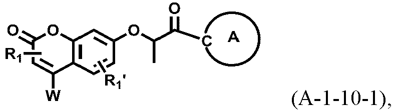 wherein the cyclic group A is aryl, cycloalkyl, heterocycloalkyl or heteroaryl which may be substituted or unsubstituted with one or two or more groups selected from the group consisting of halogen, cyano, hydroxyl, mercapto, ether group, nitro, alkoxy, amino, amine group, -C₀₋₄ alkyl-carboxyl, sulfonic acid group, ester group, amide, sulfonic acid ester group, sulfonamido, linear alkyl, linear heteroalkyl, cycloalkyl and heterocycloalkyl; wherein the cycloalkyl is preferably C₃₋₆ cycloalkyl; the heterocycloalkyl is preferably any one of oxiranyl, oxetanyl, aziridinyl, azetidinyl and thietanyl; the heteroaryl comprises 5-membered heteroaryl such as furyl, thienyl, pyrrolyl, oxazolyl, thiazolyl, imidazolyl or pyrazolyl, or oxygen-containing imidazolyl or pyrazolyl, 6-membered heteroaryl such as pyridyl, pyrimidinyl, pyranyl, pyridazinyl, pyrazinyl, pyranone-forming group or pyranyl, benzoheterocyclyl such as benzofuranyl, benzothienyl, benzopyrrolyl, indolyl, quinolinyl, isoquinolinyl, benzopyranyl, a group formed by benzo-γ-pyranone, heterocycloheterocyclyl such as purinyl; W is preferably 2-chloro-4-fluoro-substituted phenyl or 2-methylphenyl; R₁ and R₁' are independently selected from the group consisting of hydrogen, C₁₋₄ alkyl, C₁₋₄ alkoxy, C₁₋₄ alkylphenyl; 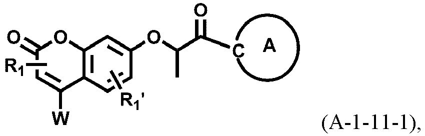 wherein the cyclic group A is preferably fused cyclic group or aromatic fused heterocyclic group, and more preferably, the benzoheterocyclyl is benzofuranyl, benzothienyl, benzopyrrolyl, benzoheterocyclyl such as indolyl, quinolinyl, isoquinolinyl, benzopyranyl, a group formed by benzo-γ-pyrone; W is preferably 2-chloro-4-fluoro-substituted phenyl or 2-methylphenyl; R₁ and R₁' are independently selected from the group consisting of hydrogen, C₁₋₄ alkyl, C₁₋₄ alkoxy, C₁₋₄ alkylphenyl; 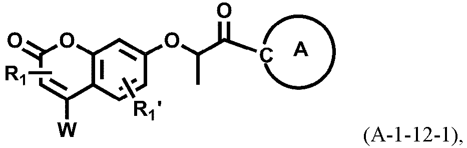 wherein the cyclic group A is preferably selected from the group consisting of spirocyclic group or bridged cyclic group; W is preferably 2-chloro-4-fluoro-substituted benzene or 2-methylphenyl; R₁ and R₁' are independently selected from the group consisting of hydrogen, C₁₋₄ alkyl, C₁₋₄ alkoxy, C₁₋₄ alkylphenyl; 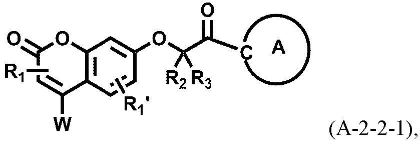 wherein the cyclic group A is preferably phenyl, halophenyl, -C₀₋₄ alkylcarboxyl-substituted phenyl, further preferably phenyl or carboxyl-substituted phenyl; W is preferably 2-chloro-4-fluoro-substituted benzene or 2-methylphenyl; R₁ and R₁' are independently selected from the group consisting of hydrogen, C₁₋₄ alkyl, C₁₋₄ alkoxy, C₁₋₄ alkylphenyl; 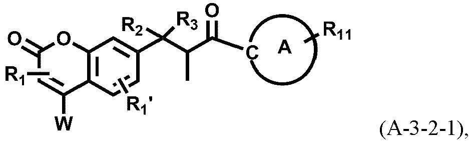 wherein the cyclic group A is preferably phenyl substituted or unsubstituted with any one or two or more of F, Cl, Br, I, oxo, C₁₋₆ alkoxy, C₁₋₆ alkyl, C₃₋₁₀ cycloalkyl, sulfonic acid group, nitro, amino, cyano, amine group or -C₀₋₄ alkyl-carboxyl as R₁₁; further preferably, the cyclic group A is phenyl substituted with R₁₁ as carboxyl group; W is preferably 2-chloro-4-fluoro-substituted benzene or 2-methylphenyl; R₁ and R₁' are independently selected from the group consisting of hydrogen, C₁₋₄ alkyl, C₁₋₄ alkoxy, C₁₋₄ alkylphenyl; 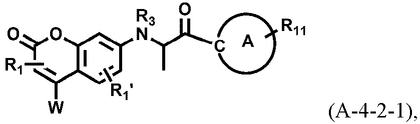 wherein the cyclic group A is preferably phenyl substituted or unsubstituted with any one or two or more of F, Cl, Br, I, oxo, C₁₋₆ alkoxy, C₁₋₆ alkyl, C₃₋₁₀ cycloalkyl, sulfonic acid group, nitro, amino, cyano, amine group or -C₀₋₄ alkyl-carboxyl as R₁₁; further preferably, the cyclic group A is phenyl substituted with R₁₁ as carboxyl group; W is preferably 2-chloro-4-fluoro-substituted benzene or 2-methylphenyl; R₁ and R₁' are independently selected from the group consisting of hydrogen, C₁₋₄ alkyl, C₁₋₄ alkoxy, C₁₋₄ alkylphenyl; 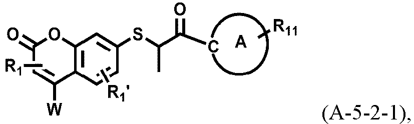 wherein the cyclic group A is preferably phenyl substituted or unsubstituted with any one or two or more of F, Cl, Br, I, oxo, C₁₋₆ alkoxy, C₁₋₆ alkyl, C₃₋₁₀ cycloalkyl, sulfonic acid group, nitro, amino, cyano, amine group or -C₀₋₄ alkyl-carboxyl as R₁₁; further preferably, the cyclic group A is phenyl substituted with R₁₁ as carboxyl group; W is preferably 2-chloro-4-fluoro-substituted benzene or 2-methylphenyl; R₁ and R₁' are independently selected from the group consisting of hydrogen, C₁₋₄ alkyl, C₁₋₄ alkoxy, C₁₋₄ alkylphenyl; 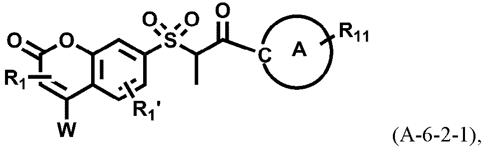 wherein the cyclic group A is preferably phenyl substituted or unsubstituted with any one or two or more of F, Cl, Br, I, oxo, C₁₋₆ alkoxy, C₁₋₆ alkyl, C₃₋₁₀ cycloalkyl, sulfonic acid group, nitro, amino, cyano, amine group or -C₀₋₄ alkylcarboxyl as R₁₁; further preferably, the cyclic group A is phenyl substituted with R₁₁ as carboxyl group; W is preferably 2-chloro-4-fluoro-substituted benzene; R₁ and R₁' are independently selected from the group consisting of hydrogen, C₁₋₄ alkyl, C₁₋₄ alkoxy, C₁₋₄ alkylphenyl; 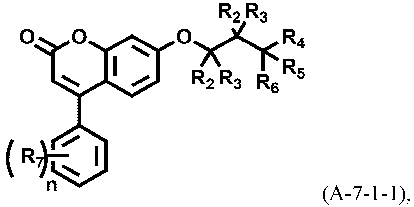 wherein R₇ is selected from the group consisting of hydrogen, C₁₋₄ alkyl, C₁₋₄ alkoxy; n is an integer from 0 to 5; R₂ and R₃ are independently selected from the group consisting of hydrogen, C₁₋₄ alkyl; R₄ and R₅ together form an oxo group; 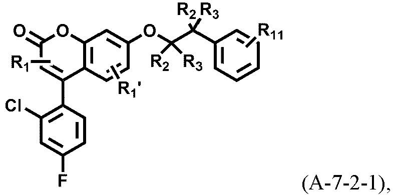 wherein R₂ and R₃ are each independently preferably hydrogen, C₁₋₆ alkyl; or one set of R₂ and R₃ together form an oxo group, and the other set of R₂ and R₃ is independently preferably hydrogen, C₁₋₆ alkyl; R₁ and R₁' are independently selected from the group consisting of hydrogen, C₁₋₄ alkyl, C₁₋₄ alkoxy, C₁₋₄ alkylphenyl; 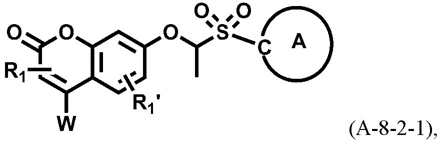 wherein the cyclic group A is preferably phenyl that may be substituted or unsubstituted with any one or two or more of F, Cl, Br, I, oxo, C₁₋₆ alkoxy, C₁₋₆ alkyl, C₃₋₁₀ cycloalkyl, sulfonic acid group, nitro, amino, cyano, amine group or carboxyl; further preferably, the cyclic group A is phenyl substituted with R₁₁ as carboxyl group; W is preferably 2-chloro-4-fluoro-substituted benzene or 2-methylphenyl; R₁ and R₁' are both preferably hydrogen; 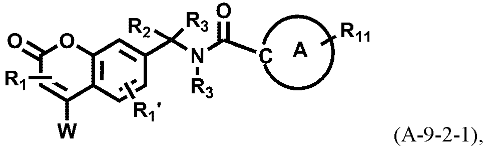 wherein the cyclic group A is preferably phenyl substituted with any one or two or more of F, Cl, Br, I, oxo, C₁₋₆ alkoxy, C₁₋₆ alkyl, C₃₋₁₀ cycloalkyl, sulfonic acid group, nitro, amino, cyano, amine group or C₀₋₄ alkylcarboxyl as R₁₁; further preferably, the cyclic group A is phenyl substituted with R₁₁ as carboxyl group; W is preferably 2-chloro-4-fluoro-substituted benzene or 2-methylphenyl; R₁ and R₁' are independently selected from the group consisting of hydrogen, C₁₋₄ alkyl, C₁₋₄ alkoxy, C₁₋₄ alkylphenyl; 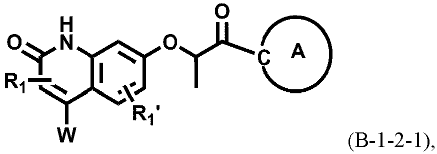 wherein the cyclic group A is preferably phenyl substituted or unsubstituted with any one or two or more of F, Cl, Br, I, oxo, C₁₋₆ alkoxy, C₁₋₆ alkyl, C₃₋₁₀ cycloalkyl, sulfonic acid group, nitro, amino, cyano, amine group or C₀₋₄ alkylcarboxyl; further preferably, the cyclic group A is phenyl substituted with R₁₁ as carboxyl group; W is preferably 2-chloro-4-fluoro-substituted benzene or 2-methylphenyl; R₁ and R₁' are independently selected from the group consisting of hydrogen, C₁₋₄ alkyl, C₁₋₄ alkoxy, C₁₋₄ alkylphenyl; 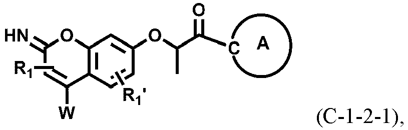 wherein the cyclic group A is preferably phenyl substituted with any one or two or more of F, Cl, Br, I, oxo, C₁₋₆ alkoxy, C₁₋₆ alkyl, C₃₋₁₀ cycloalkyl, sulfonic acid group, nitro, amino, cyano, amine group or C₀-₄ alkylcarboxyl; further preferably, the cyclic group A is phenyl substituted with R₁₁ as carboxyl group; W is preferably 2-chloro-4-fluoro-substituted benzene or 2-methylphenyl; R₁ and R₁' are independently selected from the group consisting of hydrogen, C₁₋₄ alkyl, C₁₋₄ alkoxy, C₁₋₄ alkylphenyl;
and 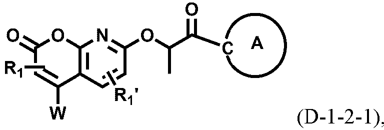 wherein the cyclic group A is preferably phenyl substituted with any one or two or more of F, Cl, Br, I, oxo, C₁₋₆ alkoxy, C₁₋₆ alkyl, C₃₋₁₀ cycloalkyl, sulfonic acid group, nitro, amino, cyano, amine group or C₀₋₄ alkylcarboxyl; further preferably, the cyclic group A is phenyl substituted with R₁₁ as carboxyl group; W is preferably 2-chloro-4-fluoro-substituted benzene or 2-methylphenyl; R₁ and R₁' are independently selected from the group consisting of hydrogen, C₁₋₄ alkyl, C₁₋₄ alkoxy, C₁₋₄ alkylphenyl.

10. A pharmaceutical composition comprising the compound, or stereoisomer thereof, or salt thereof, or prodrug thereof, or deuteride thereof, or hydrate thereof, or solvate thereof or pharmaceutically acceptable salt thereof according to any one of claims 1 to 9; and pharmaceutically acceptable carriers.

11. Use of the compound, or stereoisomer thereof, or salt thereof, or prodrug thereof, or deuteride thereof, or hydrate thereof, or solvate thereof or pharmaceutically acceptable salt thereof according to any one of claims 1 to 9; or the pharmaceutical composition according to claim 10, in the manufacture of drug as POLRMT inhibitor.

12. Use of the compound, or stereoisomer thereof, or salt thereof, or prodrug thereof, or deuteride thereof, or hydrate thereof, or solvate thereof or pharmaceutically acceptable salt thereof according to any one of claims 1 to 9; ,or the pharmaceutical composition according to claim 3, in the manufacture of drug for diseases associated with abnormally high expression of POLRMT in oxidative phosphorylation.

13. The use according to claim 12, wherein the diseases are cancers; preferably, the cancers are melanoma, metastatic melanoma, pancreatic cancer, pancreatic ductal adenocarcinoma, prostate cancer, lung cancer, hepatocellular carcinoma, lymphoma, leukemia, multiple myeloma, breast cancer, glioma, glioblastoma, cervical cancer, renal cancer, colorectal cancer or ovarian cancer.
